(19) Europäisches Patentamt
European Patent Office
Office européen des brevets

(11) EP 4 707 284 A1

(12) **EUROPEAN PATENT APPLICATION**
published in accordance with Art. 153(4) EPC

(43) Date of publication:
**11.03.2026 Bulletin 2026/11**

(21) Application number: **24800130.7**

(22) Date of filing: **02.05.2024**

(51) International Patent Classification (IPC):
***C07F 9/6561*** *(2006.01)* ***A61K 31/675*** *(2006.01)*
***A61P 25/00*** *(2006.01)* ***A61P 25/16*** *(2006.01)*
***A61P 25/18*** *(2006.01)* ***A61P 25/20*** *(2006.01)*
***A61P 25/22*** *(2006.01)* ***A61P 25/24*** *(2006.01)*
***A61P 25/28*** *(2006.01)* ***A61P 43/00*** *(2006.01)*

(52) Cooperative Patent Classification (CPC):
**A61K 31/675; A61P 25/00; A61P 25/16; A61P 25/18; A61P 25/20; A61P 25/22; A61P 25/24; A61P 25/28; A61P 43/00; C07F 9/6561**

(86) International application number:
**PCT/JP2024/016879**

(87) International publication number:
**WO 2024/228399 (07.11.2024 Gazette 2024/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.05.2023 JP 2023076322**
**29.03.2024 JP 2024058286**

(71) Applicant: **Otsuka Pharmaceutical Co., Ltd.**
**Tokyo 101-8535 (JP)**

(72) Inventors:
- **ITO, Nobuaki**
  **Osaka-shi, Osaka 540-0021 (JP)**
- **OKADA, Minoru**
  **Osaka-shi, Osaka 540-0021 (JP)**
- **YUKI, Yohei**
  **Osaka-shi, Osaka 540-0021 (JP)**
- **SHINOHARA, Toshio**
  **Osaka-shi, Osaka 540-0021 (JP)**
- **HAYASHI, Masashi**
  **Osaka-shi, Osaka 540-0021 (JP)**
- **SATO, Tetsuya**
  **Osaka-shi, Osaka 540-0021 (JP)**
- **MINOWA, Takuya**
  **Osaka-shi, Osaka 540-0021 (JP)**
- **KANEKO, Daiki**
  **Osaka-shi, Osaka 540-0021 (JP)**
- **SAKO, Nobutomo**
  **Osaka-shi, Osaka 540-0021 (JP)**
- **OKAWA, Shinya**
  **Osaka-shi, Osaka 540-0021 (JP)**
- **OKADA, Akie**
  **Osaka-shi, Osaka 540-0021 (JP)**
- **KIMURA, Naoji**
  **Osaka-shi, Osaka 540-0021 (JP)**
- **HOSOMI, Atsushi**
  **Osaka-shi, Osaka 540-0021 (JP)**
- **MATSUDA, Takakuni**
  **Osaka-shi, Osaka 540-0021 (JP)**
- **KOGA, Toshihisa**
  **Osaka-shi, Osaka 540-0021 (JP)**
- **KAWATA, Reo**
  **Osaka-shi, Osaka 540-0021 (JP)**
- **YODA, Noriaki**
  **Osaka-shi, Osaka 540-0021 (JP)**

(74) Representative: **Hoffmann Eitle**
**Patent- und Rechtsanwälte PartmbB**
**Arabellastraße 30**
**81925 München (DE)**

(54) **HETEROCYCLIC COMPOUND AND USE THEREOF**

(57) Provided is a novel therapeutic agent for central nervous system diseases. Provided is a compound represented by formula (I) or a salt thereof:



Processed by Luminess, 75001 PARIS (FR)

**(Cont. next page)**

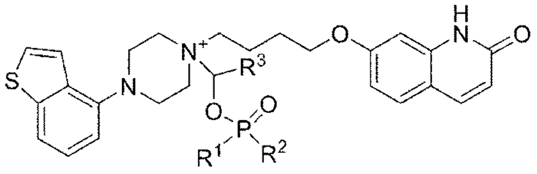

(I)

wherein $R^1$ and $R^2$ each independently represent -O$^-$, -OR$^4$, or - NR$^{4Na}$R$^{4Nb}$; $R^3$ represents hydrogen or alkyl; $R^4$, $R^{4Na}$, and $R^{4Nb}$ each independently represent hydrogen, or alkyl, alkenyl, alkadienyl, or alkynyl each optionally having 1 to 3 substituents, wherein alkyl represented by $R^4$, $R^{4Na}$, or $R^{4Nb}$ may have a structure in which one or more methylene groups (-$CH_2$-) are replaced with -O-, -S-, - CO-, -NH-, -SiR$^{sia}$R$^{sib}$-, or -(CO)O-; if $R^1$ and $R^2$ both represent - OR$^4$, $R^1$ and $R^2$ may be identical or different; and if $R^1$ and $R^2$ both represent -NR$^{4Na}$R$^{4Nb}$, $R^1$ and $R^2$ may be identical or different.

## Description

Technical Field

The present disclosure relates to a specific heterocyclic compound and use thereof.

Background Art

7-[4-(4-benzo[b]thiophen-4-yl-piperazin-1-yl)butoxy]-1H-quinolin-2-one ("brexpiprazole" below) or a salt thereof has dopamine $D_2$ receptor partial agonistic action, serotonin 5-$HT_{2A}$ receptor antagonistic action, and adrenergic $\alpha_1$ receptor antagonistic action. In addition to those actions, brexpiprazole or a salt thereof also has serotonin uptake inhibition action (or serotonin reuptake inhibition action), and is known to have a broad therapeutic spectrum for central nervous system diseases (particularly schizophrenia) (Patent Literature (PTL) 1 and 2).
One of the drug design methods in the field of pharmaceuticals is the development of a drug that aims to improve the properties of a known drug molecule (e.g., to increase its efficacy or slow its metabolism in vivo) by chemically converting or modifying some functional groups of the drug molecule.
It is eagerly awaited that such drug development using brexpiprazole will find a novel compound with improved properties and offer new options for therapeutics for central nervous system diseases.

Citation List

Patent Literature

PTL 1: JP2006-316052A
PTL 2: WO2013/035892A

Summary of Invention

Technical Problem

One of the objects to be achieved by the present disclosure is to provide a novel therapeutic agent for central nervous system diseases.

Solution to Problem

The present inventors conducted extensive research and successfully developed a novel heterocyclic compound represented by the following formula (I) and a salt thereof:

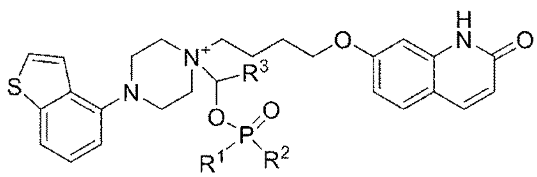

(I)

and found that this compound can be a new therapeutic agent for central nervous system diseases.
The present disclosure includes, for example, the subject matter described in the following items.

Item 1.

A compound represented by formula (I) or a salt thereof:

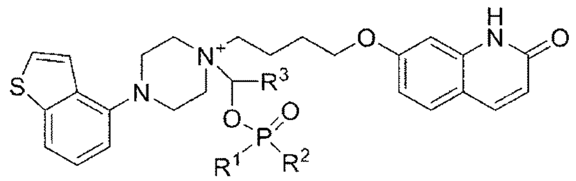

(I)

wherein

$R^1$ and $R^2$ each independently represent $-O^-$, $-OR^4$, or $-NR^{4Na}R^{4Nb}$,
$R^3$ represents hydrogen or alkyl,
$R^4$, $R^{4Na}$, and $R^{4Nb}$ each independently represent hydrogen, or alkyl, alkenyl, alkadienyl, or alkynyl each optionally having 1 to 3 substituents, wherein alkyl represented by $R^4$, $R^{4Na}$, or $R^{4Nb}$ may have a structure in which one or more methylene groups ($-CH_2-$) are replaced with -O-, -S-, -CO-, -NH-, $-SiR^{sia}R^{sib}-$, or -(CO)O-,
if $R^1$ and $R^2$ both represent $-OR^4$, $R^1$ and $R^2$ may be identical or different, and
if $R^1$ and $R^2$ both represent $-NR^{4Na}R^{4Nb}$, $R^1$ and $R^2$ may be identical or different.

Item 2.

The compound or salt thereof according to Item 1, wherein $R^1$ represents $-O^-$, $R^2$ represents $-OR^4$ or $-NR^{4Na}R^{4Nb}$ wherein $R^4$, $R^{4Na}$, and $R^{4Nb}$ are as defined above.

Item 3.

A compound represented by formula (I) or a salt thereof:

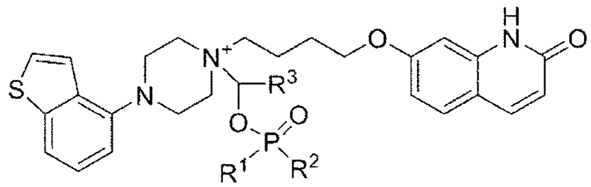

(I)

wherein

$R^1$ represents $-O^-$,
$R^2$ represents $-OR^4$ or $-NR^{4Na}R^{4Nb}$,
$R^3$ represents hydrogen or $C_{1-6}$ alkyl,
$R^4$ represents hydrogen or any of the following (0-1) to (5),
$-NR^{4Na}R^{4Nb}$ represents the following (i) or (ii):

(0-1): $C_{1-18}$ alkenyl optionally having 1 to 3 substituents selected from halogen, hydroxy, $C_{1-6}$ alkyl, and $C_{1-6}$ alkoxy,
(0-2): $C_{1-18}$ alkadienyl optionally having 1 to 3 substituents selected from halogen, hydroxy, $C_{1-6}$ alkyl, and $C_{1-6}$ alkoxy,
(0-3): $C_{1-18}$ alkynyl optionally having 1 to 3 substituents selected from halogen, hydroxy, $C_{1-6}$ alkyl, and $C_{1-6}$ alkoxy,
(1-1): $R^{4a1}$,

wherein $R^{4a1}$ represents

$-C_nH_{2n+1}$,

$-C_nH_{2n-1}$,

$-C_nH_{2n-3}$,

$-C_nH_{2n}-OH$,

$-C_nH_{2n-2}-OH$,

or

$-C_nH_{2n-4}-OH$,

wherein n represents 1 to 24, with the proviso that in $-C_nH_{2n-1}$, n is 2 or more; in $-C_nH_{2n-3}$, n is 2 or more; in $-C_nH_{2n-2}-$, n is 2 or more; and in $-C_nH_{2n-4}-$, n is 2 or more,

(1-2): $R^{4a2}$,

wherein $R^{4a2}$ represents

$-CHX^1X^2$,

$-C_{n-1}H_{2n-2}-CHX^1X^2$,

$-C_{n-1}H_{2n-4}-CHX^1X^2$,

or

$-C_{n-1}H_{2n-6}-CHX^1X^2$,

wherein
n represents 2 to 24, with the proviso that in $-C_{n-1}H_{2n-4}-$, n represents 3 or more; in $-C_{n-1}H_{2n-5}-$, n represents 3 or more,
$X^1$ and $X^2$ are identical or different and represent hydrogen or halogen (F, Cl, Br, or I), with the proviso that either $X^1$ or $X^2$, or both, represent halogen,

(1-3): $R^{4a3}$,

wherein $R^{4a3}$ represents

$-C_nH_{2n}-R^{4-1}$,

$-C_{n-1}H_{2n-2}-CHR^{4-1a}R^{4-1b}$,

$-C_nH_{2n-2}-R^{4-1}$,

or

$-C_nH_{2n-4}-R^{4-1}$,

wherein
n represents 1 to 24, with the proviso that in $-C_nH_{2n-2}-$, n represents 2 or more; in $-C_nH_{2n-4}-$, n represents 2 or more,
$R^{4-1}$ represents $C_{1-6}$ alkoxy, -O-phenyl, or a heterocyclic group optionally substituted with $C_{1-6}$ alkyl or halogen,
$R^{4-1a}$ and $R^{4-1b}$ are identical or different and represent $-C_{1-3}$ alkylene-$C_{1-3}$ alkoxy, or identical or different and represent -COO-$C_{1-3}$ alkyl or $-CH_2$-CO-O-$C_{1-3}$ alkyl,

(2): $R^{4b}$,

wherein $R^{4b}$ represents

$-(C_pH_{2p}-O)_q-C_rH_{2r}-R^{4-2}$,

$-(C_pH_{2p}-O)_q-C_rH_{2r-2}-R^{4-2}$,

$-(C_pH_{2p-2}-O)_q-C_rH_{2r}-R^{4-2}$,

or

$-(C_pH_{2p-2}-O)_q-C_rH_{2r-2}-R^{4-2}$,

wherein p represents 1 to 4, q represents 1 to 4, r represents 1 to 4, with the proviso that in $C_pH_{2p-2}$, p represents 2 or more, in $C_rH_{2r-2}$, r represents 2 or more,
when q is 2 to 4, 2 to 4 repeating structures, the number of repetitions of which is represented by q, may be identical or different,
$R^{4-2}$ represents hydrogen, hydroxy, or cycloalkyl, phenyl, benzyl, or a heterocyclic group each optionally having 1 to 3 substituents selected from $C_{1-6}$ alkyl, $C_{1-6}$ alkoxy, $-C_{1-3}$ alkylene-$C_{1-3}$ alkoxy, and nitro,

(3): $-C_\alpha H_{2\alpha}-CO-O-R^{COO}$ or $-C_\alpha H_{2\alpha}-CO-O-CH_2-R^{COO}$ wherein $\alpha$ represents 1 to 10,
wherein $-R^{COO}$ represents hydrogen, $C_{1-6}$ alkyl or $C_{1-6}$ alkoxy each optionally substituted with 1 to 3 $C_{1-6}$ alkoxy groups, or cycloalkyl, phenyl, benzyl, or a heterocyclic group each optionally having 1 to 3 substituents selected from $C_{1-6}$ alkyl, $C_{1-6}$ alkoxy, $-C_{1-3}$ alkylene-$C_{1-3}$ alkoxy, and nitro,
(4): $-C_\beta H_{2\beta}-O-CO-R^{OCO}$ wherein $\beta$ represents 1 to 10,

wherein $-R^{OCO}$ represents $R^{4a1}$, $R^{4b}$, or $-C_\alpha H_{2\alpha}-CO-O-R^{COO}$, or $-C_\alpha H_{2\alpha}-CO-NH-R^{COO}$, $-C_\alpha H_{2\alpha}-NH-CO-R^{COO}$, or $-C_\alpha H_{2\alpha}-NH-CO-O-R^{COO}$ wherein - $R^{COO}$ is as defined above, or
cycloalkyl, phenyl, benzyl, or a heterocyclic group each optionally having 1 to 3 substituents selected from $C_{1-6}$ alkyl, $C_{1-6}$ alkoxy, $-C_{1-3}$ alkylene-$C_{1-3}$ alkoxy, and nitro,

(5): $-C_\gamma H_{2\gamma}-O-CO-O-R^{OCOO}$ wherein $\gamma$ represents 1 to 10,

wherein $-R^{OCOO}$ represents hydrogen, $R^{4a1}$, $R^{4b}$, or $-C_\alpha H_{2\alpha}-CO-O-R^{COO}$ wherein $-R^{COO}$ is as defined above, or
cycloalkyl, phenyl, benzyl, or a heterocyclic group each optionally having 1 to 3 substituents selected from $C_{1-6}$ alkyl, $C_{1-6}$ alkoxy, $-C_{1-3}$ alkylene-$C_{1-3}$ alkoxy, and nitro,

(i) $R^{4Na}$ and $R^{4Nb}$ are identical or different and represent $C_{1-6}$ alkyl optionally substituted with 1 to 3 $C_{1-6}$ alkoxy groups,
(ii) $R^{4Na}$ represents hydrogen, and $R^{4Nb}$ represents $C_{1-6}$ alkyl optionally substituted with 1 to 3 $C_{1-6}$ alkoxy groups, or represents $-C_\alpha H_{2\alpha}-CO-O-R^{COO}$ or $-C_\alpha H_{2\alpha}-CO-NH-R^{COO}$

wherein $-R^{COO}$ is as defined above.

Item 4.

The compound or salt thereof according to any one of Items 1 to 3, wherein the heterocyclic group has a structure in which a hydrogen atom bound to a constituent atom of a heterocycle is removed from the heterocycle, the heterocycle being selected from the group consisting of furan, tetrahydropyran, tetrahydrofuran, 1,3-dioxane, 1,4-dioxane, pyrrole, imidazole, pyrazole, pyridine, pyrimidine, pyrazine, pyridazine, pyrrolidine, imidazolidine, thiophene, piperidine, piperazine, oxazole, isoxazole, oxadiazole, morpholine, indole, indazole, benzimidazole, and quinoline.

Item 5.

The compound or salt thereof according to any one of Items 1 to 4, wherein $-OR^4$ represents any of the groups listed in the following table when asterisk "*" represents the side opposite to $R^4$ relative to the oxygen atom ($*-OR^4$):

| |
|---|
| *-OH |
| 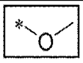 |
| 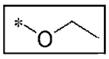 |
| 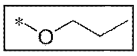 |
| 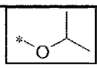 |
| 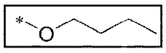 |
| 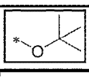 |
| 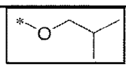 |
| 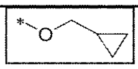 |
| 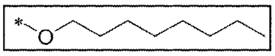 |
| 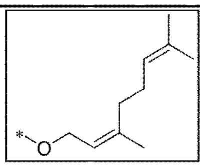 |
| 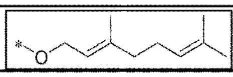 |
| 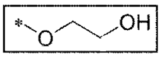 |
| 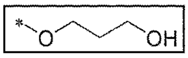 |
| 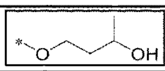 |
| 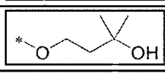 |
| 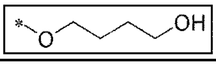 |
| 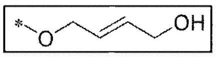 |
| 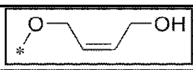 |
| 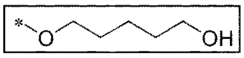 |
| 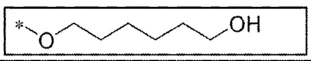 |
| 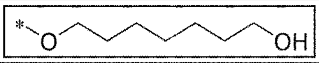 |
| 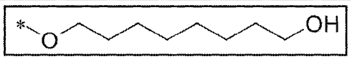 |
| 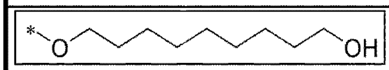 |
| 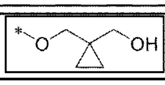 |

| |
|---|
| 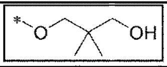 |
| 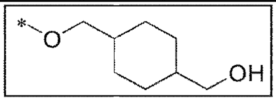 |
| 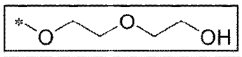 |
| 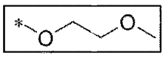 |
| 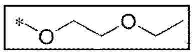 |
| 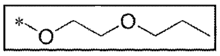 |
| 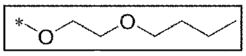 |
| 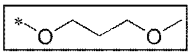 |
| 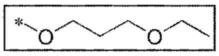 |
| 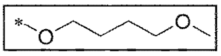 |
| 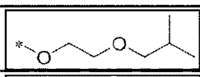 |
| 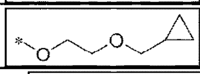 |
| 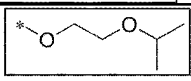 |
| 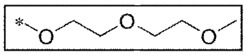 |
| 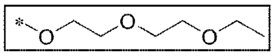 |
| 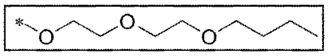 |
| 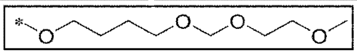 |
| 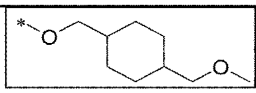 |
| 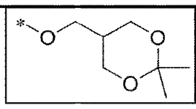 |
| 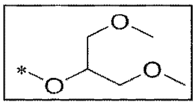 |
| 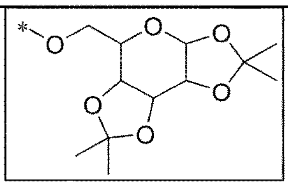 |

| |
|---|
| 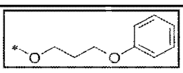 |
| 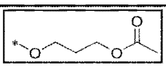 |
| 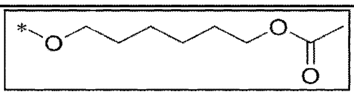 |
| 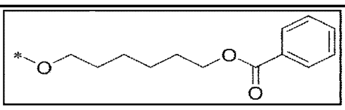 |
| 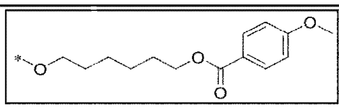 |
| 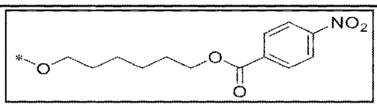 |
| 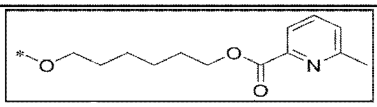 |
| 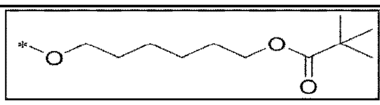 |
| 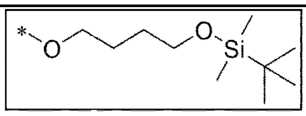 |
| 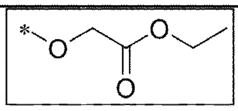 |
| 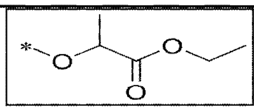 |
| 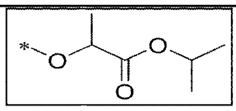 |
| 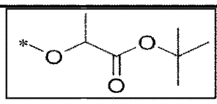 |
| 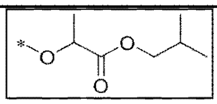 |

| |
|---|
| 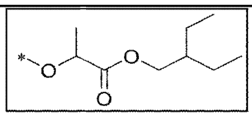 |
| 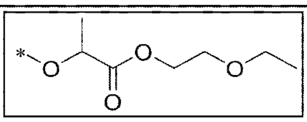 |
| 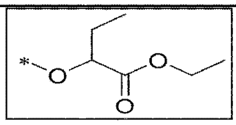 |
| 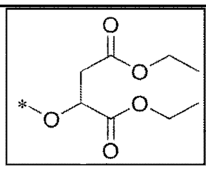 |
| 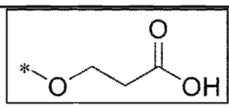 |
| 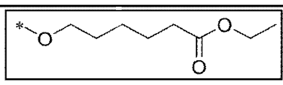 |
| 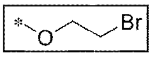 |
| 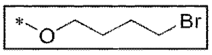 |
| 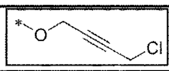 |
| 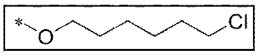 |
| 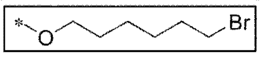 |
| 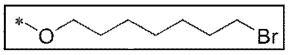 |
| 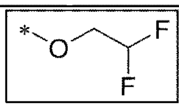 |
| 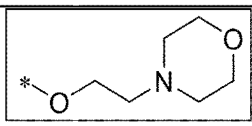 |
| 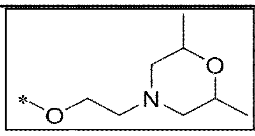 |

| |
|---|
| 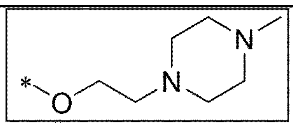 |
| 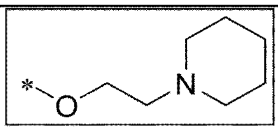 |
| 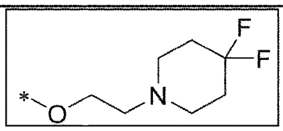 |
| 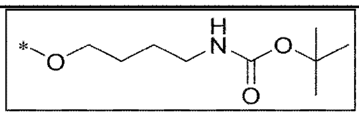 |
| 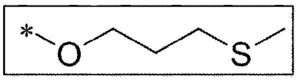 |
| 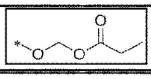 |
| 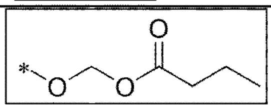 |
| 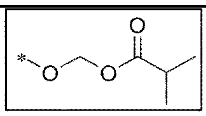 |
| 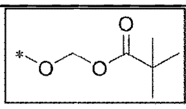 |
| 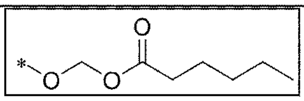 |
| 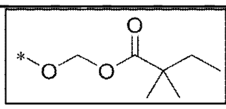 |
| 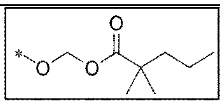 |
| 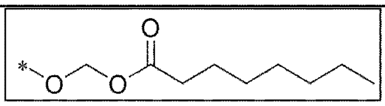 |

| |
|---|
| 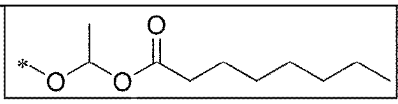 |
| 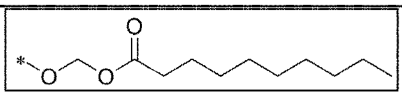 |
| 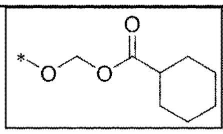 |
| 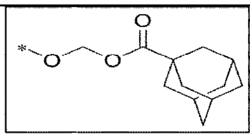 |
| 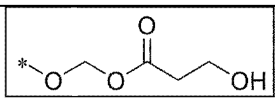 |
| 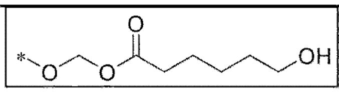 |
| 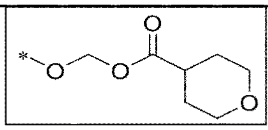 |
| 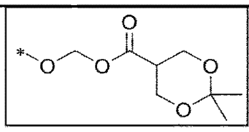 |
| 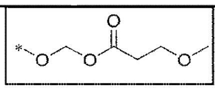 |
| 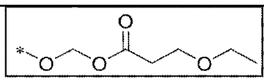 |
| 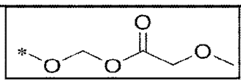 |
| 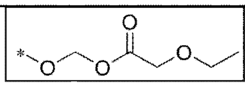 |
| 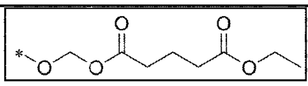 |
| 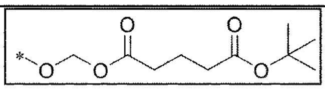 |

| |
|---|
| 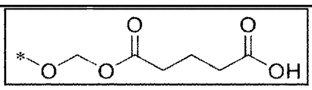 |
| 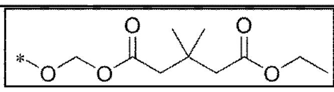 |
| 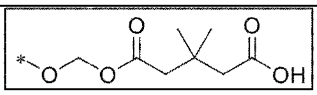 |
| 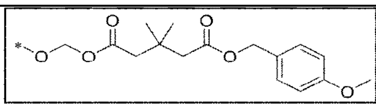 |
| 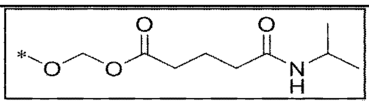 |
| 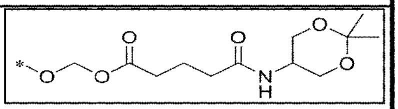 |
| 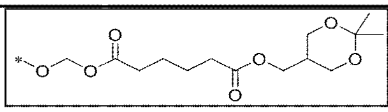 |
| 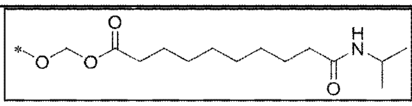 |
| 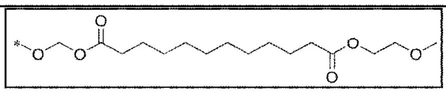 |
| 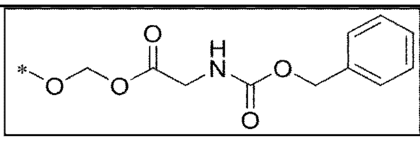 |
| 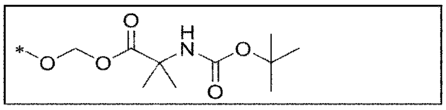 |
| 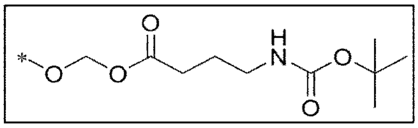 |

| |
|---|
| 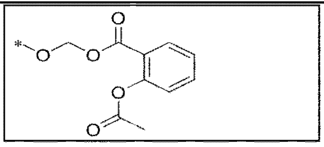 |
| 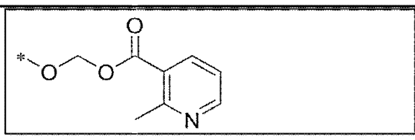 |
| 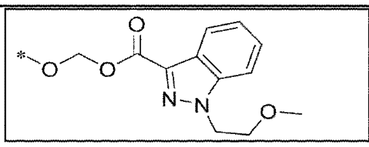 |
| 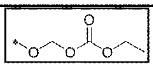 |
| 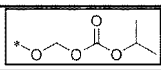 |
| 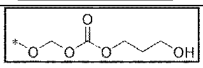 |
| 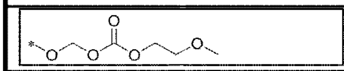 |
| 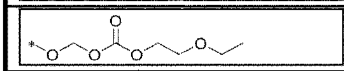 |
| 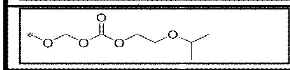 |
| 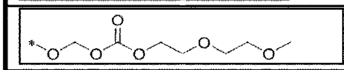 |
| 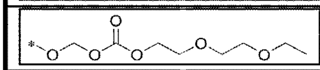 |
| 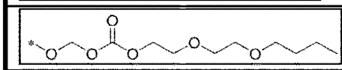 |
| 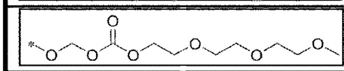 |
| 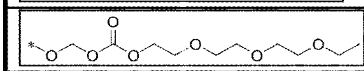 |
| 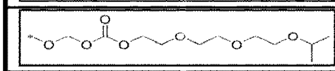 |
| 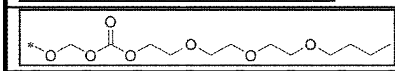 |
| 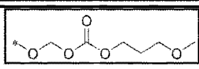 |
| 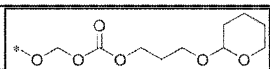 |
| 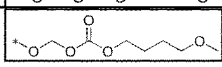 |
| 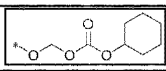 |
| 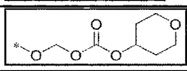 |
| 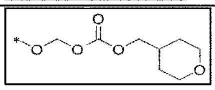 |

| |
|---|
| 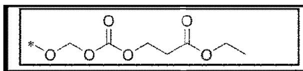 |
| 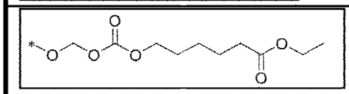 |
| 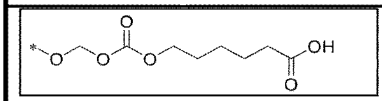 |
| 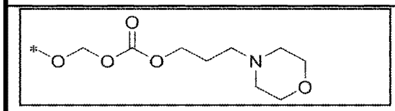 |
| 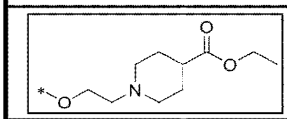 |
| 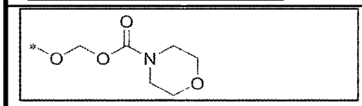 |
| 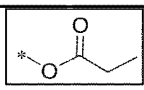 |
| 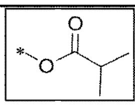 |
| 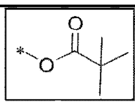 |
| 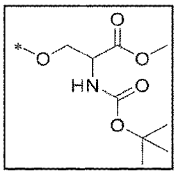 |
| 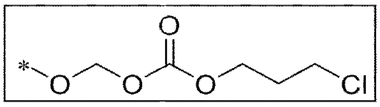 |

and $-NR^{4Na}R^{4Nb}$ represents any of the groups listed in the following table when asterisk "*" represents the side opposite to $R^{4Na}R^{4Nb}$ relative to the nitrogen atom ($*-NR^{4Na}R^{4Nb}$):

| |
|---|
| 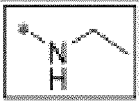 |
| 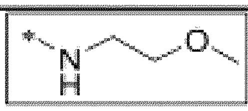 |
| 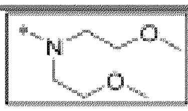 |
| 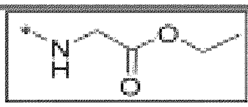 |
| 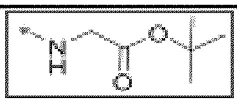 |
| 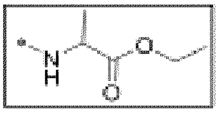 |
| 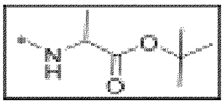 |
| 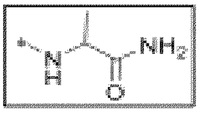 |
| 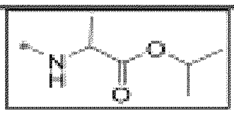 |

.

Item 6.

A pharmaceutical composition comprising the compound or salt thereof of any one of Items 1 to 5 and a pharmaceutically acceptable carrier.

Item 7.

The pharmaceutical composition according to Item 6, comprising the compound or salt thereof of any one of Items 1 to 5, a suspending agent, and a dispersion medium, wherein the pharmaceutical composition is in the form of a suspension.

Item 8.

The pharmaceutical composition according to Item 7, wherein the average particle diameter of particles in the suspension is 0.5 to 30 $\mu$m.

Item 9.

The pharmaceutical composition according to Item 7, wherein the average particle diameter of particles in the suspension is 50 to 500 nm.

Item 10.

The pharmaceutical composition according to any one of Items 7 to 9, wherein the suspending agent is carboxymethyl cellulose or a salt thereof, and the dispersion medium is a liquid containing water for injection.

Item 11.

A pharmaceutical composition comprising a viscous mixture of

a) the compound or salt thereof of any one of Items 1 to 5,
b) two or more liquid crystal-forming lipids or gel-forming lipids, and
c) a biocompatible organic solvent,

wherein the pharmaceutical composition is a precursor preparation used to form a liquid crystal phase structure or lipid gel by bringing the pharmaceutical composition into contact with an aqueous fluid in vivo.

Item 12.

The pharmaceutical composition according to Item 11, wherein b) the two or more liquid crystal-forming lipids or gel-forming lipids contain

b-1) at least one diacylglycerol, and
b-2) at least one phosphatidylcholine.

Item 13.

A pharmaceutical composition comprising a viscous mixture of

a) the compound or salt thereof of any one of Items 1 to 5,
b-1) at least one diacylglycerol,
b-2) at least one phosphatidylcholine, and
c) a biocompatible organic solvent.

Item 14.

A microsphere comprising the compound or salt thereof of any one of Items 1 to 5 as an active ingredient.

Item 15.

The microsphere according to Item 14, comprising the compound of any one of Items 1 to 5 and a biodegradable polymer. Item 16.
The microsphere according to Item 15, wherein the biodegradable polymer is at least one member selected from the group consisting of polylactic acid and a lactic acid-glycol copolymer.

Item 17.

The microsphere according to any one of Items 14 to 16, having an average particle diameter of 5 to 150 μm (preferably 30 to 100 μm).

Item 18.

A pharmaceutical composition comprising the microsphere of any one of Items 14 to 17 in the form of a suspension.

Item 19.

A pharmaceutical composition comprising the microsphere of any one of Items 14 to 17 in the form of a suspension in oil. Item 20.

The pharmaceutical composition according to Item 19, wherein the oil is a medium-chain fatty acid triglyceride.

Item 21.

The pharmaceutical composition according to any one of Items 6 to 13 and 18 to 20, which is for use in intramuscular administration or subcutaneous administration.

Item 22.

The pharmaceutical composition according to any one of Items 6 to 13 and 18 to 21, which is for use in the prevention and/or treatment of a central nervous system disease.

Item 23.

The pharmaceutical composition according to Item 22, wherein the central nervous system disease is selected from the group consisting of schizophrenia, treatment-resistant, refractory, or chronic schizophrenia, schizoaffective disorder, psychotic disorder, mood disorder, bipolar disorder, mania, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, Alzheimer's disease, Parkinson's disease, cognitive impairment, cognitive impairment associated with neurodegenerative disease, cognitive impairment due to neurodegenerative disease, cognitive impairment in schizophrenia, cognitive impairment caused by treatment-resistant, refractory, or chronic schizophrenia, vomiting, motion sickness, obesity, migraine, pain, mental retardation, autism disorder, Tourette's disorder, tic disorder, attention deficit hyperactivity disorder, conduct disorder, Down syndrome, impulsive symptoms associated with dementia, and borderline personality disorder.

Item 24.

The pharmaceutical composition according to any one of Items 6 to 13 and 18 to 23, which is administered at intervals of 1 day or 2 to 3 days.

Item 25.

The pharmaceutical composition according to any one of Items 6 to 13 and 18 to 23, which is administered at intervals of 2 weeks or more.

Item 26.

The pharmaceutical composition according to any one of Items 6 to 13 and 18 to 23, which is administered at intervals of 4 weeks or more.

Item 27.

An agent for preventing and/or treating a central nervous system disease, comprising the compound or salt thereof of any one of Items 1 to 5 as an active ingredient.

Item 28.

The agent according to Item 27, wherein the central nervous system disease is selected from the group consisting of schizophrenia, treatment-resistant, refractory, or chronic schizophrenia, schizoaffective disorder, psychotic disorder, mood disorder, bipolar disorder, mania, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, Alzheimer's disease, Parkinson's disease, cognitive impairment, cognitive impairment associated with neurodegenerative disease, cognitive impairment due to neurodegenerative disease, cognitive impairment in schizophrenia, cognitive impairment caused by treatment-resistant, refractory, or chronic schizophrenia, vomiting, motion sickness, obesity, migraine, pain, mental retardation, autism disorder, Tourette's disorder, tic disorder, attention deficit hyperactivity disorder, conduct disorder, Down syndrome, impulsive symptoms associated with dementia,

and borderline personality disorder.

Item 29.

Use of the compound or salt thereof of any one of Items 1 to 5 as a medicament.

Item 30.

A method for preventing and/or treating a central nervous system disease, comprising administering the compound or salt thereof of any one of Items 1 to 5 to a human or animal. Item 31.

The method according to Item 30, wherein the central nervous system disease is selected from the group consisting of schizophrenia, treatment-resistant, refractory, or chronic schizophrenia, schizoaffective disorder, psychotic disorder, mood disorder, bipolar disorder, mania, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, Alzheimer's disease, Parkinson's disease, cognitive impairment, cognitive impairment associated with neurodegenerative disease, cognitive impairment due to neurodegenerative disease, cognitive impairment in schizophrenia, cognitive impairment caused by treatment-resistant, refractory, or chronic schizophrenia, vomiting, motion sickness, obesity, migraine, pain, mental retardation, autism disorder, Tourette's disorder, tic disorder, attention deficit hyperactivity disorder, conduct disorder, Down syndrome, impulsive symptoms associated with dementia, and borderline personality disorder.

Item A-1.

A compound represented by formula (I) or a salt thereof:

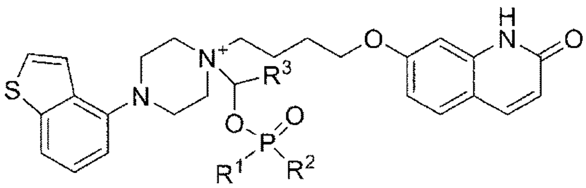

(I)

wherein

$R^1$ and $R^2$ each independently represent -O$^-$ or -O$R^4$,
$R^3$ represents hydrogen or alkyl,
$R^4$ represents hydrogen or alkyl optionally having 1 to 3 substituents, wherein alkyl represented by $R^4$ may have some carbon atoms replaced with -O- and/or -(CO)O-, and
if $R^1$ and $R^2$ both represent -O$R^4$, $R^4$ may be identical or different.

Item A-2.

The compound or salt thereof according to Item A-1, wherein

alkyl represented by $R^4$ has the carbon atom of a terminal methyl group replaced with -O- or -(CO)O-,
the -O- or -(CO)O- has substituent $R^{C1}$ bound at one end thereof, and
$R^{C1}$ represents hydrogen, lower alkyl, optionally substituted cycloalkyl, or an optionally substituted heterocyclic group.

Item A-3.

The compound or salt thereof according to Item A-1 or A-2, wherein the substituent of the alkyl optionally having 1 to 3 substituents is

(i) a substituent bound to a carbon atom of alkyl, or
(ii) a substituent ($R^{C1}$) bound to one end of the -O- or -(CO)O-,

wherein

the substituent in (i) is halogen, hydroxy, lower alkyl, lower alkoxy, cycloalkyl, carboxy, lower alkyl amino carbonyl, or an optionally substituted heterocyclic group, and
the substituent ($R^{C1}$) in (ii) is hydrogen, lower alkyl, optionally substituted cycloalkyl, or an optionally substituted heterocyclic group.

Item A-4.

The compound or salt thereof according to any one of Items A-1 to A-3, wherein the compound represented by formula (I) is a compound represented by formula (II):

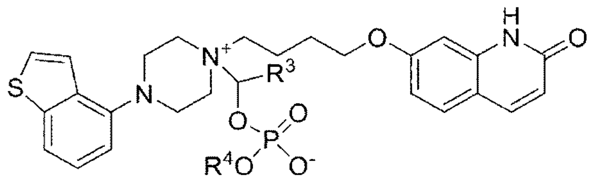

(II)

wherein $R^3$ and $R^4$ are as defined above.

Item A-5.

The compound or salt thereof according to any one of Items A-1 to A-4, wherein

$R^3$ represents hydrogen or $C_{1-6}$ alkyl,
$R^4$ represents hydrogen or $C_{1-18}$ alkyl optionally having 1 to 3 substituents, wherein alkyl represented by $R^4$ may have 1 to 5 carbon atoms replaced with -O- and/or -(CO)O-, and
if $R^1$ and $R^2$ both represent $-OR^4$, $R^4$ may be identical or different.

Item A-6.

The compound or salt thereof according to any one of Items A-1 to A-5, wherein

$R^4$ represents hydrogen or $C_{1-18}$ alkyl optionally having 1 to 3 substituents,
the substituents each independently represent hydrogen, halogen, hydroxy, lower alkoxy, $-O-(CH_2)_n-OR^5$, $-O(CO)-(CH_2)_n-OR^{5a}$,$-(CO)OR^6$, $-(CO)NR^7R^8$, $-O(CO)R^9$, $-O(CO)OR^{10}$, $-O(CO)(CR^{11}R^{12})_n(CO)OR^{13}$, $C_{3-7}$ cycloalkyl, or an optionally substituted heterocyclic group,
$R^5$, $R^{5a}$, $R^6$, $R^9$, $R^{10}$, and $R^{13}$ independently represent hydrogen, $C_{1-16}$ alkyl, or $C_{3-7}$ cycloalkyl,
$R^7$ and $R^8$ independently represent hydrogen or lower alkyl optionally having 1 or 2 carbon atoms replaced with an oxygen atom or a nitrogen atom,
$R^7$ and $R^8$ may be bound to form a ring,
$R^{11}$ and $R^{12}$ independently represent hydrogen or $C_{1-16}$ alkyl, wherein if two or more $R^{11}$s are present, the two or more $R^{11}$s independently represent hydrogen or $C_{1-16}$ alkyl, and if two or more $R^{12}$s are present, the two or more $R^{12}$s independently represent hydrogen or $C_{1-16}$ alkyl,
n represents an integer of 1 to 10 (1, 2, 3, 4, 5, 6, 7, 8, 9, or 10), and
if $R^1$ and $R^2$ both represent $-OR^4$, $R^4$ may be identical or different.

Item A-7.

The compound or salt thereof according to any one of Items A-1 to A-6, wherein

$R^4$ represents hydrogen or $-(A-Y)_m-R^{14}$,
A represents $C_{1-18}$ alkylene,
Y represents a bond, -O-, -(CO)O-, -O(CO)O-, or -(CO)NH-,

$R^{14}$ represents hydrogen, $C_{1-18}$ alkyl, hydroxy, cycloalkyl, or a heterocyclic group, and
m represents an integer of 0 to 5 (0, 1, 2, 3, 4 or 5).

Item A-8.

The compound or salt thereof according to any one of Items A-4 to A-6, wherein

$R^4$ represents hydrogen or $C_{1-8}$ alkyl optionally having one substituent,
the substituent independently represents hydroxy, lower alkoxy, $-O-(CH_2)_n-OR^5$, $-O(CO)-(CH_2)_n-OR^{5a}$, $-(CO)OR^6$, $-O(CO)R^9$, - $O(CO)OR^{10}$, $-O(CO)-A-(CO)OR^{13}$, or morpholinyl optionally having two $C_{1-6}$ alkyl groups,
n represents an integer of 1 to 10,
$R^5$, $R^{5a}$, $R^6$, $R^9$, $R^{10}$, and $R^{13}$ independently represent hydrogen, $C_{1-16}$ alkyl, an optionally substituted heterocyclic group, or $C_{3-7}$ cycloalkyl, and
A represents $C_{1-6}$ alkylene.

Item A-9.

The compound or salt thereof according to any one of Items A-4 to A-6, wherein

$R^4$ represents hydrogen or $C_{1-8}$ alkyl optionally having one substituent,
the substituent independently represents hydroxy, $C_{1-6}$ alkoxy, $-O-(CH_2)_n-OR^5$, $-O(CO)-(CH_2)_n-OR^{5a}$, $-(CO)OR^6$, $-O(CO)R^9$, $-O(CO)-A-(CO)OR^{13}$, or a heterocyclic group optionally having two $C_{1-6}$ alkyl groups,
n represents an integer of 1 to 10,
$R^5$ represents $C_{1-6}$ alkyl,
$R^{5a}$ represents $C_{1-6}$ alkyl,
$R^6$ represents $C_{1-6}$ alkyl,
$R^9$ represents $C_{1-16}$ alkyl,
$R^{13}$ represents $C_{1-6}$ alkyl or pyridyl optionally having $C_{1-6}$ alkyl, and
A represents $C_{1-6}$ alkylene.

Item A-10.

The compound or salt thereof according to any one of Items A-4 to A-9, wherein the heterocyclic group has a structure in which a hydrogen atom bound to a constituent atom of a heterocycle is removed from the heterocycle, the heterocycle being selected from the group consisting of furan, tetrahydropyran, tetrahydrofuran, 1,3-dioxane, 1,4-dioxane, pyrrole, imidazole, pyrazole, pyridine, pyrimidine, pyrazine, pyridazine, pyrrolidine, imidazolidine, thiophene, piperidine, piperazine, oxazole, isoxazole, oxadiazole, morpholine, indole, indazole, benzimidazole, and quinoline.

Item A-11.

A pharmaceutical composition comprising the compound or salt thereof of any one of Items A-1 to A-10 and a pharmaceutically acceptable carrier.

Item A-12.

The pharmaceutical composition according to Item A-11, comprising the compound or salt thereof of any one of Items A-1 to A-10, a suspending agent, and a dispersion medium, wherein the pharmaceutical composition is in the form of a suspension.

Item A-13.

The pharmaceutical composition according to Item A-12, wherein the average particle diameter of particles in the suspension is 0.5 to 30 $\mu$m.

Item A-14.

The pharmaceutical composition according to Item A-12, wherein the average particle diameter of particles in the

suspension is 50 to 500 nm.

Item A-15.

The pharmaceutical composition according to any one of Items A-12 to A-14, wherein the suspending agent is carboxymethyl cellulose or a salt thereof, and the dispersion medium is a liquid containing water for injection.

Item A-16.

A pharmaceutical composition comprising a viscous mixture of

a) the compound or salt thereof of any one of Items A-1 to A-10,
b) two or more liquid crystal-forming lipids or gel-forming lipids, and
c) a biocompatible organic solvent,

wherein the pharmaceutical composition is a precursor preparation used to form a liquid crystal phase structure or lipid gel by bringing the pharmaceutical composition into contact with an aqueous fluid in vivo.

Item A-17.

The pharmaceutical composition according to Item A-16, wherein b) the two or more liquid crystal-forming lipids or gel-forming lipids contain

b-1) at least one diacylglycerol, and
b-2) at least one phosphatidylcholine.

Item A-18.

A pharmaceutical composition comprising a viscous mixture of

a) the compound or salt thereof of any one of Items A-1 to A-10,
b-1) at least one diacylglycerol,
b-2) at least one phosphatidylcholine, and
c) a biocompatible organic solvent.

Item A-19.

A microsphere comprising the compound or salt thereof of any one of Items A-1 to A-10 as an active ingredient.

Item A-20.

The microsphere according to Item A-19, comprising the compound of any one of Items A-1 to A-10 and a biodegradable polymer.

Item A-21.

The microsphere according to Item A-20, wherein the biodegradable polymer is at least one member selected from the group consisting of polylactic acid and a lactic acid-glycol copolymer.

Item A-22.

The microsphere according to any one of Items A-19 to A-21, having an average particle diameter of 5 to 150 μm (preferably 30 to 100 μm).

Item A-23.

A pharmaceutical composition comprising the microsphere of any one of Items A-19 to A-22 in the form of a suspension.

Item A-24.

A pharmaceutical composition comprising the microsphere of any one of Items A-19 to A-22 in the form of a suspension in oil.

Item A-25.

The pharmaceutical composition according to Item A-24, wherein the oil is a medium-chain fatty acid triglyceride.

Item A-26.

The pharmaceutical composition according to any one of Items A-11 to A-18 and A-23 to A-25, which is for use in intramuscular administration or subcutaneous administration.

Item A-27.

The pharmaceutical composition according to any one of Items A-11 to A-18 and A-23 to A-26, which is for use in the prevention and/or treatment of a central nervous system disease.

Item A-28.

An agent for preventing and/or treating a central nervous system disease, comprising the compound or salt thereof of any one of Items A-1 to A-10 as an active ingredient.

Item A-29.

The agent according to Item A-28, wherein the central nervous system disease is selected from the group consisting of schizophrenia, treatment-resistant, refractory, or chronic schizophrenia, schizoaffective disorder, psychotic disorder, mood disorder, bipolar disorder, mania, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, Alzheimer's disease, Parkinson's disease, cognitive impairment, cognitive impairment associated with neurodegenerative disease, cognitive impairment due to neurodegenerative disease, cognitive impairment in schizophrenia, cognitive impairment caused by treatment-resistant, refractory, or chronic schizophrenia, vomiting, motion sickness, obesity, migraine, pain, mental retardation, autism disorder, Tourette's disorder, tic disorder, attention deficit hyperactivity disorder, conduct disorder, Down syndrome, impulsive symptoms associated with dementia, and borderline personality disorder.

Item A-30.

Use of the compound or salt thereof of any one of Items A-1 to A-10 as a medicament.

Item A-31.

A method for preventing or treating a central nervous system disease, comprising administering the compound or salt thereof of any one of Items A-1 to A-10 to a human or animal.

Item A-32.

The method according to Item A-31, wherein the central nervous system disease is selected from the group consisting of schizophrenia, treatment-resistant, refractory, or chronic schizophrenia, schizoaffective disorder, psychotic disorder, mood disorder, bipolar disorder, mania, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, Alzheimer's disease, Parkinson's disease, cognitive impairment, cognitive impairment associated with neurodegenerative disease, cognitive impairment due to neurodegenerative disease, cognitive impairment in schizophrenia, cognitive impairment caused by treatment-resistant, refractory, or chronic schizophrenia, vomiting, motion sickness, obesity, migraine, pain, mental retardation, autism disorder, Tourette's disorder, tic disorder,

attention deficit hyperactivity disorder, conduct disorder, Down syndrome, impulsive symptoms associated with dementia, and borderline personality disorder.

In the Items above, the "heterocycle" for the optionally substituted heterocyclic group is, for example, a saturated or unsaturated, monocyclic or polycyclic heterocycle containing as ring-constituting atoms 1 to 5 heteroatoms independently selected from the group consisting of nitrogen, oxygen, and sulfur, and includes, for example, a saturated or unsaturated, 3- to 15-membered, preferably 5- to 10-membered, monocyclic, bicyclic, or tricyclic heterocycle.

Specifically, examples of heterocycles include furan, tetrahydropyran, tetrahydrofuran, 1,3-dioxane, 1,4-dioxane, pyrrole, imidazole, pyrazole, pyridine, pyrimidine, pyrazine, pyridazine, pyrrolidine, imidazolidine, thiophene, piperidine, piperazine, oxazole, isoxazole, oxadiazole, morpholine, indole, indazole, benzimidazole, and quinoline. A heterocyclic group has a structure in which a hydrogen atom bound to a constituent atom of a heterocycle is removed from the heterocycle.

In the Items above, the substituent of the optionally substituted heterocyclic group includes halogen, hydroxy, $C_{1-6}$ alkyl optionally substituted with halogen, hydroxy, oxo, or $C_{1-6}$ alkoxy, and $C_{1-6}$ alkoxy optionally substituted with halogen, hydroxy, or oxo.

The number of substituents is, for example, 1, 2, or 3.

Advantageous Effects of Invention

The compound represented by formula (I) has a structure in which a specific nitrogen atom of brexpiprazole is chemically modified. After the compound represented by formula (I) is administered in vivo, the compound is converted into brexpiprazole under physiological conditions.

The compound represented by formula (I) encompasses (1) compounds with relatively low cytotoxicity. The compound represented by formula (I) encompasses (2) compounds that are highly stable and easy to handle. The compound represented by formula (I) encompasses (3) compounds that are absorbed relatively fast subcutaneously. The compound represented by formula (I) encompasses (4) compounds that are rapidly converted to brexpiprazole in vivo. The compound represented by formula (I) encompasses (5) compounds that are suitable for injection, in particular, subcutaneous injection. The compound represented by formula (I) encompasses (6) compounds that are suitable for long-acting injection that maintains the brexpiprazole concentration in blood for one to four weeks or more, in particular, subcutaneous long-acting injection.

Brief Description of Drawings

Fig. 1 is a graph showing the changes in the plasma drug concentration after subcutaneous administration of the pharmaceutical composition of Example A to rats (dose: 1.25 mg/kg) (N=3, mean ± standard deviation).

Fig. 2 is a graph showing the changes in the plasma drug concentration after subcutaneous administration of the pharmaceutical composition of Example D (aqueous suspension of the Example 1 compound) to rats (dose: 31.3 mg/kg) (N=3, mean ± standard deviation).

Fig. 3 is a graph showing the changes in the plasma drug concentration after subcutaneous administration of the pharmaceutical composition of Example E (aqueous suspension of the zinc salt of the Example 1 compound) to rats (dose: 33.2 mg/kg) (N=3, mean ± standard deviation).

Fig. 4 is a graph showing the changes in the plasma drug concentration after subcutaneous administration of the pharmaceutical composition of Example F (liquid crystal/lipid gel formulation of the Example 1 compound) to rats (dose: 31.3 mg/kg) (N=3, mean ± standard deviation).

Fig. 5 is a graph showing the changes in the plasma drug concentration after subcutaneous administration of the pharmaceutical composition of Example G (liquid crystal/lipid gel formulation of the zinc salt of the Example 1 compound) to rats (dose: 33.2 mg/kg) (N=3, mean ± standard deviation).

Fig. 6 shows the results of polarized light microscopy observation of the microspheres of Production Example I.

Fig. 7 shows the results of polarized light microscopy observation of the microspheres of Production Example J.

Fig. 8 shows the results of polarized light microscopy observation of the microspheres of Production Example K.

Fig. 9 is a graph showing the changes in the plasma drug concentration after subcutaneous administration of the pharmaceutical composition of Example H to rats (dose: 31.3 mg/kg) (microsphere formulation of the Example 1 compound (RG 505, 1.5-fold relative to API)) (N=5 until day 14, N=3 after day 21, mean ± standard deviation).

Fig. 10 is a graph showing the changes in the plasma drug concentration after subcutaneous administration of the pharmaceutical composition of Example J to rats (dose: 31.3 mg/kg) (microsphere formulation of the Example 1 compound (RG 504, 2-fold relative to API)) (N=5 until day 14, N=3 after day 21, mean ± standard deviation).

Fig. 11 is a graph showing the changes in the plasma drug concentration after subcutaneous administration of the

pharmaceutical composition of Example K to rats (dose: 31.3 mg/kg) (microsphere formulation of the Example 1 compound (RG 503, 1.5-fold relative to API)) (N=5 until day 14, N=3 after day 21, mean ± standard deviation).

Fig. 12 is a graph showing the changes in the plasma drug concentration after subcutaneous administration of the pharmaceutical composition of Example L to rats (dose: 31.3 mg/kg) (microsphere formulation of the Example 1 compound (RG 503, 2-fold relative to API)) (N=3, mean ± standard deviation).

Fig. 13 is a graph showing the changes in the plasma drug concentration after subcutaneous administration of the pharmaceutical composition of Example P (aqueous suspension of the Example 3 compound) to rats (dose: 29.8 mg/kg) (N=5, mean ± standard deviation).

Fig. 14 is a graph showing the changes in the plasma drug concentration after subcutaneous administration of the pharmaceutical composition of Example Q (aqueous suspension of the Example 10 compound) to rats (dose: 35.5 mg/kg) (N=3, mean ± standard deviation).

Fig. 15 is a graph showing the changes in the plasma drug concentration after subcutaneous administration of the pharmaceutical composition of Example R (aqueous suspension of the Example 12 compound) to rats (dose: 34.7 mg/kg) (N=3, mean ± standard deviation).

Fig. 16 is a graph showing the changes in the plasma drug concentration after subcutaneous administration of the pharmaceutical composition of Example S (aqueous suspension of the Example 13 compound) to rats (dose: 36.3 mg/kg) (N=3, mean ± standard deviation).

Fig. 17 is a graph showing the changes in the plasma drug concentration after subcutaneous administration of the pharmaceutical composition of Example T (aqueous suspension of the Example 44 compound) to rats (dose: 37.1 mg/kg) (N=3, mean ± standard deviation).

Fig. 18 is a graph showing the changes in the plasma drug concentration after subcutaneous administration of the pharmaceutical composition of Example U (aqueous suspension of the Example 92 compound) to rats (dose: 36.3 mg/kg) (N=3, mean ± standard deviation).

Fig. 19 is a graph showing the changes in the plasma drug concentration after subcutaneous administration of the pharmaceutical composition of Example V (aqueous suspension of the Example 121 compound) to rats (dose: 37.1 mg/kg) (N=3, mean ± standard deviation).

Description of Embodiments

The following describes in more detail embodiments falling within the scope of the present disclosure. Although the present disclosure includes a specific novel heterocyclic compound and a salt thereof (in particular, a pharmaceutically acceptable salt), a pharmaceutical composition containing the compound and/or salt thereof as an active ingredient, and use thereof, the present disclosure is not limited to these. The present disclosure encompasses all the matter that is disclosed in the present specification and that can be recognized by a person skilled in the art.

The specific novel heterocyclic compound falling within the scope of the present disclosure is a heterocyclic compound represented by formula (I):

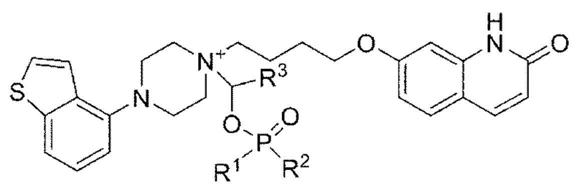

(I)

wherein

$R^1$ and $R^2$ each independently represent $-O^-$, $-OR^4$, or $-NR^{4Na}R^{4Nb}$ ,

$R^3$ represents hydrogen or alkyl,

$R^4$, $R^{4Na}$, and $R^{4Nb}$ each independently represent hydrogen, or alkyl, alkenyl, alkadienyl, or alkynyl each optionally having 1 to 3 substituents, wherein alkyl represented by $R^4$, $R^{4Na}$, or $R^{4Nb}$ may have a structure in which one or more methylene groups ($-CH_2-$) are replaced with -O-, -S-, -CO-, -NH-, $-SiR^{sia}R^{sib}-$, or -(CO)O-,

$R^{sia}$ and $R^{sib}$ are identical or different and represent hydrogen or $C_{1-6}$ alkyl,

if $R^1$ and $R^2$ both represent $-OR^4$, $R^1$ and $R^2$ may be identical or different, and

if $R^1$ and $R^2$ both represent $-NR^{4Na}R^{4Nb}$, $R^1$ and $R^2$ may be identical or different.

$R^{sia}$ and $R^{sib}$ are identical or different and represent hydrogen or $C_{1-6}$ alkyl.

In the present specification, the compound represented by formula (I) is also referred to as "compound (I)."

In the present specification, alkenyl is, for example, linear or branched alkenyl having 1 to 18 (1, 2, 3, 4, 5, 6, 7, 8, 9,

10, 11, 12, 13, 14, 15, 16, 17, or 18) carbon atoms ($C_{1-18}$ alkenyl), more preferably $C_{1-12}$ alkenyl, and still more preferably $C_{1-8}$ alkenyl or $C_{1-6}$ alkenyl. Alkenyl also includes both *cis* forms and *trans* forms, among stereoisomers due to a carbon-carbon double bond. In the present specification, alkadienyl is, for example, linear or branched alkadienyl having 1 to 18 (1, 2, 3, 4, 5, 6, 7, 8, 9, 10, 11, 12, 13, 14, 15, 16, 17, or 18) carbon atoms ($C_{1-18}$ alkadienyl), more preferably $C_{1-12}$ alkadienyl, and still more preferably $C_{1-8}$ alkadienyl or $C_{1-6}$ alkadienyl. Alkadienyl also includes both *cis* forms and *trans* forms, among stereoisomers due to a carbon-carbon double bond.

Specifically, alkadienyl includes those with any structure of (cis, cis), (cis, trans), (trans, cis), or (trans, trans), which are in order based on the carbon-carbon double bond present closer to the end of alkadienyl.

In the present specification, alkynyl is, for example, linear or branched alkynyl having 1 to 18 (1, 2, 3, 4, 5, 6, 7, 8, 9, 10, 11, 12, 13, 14, 15, 16, 17, or 18) carbon atoms ($C_{1-18}$ alkynyl), more preferably $C_{1-12}$ alkynyl, and still more preferably $C_{1-8}$ alkynyl or $C_{1-6}$ alkynyl.

If $R^4$, $R^{4Na}$, or $R^{4Nb}$ represents alkenyl, alkadienyl, or alkynyl, the alkenyl may have one or multiple (e.g., 1 to 3, preferably 1) substituents (substituent groups), the alkadienyl may have one or multiple (e.g., 1 to 3, preferably 1) substituents (substituent groups), and the alkynyl may have one or multiple (e.g., 1 to 3, preferably 1) substituents (substituent groups). Examples of substituents of these include halogen, hydroxy, lower alkyl, and lower alkoxy.

In the present specification, alkyl is, for example, linear or branched alkyl having 1 to 24 (1, 2, 3, 4, 5, 6, 7, 8, 9, 10, 11, 12, 13, 14, 15, 16, 17, 18, 19, 20, 21, 22, 23, or 24) carbon atoms ($C_{1-24}$ alkyl), more preferably $C_{1-18}$ alkyl, and still more preferably $C_{1-16}$ alkyl, $C_{1-12}$ alkyl, $C_{1-8}$ alkyl, or $C_{1-6}$ alkyl.

More specifically, alkyl can be methyl, ethyl, propyl, isopropyl, butyl, isobutyl, sec-butyl, tert-butyl, pentyl, isopentyl, neopentyl, hexyl, isohexyl, 3-methyl pentyl, heptyl, octyl, nonyl, decyl, tetradecyl, or the like.

In the present specification, alkyl represented by $R^4$, $R^{4Na}$, or $R^{4Nb}$ includes alkyl with a structure in which one or more methylene groups ($-CH_2-$) are replaced with -O-, -S-, -CO-, -NH-, $-SiR^{sia}R^{sib}-$, or -(CO)O-. $R^{sia}$ and $R^{sib}$ are identical or different and represent hydrogen or $C_{1-6}$ alkyl.

More specifically, in the present specification, alkyl represented by $R^4$, $R^{4Na}$, or $R^{4Nb}$ includes alkyl with the replacement structure described above and alkyl with no such a replacement structure. Alkyl groups other than the alkyl represented by $R^4$, $R^{4Na}$, or $R^{4Nb}$ are alkyl with no such a replacement structure unless indicated otherwise.

In alkyl with the replacement structure, the number of methylene groups replaced depends on the number of carbons of the alkyl before replacement, but is preferably 1 to 8 (1, 2, 3, 4, 5, 6, 7, or 8), more preferably 1 to 6, and still more preferably 1 to 5 or 1 to 4.

It is preferred that $R^1$ and $R^2$ are both $-OR^4$, or that $R^1$ is $-O^-$, and $R^2$ is $-OR^4$ or $NR^{4Na}R^{4Nb}$.

If $R^3$ is alkyl, lower alkyl is particularly preferable; more specifically, $C_{1-4}$ alkyl is preferable, and methyl or ethyl is more preferable.

In the present specification, "lower" as used for substituents with a carbon chain indicates the number of carbon atoms from one to six (1, 2, 3, 4, 5, or 6).

If $R^4$, $R^{4Na}$, or $R^{4Nb}$ is alkyl, specifically $C_{1-24}$ alkyl is preferable, and $C_{1-18}$ alkyl is more preferable. The alkyl, as described above, may have the replacement structure (a structure in which one or more methylene groups ($-CH_2-$) are replaced with - O-, -S-, -CO-, -NH-, $-SiR^{sia}R^{sib}-$, or -(CO)O-). As described above, the number of methylene groups replaced depends on the number of carbons of the alkyl before replacement, but is preferably 1 to 8 (1, 2, 3, 4, 5, 6, 7, or 8), more preferably 1 to 6, and still more preferably 1 to 5 or 1 to 4. In $-OR^4$ or $-NR^{4Na}R^{4Nb}$ independently represented by $R^1$ and $R^2$, if asterisk "*" is on the side of the oxygen atom (i.e., on the side in which $-OR^4$ or $-NR^{4Na}R^{4Nb}$ is bound to a phosphorus atom), -(CO)O- may be either *-(CO)O- or *-O(CO)-. If multiple methylene groups are replaced, the structures after replacement (i.e., -O-, -S-, -CO-, -NH-, $-SiR^{sia}R^{sib}-$, or -(CO)O-) may be identical or different.

For example, $-(CH_2)_2-O-(CH_2)_2-O-CH_3$ is an example of replacement of two methylene groups with -O- in $-(CH_2)_6-CH_3$. For another example, $-CH_2-(CO)O-CH_2-CH_3$ is an example of replacement of one methylene group with *-(CO)O- in $-(CH_2)_3-CH_3$. For another example, $-(CH_2)_3-O(CO)-CH_3$ is an example of replacement of one methylene group with *-O(CO)- in$-(CH_2)_4-CH_3$.

If $R^4$, $R^{4Na}$, or $R^{4Nb}$ represents alkyl, the alkyl may have one or multiple (e.g., 1 to 3) substituents (substituent groups).

The substituent is present bonding to a carbon atom of alkyl or to the nitrogen atom of -NH- replacing a methylene group; or if the methylene group in a methyl group at an end of alkyl (the underlined part of $-CH_2-^{C1}$," the substituent is present in the form of $-O-R^{C1}$, $-S-R^{C1}$, $-CO-R^{C1}$, $-NH-R^{C1}$, $-SiR^{sia}R^{sib}-R^{C1}$, or $-(CO)O-R^{C1}$, or $-O(CO)-R^{C1}$).

Examples of substituents bound to a carbon atom of alkyl include halogen, hydroxy, alkyl (preferably lower alkyl), alkoxy (preferably lower alkoxy), cycloalkyl, carboxy, alkyl amino carbonyl (preferably lower alkyl amino carbonyl), optionally substituted phenyl or benzyl, and optionally substituted heterocyclic groups (a group with a structure in which one hydrogen atom bound to a constituent atom of an optionally substituted heterocyclic group is removed from the heterocycle).

In the present specification, examples of halogens include fluorine, chlorine, bromine, and iodine (F, Cl, Br, and I), with fluorine, chlorine, or bromine being preferred.

In the present specification, alkylene is, for example, a linear or branched alkylene group having 1 to 20 carbon atoms. More specific examples include methylene, ethylene, trimethylene, tetramethylene, hexamethylene, heptamethylene, octamethylene, decamethylene, undecamethylene, dodecamethylene, 1-methylethylene, 2-ethyltrimethylene, 1-methylheptamethylene, 2-methylheptamethylene, 1-butylhexamethylene, 2-methyl-5-ethylheptamethylene, 2,3,6-trimethylheptamethylene, and 6-ethyldecamethylene.

In the present specification, alkoxy is, for example, linear or branched alkoxy having 1 to 6 carbon atoms ($C_{1-6}$ alkoxy); specific examples include methoxy, ethoxy, propoxy, isopropoxy, butoxy, isobutoxy, sec-butoxy, tert-butoxy, pentyloxy, isopentyloxy, neopentyloxy, hexyloxy, isohexyloxy, and 3-methylpentyloxy.

In the present specification, cycloalkyl is, for example, cyclic alkyl having 3 to 7 (3, 4, 5, 6, or 7) carbon atoms ($C_{3-7}$ cycloalkyl); specific examples include cyclopropyl, cyclobutyl, cyclopentyl, cyclohexyl, and cycloheptyl.

A cycloalkyl group has a structure in which a hydrogen atom bound to a carbon atom among the carbon atoms constituting the cycloalkyl is removed.

In the present specification, a "heterocycle" is, for example, a saturated or unsaturated, monocyclic or polycyclic heterocycle containing as ring-constituting atoms 1 to 5 heteroatoms independently selected from the group consisting of nitrogen, oxygen, and sulfur, and includes, for example, a saturated or unsaturated, 3- to 15-membered, preferably 5- to 10-membered, monocyclic, bicyclic, or tricyclic heterocycle.

Specifically, examples of heterocycles include furan, tetrahydropyran, tetrahydrofuran, 1,3-dioxane, 1,4-dioxane, pyrrole, imidazole, pyrazole, pyridine, pyrimidine, pyrazine, pyridazine, pyrrolidine, imidazolidine, thiophene, piperidine, piperazine, oxazole, isoxazole, oxadiazole, morpholine, indole, indazole, benzimidazole, and quinoline. A heterocyclic group has a structure in which a hydrogen atom bound to a constituent atom of a heterocycle is removed from the heterocycle.

In the present specification, examples of lower alkyl aminocarbonyl groups include a methylaminocarbonyl group, a dimethylamino carbonyl group, an ethylaminocarbonyl group, a propylaminocarbonyl group, and an isopropylaminocarbonyl group.

The substituents of optionally substituted phenyl or benzyl or of an optionally substituted heterocyclic group may be, for example, the same substituents for alkyl represented by $R^4$. The substituent also includes halogen, hydroxy, $C_{1-6}$ alkyl optionally substituted with halogen, hydroxy, oxo, or $C_{1-6}$ alkoxy, and $C_{1-6}$ alkoxy optionally substituted with halogen, hydroxy, or oxo.

The number of substituents of optionally substituted phenyl or benzyl or of an optionally substituted heterocyclic group is, for example, 1, 2, or 3.

Among those falling within the scope of compound (I), a compound in which $R^1$ represents $-O^-$, $R^2$ represents $-OR^4$ or $-NR^{4Na}R^{4Nb}$, and $R^3$ represents hydrogen or methyl is preferable.

In this case, $R^4$ or $-NR^{4Na}R^{4Nb}$ is more preferably, for example, the following group.

($R^4$: Preferable Example 1)
$R^4$ represents $R^{4a1}$, $R^{4a2}$, or $R^{4a3}$.
$R^{4a1}$ represents

$-C_nH_{2n+1}$,

$-C_nH_{2n-1}$,

$-C_nH_{2n-3}$,

$-C_nH_{2n}$-OH,

$-C_nH_{2n-2}$-OH (preferably having a single carbon-carbon double bond in $-C_nH_{2n-2}$-), or
$-C_nH_{2n-4}$-OH (more preferably having two carbon-carbon double bonds or a single carbon-carbon triple bond in $-C_nH_{2n-4}$-
(In the formulas above, n represents 1 to 24. n may represent, but is not limited to, for example, 1 to 18, 1 to 12, 1 to 10, or 1 to 6. However, n represents 2 or more in $-C_nH_{2n-1}$, n represents 2 or more in $-C_nH_{2n-3}$, n represents 2 or more in $-C_nH_{2n-2}$-, and n
represents 2 or more in $-C_nH_{2n-4}$-).
$R^{4a2}$ represents
$-CHX^1X^2$,
$-C_{n-1}H_{2n-2}-CHX^1X^2$,
$-C_{n-1}H_{2n-4}-CHX^1X^2$ (more preferably having a single carbon-carbon double bond in $-C_{n-1}H_{2n-4}$-), or
$-C_{n-1}H_{2n-6}-CHX^1X^2$ (more preferably having two carbon-carbon double bonds or a single carbon-carbon triple bond in

$-C_{n-1}H_{2n-6}-$)

(In the formulas above, n represents 2 to 24. n may represent, but is not limited to, for example, 2 to 18, 2 to 12, 2 to 10, or 2 to 6. However, n represents 3 or more in $-C_{n-1}H_{2n-6}-$.)

$X^1$ and $X^2$ may be identical or different and represent hydrogen or halogen (F, Cl, Br, or I). However, at least either one is preferably halogen.

$R^{4a3}$ represents

$-C_nH_{2n}-R^{4-1}$,

$-C_{n-1}H_{2n-2}-CHR^{4-1a}R^{4-1b}$,

$-C_nH_{2n-2}-R^{4-1}$,

or

$-C_nH_{2n-4}-R^{4-1}$

(wherein n represents 1 to 24. n may represent, but is not limited to, for example, 1 to 18, 1 to 12, 1 to 10, or 1 to 6. However, n represents 2 or more in $-C_nH_{2n-2}-$, and n represents 2 or more in $-C_nH_{2n-4}-$. $R^{4-1}$ represents $C_{1-6}$ alkoxy, -O-phenyl, or a heterocyclic group optionally substituted with $C_{1-6}$ alkyl or halogen. $R^{4-1a}$ and $R^{4-1b}$ may be identical or different and represent $-C_{1-3}$ alkylene-$C_{1-3}$ alkoxy, or identical or different and represent $-CO-O-C_{1-3}$ alkyl or $-CH_2-CO-O-C_{1-3}$ alkyl.). The same also applies to the following.

If $R^{4-1}$ is the heterocyclic group described above, preferable examples of $R^{4-1}$ include, but are not particularly limited to, the following groups.

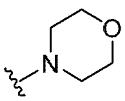
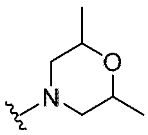
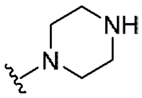
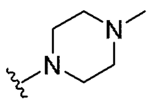
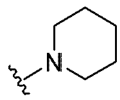
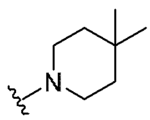
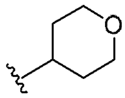

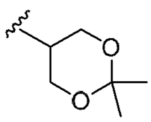
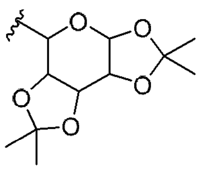
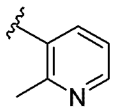
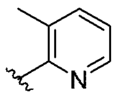
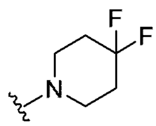
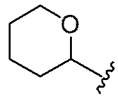

($R^4$: Preferable Example 2)
$R^4$ represents $R^{4b}$, and
$R^{4b}$ represents

$-(C_pH_{2p}-O)_q-C_rH_{2r}-R^{4-2}$,

$-(C_pH_{2p}-O)_q-C_rH_{2r-2}-R^{4-2}$,

$-(C_pH_{2p-2}-O)_q-C_rH_{2r}-R^{4-2}$,

or

$-(C_pH_{2p-2}-O)_q-C_rH_{2r-2}-R^{4-2}$

(In the formulas above, p represents 1 to 4, q represents 1 to 4, and r represents 1 to 4. However, p represents 2 or more in $C_pH_{2p-2}$, and r represents 2 or more in $C_rH_{2r-2}$. When q is 2 to 4, 2 to 4 repeating structures, the number of repetitions

of which is represented by q, may be identical or different. $R^{4-2}$ represents hydrogen, hydroxy, or "cycloalkyl, phenyl, benzyl, or a heterocyclic group each optionally having 1 to 3 substituents selected from $C_{1-6}$ alkyl, $C_{1-6}$ alkoxy, $-C_{1-3}$ alkylene-$C_{1-3}$ alkoxy, and nitro"). The same applies to the following.

($R^4$: Preferable Example 3)

$R^4$ represents $-C_{\alpha}H_{2\alpha}$-CO-O-$R^{COO}$ or $-C_{\alpha}H_{2\alpha}$-CO-O-$CH_2$-$R^{COO}$ ($\alpha$ represents 1 to 10).

-$R^{COO}$ represents hydrogen, $C_{1-6}$ alkyl or $C_{1-6}$ alkoxy each optionally substituted with 1 to 3 $C_{1-6}$ alkoxy groups, or cycloalkyl, phenyl, benzyl, or a heterocyclic group each optionally having 1 to 3 substituents selected from $C_{1-6}$ alkyl, $C_{1-6}$ alkoxy, $-C_{1-3}$ alkylene-$C_{1-3}$ alkoxy, and nitro. The same applies to the following.

$R^4$ preferably represents $-C_{\alpha}H_{2\alpha}$-CO-O-$R^{COO}$,

-$R^{COO}$ preferably represents hydrogen or $C_{1-6}$ alkyl optionally substituted with 1 to 3 $C_{1-6}$ alkoxy groups.

($R^4$: Preferable Example 4)

$R^4$ represents $-C_{\beta}H_{2\beta}$-O-CO-$R^{OCO}$ ($\beta$ represents 1 to 10).

-$R^{OCO}$ represents $R^{4a1}$, $R^{4b}$, or $-C_{\alpha}H_{2\alpha}$-CO-O-$R^{COO}$; or $-C_{\alpha}H_{2\alpha}$-CO-NH-$R^{COO}$, $-C_{\alpha}H_{2\alpha}$-NH-CO-$R^{COO}$, or $-C_{\alpha}H_{2\alpha}$-NH-CO-O-$R^{COO}$; or cycloalkyl, phenyl, benzyl, or a heterocyclic group each optionally having 1 to 3 substituents selected from $C_{1-6}$ alkyl, $C_{1-6}$ alkoxy, $-C_{1-3}$ alkylene-$C_{1-3}$ alkoxy, and nitro.

The same applies to the following.

($R^4$: Preferable Example 5)

$R^4$ represents $-C_{\gamma}H_{2\gamma}$-O-CO-O-$R^{OCOO}$ ($\gamma$ represents 1 to 10).

-$R^{OCOO}$ represents hydrogen, $R^{4a1}$, $R^{4b}$, or $-C_{\alpha}H_{2\alpha}$-CO-O-$R^{COO}$; or cycloalkyl, phenyl, benzyl, or a heterocyclic group each optionally having 1 to 3 substituents selected from $C_{1-6}$ alkyl, $C_{1-6}$ alkoxy, $-C_{1-3}$ alkylene-$C_{1-3}$ alkoxy, and nitro. The same applies to the following.

($NR^{4Na}R^{4Nb}$: Preferable Example i)

$R^{4Na}$ and $R^{4Nb}$ are identical or different and represent $C_{1-6}$ alkyl optionally substituted with 1 to 3 $C_{1-6}$ alkoxy groups.

($NR^{4Na}R^{4Nb}$: Preferable Example ii)

$R^{4Na}$ represents hydrogen,

$R^{4Nb}$ represents $C_{1-6}$ alkyl optionally substituted with 1 to 3 $C_{1-6}$ alkoxy; or $-C_{\alpha}H_{2\alpha}$-CO-O-$R^{COO}$ or $-C_{\alpha}H_{2\alpha}$-CO-NH-$R^{COO}$,

The alphabetical symbols used in the preferable examples may be identical or different. For example, -$R^{COO}$ is explained in Preferable Example 3, and also in Preferable Examples 4 and 5. -$R^{COO}$ in Preferable Examples 3 to 5 may be identical or different within the scope described above.

Additionally, the following table shows preferable examples of $R^2$ in regards to $R^1$ and $R^2$ when $R^1$ is $-O^-$, and $R^2$ is $-OR^4$ or $NR^{4Na}R^{4Nb}$. In the table, asterisk "*" indicates the side on which $R^2$ is bound to the phosphorus atom. Any optical isomers of the structural formulas shown in the table are also encompassed by the present disclosure.

| |
|---|
| *-OH |
| 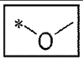 |
| 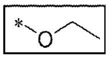 |
| 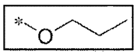 |
| 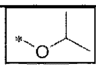 |
| 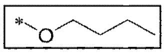 |
| 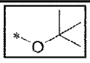 |
| 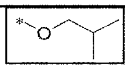 |
| 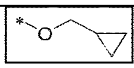 |
| 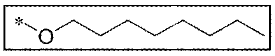 |
| 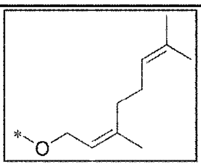 |
| 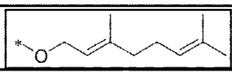 |
| 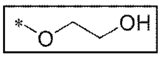 |
| 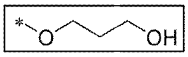 |
| 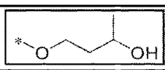 |
| 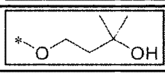 |
| 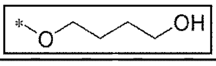 |
| 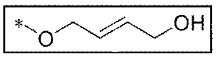 |
| 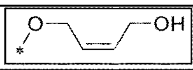 |
| 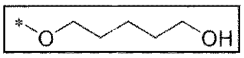 |
| 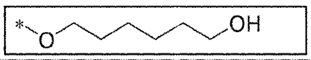 |
| 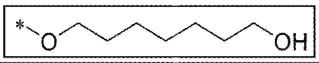 |
| 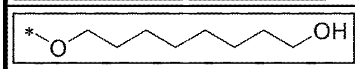 |
| 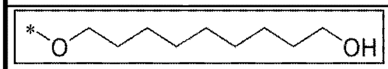 |
| 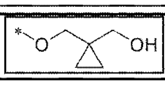 |

| |
|---|
| 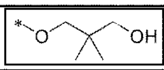 |
| 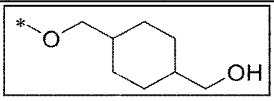 |
| 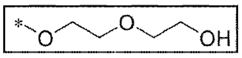 |
| 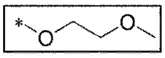 |
| 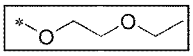 |
| 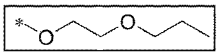 |
| 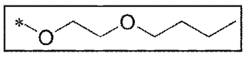 |
| 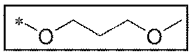 |
| 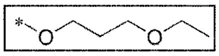 |
| 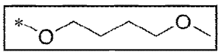 |
| 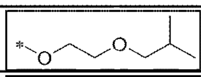 |
| 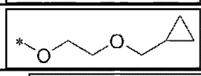 |
| 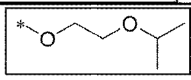 |
| 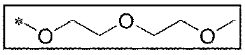 |
| 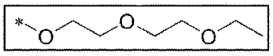 |
| 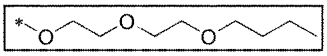 |
| 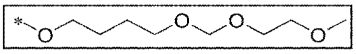 |
| 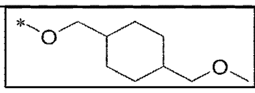 |
| 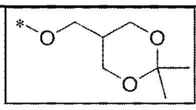 |
| 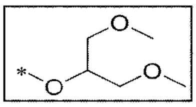 |
| 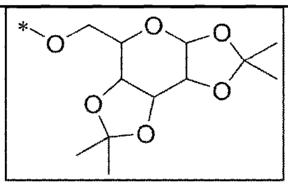 |

| |
|---|
| 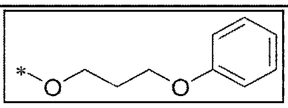 |
| 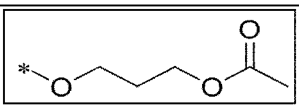 |
| 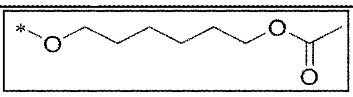 |
| 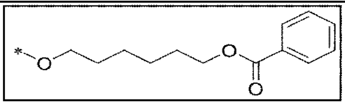 |
| 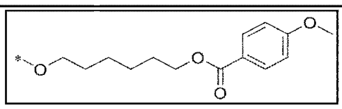 |
| 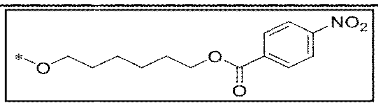 |
| 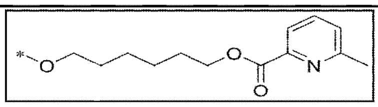 |
| 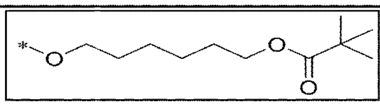 |
| 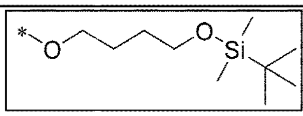 |
| 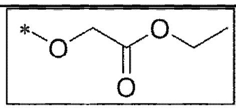 |
| 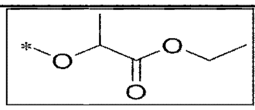 |
| 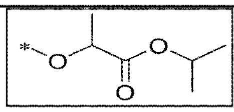 |
| 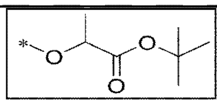 |
| 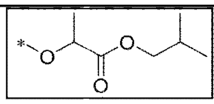 |

| |
|---|
| 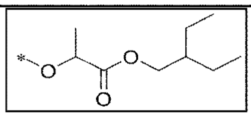 |
| 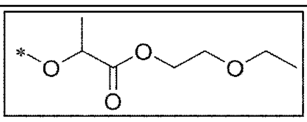 |
| 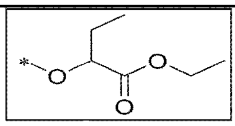 |
| 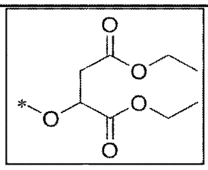 |
| 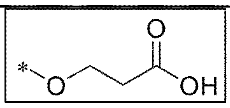 |
| 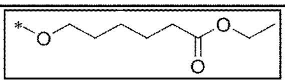 |
| 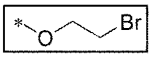 |
| 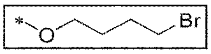 |
| 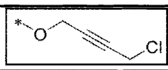 |
| 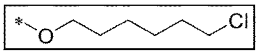 |
| 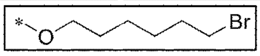 |
| 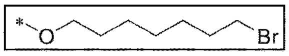 |
| 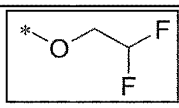 |
| 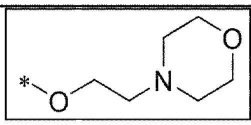 |
| 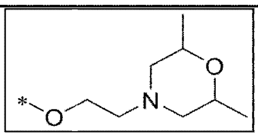 |

| |
|---|
| 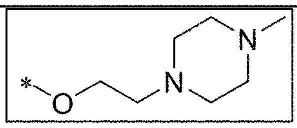 |
| 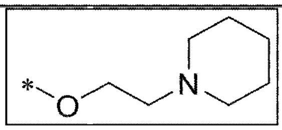 |
| 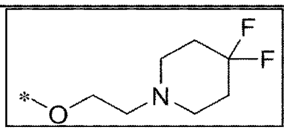 |
| 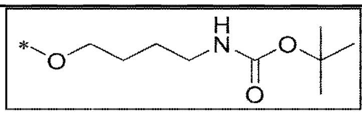 |
| 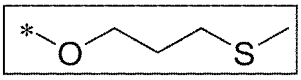 |
| 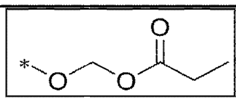 |
| 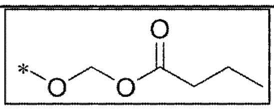 |
| 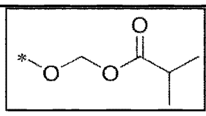 |
| 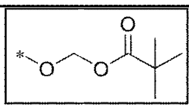 |
| 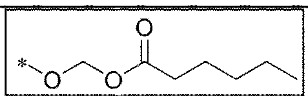 |
| 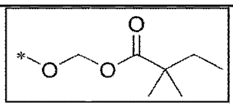 |
| 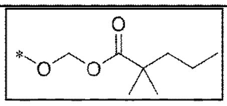 |
| 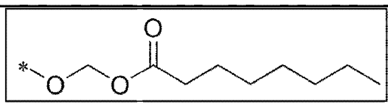 |

| |
|---|
| 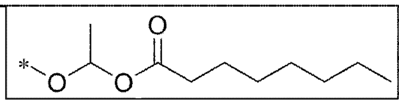 |
| 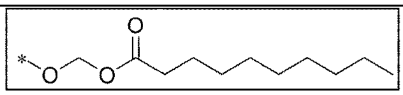 |
| 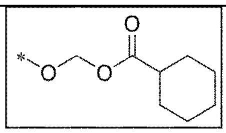 |
| 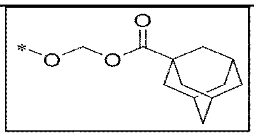 |
| 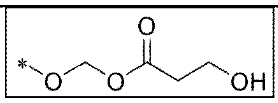 |
| 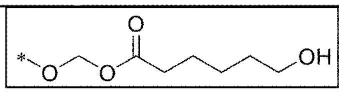 |
| 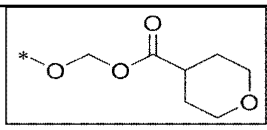 |
| 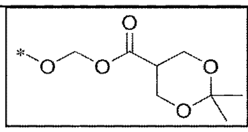 |
| 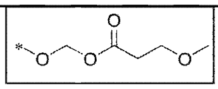 |
| 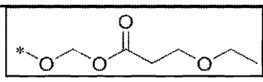 |
| 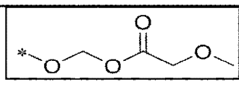 |
| 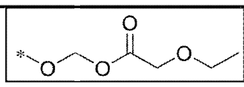 |
| 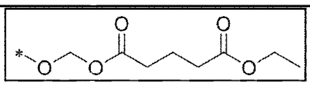 |
| 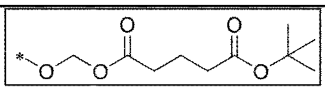 |

| |
|---|
| 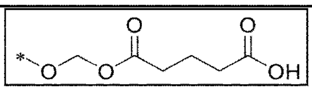 |
| 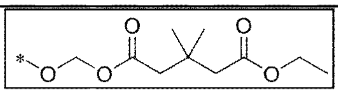 |
| 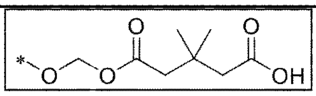 |
| 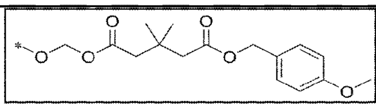 |
| 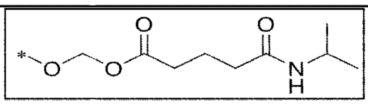 |
| 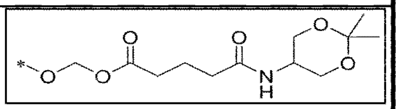 |
| 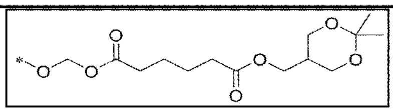 |
| 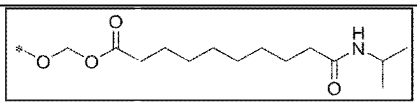 |
| 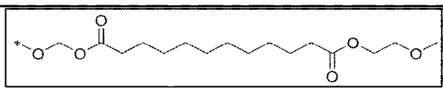 |
| 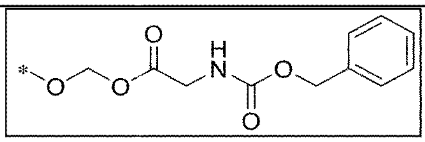 |
| 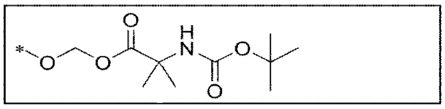 |
| 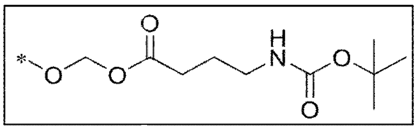 |

| |
|---|
| 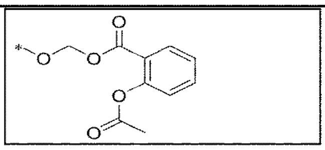 |
| 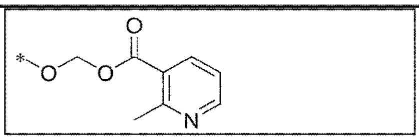 |
| 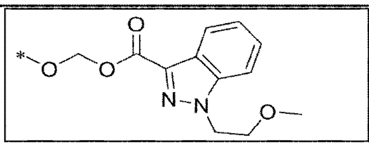 |
| 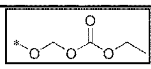 |
| 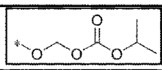 |
| 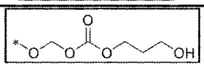 |
| 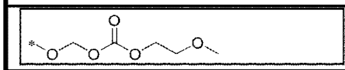 |
| 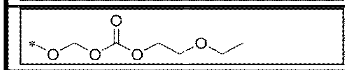 |
| 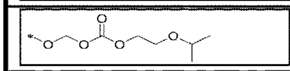 |
| 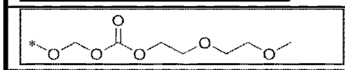 |
| 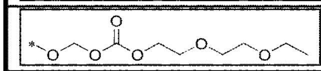 |
| 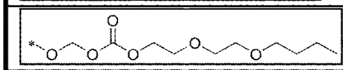 |
| 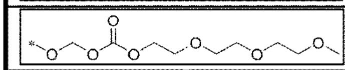 |
| 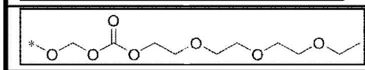 |
| 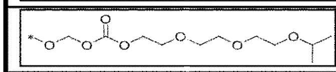 |
| 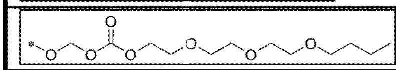 |
| 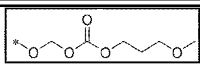 |
| 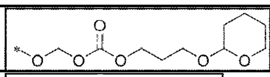 |
| 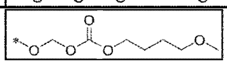 |
| 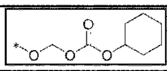 |
| 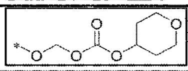 |
| 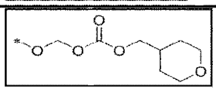 |

| |
|---|
| 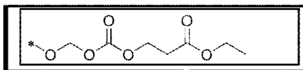 |
| 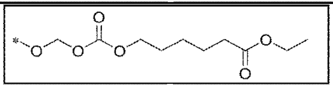 |
| 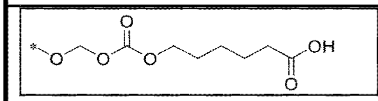 |
| 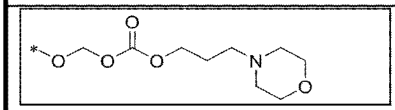 |
| 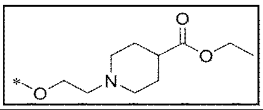 |
| 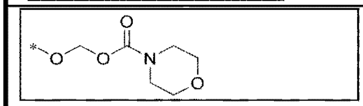 |
| 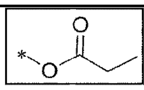 |
| 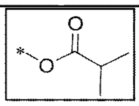 |
| 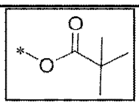 |
| 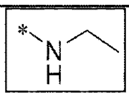 |
| 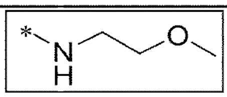 |
| 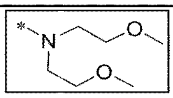 |
| 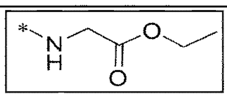 |
| 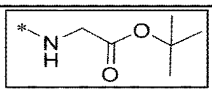 |
| 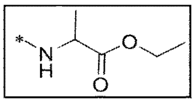 |
| 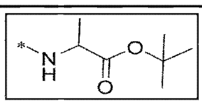 |

| |
|---|
| 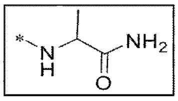 |
| 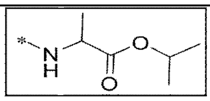 |
| 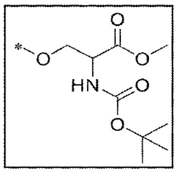 |
| 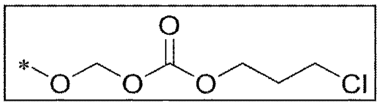 |

In a more preferable embodiment, compound (I) is a compound represented by the following formula (II):

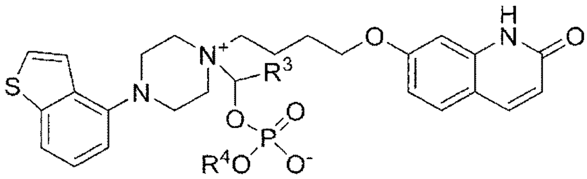

(I I)

(wherein $R^3$ and $R^4$ are as defined above). In the present specification, among the compounds falling within the scope of compound (I), the compound represented by formula (II) is also particularly referred to as "compound (II)." Compound (II) has a structure in which $R^1$ is $-O^-$, and $R^2$ is $-OR^4$ in formula (I).

The following further describes a preferable example of compound (II). However, the description above also includes the description of compound (II), and compound (II) is not limited by the following explanation.

$R^4$ can be, specifically, hydrogen or $C_{1-18}$ alkyl optionally having 1 to 3 substituents.

The substituents independently represent halogen, hydroxy, lower alkoxy, $-O-(CH_2)_n-OR^5$, $-O(CO)-(CH_2)_n-OR^{5a}$, $-(CO)OR^6$, $-(CO)NR^7R^8$, $-O(CO)R^9$, $-O(CO)OR^{10}$, $-O(CO)-(CR^{11}R^{12})_n-(CO)OR^{13}$, $C_{3-7}$ cycloalkyl, or an optionally substituted heterocyclic group. $R^5$, $R^{5a}$, $R^6$, $R^9$, $R^{10}$, and $R^{13}$ independently represent hydrogen, $C_{1-16}$ alkyl, an optionally substituted heterocyclic group, or $C_{3-7}$ cycloalkyl. $R^7$ and $R^8$ independently represent hydrogen or lower alkyl optionally having 1 or 2 carbon atoms replaced with an oxygen atom or nitrogen atom. $R^7$ and $R^8$ may be bound to form a ring. $R^{11}$ and $R^{12}$ independently represent hydrogen, $C_{1-16}$ alkyl, or $C_{3-7}$ cycloalkyl, wherein if two or more $R^{11}$s are present, the two or more $R^{11}$s independently represent hydrogen, $C_{1-16}$ alkyl, or $C_{3-7}$ cycloalkyl, and if two or more $R^{12}$s are present, the two or more $R^{12}$s independently represent hydrogen, $C_{1-16}$ alkyl, or $C_{3-7}$ cycloalkyl. n represents an integer of 1 to 10 (1, 2, 3, 4, 5, 6, 7, 8, 9, or 10).

For alkyl, "optionally having 1 or 2 carbon atoms replaced with an oxygen atom or a nitrogen atom" is synonymous with "one or more methylene groups ($-CH_2-$) may be replaced with -O- or -NH."

Although not particularly limited, examples of the optionally substituted heterocyclic group include the groups listed above as $R^{4-1}$ representing a heterocyclic group optionally substituted with $C_{1-6}$ alkyl or halogen. Without any particular limitation, if $R^7$ and $R^8$ are bound to form a ring, examples of - $NR^7R^8$ include groups with a structure in which one hydrogen atom bound to a nitrogen atom is removed, among the groups listed as $R^{4-1}$ representing a heterocyclic group optionally substituted with $C_{1-6}$ alkyl or halogen.

$R^4$ is more preferably hydrogen or $C_{1-8}$ alkyl optionally having one substituent.

The substituent independently represents hydroxy, lower alkoxy, $-O-(CH_2)_n-OR^5$, $-O(CO)-(CH_2)_n-OR^{5a}$, $-(CO)OR^6$, $-O(CO)R^9$, $-O(CO)OR^{10}$, $-O(CO)-A-(CO)OR^{13}$, or morpholinyl optionally having two $C_{1-6}$ alkyl groups. $R^5$, $R^{5a}$, $R^6$, $R^9$, $R^{10}$, and $R^{13}$ independently represent hydrogen, $C_{1-16}$ alkyl, an optionally substituted heterocyclic group, or $C_{3-7}$ cycloalkyl. A represents $C_{1-6}$ alkylene. n is as defined above.

$R^4$ is more preferably hydrogen or $C_{1-8}$ alkyl optionally having one substituent. The substituent independently

represents hydroxy, $C_{1-6}$ alkoxy, -O-$(CH_2)_n$$OR^5$, -O(CO)-$(CH_2)_n$-$OR^{5a}$, -(CO)$OR^6$, -O(CO)$R^9$, -O(CO)-A-(CO)$OR^{13}$, or a heterocyclic group optionally having two $C_{1-6}$ alkyl groups. $R^5$ represents $C_{1-6}$ alkyl. $R^{5a}$ represents $C_{1-6}$ alkyl. $R^6$ represents $C_{1-6}$ alkyl. $R^9$ represents $C_{1-16}$ alkyl. $R^{13}$ represents $C_{1-6}$ alkyl or pyridyl optionally having $C_{1-6}$ alkyl. A represents $C_{1-6}$ alkylene. n is as defined above.

$R^4$ also represents hydrogen or -$(A\text{-}Y)_m$-$R^{14}$ wherein A represents $C_{1-18}$ alkylene, Y represents a bond, -O-, -(CO)O-, or -(CO)NH-, $R^{14}$ represents hydrogen, halogen, $C_{1-18}$ alkyl, hydroxy, cycloalkyl, or a heterocyclic group, and m represents an integer of 0 to 5 (0, 1, 2, 3, 4, or 5).

In -$OR^4$ independently represented by $R^1$ and $R^2$, if asterisk "*" is on the side of the oxygen atom (i.e., on the side of the phosphorus atom to which -$OR^4$ is bound), -(CO)O- may be either *-(CO)O- or *-O(CO)-, and -(CO)NH- may be either *-(CO)NH- or *-NH(CO)-.

The following table shows more preferable examples of specific compound (I).

| |
|---|
| 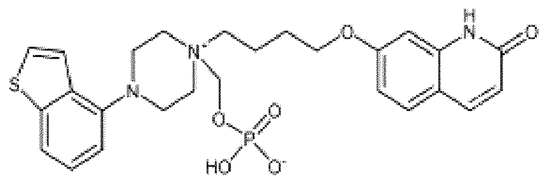 |
| 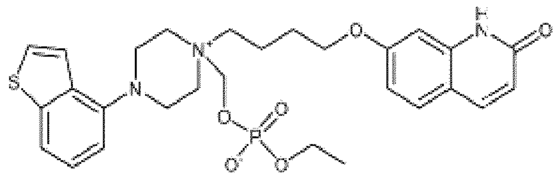 |
| 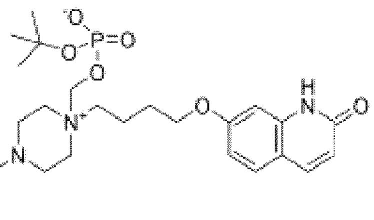 |

| |
|---|
| 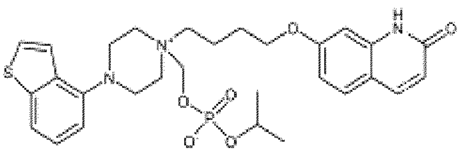 |
| 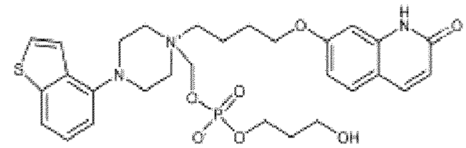 |
| 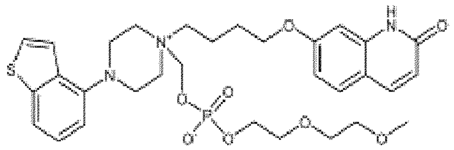 |
| 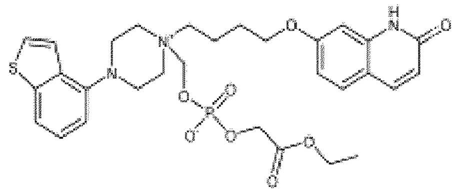 |

| |
|---|
| 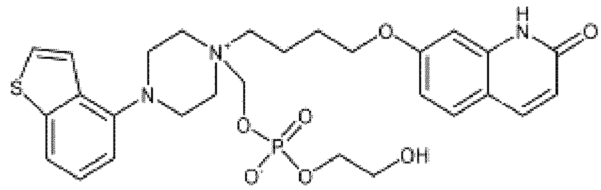 |
| 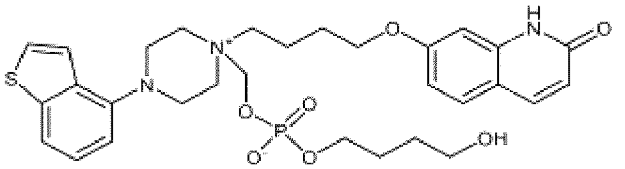 |
| 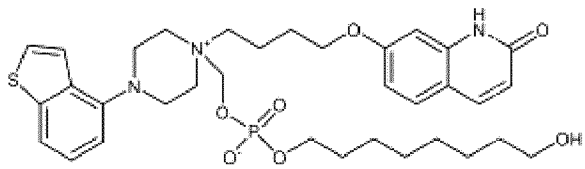 |
| 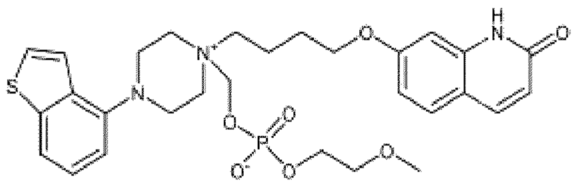 |

| |
|---|
| 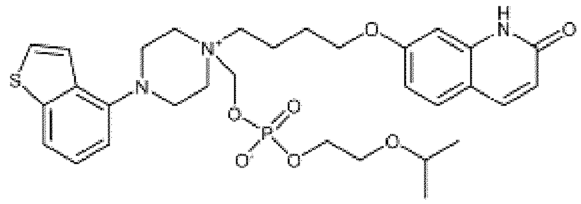 |
| 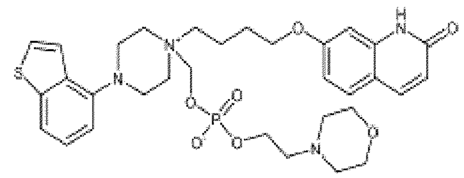 |
| 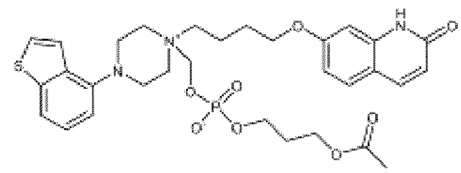 |
| 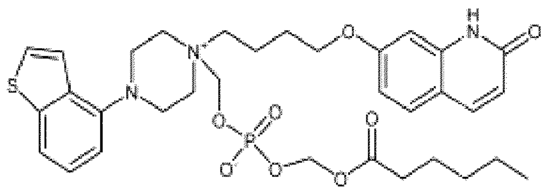 |

| |
|---|
| 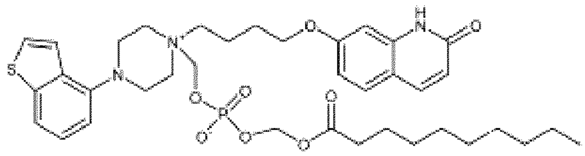 |
| 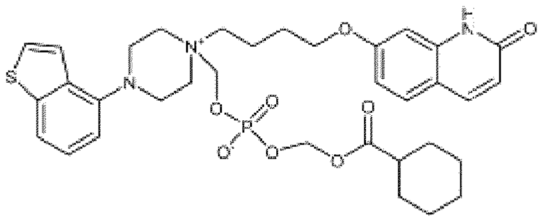 |
| 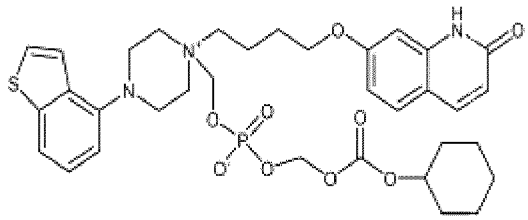 |

| |
|---|
| 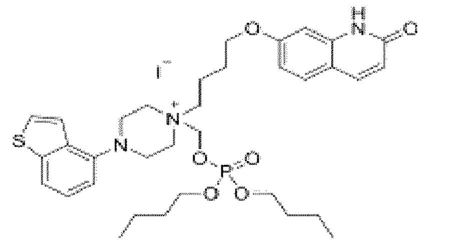 |
| 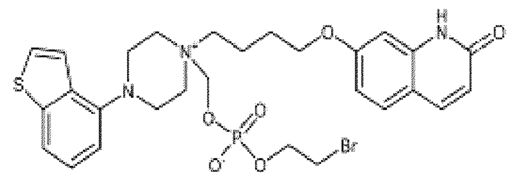 |
| 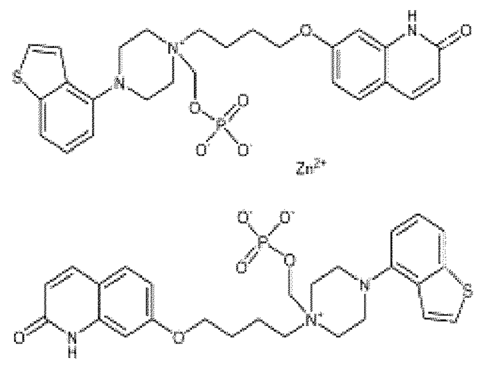 |

| |
|---|
| 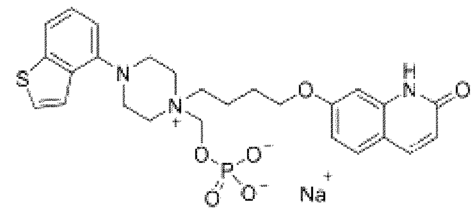 |
| 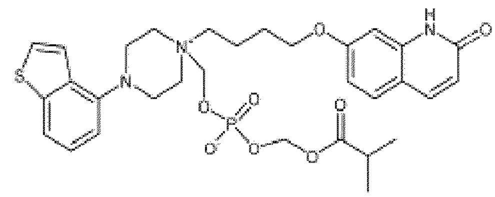 |
| 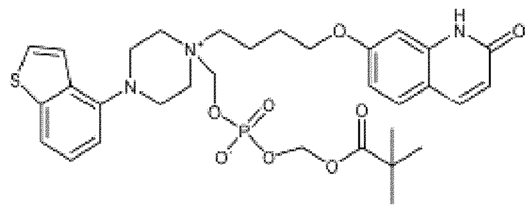 |
| 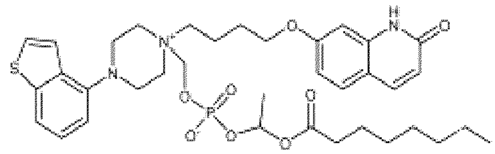 |
| 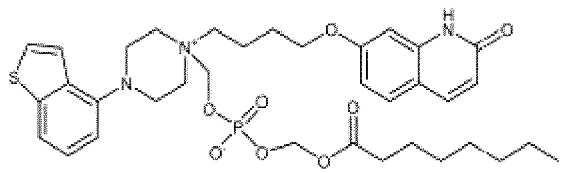 |

| |
|---|
| 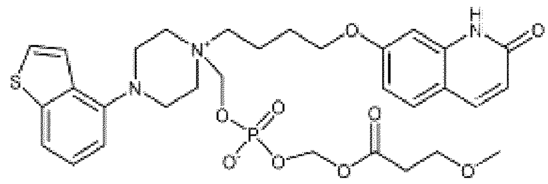 |
| 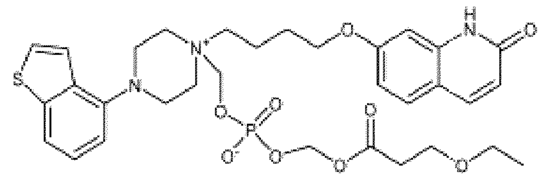 |
| 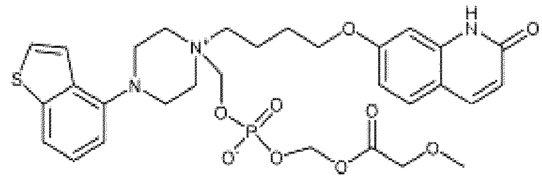 |
| 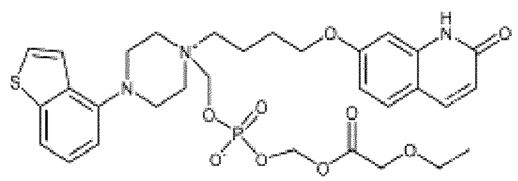 |

| |
|---|
| 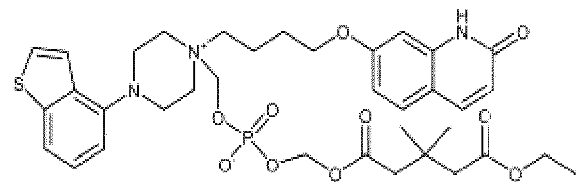 |
| 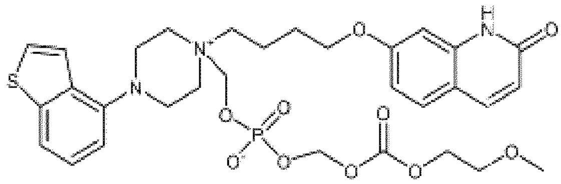 |
| 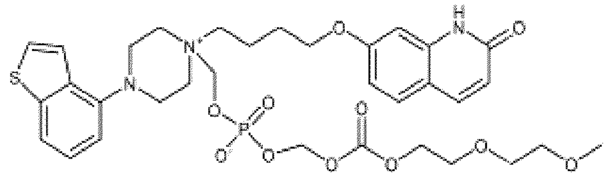 |
| 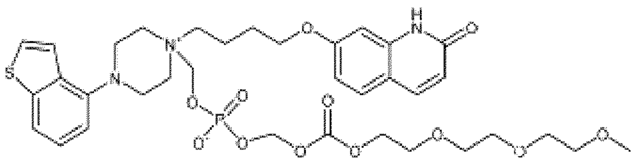 |

| |
|---|
| 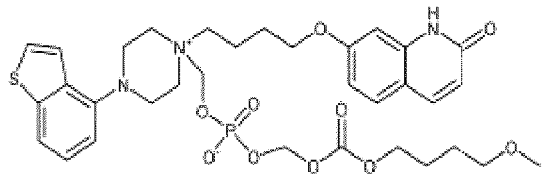 |
| 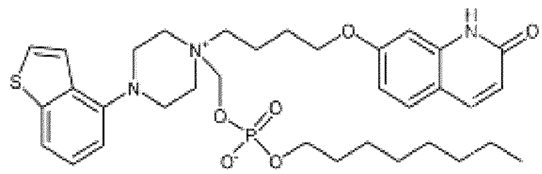 |
| 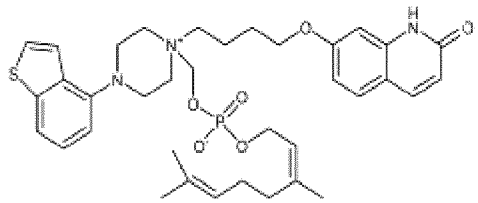 |
| 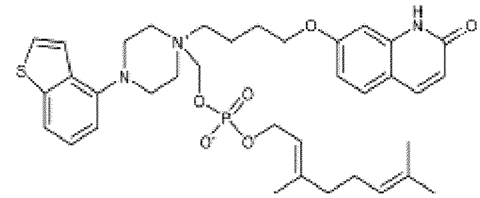 |

| |
|---|
| 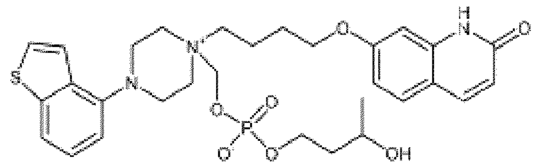 |
| 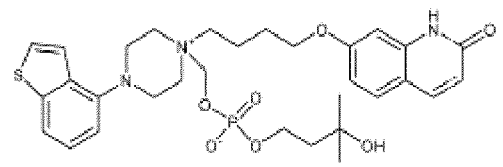 |
| 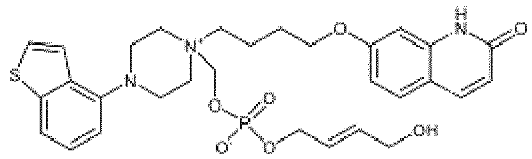 |
| 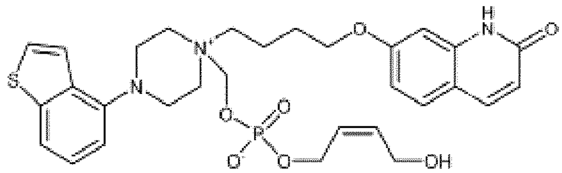 |

| |
|---|
| 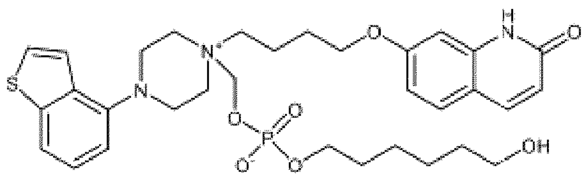 |
| 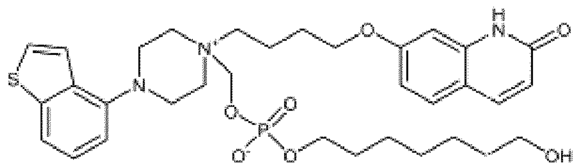 |
| 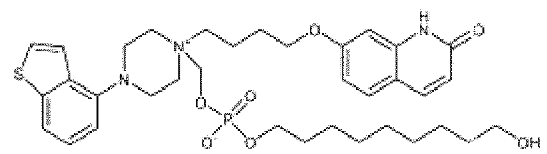 |
| 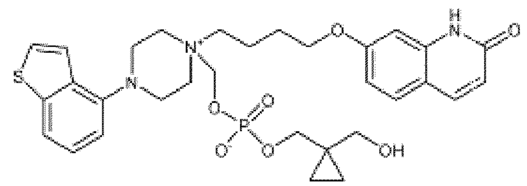 |

| |
|---|
| 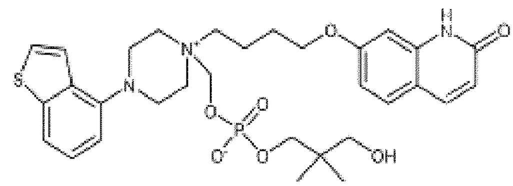 |
| 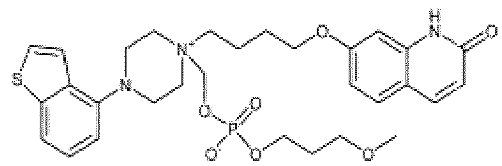 |
| 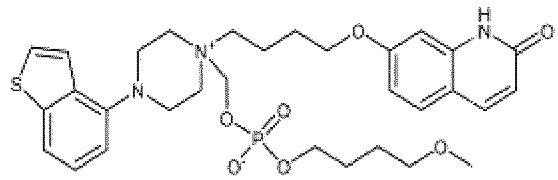 |
| 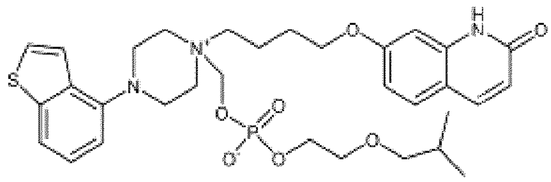 |

| |
|---|
| 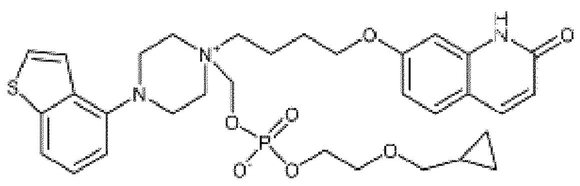 |
| 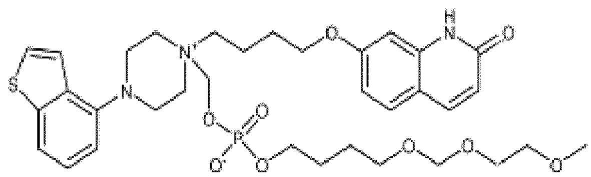 |
| 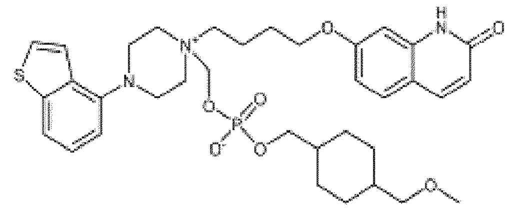 |
| 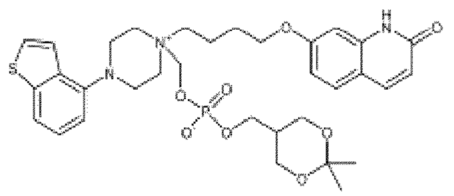 |

| |
|---|
| 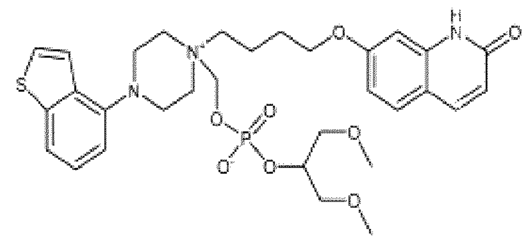 |
| 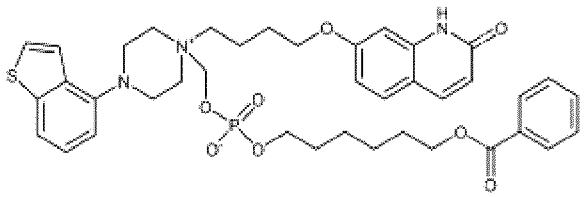 |
| 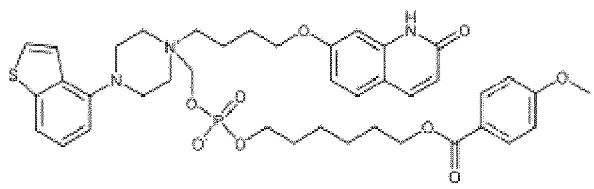 |
| 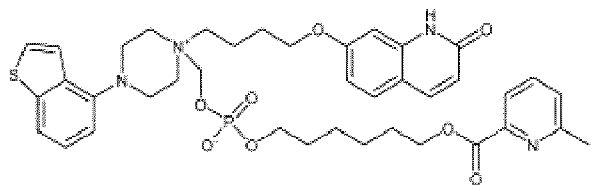 |

| |
|---|
| 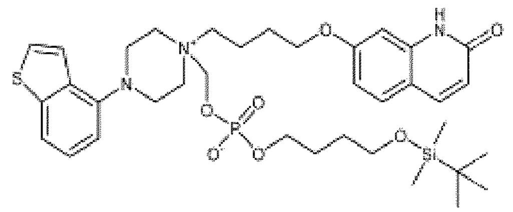 |
| 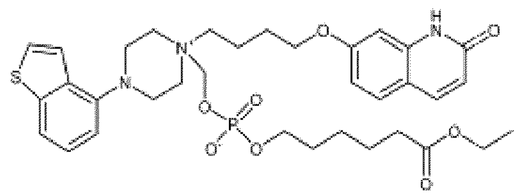 |
| 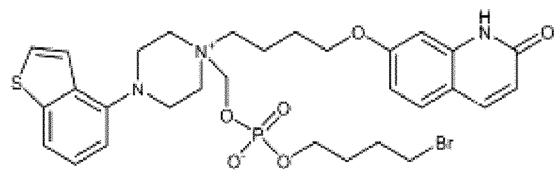 |
| 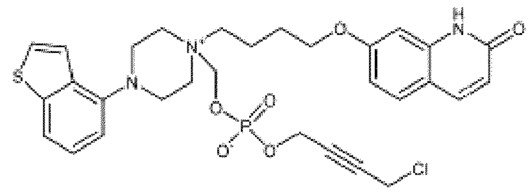 |

| |
|---|
| 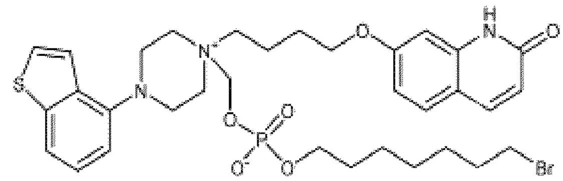 |
| 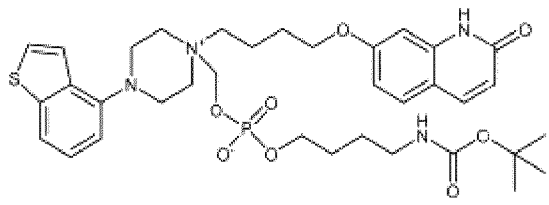 |
| 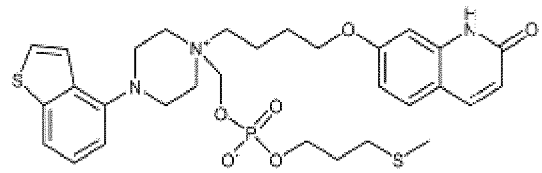 |
| 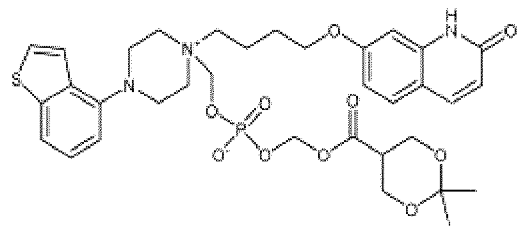 |

| |
|---|
| 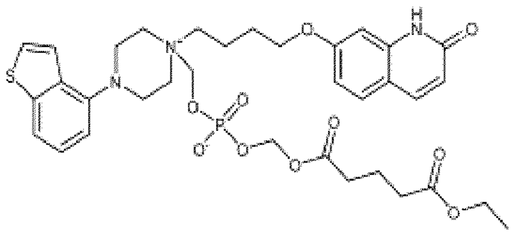 |
| 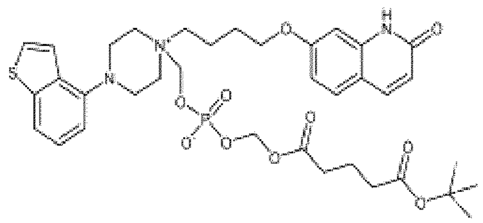 |
| 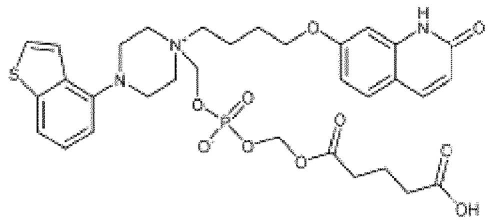 |

| |
|---|
| 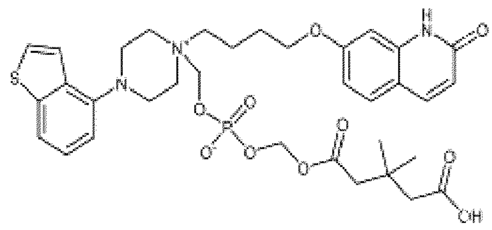 |
| 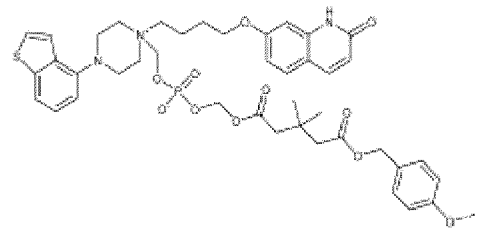 |
| 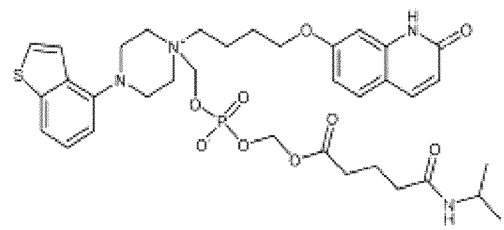 |

| |
|---|
| 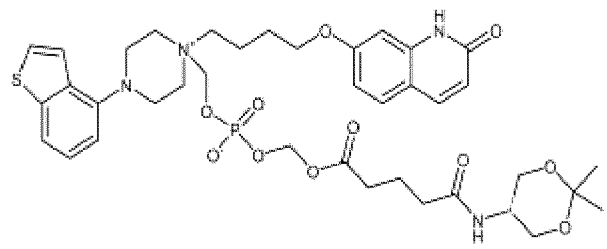 |
| 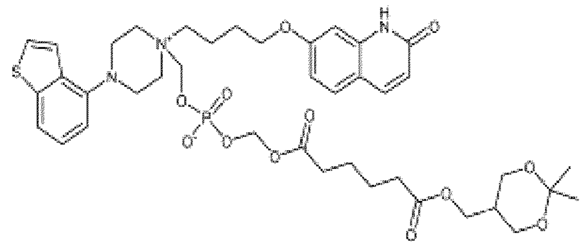 |
| 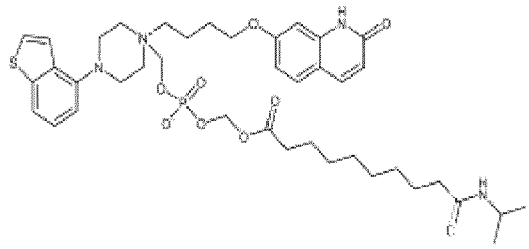 |

| |
|---|
| 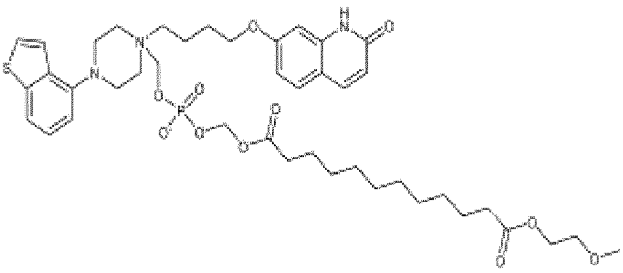 |
| 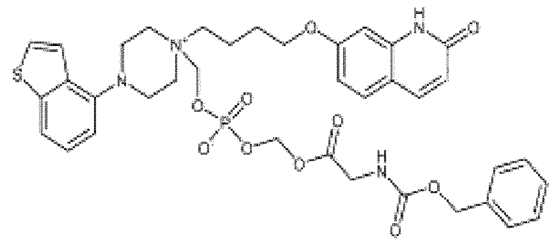 |
| 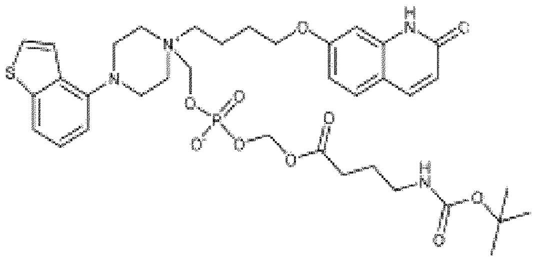 |

| |
|---|
| 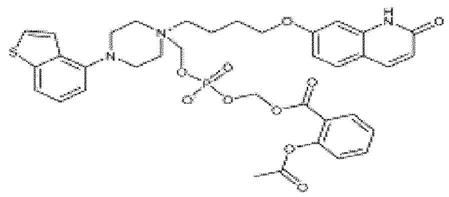 |
| 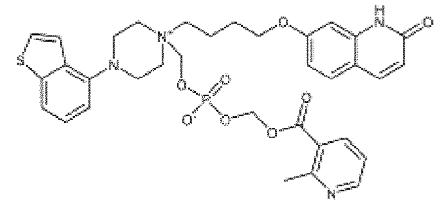 |
| 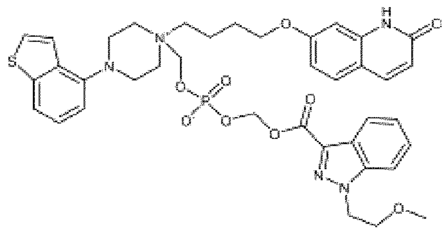 |

| |
|---|
| 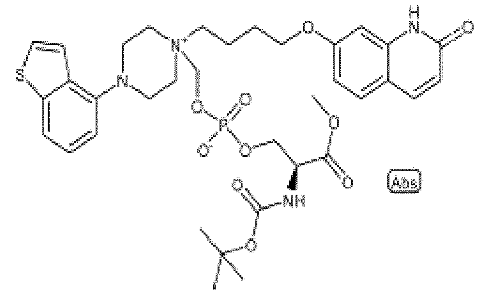 |
| 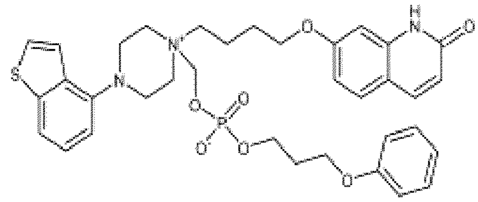 |
| 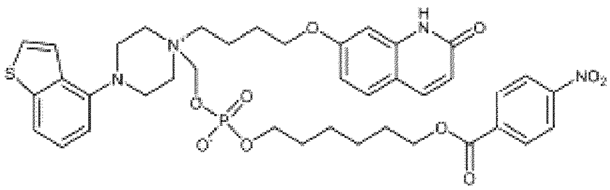 |

| |
|---|
| 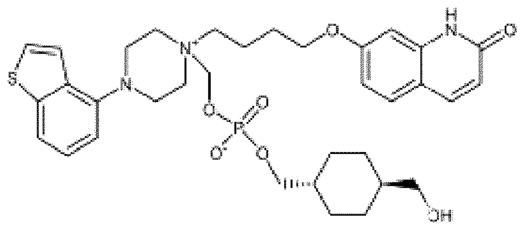 |
| 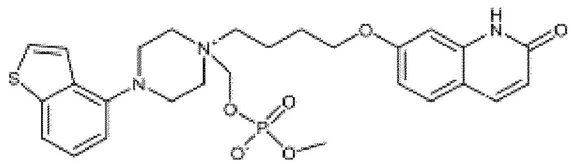 |
| 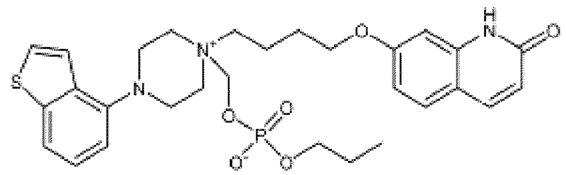 |
| 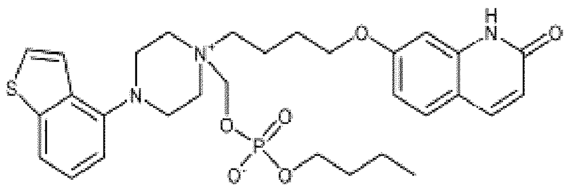 |

| |
|---|
| 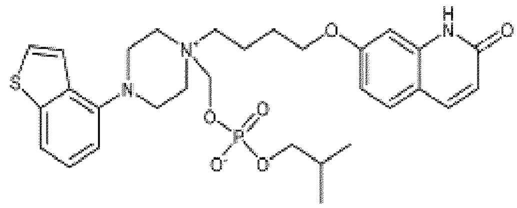 |
| 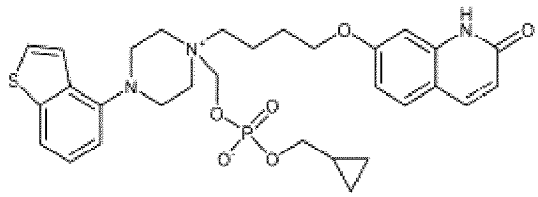 |
| 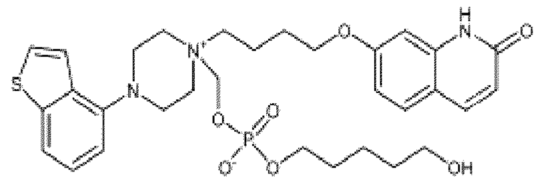 |
| 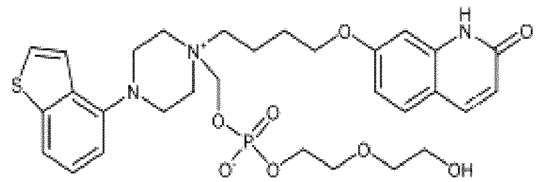 |

| |
|---|
| 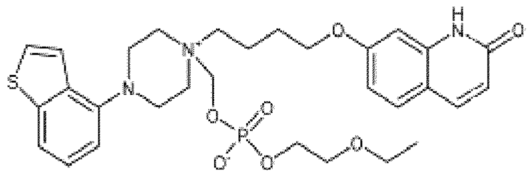 |
| 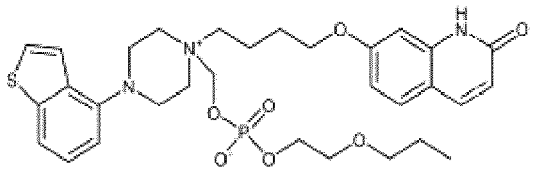 |
| 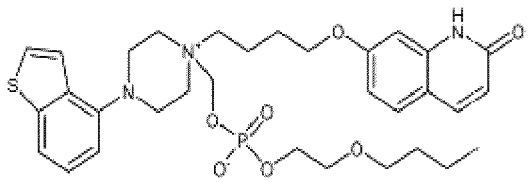 |

| |
|---|
| 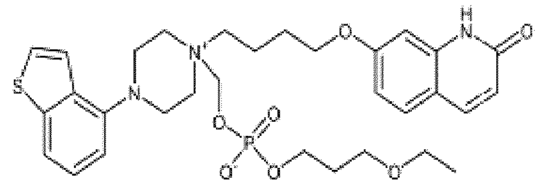 |
| 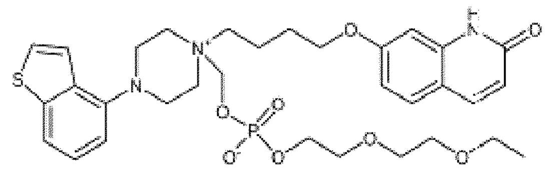 |
| 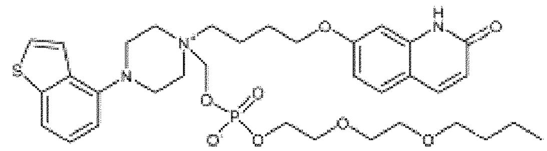 |

| |
|---|
| 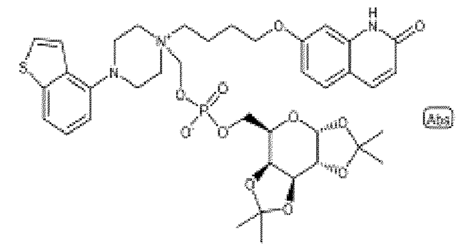 |
| 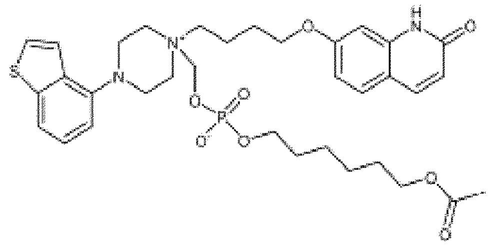 |
| 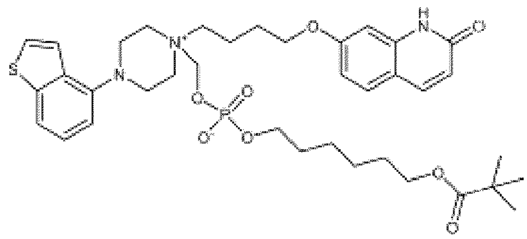 |

| |
|---|
| 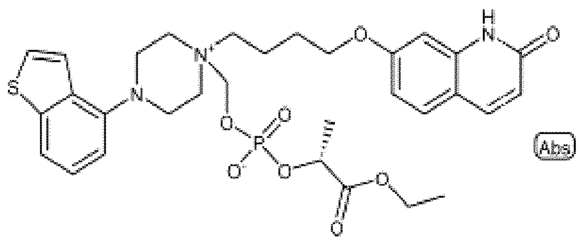 |
| 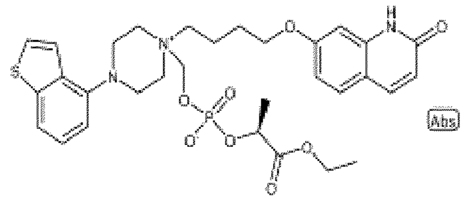 |
| 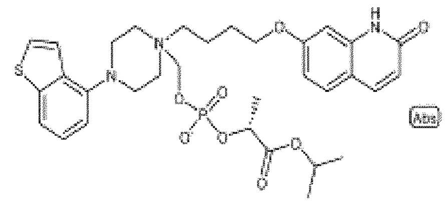 |

| |
|---|
| 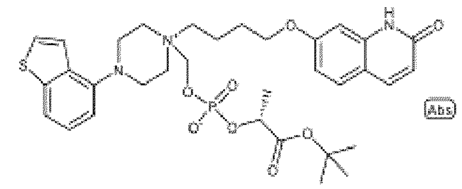 |
| 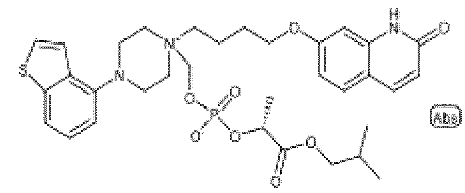 |
| 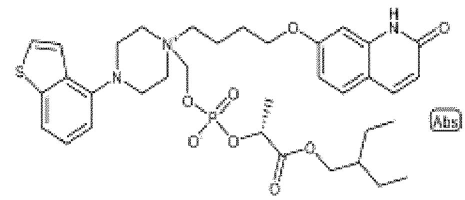 |
| 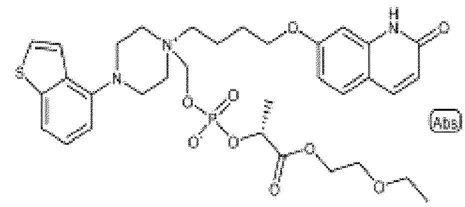 |

| |
|---|
| 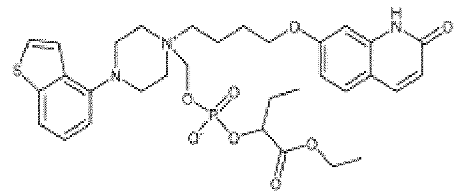 |
| 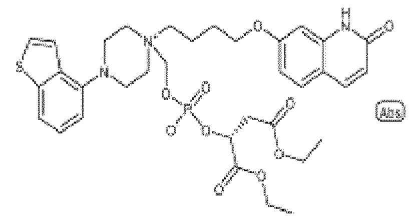 |
| 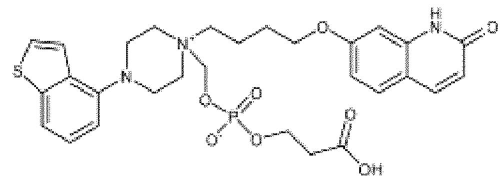 |
| 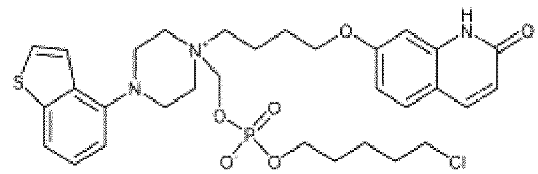 |

| |
|---|
| 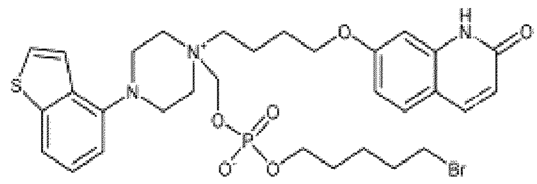 |
| 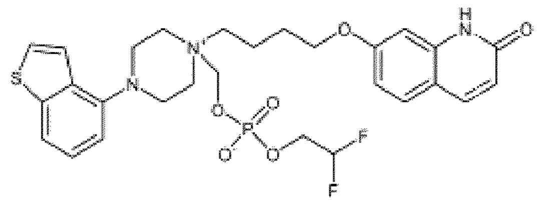 |
| 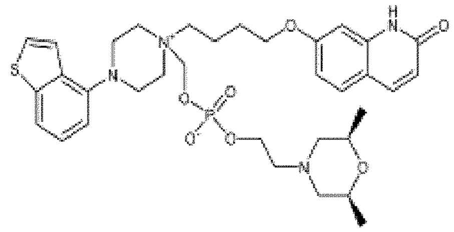 |
| 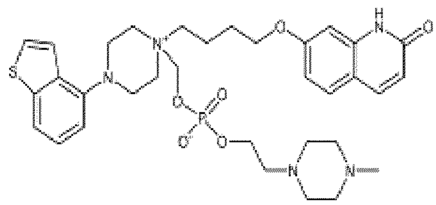 |

| |
|---|
| 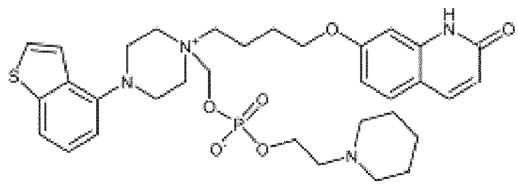 |
| 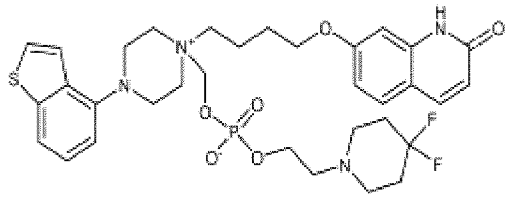 |
| 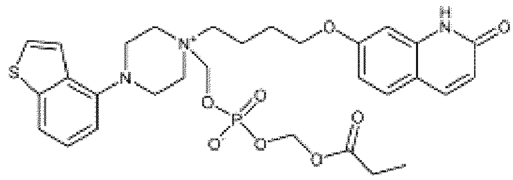 |
| 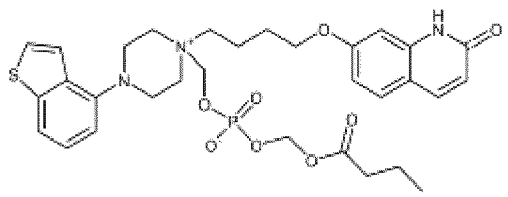 |

| |
|---|
| 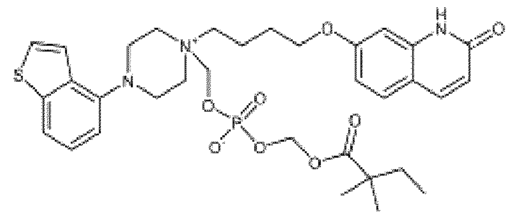 |
| 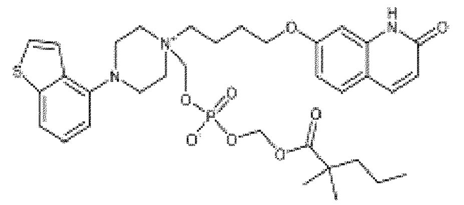 |
| 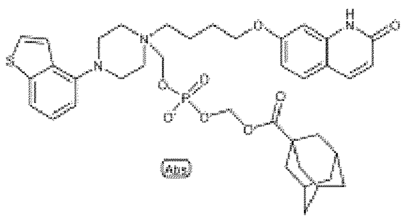 |
| 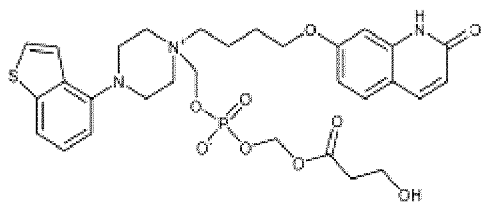 |

| |
|---|
| 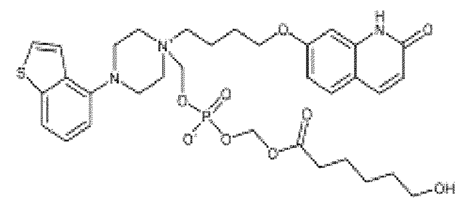 |
| 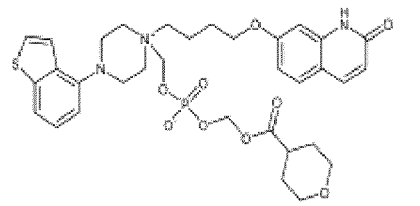 |
| 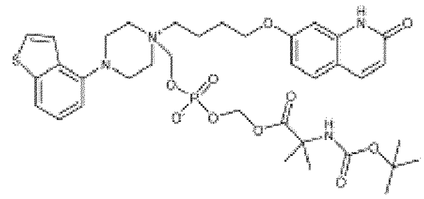 |
| 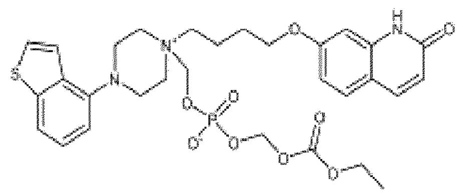 |

| |
|---|
| 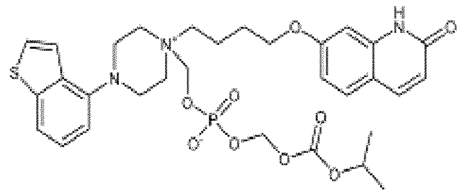 |
| 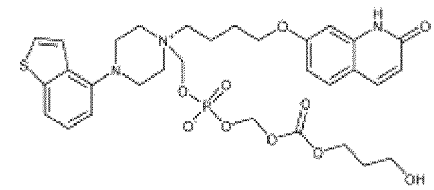 |
| 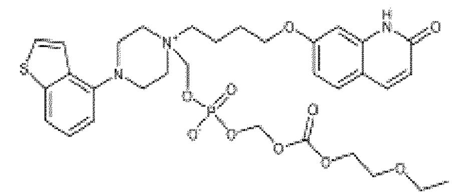 |
| 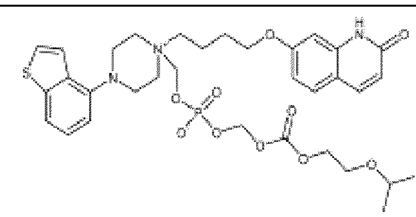 |

| |
|---|
| 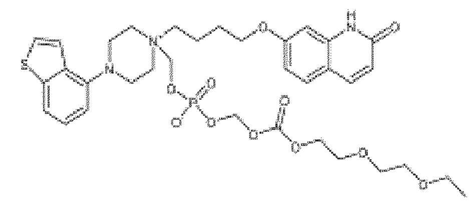 |
| 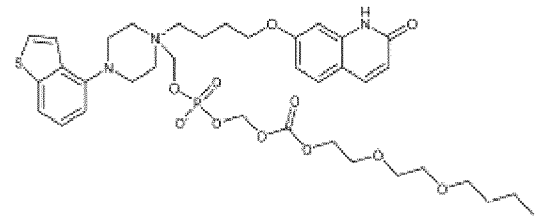 |
| 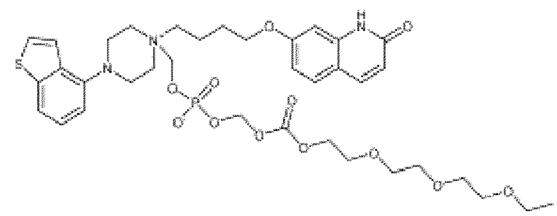 |
| 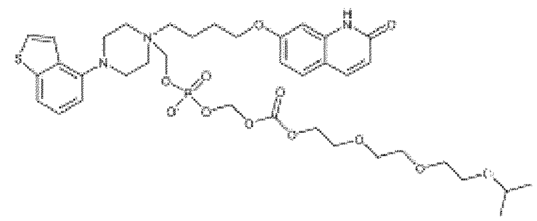 |

| |
|---|
| 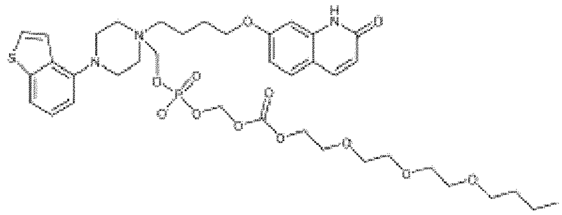 |
| 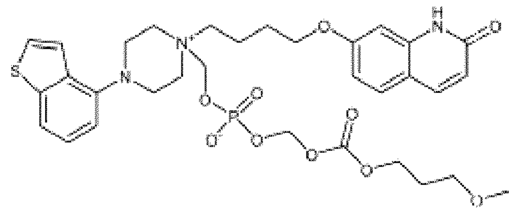 |
| 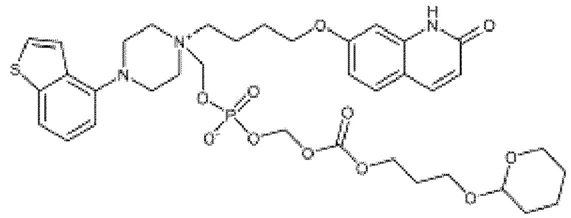 |
| 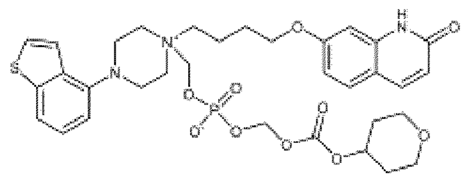 |

| |
|---|
| 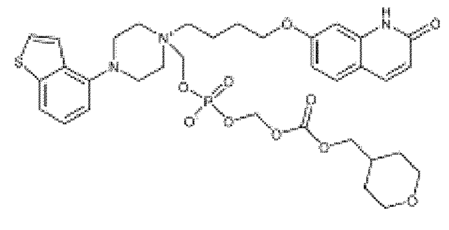 |
| 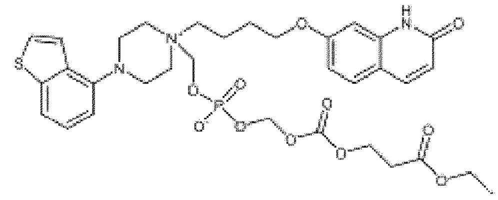 |
| 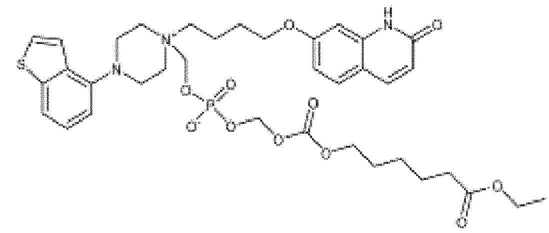 |

| |
|---|
| 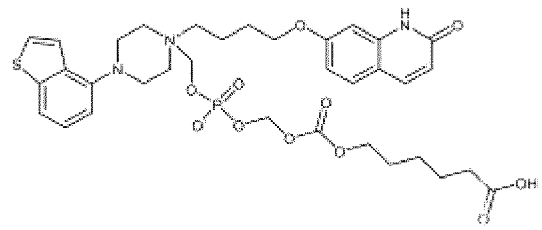 |
| 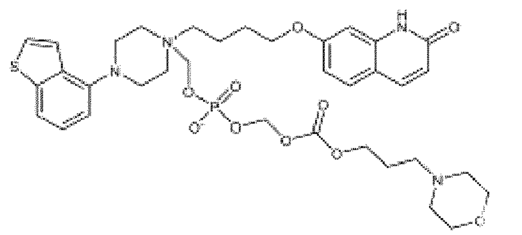 |
| 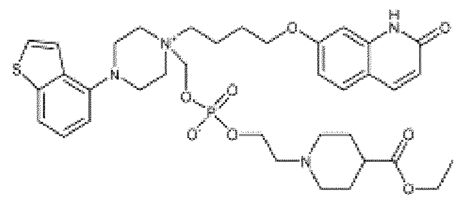 |

| |
|---|
| 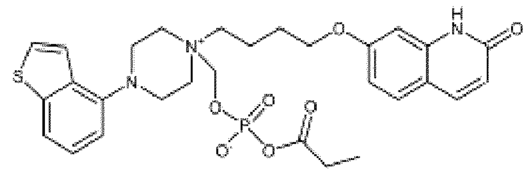 |
| 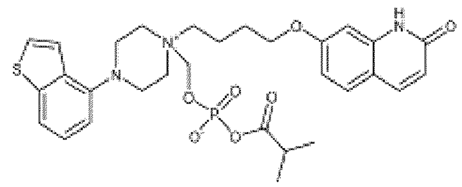 |
| 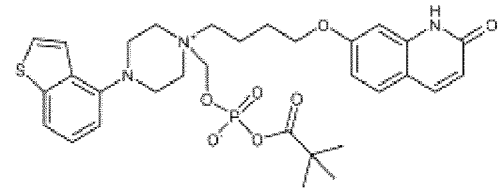 |
| 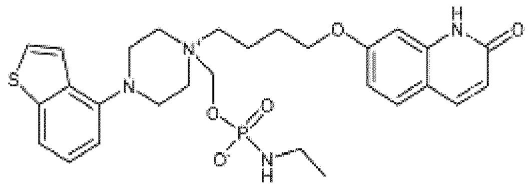 |

| |
|---|
| 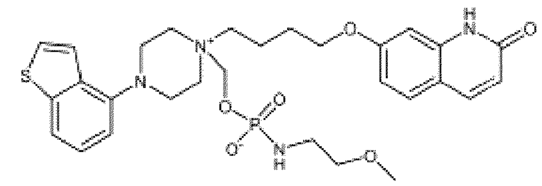 |
| 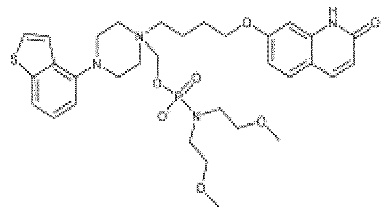 |
| 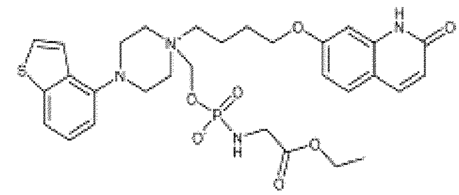 |
| 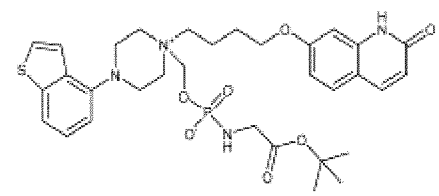 |

| |
|---|
| 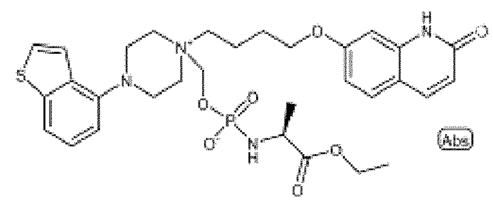 |
| 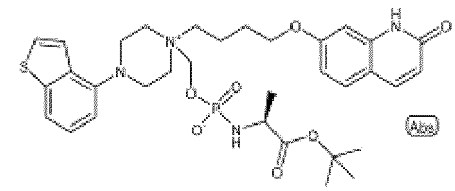 |
| 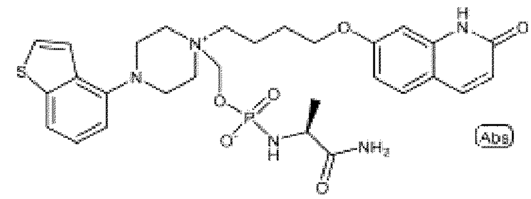 |
| 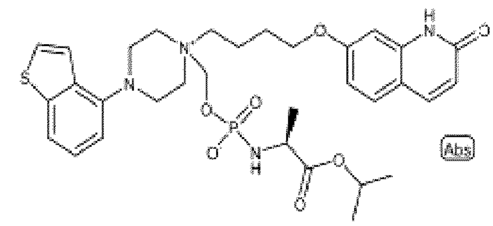 |

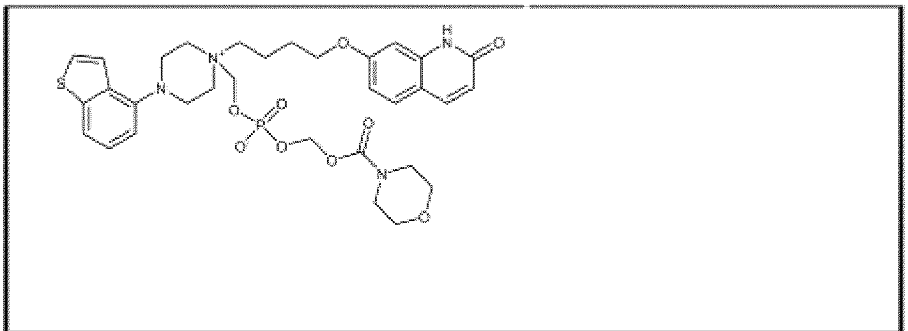

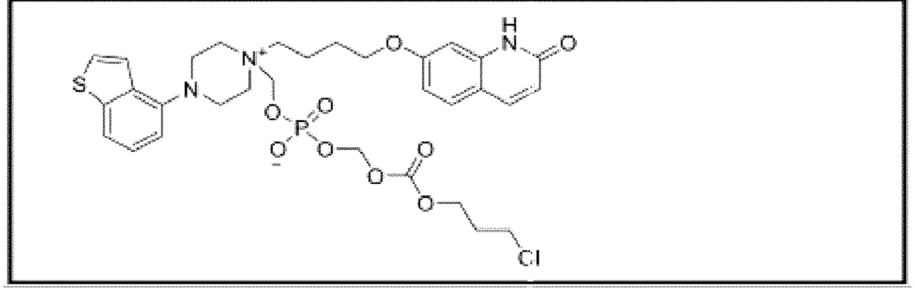

In an embodiment, examples of diseases that can be prevented and/or treated by compound (I) or a salt thereof include the following central nervous system diseases: schizophrenia, treatment-resistant, refractory, or chronic schizophrenia, schizoaffective disorder, psychotic disorder, mood disorder, bipolar disorder, mania, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, Alzheimer's disease, Parkinson's disease, cognitive impairment, cognitive impairment associated with neurodegenerative disease, cognitive impairment due to neurodegenerative disease, cognitive impairment in schizophrenia, cognitive impairment caused by treatment-resistant, refractory, or chronic schizophrenia, vomiting, motion sickness, obesity, migraine, pain, mental retardation, autism disorder, Tourette's disorder, tic disorder, attention deficit hyperactivity disorder, conduct disorder, Down syndrome, impulsive symptoms associated with dementia, and borderline personality disorder .

General Production Method

Compound (I) can be produced, for example, according to the following general production methods. However, the method is not limited to these methods.

The starting material compounds for use may be commercial products, or may be synthesized according to a known method or a method equivalent to a known method.

The solvents, acids, bases, protective groups, and leaving groups used in the production of compound (I) can be any of those that are commonly used in the field of synthetic organic chemistry.

In the production of compound (I), the product in the form of a reaction solution or of a crude product may be used in the subsequent reaction, or the product may be isolated from the reaction mixture according to a common method and easily purified by ordinary separation techniques. Examples of ordinary separation techniques include filtration, extraction, condensation, evaporation, crystallization, recrystallization, reprecipitation, distillation, chromatography, and optical resolution.

In the production of compound (I), reactions such as alkylation, hydrolysis, amination, esterification, amidation, etherification, oxidation, and reduction can be performed according to known methods.

These various commonly used reagents and methods are described, for example, in Organic Functional Group Preparations (2nd ed., Academic Press, Inc., 1989); Comprehensive Organic Transformations (VCH Publishers, Inc., 1989, P. G. M. Wuts); and Greene's Protective Groups in Organic Synthesis (4th ed., 2006, T. W. Greene, John Wiley & Sons, New York, 1991).

In the general production method of compound (I), the starting material compounds, intermediate compounds, and compound (I) may be in their salt form; the target compound obtained in each reaction may also be in a salt form.

If these compounds are free compounds, they can be converted to their desired salt form according to a known method. If the compounds are in a salt form, they can be converted to their free form or other desired salt form according to a known method.

The following describes, in particular, a method for producing compound (II). The following method for producing

compound (II) is a specific example of the production method for compound (I). A person skilled in the art will understand that compound (I) can be produced by performing the same operations in accordance with the specific example. A person skilled in the art will also understand that compound (I) can be produced in accordance with the production methods described in the Examples, methods known in the art, or methods readily conceivable from known methods.

Compound (II) can be produced, for example, according to the production method described below. The production method described below is an example, and is not intended to limit the production method for compound (II). In the following reaction formulas, each starting material compound may form a salt if it does not inhibit reactions. The salt for use can be those listed as examples of salts of compound (II).

For starting material compounds whose specific production method is not described, commercial products may be used, or the starting material compounds may be produced according to a known method or a method equivalent to a known method. The solvents, acids, bases, protective groups, and leaving groups used in the production of compound (II) can be any of those that are commonly used in the field of synthetic organic chemistry.

In the production of compound (II), the product in the form of a reaction solution or of a crude product may be used in the subsequent reaction, or the product may be isolated from the reaction mixture according to a common method and easily purified by ordinary separation techniques. Examples of ordinary separation techniques include filtration, extraction, condensation, evaporation, crystallization, recrystallization, reprecipitation, distillation, chromatography, and optical resolution.

More specifically, compound (II) can be synthesized, for example, according to a method classified into the following step A and step B.

Step A: Phosphoric Acid Coupling Reaction

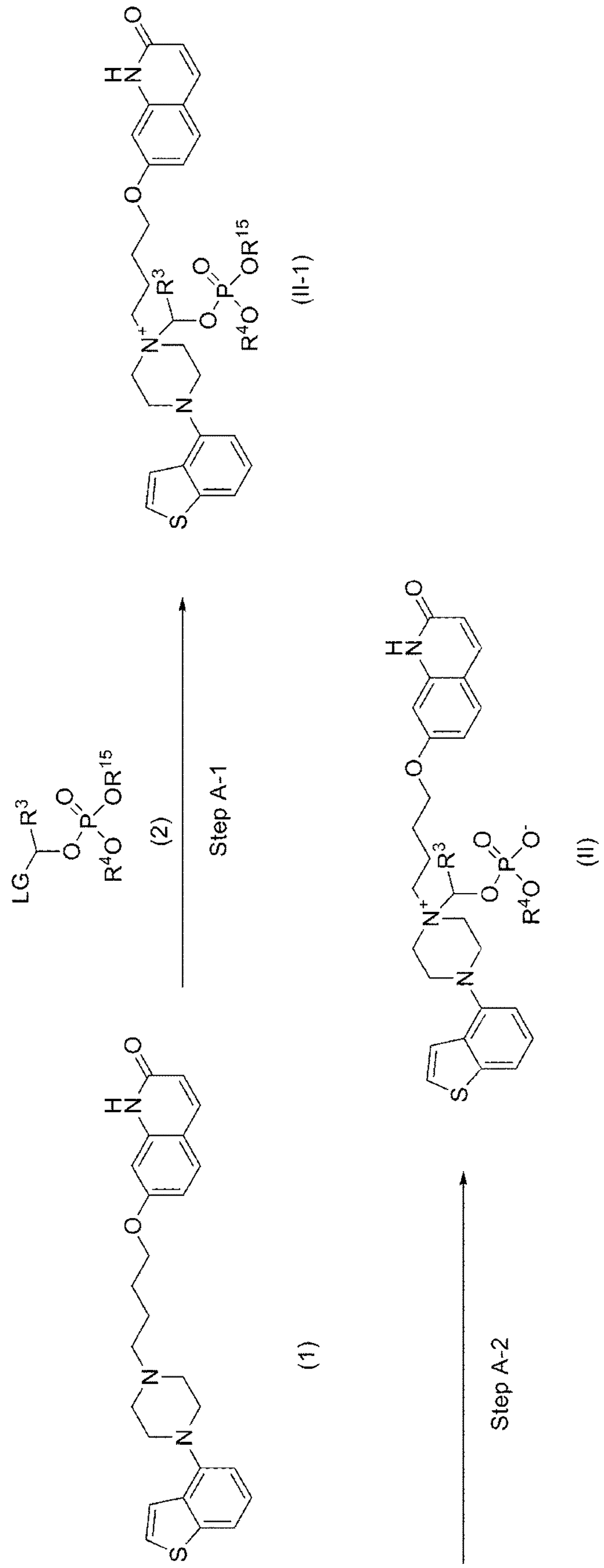


In the formula above, LG represents a leaving group, and $R^{15}$ represents alkyl optionally having 1 to 3 substituents wherein alkyl may have some carbon atoms replaced with -O- and/or -(CO)O-. Other alphabetical symbols

are as defined above. For alkyl represented by $R^{15}$, the explanation for $R^4$ above applies directly. $R^4$ and $R^{15}$ may be identical or different, and are preferably different. $R^{15}$ is more preferably optionally substituted, linear or branched alkyl.

Compound (II-1) can be obtained by reacting compound (1) with compound (2) in an inert solvent, in the presence or absence of a base depending on reaction conditions (Step A-1). Compound (1) is brexpiprazole.

Compound (1) can be produced according to the method described in PTL 1 (JP2006-316052A), and can be used in a free form or a salt form. Examples of compound (1) in a salt form include inorganic acid salts, such as hydrochloride, sulfate, phosphate, hydrobromide, hydroiodide, and nitrate; organic acid salts, such as formate, propionate, oxalate, carbonate, picrate, methanesulfonate, ethanesulfonate, p-toluenesulfonate, acetate, citrate, tartrate, malonate, succinate, maleate, fumarate, malate, and lactate; and amino acid salts, such as aspartate, and glutamate.

Examples of leaving groups include halogen atoms (e.g., chlorine, bromine, and iodine), alkylsulfonyloxy groups (e.g., methylsulfonyloxy, ethylsulfonyloxy, and trifluoromethylsulfonyloxy), and arylsulfonyloxy groups (e.g., benzenesulfonyloxy, p-toluenesulfonyloxy, 2,4,6-trimethylbenzenesulfonyloxy, 2-nitrobenzenesulfonyloxy, and 4-nitrobenzenesulfonyloxy).

Examples of inert solvents include water; alcohol solvents, such as MeOH, EtOH, isopropanol, n-butanol, trifluoroethanol, and ethylene glycol; ketone solvents, such as acetone, and methyl ethyl ketone; ether solvents, such as THF, dioxane, $Et_2O$, diisopropyl ether, cyclopentyl methyl ether, and diglyme; ester solvents, such as AcOMe, and AcOEt; aprotic polar solvents, such as MeCN, DMF, and DMSO; hydrocarbon solvents, such as n-pentane, n-hexane, n-heptane, and cyclohexane; halogenated hydrocarbon solvents, such as chloroform, DCE, and DCM; other organic solvents; and mixed solvents of these, with halogenated hydrocarbon solvents (e.g., DCM) or aprotic polar solvents (e.g., MeCN) being preferred.

The base for use can be selected, for example, from a wide range of known inorganic bases and organic bases. Examples of inorganic bases include alkali metals (e.g., sodium and potassium), alkali metal hydrogen carbonates (e.g., lithium hydrogen carbonate, sodium hydrogen carbonate, and potassium hydrogen carbonate), alkali metal hydroxides (e.g., LiOH, NaOH, and KOH), alkali metal carbonates (e.g., $Li_2CO_3$, $Na_2CO_3$, $K_2CO_3$, and $Cs_2CO_3$), alkali metal lower alkoxides (e.g., sodium methoxide, and sodium ethoxide), and alkali metal hydrides (e.g., NaH, and KH). Examples of organic bases include trialkyl amines (e.g., trimethylamine, TEA, and DIPEA), pyridine, quinoline, piperidine, imidazole, picoline, dimethylaminopyridine, dimethylaniline, N-methylmorpholine, DBN, DABCO, and DBU. If these bases are liquid, they can also be used as a solvent. These bases may be used alone or in a combination of two or more. The amount of the base for use is typically 0.1 to 10 mol, and preferably 0.1 to 5 mol, per mole of compound (1).

Reaction conditions are not particularly limited, and the reaction can usually proceed under cooling, room-temperature, or heating conditions. Preferably, the reaction is performed at a temperature ranging from room temperature to 100°C for 30 minutes to 350 hours, more preferably 1 hour to 200 hours, and particularly preferably 1 hour to 48 hours. Compound (II) can be obtained by reacting compound (II-1) in an inert solvent, under acidic or basic conditions, or with an alkali metal halide, although reaction conditions are not limited (Step A-2).

Examples of inert solvents include water; alcohol solvents, such as MeOH, EtOH, isopropanol, n-butanol, trifluoroethanol, and ethylene glycol; ketone solvents, such as acetone, and methyl ethyl ketone; ether solvents, such as THF, dioxane, $Et_2O$, diisopropyl ether, cyclopentyl methyl ether, and diglyme; ester solvents, such as AcOMe, and AcOEt; aprotic polar solvents, such as MeCN, DMF, and DMSO; hydrocarbon solvents, such as n-pentane, n-hexane, n-heptane, and cyclohexane; halogenated hydrocarbon solvents, such as chloroform, DCE, and DCM; other organic solvents; and mixed solvents of these, with halogenated hydrocarbon solvents (e.g., DCM), aprotic polar solvents (e.g., MeCN), or ketone solvents (e.g., acetone) being preferred.

The base for use can be selected, for example, from a wide range of known inorganic bases and organic bases. Examples of inorganic bases include alkali metals (e.g., sodium and potassium), alkali metal hydrogen carbonates (e.g., lithium hydrogen carbonate, sodium hydrogen carbonate, and potassium hydrogen carbonate), alkali metal hydroxides (e.g., LiOH, NaOH, and KOH), alkali metal carbonates (e.g., $Li_2CO_3$, $Na_2CO_3$, $K_2CO_3$, and $Cs_2CO_3$), alkali metal lower alkoxides (e.g., sodium methoxide, and sodium ethoxide), and alkali metal hydrides (e.g., NaH, and KH). Examples of organic bases include trialkyl amines (e.g., trimethylamine, TEA, and DIPEA), pyridine, quinoline, piperidine, imidazole, picoline, dimethylaminopyridine, dimethylaniline, N-methylmorpholine, DBN, DABCO, and DBU. If these bases are liquid, they can also be used as a solvent. These bases may be used alone or in a combination of two or more. The amount of the base for use is typically 0.1 to 10 mol, and preferably 0.1 to 5 mol, per mole of compound (II-1).

Examples of acids include inorganic acids, such as hydrochloric acid, sulfuric acid, nitric acid, hydrobromic acid, and phosphoric acid; and organic acids, such as acetic acid, trifluoroacetic acid, oxalic acid, phthalic acid, fumaric acid, tartaric acid, maleic acid, citric acid, succinic acid, methanesulfonic acid, p-toluenesulfonic acid, and 10-camphorsulfonic acid. These acids may be used in a combination of two or more in appropriate proportions. The amount of the acid for use typically ranges from 1 molar equivalent to an excess amount relative to compound (II-1). Examples of alkali metal halides include LiI, NaI, and KI. The amount of the alkali metal halide for use typically ranges from 1 molar equivalent to an excess amount relative to compound (II-1).

Reaction conditions are not limited, and the reaction can usually proceed under cooling, room-temperature, or

heating conditions. Preferably, the reaction is performed at a temperature ranging from room temperature to 100°C for 0.5 to 350 hours.

Step B: Protection-Deprotection

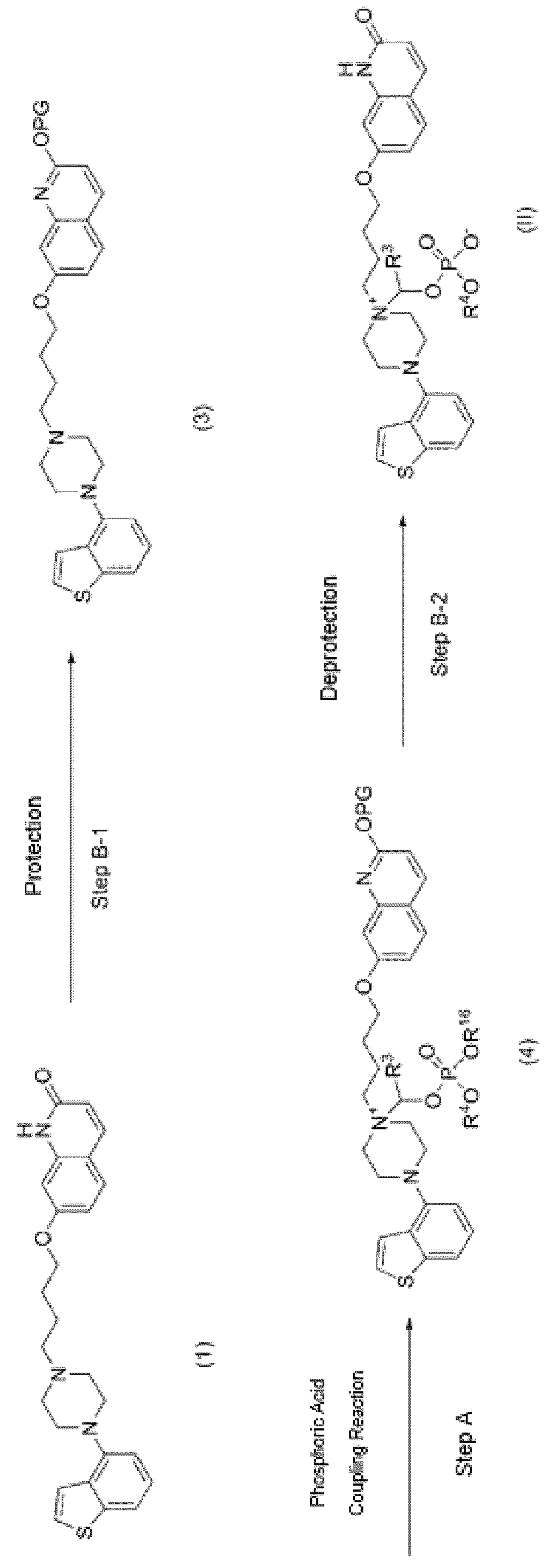
Protection
Step B-1
Phosphoric Acid
Coupling Reaction
Step A
Deprotection
Step B-2

(1) (3) (4) (II)

(In the formula above, PG represents a protective group for a hydroxy group, $-OR^{16}$ represents an alkoxy group or $-O^-$, and other symbols are as defined above.)

Compound (1) may be protected with an appropriate protective group to form compound (3) (Step B-1), and subjected to the phosphoric acid coupling reaction (step A), followed by releasing the protective group (Step B-2), thereby synthesizing compound (II), which is a final product (step B). In performing step A, the operation may be ended after step A-1 (i.e., reacting compound (3) with compound (2) to obtain a compound corresponding to compound (II-1) (i.e., compound (4) in which $-OR^{16}$ is an alkoxy group)), or subsequently step A-2 may be performed to obtain a compound corresponding to compound (II) (i.e., compound (4) in which $-OR^{16}$ is $-O^-$).

Step B-1: Protection Reaction

The protective group (-OPG) for a hydroxy group can be any of those used in the field of synthetic organic chemistry. Examples include ethers (e.g., methyl, methoxymethyl, benzyloxymethyl, methoxyethoxymethyl, 2-(trimethylsilyl)ethoxymethyl, methylthiomethyl, tetrahydropyranyl, phenacyl, cyclopropylmethyl, allyl, prenyl, propargyl, t-butyl, benzyl, 4-(dimethylamino)carbonylbenzyl, 4-methylsulfinylbenzyl, 9-anthrylmethyl, and 4-picolyl); silyl ethers (e.g., trimethylsilyl, t-butyldimethylsilyl, t-butyldiphenylsilyl, triisopropylsilyl); esters (e.g., formate, acetate, levulinate, pivaloate, benzoate, and 9-fluorenecarboxylate); carbonates (e.g., methyl, t-butyl,, isopropyl, allyl, 4-methylsulfinylbenzyl, 2,2,2-trichloroethyl, vinyl, and benzyl); aryl carbamates (e.g., phenylcarbamate); phosphinates (e.g., dimethylphosphinyl, and dimethylphosphinothioyl); and sulfonates (e.g., methanesulfonate, trifluoromethanesulfonate, toluenesulfonate, and benzylsulfonate). An example of preferable protective groups is an ether.

For example, the protection reaction can be performed with compound (1) in an inert solvent in the presence of a base to obtain compound (3).

The base for use can be, for example, a known inorganic base. Examples of inorganic bases include alkali metals (e.g., sodium and potassium), alkali metal hydrogen carbonates (e.g., lithium hydrogen carbonate, sodium hydrogen carbonate, and potassium hydrogen carbonate), alkali metal hydroxides (e.g., LiOH, NaOH, and KOH), alkali metal carbonates (e.g., $Li_2CO_3$, $Na_2CO_3$, $K_2CO_3$, and $Cs_2CO_3$), alkali metal lower alkoxides (e.g., sodium methoxide, and sodium ethoxide), alkali metal hydrides (e.g., NaH, and KH), and silver carbonate. The amount of the base for use typically ranges from 1 molar equivalent to an excess amount relative to compound (1).

Examples of inert solvents include water; alcohol solvents, such as MeOH, EtOH, isopropanol, n-butanol, trifluoroethanol, and ethylene glycol; ketone solvents, such as acetone, and methyl ethyl ketone; ether solvents, such as THF, dioxane, $Et_2O$, diisopropyl ether, cyclopentyl methyl ether, and diglyme; ester solvents, such as AcOMe, and AcOEt; aprotic polar solvents, such as MeCN, DMF, and DMSO; hydrocarbon solvents, such as n-pentane, n-hexane, n-heptane, and cyclohexane; halogenated hydrocarbon solvents, such as chloroform, DCE, and DCM; other organic solvents; and mixed solvents of these. These inert solvents may be used in a combination of two or more in appropriate proportions. Preferably, the inert solvent is an ether solvent, such as cyclopentyl methyl ether.

The reaction temperature is typically -80 to 150°C.

The reaction time is typically 0.1 to 200 hours.

Step B-2: Deprotection Reaction

The deprotection reaction of compound (4) may be a known reaction according to the type of the protective group. For example, the deprotection reaction can be performed by deprotecting compound (4) in an inert solvent or without a solvent in the presence or absence of an acid to obtain compound (II).

Examples of acids include inorganic acids, such as hydrochloric acid, sulfuric acid, nitric acid, hydrobromic acid, and phosphoric acid; and organic acids, such as acetic acid, trifluoroacetic acid, oxalic acid, phthalic acid, fumaric acid, tartaric acid, maleic acid, citric acid, succinic acid, methanesulfonic acid, p-toluenesulfonic acid, and 10-camphorsulfonic acid. These acids may be used in a combination of two or more in appropriate proportions. The amount of the acid for use typically ranges from 1 molar equivalent to an excess amount relative to intermediate (4).

Examples of inert solvents include water; alcohol solvents, such as MeOH, EtOH, isopropanol, n-butanol, trifluoroethanol, and ethylene glycol; ketone solvents, such as acetone, and methyl ethyl ketone; ether solvents, such as THF, dioxane, $Et_2O$, diisopropyl ether, cyclopentyl methyl ether, and diglyme; ester solvents, such as AcOMe, and AcOEt; aprotic polar solvents, such as MeCN, DMF, and DMSO; hydrocarbon solvents, such as n-pentane, n-hexane, n-heptane, and cyclohexane; halogenated hydrocarbon solvents, such as chloroform, DCE, and DCM; other organic solvents; and mixed solvents of these. These inert solvents may be used in a combination of two or more in appropriate proportions.

The reaction temperature is typically -80 to 150°C.

The reaction time is typically 0.1 to 200 hours.

Furthermore, various compounds (II) can also be synthesized by performing substitution reaction or condensation reaction by using a compound in which $R^4$ is a hydrogen atom in compound (II) (represented as compound (5) in the

formula below). The following describes an example.

Step C-1: Substitution Reaction

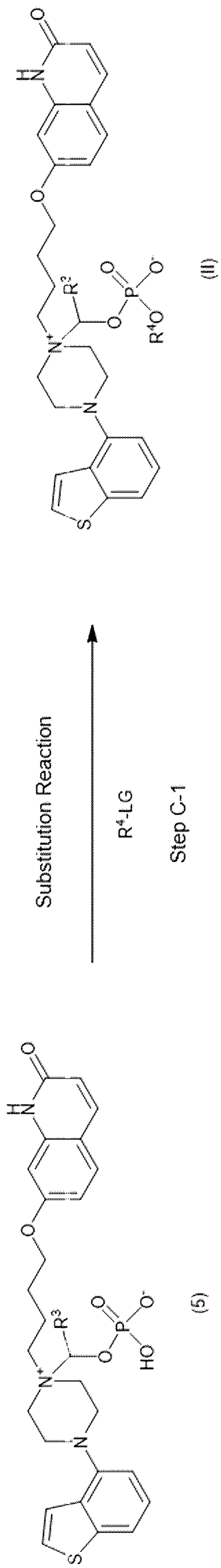


(5)

(II)

In the formula above, LG represents a leaving group, and $R^4$ is as defined above (excluding, however, a hydrogen atom).

Compound (5) may be reacted with $R^4$-LG in an inert solvent or without a solvent in the presence or absence of a base according to reaction conditions to obtain compound (II) (step C-1: substitution reaction).

Examples of leaving groups include halogen atoms (e.g., chlorine, bromine, and iodine), alkylsulfonyloxy groups (e.g., methylsulfonyloxy, ethylsulfonyloxy, and trifluoromethylsulfonyloxy), arylsulfonyloxy groups (e.g., benzenesulfonyloxy, p-toluenesulfonyloxy, 2,4,6-trimethylbenzenesulfonyloxy, 2-nitrobenzenesulfonyloxy, and 4-nitrobenzenesulfonyloxy).

Examples of inert solvents include water; alcohol solvents, such as MeOH, EtOH, isopropanol, n-butanol, trifluoroethanol, and ethylene glycol; ketone solvents, such as acetone, and methyl ethyl ketone; ether solvents, such as THF, dioxane, $Et_2O$, diisopropyl ether, cyclopentyl methyl ether, and diglyme; ester solvents, such as AcOMe, and AcOEt; aprotic polar solvents, such as MeCN, DMF, and DMSO; hydrocarbon solvents, such as n-pentane, n-hexane, n-heptane, and cyclohexane; halogenated hydrocarbon solvents, such as chloroform, DCE, and DCM; other organic solvents; and mixed solvents of these, with aprotic polar solvents (e.g., MeCN), ketone solvents (e.g., acetone), or mixed solvents of any of these with water being preferred.

The base for use can be selected, for example, from a wide range of known inorganic bases and organic bases.

Examples of inorganic bases include alkali metals (e.g., sodium and potassium), alkali metal hydrogen carbonates (e.g., lithium hydrogen carbonate, sodium hydrogen carbonate, and potassium hydrogen carbonate), alkali metal hydroxides (e.g., LiOH, NaOH, and KOH), alkali metal carbonates (e.g., $Li_2CO_3$, $Na_2CO_3$, $K_2CO_3$, and $Cs_2CO_3$), alkali metal lower alkoxides (e.g., sodium methoxide, and sodium ethoxide), and alkali metal hydrides (e.g., NaH, and KH). Examples of organic bases include trialkyl amines (e.g., trimethylamine, TEA, and DIPEA), pyridine, quinoline, piperidine, imidazole, picoline, dimethylaminopyridine, dimethylaniline, N-methylmorpholine, DBN, DABCO, and DBU. If these bases are liquid, they can also be used as a solvent. These bases may be used alone or in a combination of two or more.

The amount of the base for use typically ranges from 1 molar equivalent to an excess amount per mole of compound (5) to be reacted.

The reaction conditions are not particularly limited. The reaction temperature is typically -80°C to 150°C. The reaction time is typically 0.1 hours to 200 hours.

Step C-2, C-2': Condensation Reaction

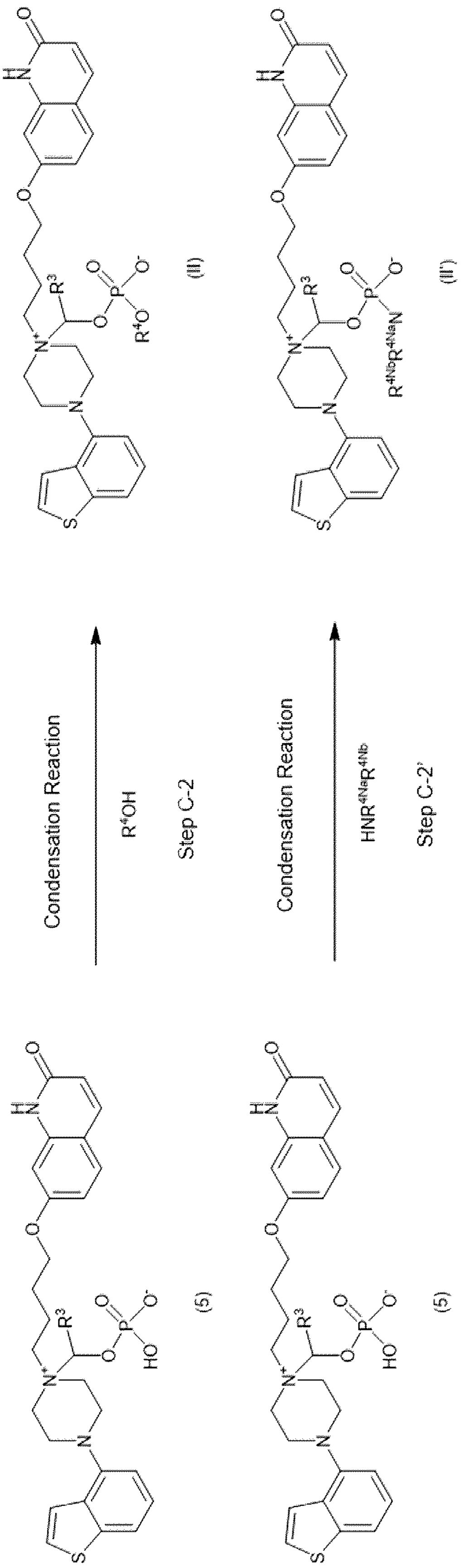


In the formula above, $R^4$ is as defined above (excluding, however, a hydrogen atom).

For example, compound (5) may be reacted with an alcohol ($R^4OH$) in an inert solvent or without a solvent in the

presence or absence of a base according to reaction conditions, in the presence of a condensation agent, to obtain compound (II) (step C-2: condensation reaction).

Using an amine ($HNR^{4Na}R^{4Nb}$) instead of the alcohol also yields compound (II') in which $R^1$ is $-O^-$, and $R^2$ is $NR^{4Na}R^{4Nb}$ (step C-2': condensation reaction).

Examples of inert solvents include water; alcohol solvents, such as MeOH, EtOH, isopropanol, n-butanol, trifluoroethanol, and ethylene glycol; ketone solvents, such as acetone, and methyl ethyl ketone; ether solvents, such as THF, dioxane, $Et_2O$, diisopropyl ether, cyclopentyl methyl ether, and diglyme; ester solvents, such as AcOMe, and AcOEt; aprotic polar solvents, such as MeCN, DMF, and DMSO; hydrocarbon solvents, such as n-pentane, n-hexane, n-heptane, and cyclohexane; halogenated hydrocarbon solvents, such as chloroform, DCE, and DCM; other organic solvents; and mixed solvents of these, with halogenated hydrocarbon solvents, (e.g., DCM), aprotic polar solvents (e.g., MeCN, and DMF), ketone solvents (e.g., acetone), or mixed solvents of any of these with water being preferred.

The base for use can be selected, for example, from a wide range of known inorganic bases and organic bases. Examples of inorganic bases include alkali metals (e.g., sodium and potassium), alkali metal hydrogen carbonates (e.g., lithium hydrogen carbonate, sodium hydrogen carbonate, and potassium hydrogen carbonate), alkali metal hydroxides (e.g., LiOH, NaOH, and KOH), alkali metal carbonates (e.g., $Li_2CO_3$, $Na_2CO_3$, $K_2CO_3$, and $Cs_2CO_3$), alkali metal lower alkoxides (e.g., sodium methoxide, and sodium ethoxide), and alkali metal hydrides (e.g., NaH, and KH). Examples of organic bases include trialkyl amines (e.g., trimethylamine, TEA, and DIPEA), pyridine, quinoline, piperidine, imidazole, picoline, dimethylaminopyridine, dimethylaniline, N-methylmorpholine, DBN, DABCO, and DBU. If these bases are liquid, they can also be used as a solvent. These bases may be used alone or in a combination of two or more.

The amount of the base for use is typically ranges from 1 molar equivalent to an excess amount per mole of compound (5) to be reacted.

The condensation agent for use can be selected, for example, from a wide range of known condensation agents. Examples include 3-ethyl-1-(3-dimethylaminopropyl)carbodiimide (WSC) or a HCl salt thereof; 1-[bis(dimethylamino) methylene]-1H-1,2,3-triazolo[4,5-b]pyridinium 3-oxide hexafluorophosphate (HATU); 1-(chloro-1-pyrrolidinylmethylene) pyrrolidinium hexafluorophosphate; N-cyclohexyl-N'-morpholinoethyl carbodiimide; N-cyclohexyl-N'-(4-diethylaminocyclohexyl) carbodiimide; N,N'-diethyl carbodiimide; N,N'-diisopropyl carbodiimide; N,N'-carbonylbis(2-methylimidazole); pentamethylene ketene-N-cyclohexylimine; diphenylketene-N-cyclohexylimine; ethoxyacetylene; 1-alkoxy-1-chloroethylene; trialkyl phosphite; ethyl polyphosphate; isopropyl polyphosphate; phosphorus oxychloride (phosphoryl chloride); phosphorus trichloride; diphenylphosphoryl azide; thionyl chloride; oxalyl chloride; alkyl haloformates, such as ethyl chloroformate, and isopropyl chloroformate; triphenylphosphine; 2-ethyl-7-hydroxybenzisoxazolium salts; 2-ethyl-5-(m-sulfophenyl) isoxazolium hydroxide inner salts; benzotriazol-1-yloxy-tris(dimethylamino)phosphonium hexafluorophosphate; 1-(p-chlorobenzenesulfonyloxy)-6-chloro-1H-benzotriazole; and Vilsmeier reagents prepared by reacting DMF with thionyl chloride, phosgene, trichloromethyl chloroformate, phosphorus oxychloride, etc.

The reaction conditions are not particularly limited. The reaction temperature is typically -80°C to 150°C. The reaction time is typically 0.1 hours to 200 hours.

Furthermore, various compounds (II) can also be synthesized by performing acylation reaction by using compound (5). The acylation reaction is an embodiment of C-1 (substitution reaction). The following describes an example.

Step C-3: Acylation Reaction

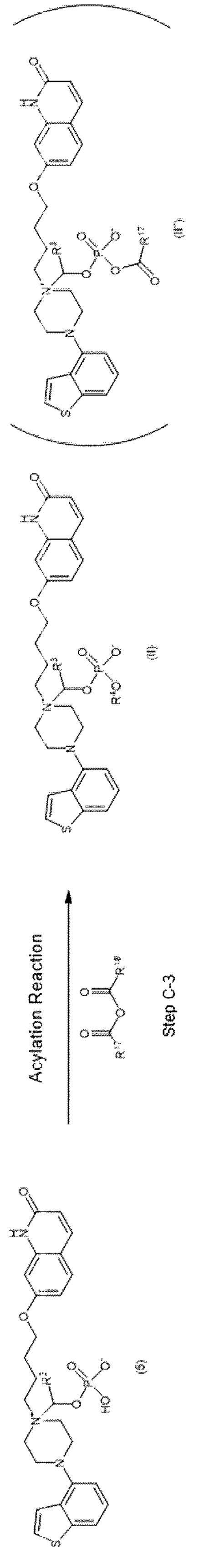


In the formula above, $R^{17}$ and $R^{18}$ are preferably, for example, alkyl optionally having 1 to 3 substituents (e.g., $C_{1-18}$ alkyl). $R^{17}$ and $R^{18}$ may be identical or different.

For example, compound (5) may be reacted with an acylating agent such as carboxylic anhydride in an inert

solvent or without a solvent in the presence of a base according to reaction conditions to obtain compound (II) (Step C-3: acylation reaction).

Examples of inert solvents include water; alcohol solvents, such as MeOH, EtOH, isopropanol, n-butanol, trifluoroethanol, and ethylene glycol; ketone solvents, such as acetone, and methyl ethyl ketone; ether solvents, such as THF, dioxane, $Et_2O$, diisopropyl ether, cyclopentyl methyl ether, and diglyme; ester solvents, such as AcOMe, and AcOEt; aprotic polar solvents, such as MeCN, DMF, and DMSO; hydrocarbon solvents, such as n-pentane, n-hexane, n-heptane, and cyclohexane; halogenated hydrocarbon solvents, such as chloroform, DCE, and DCM; other organic solvents; and mixed solvents of these, with halogenated hydrocarbon solvents (e.g., DCM), aprotic polar solvents (e.g., MeCN and DMF), or ketone solvents (e.g., acetone) being preferred.

The base for use can be selected, for example, from a wide range of known inorganic bases and organic bases. Examples of inorganic bases include alkali metals (e.g., sodium and potassium), alkali metal hydrogen carbonates (e.g., lithium hydrogen carbonate, sodium hydrogen carbonate, and potassium hydrogen carbonate), alkali metal hydroxides (e.g., LiOH, NaOH, and KOH), alkali metal carbonates (e.g., $Li_2CO_3$, $Na_2CO_3$, $K_2CO_3$, and $Cs_2CO_3$), alkali metal lower alkoxides (e.g., sodium methoxide, and sodium ethoxide), and alkali metal hydrides (e.g., NaH, and KH). Examples of organic bases include trialkyl amines (e.g., trimethylamine, TEA, and DIPEA), pyridine, quinoline, piperidine, imidazole, picoline, dimethylaminopyridine, dimethylaniline, N-methylmorpholine, DBN, DABCO, and DBU. If these bases are liquid, they can also be used as a solvent. These bases may be used alone or in a combination of two or more.

The amount of the base for use typically ranges from 1 molar equivalent to an excess amount per mole of compound (5) to be reacted.

The reaction conditions are not particularly limited. The reaction temperature is typically -80°C to 150°C. The reaction time is typically 0.1 hours to 200 hours.

In the present specification, compound (I), starting material compounds, and intermediate compounds may be used in the form of a chemically acceptable geometric isomer, stereoisomer, optical isomer, or tautomer. These isomers can be isolated according to a common optical resolution method or produced from corresponding optically active starting material compounds.

In the present specification, compound (I), starting material compounds, and intermediate compounds may be in the form of a salt. The target compound obtained in each reaction may also form a salt. If the compound obtained in each reaction is a free compound, the free compound can be converted into a target salt according to a known method. If the compound is a salt, the compound can be converted into its free form or another target salt according to a known method. Examples of such a salt are described below.

The salt is preferably a pharmaceutically acceptable salt; for example, metal salts (e.g., alkali metal salts, alkaline earth metal salts, and zinc salts) are preferred. Acid addition salts and base addition salts are also preferred. Examples of acids for acid addition salts include inorganic acids, such as hydrochloric acid, hydrobromic acid, hydroiodic acid, nitric acid, sulfuric acid, and phosphoric acid; organic acids, such as formic acid, propionic acid, oxalic acid, carbonic acid, picric acid, methanesulfonic acid, ethanesulfonic acid, p-toluenesulfonic acid, acetic acid, citric acid, tartaric acid, malonic acid, succinic acid, maleic acid, fumaric acid, malic acid, and lactic acid; and amino acids, such as aspartic acid, and glutamic acid. Examples of bases for base addition salts include metals, such as alkali metals (e.g., sodium, and potassium), and alkaline earth metals (e.g., calcium, and magnesium); inorganic bases, such as alkali metal carbonates (e.g., lithium carbonate, potassium carbonate, sodium carbonate, and cesium carbonate), alkali metal hydrogen carbonates (e.g., lithium hydrogen carbonate, sodium hydrogen carbonate, and potassium hydrogen carbonate), and alkali metal hydroxides (e.g., lithium hydroxide, sodium hydroxide, potassium hydroxide, calcium hydroxide, and cesium hydroxide); organic bases, such as methylamine, diethylamine, trimethylamine, triethylamine, N-ethyldiisopropylamine, ethanolamine, diethanolamine, triethanolamine, ethylenediamine, tris(hydroxymethy)methyl amine, dicyclohexylamine, N,N'-dibenzylethylenediamine, guanidine, pyridine, quinoline, piperidine, imidazole, dimethylaminopyridine, dimethylaniline, picoline, choline, N-methylmorpholine, DBN, DBU, and DABCO; amino acids, such as lysine, and arginine; and ammonia.

Compound (I) or a salt thereof may also be used in a form of various solvates (in particular, hydrate). Compound (I) or a salt thereof may also be used in a crystalline polymorphic form. Compound (I) or a salt thereof may also be used in a form of a pharmaceutically acceptable cocrystal or cocrystal salt. A cocrystal or cocrystal salt means a crystalline substance composed of two or more unique substances that are solids at room temperature, each with different physical properties (e.g., structure, melting point, and heat of melting). Cocrystals and cocrystal salts can be produced according to a known co-crystallization method.

Compound (I) includes compounds in which any one or multiple atoms of formula (I) are replaced with one or multiple isotope atoms. Examples of isotope atoms include deuterium ($^2H$), tritium ($^3H$), $^{13}C$, $^{15}N$, and $^{18}O$. Compound (I) also includes compounds labeled with various radioactive or non-radioactive isotopes.

Compound (I) may be used in combination with various therapeutic or prophylactic agents for diseases that can be treated by compound (I). Compound (I) and a therapeutic or prophylactic agent in combination may be administered simultaneously, or these may be administered consecutively or at desired time intervals. A simultaneous administration formulation of compound (I) and a therapeutic or prophylactic agent may be a combination drug or a combination of

separately formulated preparations.

The following describes a pharmaceutical formulation containing compound (I) as an active ingredient ("pharmaceutical composition" below).

The pharmaceutical formulation is a formulation of compound (I) in a common pharmaceutical formulation form and is prepared by using compound (I) or a salt thereof and a pharmaceutically acceptable carrier. Such carriers include commonly used fillers, bulking agents, binders, humectants, disintegrants, surface active agents, lubricants, suspending agents, dissolution aids, isotonic agents, diluents such as solvents, and excipients.

Such a pharmaceutical formulation can be a form selected from a variety of forms depending on the therapeutic purpose, such as tablets, pills, powders, liquids, suspensions, emulsions, granules, capsules, suppositories, injections, nasal sprays, and inhalants, with injections being particularly preferred. The injections include an injection for intramuscular administration and an injection for subcutaneous administration, with an injection for subcutaneous administration being particularly preferred.

Carriers for use in forming tablets can be selected from a wide range of known carriers. Examples include excipients, such as lactose, sucrose, sodium chloride, glucose, urea, starch, calcium carbonate, kaolin, and crystalline cellulose; binders, such as water, ethanol, propanol, simple syrup, dextrose solutions, starch solutions, gelatin solutions, carboxymethyl cellulose, shellac, methylcellulose, potassium phosphate, and polyvinyl pyrrolidone; disintegrants, such as dry starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch, and lactose; disintegration inhibitors, such as sucrose, stearin, cocoa butter, and hydrogenated oil; absorption promoters, such as quaternary ammonium bases, and sodium lauryl sulfate; humectants, such as glycerin, and starch; adsorbents, such as starch, lactose, kaolin, bentonite, and colloidal silicic acid; and lubricants, such as purified talc, stearates, boric acid powder, and polyethylene glycol.

Furthermore, tablets can optionally be made into tablets with common coating, such as sugar-coated tablets, gelatin-coated tablets, enteric-coated tablets, film-coated tablets, or double- or multi-layered tablets.

Carriers for use in forming pills can be selected from a wide range of known carriers. Examples include excipients, such as glucose, lactose, starch, cocoa butter, hydrogenated vegetable oil, kaolin, and talc; binders, such as gum arabic powder, tragacanth powder, gelatin, and ethanol; and disintegrants, such as laminaran, and agar.

Carriers for use in forming suppositories can be selected from a wide range of known carriers. Examples include polyethylene glycol, cocoa butter, higher alcohols, esters of higher alcohols, gelatin, and semisynthetic glycerides.

If the pharmaceutical formulation is prepared in the form of an injection, the liquid, emulsion, or suspension is sterilized and preferably isotonic to blood.

Diluents for use in forming these liquids, emulsions, or suspensions can be selected from widely used known diluents, such as water, ethanol, propylene glycol, ethoxylated isostearyl alcohol, polyoxylated isostearyl alcohol, and polyoxyethylene sorbitan fatty acid esters.

In this case, the pharmaceutical formulation may contain a salt, glucose, amino acids, or glycerin in an amount sufficient to prepare an isotonic solution, as well as a commonly used dissolution aid, buffering agent, pain reliever, etc., and may further contain, if necessary, a preservative, a stabilizer, an antioxidant, a solubilizer, a pH adjuster, and other pharmaceuticals.

Brexpiprazole is known to be useful for alleviating symptoms accompanied with severe agitation, destruction, and violent behavior in Alzheimer's dementia. Since immediate-release injections of antipsychotic agents are known to be useful as a therapeutic agent for alleviating symptoms in acute-phase patients with severe agitation, destruction, and violent behavior in schizophrenia etc., injections that immediately release brexpiprazole into the blood are expected to be useful as a therapeutic agent for alleviating symptoms in acute-phase patients with agitation in schizophrenia and Alzheimer's dementia. Pharmaceutical compositions that comprise compound (1) and that are designed to be in a preferable injectable form according to medical needs are expected to achieve immediate efficacy by subcutaneous or intramuscular administration and by immediately delivering brexpiprazole in a therapeutically effective amount into the patient's bloodstream for a short period of time (e.g., a few hours) for acute-phase use in agitation etc. Examples of a preferable injectable form of such injections for acute-phase use include an injection obtained by dissolving compound (1) in water for injection, a biocompatible organic solvent, or a mixture of water for injection and a biocompatible organic solvent to which an appropriate isotonic agent, buffering agent, pH adjuster, solubilizer, and the like are added as necessary.

Examples of the solubilizers include polysorbate 80, polysorbate 60, polyoxyethylene hardened castor oil 60, Poloxamer, β-cyclodextrin, and sulfobutyl ether-β-cyclodextrin.

Examples of the isotonic agents includes alkali metal chlorides, such as sodium chloride and potassium chloride; sugar alcohols, such as mannitol, sorbitol, xylitol, and maltitol; sugars, such as glucose, trehalose, and maltose; and glycerin. The composition may not comprise an isotonic agent. If an isotonic agent is contained, the concentration of the isotonic agent in the composition is preferably such that the osmotic pressure of a solution of the composition is isotonic. The concentration depends on the type of isotonic agent. For example, for the case of alkali metal chlorides, the

concentration is preferably 0.5 to 20 mg/mL, and more preferably 2 to 10 mg/mL; for the case of sugar alcohols and sugars, the concentration is preferably 10 mg/mL to 200 mg/mL, and more preferably 20 to 100 mg/mL.

Examples of the buffering agent include phosphates, such as sodium phosphate, sodium dihydrogen phosphate, monosodium hydrogen phosphate, disodium hydrogen phosphate, potassium phosphate, potassium dihydrogen phosphate, and potassium dihydrogen phosphate; borates, such as sodium borate and potassium borate; citrates, such as sodium citrate and disodium citrate; acetates, such as sodium acetate and potassium acetate; carbonates, such as sodium carbonate and sodium hydrogen carbonate, and tris-hydroxymethylaminomethane (Tris). The concentration of the buffering agent in the composition is, for example, 0.01 to 5.0 mg/mL, and preferably 0.1 to 2 mg/mL.

The pH adjuster may be an acidic pH adjuster or a basic pH adjuster. Examples of acidic pH adjusters include hydrochloric acid, phosphoric acid, acetic acid, and citric acid. Examples of basic pH adjusters include sodium hydroxide, potassium hydroxide, calcium carbonate, magnesium oxide, and magnesium hydroxide. The pH adjuster is usually added in an appropriate amount depending on the desired pH of the composition.

Examples of the biocompatible organic solvent include those that are miscible with water. Examples of such solvents include alcohols, such as ethanol, propanol, isopropanol, propylene glycol, and glycerin, and dimethyl sulfoxide, N-methyl-2-pyrrolidone, polyethylene glycol 300, and polyethylene glycol 400.

The agitation associated with schizophrenia, depression, and Alzheimer's disease is often treated by administration of once-daily tablets. However, in cases that involve medication refusal or agitation, taking tablets may be difficult. In such cases, long-acting injections (preferably long-acting injections for subcutaneous administration) that are administered every day to every several days are considered to be useful formulations because drug administration is reliably conducted and they are convenient for use. The pharmaceutical composition that contains compound (1) and that is designed to be in a preferable injectable form according to medical needs is expected to achieve efficacy for one day to several days by subcutaneous or intramuscular administration and by continuously delivering brexpiprazole in a therapeutically effective amount into the patient's bloodstream for a relatively short period of time (one day to several days) even in patients who have difficulty taking tablets.

In the treatment of schizophrenia, long-acting injections are a useful dosage form to improve treatment and preventive effects in patients with poor medication adherence. In general, for drugs to exert therapeutic effects, it is desirable for the drug concentration in the blood to immediately reach the therapeutic range and to then maintain a certain level. Although aripiprazole and paliperidone palmitate formulations (trade names: ABILIFY prolonged-release aqueous suspension for IM injection and XEPLION Aqueous Suspension for IM Injection) are clinically used as typical long-acting injections for schizophrenia as the indication, these drugs are poorly soluble and have slow dissolution after administration; thus, a combined use of oral agents or shortening of injection intervals is required to compensate for the blood drug concentration at the start of treatment. The pharmaceutical composition that contains compound (1) and that is designed to be in a preferable injectable form according to medical needs is expected to improve treatment and preventive effects in patients with poor medication adherence by subcutaneous or intramuscular administration and by continuously delivering brexpiprazole in a therapeutically effective amount into the patient's bloodstream immediately and for a relatively short period of time (one to four weeks or more), even in patients in which sufficient treatment or preventive effects are not expected due to poor medication adherence.

Example of a preferable injectable form of a long-acting injection that continuously delivers brexpiprazole for one day to several days or one to four weeks or more include a pharmaceutical composition in the form of a suspension containing compound (1), a suspending agent, and a dispersion medium.

The concentration of compound (1) in the composition is not particularly limited as long as it is an effective concentration according to the use of the composition described above. The concentration of compound (1) in the composition is, for example, 50 mg/mL or more, preferably 100 mg/mL or more, more preferably 150 mg/mL or more, and even more preferably 200 mg/mL or 250 mg/mL or more, in terms of brexpiprazole. The upper limit is not particularly limited, and is, for example, 1000 mg/mL or less, 750 mg/mL or less, or 500 mg/mL or less. For example, the concentration for use can be in the range of 50 to 1000 mg/mL.

The suspending agent contained in the composition is not particularly limited as long as it is pharmaceutically acceptable and can achieve a predetermined viscosity. Examples of the suspending agent include carboxymethyl cellulose and salts thereof; polyoxyethylene-polyoxypropylene block copolymers, such as Poloxamer; and polyethylene glycol (which is also referred to as "Macrogol").

Examples of salts of carboxymethyl cellulose include metal salts, such as alkali metal salts, and ammonium salts. Specific examples include sodium carboxymethyl cellulose, potassium carboxymethyl cellulose, lithium carboxymethyl cellulose, ammonium carboxymethyl cellulose, and mixtures thereof. In one embodiment, the suspending agent preferably contains carboxymethyl cellulose and/or a sodium salt thereof, and particularly preferably contains sodium carboxymethyl cellulose. The suspending agents may be used alone or in a combination of two or more. The concentration of the suspending agent in the composition is, for example, 0.1 mg/mL or more, preferably 0.2 mg/mL or more, and more preferably 0.5 mg/mL or more. The concentration of the suspending agent in the composition is, for example, 100 mg/mL or less, preferably 50 mg/mL or less, and more preferably 25 mg/mL or less. The concentration of the suspending agent in the

composition is, for example, 0.1 to 50 mg/mL.

The dispersion medium contained in the composition is not particularly limited as long as it is pharmaceutically acceptable and is capable of dispersing the active ingredient. The dispersion medium may be used alone or in a combination of two or more different types. The dispersion medium preferably contains at least water. Examples of the dispersion medium include water, physiological saline, and a solvent containing water and the biocompatible organic solvent mentioned above. In a preferable embodiment, the dispersion medium is water, and it is particularly preferable to use purified water, sterilized purified water, water for injection, or the like.

The composition may also further comprise any additives. The additives may be any pharmaceutically acceptable additives, and may be, for example, the isotonic agents, buffering agents, and pH adjusters mentioned above. The additives may be used alone or in a combination of two or more.

The average particle diameter of the suspended compound (1) is, for example, 0.1 to 30 μm, and preferably 0.5 to 20 μm. These ranges are preferable to maintain the action for a long time. On the other hand, in order to obtain a high blood drug concentration immediately after administration, the average particle diameter of the suspended compound (1) is preferably, for example, 500 nm or less, and more preferably 50 to 500 nm. The average particle diameter is measured by a laser diffraction scattering method. For the measurement of the average particle diameter by a laser diffraction scattering method, for example, SALD-3100 or SALD-2300 (manufacturer: Shimadzu Corporation) can be used.

For the preparation method for the composition, a wet-milling method is preferably used. The wet-milling method is preferably wet ball milling, high-pressure homogenization, high-shear homogenization, bead milling (e.g., Dyno Mill), or the like. In addition to these milling methods, other methods, such as low-energy and high-energy milling (e.g., roller milling) can also be used. Examples of other preparation methods include controlled crystallization.

In one embodiment, the composition can be produced, for example, by a method comprising step 1 of mixing compound (1), a suspending agent, and a dispersion medium, step 2 of subjecting the suspension obtained by the mixing to bead milling, and step 3 of removing beads from the suspension obtained by bead milling. In step 1, the mixing order of each component is not particularly limited. In one embodiment, step 1 includes a step of mixing components other than the active ingredient to obtain a vehicle solution, and a step of mixing the vehicle solution with the active ingredient. In step 2, the method of bead milling is not particularly limited. In one embodiment, step 2 is a step of adding beads to the suspension, followed by stirring. Examples of the material of the beads include zirconia, alumina, and glass. The diameter of the beads is, for example, 0.1 to 5 mm, and preferably 0.2 to 3 mm. The average particle diameter of the particles of compound (1) obtained by bead milling can be appropriately adjusted by adjusting, for example, the size of the beads, the rotational speed (peripheral speed) during milling, and the milling time. In step 3, the method for removing the beads is not particularly limited. In one embodiment, step 3 is a step of collecting the composition by using a syringe needle (e.g., 22G or less), a pipette, or a mesh filter (e.g., a 80 μm mesh filter) with a pore size smaller than that of the beads.

Examples of another preferable injectable form of the long-acting injection that continuously delivers brexpiprazole for one day to several days or one to four weeks or more include a pharmaceutical composition that is a low-viscosity precursor preparation containing a mixture of compound (1), two or more liquid crystal-forming lipids or gel-forming lipids, and at least one biocompatible organic solvent, and that forms or is capable of forming at least one liquid crystal phase structure or lipid gel when brought into contact with an aqueous fluid in vivo.

The precursor preparation usually does not contain a significant amount of water before administration and is a low-viscosity liquid or suspension in which two or more liquid crystal-forming lipids or gel-forming lipids are dissolved in a biocompatible organic solvent. The liquid crystal-forming lipids or gel-forming lipids are amphiphilic components of specific types, and a combined use of at least one diacylglycerol and at least one phosphatidylcholine is preferred. By dissolving the liquid crystal-forming lipids or gel-forming lipids in at least one biocompatible organic solvent, a low-viscosity solution can be prepared, and by dissolving or suspending compound (1) in these solutions, a precursor preparation as a preferable low-viscosity liquid or suspension can be prepared. Such a precursor preparation forms a liquid crystal phase structure or a high-viscosity lipid gel by being brought into contact with an aqueous fluid in vivo after administration. Typically, the aqueous fluid is a body fluid, in particular, extravascular fluid, extracellular fluid/interstitial fluid, or plasma, and the precursor preparation forms a liquid crystal phase structure or a high-viscosity lipid gel by being brought into contact with such a fluid (e.g., in vivo fluid).

The precursor preparation, which is a low-viscosity liquid or suspension, represents a liquid or suspension that can be easily administered to a subject, and particularly represents a mixture that is easily administered with a device composed of a standard syringe or needle. In a particularly preferable embodiment, the precursor preparation, in which compound (1) is dissolved, or the medium before compound (1) is suspended should be a mixture that can pass through a standard sterile filter membrane, such as a 0.22 μm syringe filter. A typical range of suitable viscosity at 20°C is, for example, 0.1 to 5000 mPa·s, preferably 1 to 1000 mPa·s, and more preferably 1 to 500 mPa·s.

The above viscosity is measured with a rotational rheometer under the conditions of a temperature of 20°C and a shear rate within the range of 9000 to 10000 (1/s). Examples of the rotational rheometer include Discovery Hybrid Rheometer-2 (DHR-2) and Discovery Hybrid Rheometer-3 (DHR-3) (manufacturer: TA Instruments).

The precursor preparation may form a liquid crystal structure called a lyotropic liquid crystal when the amphiphilic

compounds (two or more liquid crystal-forming lipids or gel-forming lipids) are exposed to the aqueous fluid after being administered in vivo. Known examples of such liquid crystal structures include a hexagonal structure in which cylindrical aggregates form a hexagonal crystal system, a layered lamellar structure, an inverse hexagonal structure in which water is taken into the cylinder and the hydrophobic part is directed outward, a cubic structure in which spherical micelles form a cubic system in a water (or oil) continuous phase, and a bicontinuous cubic structure in which lipid bilayers are arranged three-dimensionally, forming a curved surface. The present inventors have discovered that after administration of the precursor preparation in vivo, the liquid crystal or lipid gel is formed, whereby the release of compound (1) after administration is controlled, and the released compound (1) is converted to brexpiprazole in vivo, making it possible to continuously deliver brexpiprazole.

The formation of the gel or liquid crystal can be confirmed by injecting the precursor preparation into water and confirming the formation of lumps. The formation of the liquid crystal can be confirmed by analyzing the obtained lumps by a small-angle X-ray scattering (SAXS) method. In some cases, a liquid crystal may be formed in a gel state (liquid crystal gel).

Diacylglycerol has two nonpolar "tail" groups. These two nonpolar groups may be the same or different, may have the same or different number of carbon atoms, and may each be independently saturated or unsaturated. Both 1,3-diacylglycerol and 1,2-diacylglycerol can be used. Examples of nonpolar groups include $C_6$-$C_{32}$ alkyl groups and $C_6$-$C_{32}$ alkenyl groups, which are usually present as esters of long-chain carboxylic acids. Descriptions thereof are often made by referring to the number of carbon atoms and the degree of unsaturation in the carbon chain. Therefore, CX:Z represents a hydrocarbon chain wherein X represents the number of carbon atoms, and Z represents the degree of unsaturation. Examples include, in particular, a caproyl (C6:0) group, capryloyl (C8:0) group, capryl (C10:0) group, lauroyl (C12:0) group, myristoyl (C14:0) group, palmitoyl (C16:0) group, phytanoyl (C16:0) group, palmitoleoyl (C16:1) group, stearoyl (C18:0) group, oleoyl (C18:1) group, elaidoyl (C18:1) group, linoleoyl (C18:2) group, linolenoyl (C18:3) group, arachidonoyl (C20:4) group, behenoyl (C22:0) group, and lignoceroyl (C24:9) group.

Therefore, typical nonpolar chains include caproic acid, caprylic acid, capric acid, lauric acid, myristic acid, palmitic acid, phytanic acid, palmitolic acid, stearic acid, oleic acid, elaidic acid, linoleic acid, linolenic acid, arachidonic acid, behenic acid, and lignoceric acid, and those based on fatty acids of natural ester lipids containing alcohols corresponding to these fatty acids. Preferable nonpolar chains include palmitic acid, stearic acid, oleic acid, and linoleic acid, and particularly preferred is oleic acid.

The diacylglycerol is not particularly limited and is preferably, for example, a diacylglycerol having the same or different nonpolar chains mentioned above. Among these, glycerol dioleate (GDO) is particularly preferred.

The diacylglycerols may be used alone or in a combination of two or more.

Phosphatidylcholine (PC) is a generic term for phospholipids having a structure in which choline is phosphate ester-bonded as the hydrophilic part of glycerophospholipid while two fatty acids are ester-bonded to the glycerol backbone as the hydrophobic part. Multiple phospholipids belong to phosphatidylcholine due to a large number of combinations of fatty acids. Examples of preferable phospholipid sources include eggs, heart (e.g., bovine heart), brain, and liver (e.g., bovine liver), and plant sources including soybeans. A single PC or a mixture of multiple PCs derived from such material sources may be used. Preferred is soy PC (SPC) or egg-derived PC, or a mixture thereof, and most preferred is essentially pure SPC (high-purity SPC).

The liquid crystal-forming lipids or gel-forming lipids for use are preferably a combination of at least one diacylglycerol and at least one phosphatidylcholine. In particular, a combined use of one or more diacylglycerols specifically mentioned above and one or more phosphatidylcholines specifically mentioned above is preferred. A particularly preferable combination of the liquid crystal-forming lipids or gel-forming lipids is the combination of GDO and PC, in particular, the combination of GDO and soy PC and/or egg PC.

The biocompatible organic solvent is preferably, for example, those that are miscible with water, and typical solvents include at least one solvent selected from alcohols, ketones, esters (including lactones), ethers, amides, and sulfoxides. Alcohols are particularly suitable, forming suitable solvent groups. Examples of suitable alcohols include ethanol, isopropanol, propylene glycol, glycerin, and glycerol formal, with ethanol being most preferred. Examples of ketones include acetone, n-methylpyrrolidone (NMP), 2-pyrrolidone, and propylene carbonate. Examples of suitable ethers include diethyl ether, glycofurol, diethylene glycol monoethyl ether, dimethyl isobarbide, and polyethylene glycol. Examples of suitable esters include ethyl acetate and isopropyl acetate. Dimethyl sulfide is a suitable sulfide solvent. Examples of suitable amides and sulfoxides include dimethylacetamide (DMA) and dimethyl sulfoxide (DMSO), respectively. The biocompatible organic solvents may be used alone or in a combination of two or more.

In the low-viscosity precursor preparation containing a mixture of two or more liquid crystal-forming lipids or gel-forming lipids and at least one biocompatible organic solvent, a combination of SPC, GDO, and ethanol is preferred, and a combination of SPC, GDO, ethanol, and DMSO is also suitable. A preferable low-viscosity precursor preparation is prepared by dissolving or suspending compound (1) in a solution containing two or more of the liquid crystal-forming lipids or gel-forming lipids and at least one biocompatible organic solvent.

Yet another example of a preferable injectable form of a long-acting injection that continuously delivers

brexpiprazole for one day to several days or one to four weeks or more include microspheres that comprise compound (1) as an active ingredient. Microspheres here generally refer to a spherical formulation with a particle diameter ranging from about a few micrometers to several tens of micrometers, and may have an uneven surface. The microsphere of the present disclosure usually comprises a carrier, such as a base polymer (e.g., a biodegradable polymer).

The microspheres of the present disclosure have an average particle diameter of preferably 5 to 150 μm, more preferably 10 to 120 μm, even more preferably 20 to 100 μm, and still more preferably 30 to 85 μm in order to obtain the desired controlled-release properties of compound (I). In order to obtain the desired controlled-release properties, the average particle diameter can be reduced if the absorption rate needs to be faster, and the average particle diameter can be increased if the absorption rate needs to be slower.

In order to obtain the desired controlled-release properties of compound (I) and desired changes in the blood drug concentration of brexpiprazole, the percentage of inclusion of compound (1) in the microsphere of the present disclosure is preferably 80% or more. The amount of the main drug added to a batch formulation may be increased to improve the percentage of inclusion.

In the microsphere of the present disclosure, the content of compound (I) is preferably 10 to 50 wt%, more preferably 20 to 45 wt%, and even more preferably 30 to 40 wt%, from the perspective of obtaining the desired controlled-release properties of compound (I) and desired changes in the blood drug concentration of brexpiprazole.

The microsphere of the present disclosure preferably comprises a biodegradable polymer as a base polymer. The biodegradable polymer for use in the present disclosure may be those that are gradually degraded in vivo to achieve desired sustained-release performance. Examples include homopolymers and copolymers thereof, such as polylactic acid, polyglycolic acid, lactic acid-glycolic acid copolymer (poly(lactic-co-glycolic acid): PLGA), polycitric acid, polymalic acid, lactic acid-aspartic acid copolymer, lactic acid-hydroxycaproic acid copolymer, glycolic acid-hydroxycaproic acid copolymer, polypropiolactone, polybutyrolactone, polyvalerolactone, polycaprolactone, polytrimethylene carbonate, poly-p-dioxanone, poly-α-cyanoacrylate, poly β-hydroxybutyrate, polytrimethylene oxalate, polyorthoester, polyorthocarbonate, polyethylene carbonate, poly-γ-benzyl-L-glutamic acid, poly-L-alanine, polyalginic acid, polycarbonate, polyesteramide, polyamino acid, polyalkylene alkylate, polyethylene glycol, and polyurethane. Among these, polylactic acids and lactic acid-glycolic acid copolymers are preferred. These biodegradable polymers may be used alone or in a combination of two or more.

The molecular weight of a polylactic acid or lactic acid-glycolic acid copolymer, when used, may be appropriately selected from a wide range, and is usually about 2000 to 200000, preferably about 4000 to 100000, and more preferably about 10000 to 70000.

The molecular weight above refers to the weight average molecular weight in terms of the weight of polystyrene, as measured by gel permeation chromatography (GPC) using polystyrene as a reference material.

The ratio of lactic acid:glycolic acid (lactide:glycolide) in a lactic acid-glycolic acid copolymer is not particularly limited and can be appropriately selected from a wide range; it is usually about 99:1 to 1:99 on a molecular number basis, and preferably about 85:15 to 50:50 on a molecular number basis.

The polylactic acid can be poly-D-lactic acid, poly-L-lactic acid, or poly-DL-lactic acid, and preferably poly DL-lactic acid. The lactic acid-glycolic acid copolymer can be a D-lactic acid-glycolic acid copolymer, L-lactic acid-glycolic acid copolymer, or DL-lactic acid-glycolic acid copolymer, and is preferably a DL-lactic acid-glycolic acid copolymer.

The polylactic acid or lactic acid-glycolic acid copolymer for use may be produced according to known methods or may be commercial products (e.g., Resomer and Lactel (produced by Evonik)).

The microsphere of the present disclosure may comprise a non-degradable biocompatible polymer, in addition to the biodegradable polymer. The microsphere of the present disclosure may also comprise any additives, such as emulsifiers.

The microsphere of the present disclosure can be produced according to methods known in the field of pharmaceutical preparations by using the components listed above. From the perspective of obtaining microspheres with excellent sustained-release performance, excellent flowability at the time of filling, and excellent syringe penetration at the time of administration, the microsphere of the present disclosure is preferably produced by a method comprising steps of dissolving or suspending compound (I) or a salt thereof and the biodegradable polymer in an organic solvent, emulsifying the solution or suspension in the presence or absence of an emulsifier, and then removing the organic solvent. This method may also further comprise steps known in the field of pharmaceutical preparations (e.g., filtration step and granulation step).

Below, the production method for the microsphere of the present disclosure is described in more detail.

The method for producing the microsphere of the present disclosure comprises the steps of: obtaining a solution or suspension containing compound (I) and the biodegradable polymer in an organic solvent; mixing the obtained solution or suspension with water and emulsifying the mixture in the presence or absence of an emulsifier to obtain an emulsion; and removing the organic solvent from the obtained emulsion.

First, compound (I) and the biodegradable polymer are dissolved or suspended in an organic solvent to obtain a homogeneous solution or suspension. Compound (I) may be dissolved or suspended in the organic solvent. The

biodegradable polymer is preferably dissolved in the organic solvent.

The organic solvent for use in the production of the microsphere of the present disclosure may be any organic solvent that is capable of dissolving the biodegradable polymer. Examples include halogenated hydrocarbons, such as chloroform, dichloroethane, trichloroethane, dichloromethane, and carbon tetrachloride; ethers, such as ethyl ether and isopropyl ether; fatty acid esters, such as ethyl acetate and butyl acetate; aromatic hydrocarbons, such as benzene, toluene, and xylene; alcohols, such as ethanol, methanol, isopropanol, and benzyl alcohol; nitriles, such as acetonitrile; amides, such as dimethylformamide; acetone; and other water-miscible or water-immiscible organic solvents. These organic solvents may be used alone or in a combination of two or more. Among these, water-immiscible organic solvents are preferred, and dichloromethane is particularly preferred.

Acids or bases can be added to these organic solvents. Examples of the acids include hydrochloric acid, phosphoric acid, acetic acid, citric acid, formic acid, gluconic acid, lactic acid, oxalic acid, tartaric acid, and oleic acid, with acetic acid being particularly preferred.

The proportions of compound (I) or a salt thereof and the organic solvent are usually such that the amount of compound (I) or a salt thereof is about 0.01 to 30 parts by weight, preferably about 0.1 to 20 parts by weight, and more preferably about 1 to 10 parts by weight, per 100 parts by weight of the organic solvent.

The proportions of the biodegradable polymer and the organic solvent are usually such that the amount of the biodegradable polymer is about 0.01 to 30 parts by weight, preferably about 0.1 to 20 parts by weight, and more preferably about 1 to 10 parts by weight, per 100 parts by weight of the organic solvent.

Next, the obtained organic solvent solution or suspension (containing compound (I), the biodegradable polymer, and the organic solvent) is emulsified. That is, the obtained organic solvent solution is mixed with water (i.e., outer phase) to obtain an O/W emulsion in which the organic solvent solution is uniformly dispersed in water, or the obtained organic solvent suspension is mixed with water (i.e., outer phase) to obtain an S (solid)/O/W emulsion in which the suspension is uniformly dispersed in water.

The water for use is not particularly limited and is preferably those that are accepted in the field of pharmaceutical preparations, such as purified water and water for injection.

The proportions of the organic solvent solution or suspension and water are not particularly limited as long as an O/W or S/O/W emulsion having the desired particle size is obtained. The proportions are usually such that the amount of the organic solvent solution or suspension is about 0.001 to 0.2 parts by volume, preferably about 0.005 to 0.1 parts by volume, and more preferably about 0.01 to 0.05 parts by volume, per part by volume of water.

In the present disclosure, an emulsifier may be used even with the use of either a water-miscible or water-immiscible organic solvent. If a water-miscible organic solvent is used, an emulsifier is preferably used. The emulsifier for use in the present disclosure may be any emulsifier that is capable of forming an O/W or S/O/W emulsion, and preferably a stable O/W or S/O/W emulsion. Examples include anionic surfactants, such as sodium oleate, sodium stearate, and sodium lauryl sulfate; nonionic surfactants, such as polyoxyethylene sorbitan fatty acid esters and polyoxyethylene castor oil derivatives; and polyvinyl pyrrolidone, polyvinyl alcohol, carboxymethyl cellulose, lecithin, gelatin, and hyaluronic acid. These emulsifiers may be used alone or in a combination of two or more.

The emulsifier may be added to the aqueous phase before emulsification.

The amount of the emulsifier for use is not particularly limited and may be selected from a wide range. The concentration of the emulsifier in the aqueous solution is, for example, about 0.0001 to 20 wt%, preferably about 0.001 to 10 wt%, and more preferably about 0.001 to 5 wt%.

The preparation method for the O/W or S/O/W emulsion is not particularly limited. The emulsion may be prepared by using a method that causes the organic solvent solution or suspension (containing compound (I) or a salt thereof, the biodegradable polymer, and the organic solvent) to be dispersed in water as droplets or micelles with a suitable size. Examples of the preparation method include a method in which a mixture of the solution or suspension and water is stirred at an appropriate rotational speed in a homogenizer or the like to refine the solution or suspension in water to thus obtain an O/W or S/O/W emulsion; a method in which a mixture of the solution or suspension and water is passed through a filter with fine through-holes, such as a ceramic filter, at a constant speed to refine the solution or suspension to thus obtain an O/W or S/O/W emulsion; and a method in which the solution or suspension is passed through a filter with fine through-holes, such as a ceramic filter, at a constant speed to refine the solution or suspension, followed by mixing with water.

The emulsion formation may be performed in multiple steps as necessary.

The desired particle diameter can be obtained by adjusting the force of stirring during the emulsion formation. That is, the particle diameter can be reduced by increasing the force of stirring during the emulsion formation.

By removing the organic solvent from the obtained O/W or S/O/W emulsion, an aqueous suspension of microspheres is obtained.

The organic solvent may be removed according to commonly used methods. For example, the removal may be performed by heating while stirring with a propeller stirrer or magnetic stirrer, by gradually reducing the pressure, or by adjusting the vacuum level with a rotary evaporator etc. for desorption.

The thus-obtained microsphere suspension may be washed by adding an appropriate solution or solvent as

necessary to remove a free compound (I) adhering to the microsphere surface. Examples of cleaning solutions and solvents include ethanol and alkaline solutions, such as a 1N sodium hydroxide solution. The microspheres separated by centrifugation or filtration are washed several times repeatedly with distilled water, and, if necessary, with an appropriate solution or solvent, to remove the free compound (I), emulsifier, etc. adhering to the microsphere surface. Examples of cleaning solutions and solvents include ethanol and alkaline solutions, such as a 1N sodium hydroxide solution. After separation, the microspheres are heated as necessary to more completely desorb the water from the microspheres under reduced pressure and desorb the solvent from the microspheres. The washed microspheres can be resuspended in water in the presence or absence of sugar, sugar alcohols, or other additives, and then freeze-dried to obtain a microsphere powder. The conditions for freeze-drying are not particularly limited as long as the microspheres can be dried.

Further, the dried microspheres may be sieved through a sieve as necessary to obtain microspheres with the desired average particle diameter.

The microsphere of the present disclosure, particularly the microsphere obtained by the production method described above, has excellent sustained-release performance. The microsphere of the present disclosure can release compound (I) or a salt thereof in a therapeutic amount for at least one week, preferably two, three, or four weeks, and more preferably six weeks or more. The microsphere of the present disclosure can exhibit the above effects, in particular, when suspended in water for injection to obtain an aqueous suspension injection.

The microsphere of the present disclosure obtained by the above production method usually have a spherical shape. Due to the spherical shape, the microsphere of the present disclosure achieves excellent flowability at the time of filling in the production and excellent syringe penetration (needle penetration) at the time of administration, and causes less irritation at the injection site when administered as an injection for muscular or subcutaneous administration.

The microsphere of the present disclosure can be administered to patients, for example, as an aqueous suspension injection containing the compound (I)-containing microsphere, a vehicle therefor, and water for injection. Alternatively, in another embodiment, the microsphere of the present disclosure can be administered to patients, for example, as an oil suspension injection containing the compound (I)-containing microsphere, optionally a vehicle therefor, and an oil. For example, a triglyceride (in particular, medium-chain fatty acid triglyceride) is suitable as the oil used here.

The content of the microsphere in the suspension injection of the present disclosure is not particularly limited as long as the microspheres are dispersed in the injection, and is usually about 5 to 50 wt%, preferably about 10 to 40 wt%, and more preferably about 10 to 30 wt%.

Examples of the vehicle used in the present disclosure include the suspending agents, isotonic agents, buffering agents, and pH adjusters mentioned above.

In the present specification, the term "therapeutically effective amount" refers to an amount that is effective to provide therapeutic usefulness, such as relief of symptoms, when administered to humans or non-human animals. The specific dose of the substance administered to obtain therapeutic usefulness, of course, will be determined, for example, based on specific environments, such as the specific substance to be administered, the route of administration, the pathological condition to be treated, and the individual to be treated. For example, the pharmaceutical formulation may comprise compound (I) or a salt thereof in an amount of 1 to 70 wt%, in terms of the amount of compound (I).

For example, for subcutaneous or intramuscular administration, a daily dose that can provide satisfactory results may be in the order of about 0.001 to 1.5 mg/kg. The daily dose instructed for subcutaneous or intramuscular administration may range from about 0.1 mg to 500 mg, and preferably 0.1 mg to 100 mg, which may be suitably administered once per day or in two to four divided doses per day. Therefore, the unit dosage form for subcutaneous or intramuscular administration may contain compound (I) or a salt thereof in an amount of, for example, about 0.1 mg to 500 mg in terms of the amount of compound (I), together with a pharmaceutically acceptable diluent or carrier. The upper limit or lower limit of this range (0.1 mg to 500 mg) may be, for example, 0.2, 0.5, 1, 2, 3, 5, 10, 20, 50, 100, 200, 300, or 400 mg. For example, the range is preferably 0.2 to 100 mg or 0.5 to 50 mg, and more preferably 0.5 to 10 mg.

In the present specification, the term "pharmaceutically acceptable" means that a compound, a composition comprising the compound, or its dosage form falls within a range having a reasonable benefit/risk ratio appropriate for application to humans or animals without causing excessive toxicity, irritation, allergic response, or the like.

Although not so particularly limited, the pharmaceutical formulation comprising compound (I) or a salt thereof is preferably, for example, an injection. The injection includes an injection for intramuscular administration or an injection for subcutaneous administration, with an injection for subcutaneous administration being particularly preferred.

In the present specification, the terms "comprising" and "containing" also include "consisting essentially of" and "consisting of." Further, the present disclosure encompasses any combination of the constituent elements described in the present specification.

In addition, the various characteristics (properties, structures, functions, etc.) described in each embodiment of the present disclosure described above may be combined in any way in specifying the subjects encompassed by the present disclosure. In other words, the present disclosure encompasses all the subjects comprising all combinations of the combinable characteristics described in the present specification.

Examples

The embodiments of the present disclosure are described with reference to Examples in more detail below. However, the embodiments of the present disclosure are not limited to the following Examples.

The following abbreviations may be used herein.

REX: Reference Example number
EX: Example number
STR: Structural formula
Data: Physical property data (NMR1: δ (ppm) in $^1$H-NMR in dimethyl sulfoxide-$d_6$; NMR2: δ (ppm) in $^1$H-NMR in $CDCl_3$; NMR3: δ (ppm) in $^1$H-NMR in $CD_3OD$; NMR4: δ (ppm) in $^1$H-NMR in $CD_3CO_2D$, or MS: mass spectrum)

In addition, the following abbreviations may be used for compound names and reagent names.

Table 1E

| Abbreviation | Reagent name |
|---|---|
| AcOEt | Ethyl acetate |
| AcOH | Acetic acid |
| AcOMe | Methyl acetate |
| AcONa | Sodium acetate |
| CDI | 1,1'-Carbonyldiimidazole |
| $Cs_2CO_3$ | Cesium carbonate |
| DABCO | 1,4-Diazabicyclo[2.2.2]octane |
| DBN | 1,5-Diazabicyclo[4.3.0]-5-nonene |
| DBU | 1,8-Diazabicyclo[5.4.0]-7-undecene |
| DCE | 1,2-Dichloroethane |
| DCM | Dichloromethane |
| DHP | 3,4-Dihydro-2H-pyran |
| DIPEA | Diisopropylethylamine |
| DMAP | 4-Dimethylaminopyridine |
| DMF | N,N-Dimethylformamide |
| DMSO | Dimethyl sulfoxide |
| $Et_2O$ | Diethyl ether |
| EtOH | Ethanol |
| HATU | 1-[Bis(dimethylamino)methylene]-1H-1,2,3-triazolo[4,5-b]pyridinium 3-oxide hexafluorophosphate |
| HCl | Hydrochloric acid |
| HOBt | 1-Hydroxytriazole |
| $K_2CO_3$ | Potassium carbonate |
| KOH | Potassium hydroxide |
| $Li_2CO_3$ | Lithium carbonate |
| LiI | Lithium iodide |
| LiOH | Lithium hydroxide |
| MeCN | Acetonitrile |
| MeOH | Methanol |
| $MgSO_4$ | Magnesium sulfate |

(continued)

| Abbreviation | Reagent name |
|---|---|
| $Na_2CO_3$ | Sodium carbonate |
| NaH | Sodium hydride |
| $NaHCO_3$ | Sodium hydrogen carbonate |
| NaI | Sodium iodide |
| NaOH | Sodium hydroxide |
| $Na_2SO_4$ | Sodium sulfate |
| NMP | N-Methylpyrrolidone |
| Pd/C | Palladium on carbon |
| PPTS | Pyridinium p-toluenesulfonate |
| TEA | Triethylamine |
| TFA | Trifluoroacetic acid |
| $Tf_2O$ | Trifluoromethanesulfonic anhydride |
| THF | Tetrahydrofuran |
| WSC | 3-Ethyl-1-(3-dimethylaminopropyl) carbodiimide |
| WSC•HCl | 3-Ethyl-1-(3-dimethylaminopropyl) carbodiimide hydrochloride |

In the following Examples, "room temperature" is usually about 10 to 35°C. Ratios for mixed solvents are volume ratios, unless otherwise noted. The unit "%" indicates weight percent, unless otherwise noted.
The $^1$H NMR (proton nuclear magnetic resonance spectrum) was measured by Fourier transform NMR (Bruker Avance III 400 (400 MHz), Bruker Avance Neo 400 (400 MHz), or Bruker Avance III HD (500 MHz)).

Example 1 (EX 1)

NaI (2.074 g), $K_2CO_3$ (1.913 g), and di-tert-butyl (chloromethyl)phosphate (3.58 g) were added to a mixture of 7-(4-(benzo[b]thiophen-4-yl-piperazin-1-yl)butoxy)-1H-quinolin-2-one (3.0 g), MeCN (30 mL), and DCM (90 mL), and the resulting mixture was stirred at room temperature. Three days later, di-tert-butyl (chloromethyl)phosphate (1.790 g) and NaI (1.037 g) were added and stirred. After another 4 days, water was added to the reaction mixture and stirred, and the organic layer was extracted and concentrated under reduced pressure. The resulting product was dissolved in DCM (90 mL), TFA (5.33 mL) was added thereto, and the mixture was stirred at room temperature for 3 hours. A 1 N NaOH aqueous solution (138 mL) was added to the reaction mixture and stirred for 20 minutes. The aqueous layer was separated from the reaction mixture and washed with DCM and $Et_2O$. AcOH (3.96 mL) was added to the aqueous layer and stirred at room temperature for 1 hour. The precipitated solid was filtered out to obtain a crude product. The crude product was purified by reversed-phase silica gel column chromatography (0.1% AcOH MeCN/0.1% AcOH $H_2O$) and concentrated under reduced pressure to give (4-(benzo[b]thiophen-4-yl)-1-((4-((2-oxo-1,2-dihydroquinolin-7-yl)oxy)butyl)piperazin-1-ium-1-yl)methyl hydrogen phosphate (1.71 g).
NMR1; 1.77-1.89 (2H, m), 1.89-2.01 (2H, m), 3.37-3.86 (11H, m), 4.10 (2H, t, J = 6.3 Hz), 5.11 (2H, d, J = 9.0 Hz), 6.29 (1H, d, J = 9.4 Hz), 6.80 (1H, dd, J = 2.4, 8.7 Hz), 6.95-7.05 (2H, m), 7.31 (1H, dd, J = 7.9, 7.9 Hz), 7.51 (1H, d, J = 5.5 Hz), 7.54 (1H, d, J = 8.7 Hz), 7.71 (1H, d, J = 8.1 Hz), 7.76 (1H, d, J = 5.5 Hz), 7.79 (1H, d, J = 9.5 Hz), 11.93 (1H, br).

Example 2 (EX 2)

In a nitrogen atmosphere, a mixture of (4-(benzo[b]thiophen-4-yl)-1-(4-(((2-(tert-butoxy)quinolin-7-yl)oxy)butyl) piperazin-1-ium-1-yl)methyl tert-butyl phosphate (15.7 g), AcOH (42.3 mL), and TFA (14.1 mL) was stirred at 40°C for 1.5 hours. The reaction mixture was cooled to 10°C or lower, a solution of AcONa (19.6 g) in water (170 mL)/MeCN (70.7 mL) was added thereto, and the remaining liquid was washed with water (30 mL) and combined. The resulting mixture was stirred at room temperature for 1 hour, water (71 mL) was further added thereto, and the mixture was stirred at 0°C for 1 hour. The precipitated crystals were subjected to solid-liquid separation, and the resulting product was washed with water (71 mL) and MeCN/water (1:4, 71 mL), and air-dried at 40°C for 12 hours to give (4-(benzo[b]thiophen-4-yl)-1-((4-((2-oxo-1,2-dihydroquinolin-7-yl)oxy)butyl)piperazin-1-ium-1-yl)methyl hydrogen phosphate (13.81 g).

NMR1; 1.75-1.90 (2H, m), 1.90-2.00 (2H, m), 3.40-3.85 (11H, m), 4.09 (2H, t, J = 6.4 Hz), 5.11 (2H, d, J = 9.2 Hz), 6.29 (1H, d, J = 9.6 Hz), 6.80 (1H, dd, J = 2.4 Hz, 8.8 Hz), 6.95-7.05 (2H, m), 7.31 (1H, dd, J = 7.9 Hz, 7.9 Hz), 7.51 (1H, d, J = 5.6 Hz), 7.54 (1H, d, J = 8.8 Hz), 7.71 (1H, d, J = 8.0 Hz), 7.76 (1H, d, J = 5.6 Hz), 7.79 (1H, d, J = 9.6 Hz), 11.93 (1H, br).

Example 3 (EX 3)

Di-tert-butyl (chloromethyl)phosphate (1.82 g) was dissolved in MeCN/DCM (1/1) (60 mL), and 7-((4-(4-(benzo[b]thiophen-4-yl)piperazin-1-yl)butoxy)-2-(tert-butoxy)quinoline (2.2 g) was added thereto. After purging with nitrogen, the mixture was stirred at 40°C overnight under light-shielding with aluminum foil. The reaction mixture was concentrated under reduced pressure to remove a large portion of the DCM, and then extracted with addition of AcOEt/MeOH (9/1) and 10% $Na_2S_2O_3$ aqueous solution. The organic layer was separated and concentrated under reduced pressure. AcOH (30 mL) was added to the residue, and the mixture was stirred at 40°C for 2 hours. The reaction mixture was concentrated under reduced pressure, toluene was then added thereto, and the mixture was concentrated under reduced pressure. The residue was purified by silica gel column chromatography (DCM/MeOH) to give 4-(benzo[b]thiophen-4-yl)-1-(((diethoxyphosphoryl)oxy)methyl)-1-(4-((2-oxo-1,2-dihydroquinolin-7-yl)oxy)butyl)piperazin-1-ium iodide (3.1 g). Acetone (120 mL) and NaI (2.55 g) were added thereto, and the mixture was stirred at 60°C overnight. The mixture was cooled to room temperature, the liquid was removed, and water (50 mL) and AcOH (2.5 mL) were added to the residue under ice-cooling. Water (80 mL) was added thereto, and the mixture was stirred at room temperature for 8 hours. The precipitate was filtered out and washed with water. After drying under reduced pressure, silica gel column chromatography (DCM/MeOH) was performed for purification. After adding MeCN/water for crystallization, the resulting product was filtered out and washed with water. The resulting solid was dried under reduced pressure to give (4-(benzo[b]thiophen-4-yl)-1-(4-((2-oxo-1,2-dihydroquinolin-7-yl)oxy)butyl)piperazin-1-ium-1-yl)methyl ethyl phosphate (1.1 g). NMR1; 1.13 (3H, t, J = 7.1 Hz), 1.78-1.89 (2H, m), 1.89-2.01 (2H, m), 3.38-3.49 (4H, m), 3.53-3.66 (4H, m), 3.70-3.82 (4H, m), 4.10 (2H, t, J = 6.1 Hz), 5.09 (2H, d, J = 9.1 Hz), 6.30 (1H, d, J = 9.4 Hz), 6.82 (1H, dd, J = 2.4 Hz, 8.7 Hz), 6.89 (1H, d, J = 2.3 Hz), 7.01 (1H, d, J = 7.4 Hz), 7.32 (1H, t, J = 7.8 Hz), 7.51 (1H, d, J = 5.5 Hz), 7.56 (1H, d, J = 8.7 Hz), 7.71 (1H, d, J = 8.0 Hz), 7.77 (1H, d, J = 5.6 Hz), 7.80 (1H, d, J = 9.1 Hz), 11.69 (1H, s).

Example 4 (EX 4)

MeCN (44 mL) and water (44 mL) were added to (4-(benzo[b]thiophen-4-yl)-1-(4-((2-(tert-butoxy)quinolin-7-yl)oxy)butyl)piperazin-1-ium-1-yl)methyl tert-butyl phosphate (11.1 g), and the mixture was stirred at 45°C, followed by addition of water (22 mL). After confirming the disappearance of the starting material, $NaHCO_3$ (1.1 g) was added, and the mixture was concentrated under reduced pressure and extracted with 10% MeOH/DCM (100 mL). The resulting organic layer was concentrated, and the crude product was purified by silica gel column chromatography (DCM/MeOH) to give (4-(benzo[b]thiophen-4-yl)-1-(4-((2-oxo-1,2-dihydroquinolin-7-yl)oxy)butyl)piperazin-1-ium-1-yl)methyl tert-butyl phosphate (8.7 g).

NMR3; 1.47 (9H, s), 1.95-2.04 (2H, m), 2.05-2.15 (2H, m), 3.40-3.60 (4H, m), 3.65-3.70 (2H, m), 3.70-3.80 (2H, m), 3.85-3.95 (2H, m), 4.20 (2H, t, J = 6.0 Hz), 5.16 (2H, d, J = 8.0 Hz), 6.44 (1H, d, J = 9.6 Hz), 6.88-6.92 (2H, m), 7.03 (1H, dd, J = 0.4, 7.6 Hz), 7.29 (1H, t, J = 8.0 Hz), 7.49 (1H, dd, J = 0.8, 5.6 Hz), 7.55-7.58 (2H, m), 7.64 (1H, d, J = 8.0 Hz), 7.87 (1H, d, J = 7.6 Hz).

Example 5 (EX 5)

Isopropyl alcohol (10 mL), water (2 mL), and WSC•HCl (317 mg) were added at room temperature to (4-(benzo[b]thiophen-4-yl)-1-(4-((2-oxo-1,2-dihydroquinolin-7-yl)oxy)butyl)piperazin-1-ium-1-yl)methyl hydrogen phosphate (300 mg), and the mixture was stirred at 70°C for 10 hours. The reaction mixture was concentrated, water was added thereto, and the mixture was extracted with DCM/MeOH (9/1). The organic layer was concentrated, and the residue was purified by silica gel column chromatography (DCM/MeOH) to give (4-(benzo[b]thiophen-4-yl)-1-(4-((2-oxo-1,2-dihydroquinolin-7-yl)oxy)butyl)piperazin-1-ium-1-yl)methyl isopropyl phosphate (44 mg).

NMR1; 1.14 (6H, d, J = 6.2 Hz), 1.78-1.89 (2H, m), 1.89-2.01 (2H, m), 3.41-3.49 (4H, m), 3.53-3.66 (4H, m), 3.66-3.82 (2H, m), 4.10 (2H, t, J = 6.1 Hz), 4.25-4.34 (1H, m), 5.08 (2H, d, J = 7.2 Hz), 6.30 (1H, dd, J = 2.0 Hz, 9.4 Hz), 6.82 (1H, dd, J = 2.4 Hz, 8.7 Hz), 6.90 (1H, d, J = 2.3 Hz), 7.01 (1H, d, J = 7.4 Hz), 7.32 (1H, t, J = 7.8 Hz), 7.51 (1H, d, J = 5.5 Hz), 7.56 (1H, d, J = 8.7 Hz), 7.71 (1H, d, J = 8.0 Hz), 7.77 (1H, d, J = 5.6 Hz), 7.80 (1H, d, J = 9.1 Hz), 11.69 (1H, s).

Example 6 (EX 6)

1,3-Propanediol (10 mL), water (2 mL), and WSC•HCl (212 mg) were added at room temperature to (4-(benzo[b]thiophen-4-yl)-1-(4-((2-oxo-1,2-dihydroquinolin-7-yl)oxy)butyl)piperazin-1-ium-1-yl)methyl hydrogen phosphate (300

mg), and the mixture was stirred at 40°C. After 30 minutes, the mixture was heated to 50°C. After another 30 minutes, the mixture was heated again to 60°C. After another 2 hours, the mixture was cooled to room temperature and azeotroped with AcOEt, and the residue was purified by silica gel column chromatography (DCM/MeOH). The resulting product was dispersed and washed with AcOEt, and the insoluble solid was filtered out, followed by drying under reduced pressure to give (4-(benzo[b]thiophen-4-yl)-1-(4-((2-oxo-1,2-dihydroquinolin-7-yl)oxy)butyl)piperazin-1-ium-1-yl)methyl (3-hydroxypropyl) phosphate (200 mg).

NMR1; 1.57-1.68 (2H, m), 1.78-1.89 (2H, m), 1.89-2.01 (2H, m), 3.40-3.52 (6H, m), 3.53-3.66 (4H, m), 3.70-3.82 (4H, m), 4.10 (2H, t, J = 6.1 Hz), 4.70 (1H, t, J = 5.6 Hz), 5.10 (2H, d, J = 9.0 Hz), 6.30 (1H, d, J = 9.4 Hz), 6.82 (1H, dd, J = 2.4 Hz, 8.7 Hz), 6.89 (1H, d, J = 2.3 Hz), 7.01 (1H, d, J = 7.4 Hz), 7.32 (1H, t, J = 7.8 Hz), 7.51 (1H, d, J = 5.5 Hz), 7.56 (1H, d, J = 8.7 Hz), 7.71 (1H, d, J = 8.0 Hz), 7.77 (1H, d, J = 5.6 Hz), 7.81 (1H, d, J = 9.4 Hz), 11.67 (1H, s).

Example 7 (EX 7)

Diethylene glycol monomethyl ether (10.00 mL), water (2 mL), and WSC•HCl (353 mg) were added at room temperature to (4-(benzo[b]thiophen-4-yl)-1-(4-((2-oxo-1,2-dihydroquinolin-7-yl)oxy)butyl)piperazin-1-ium-1-yl)methyl hydrogen phosphate (500 mg), and the mixture was stirred at 40°C. After 3 hours, WSC•HCl (176 mg) was added. The mixture was stirred at 40°C for 3 hours. Water was added to the reaction mixture and extracted with DCM/MeOH (9/1). The organic layer was concentrated, and the residue was purified by silica gel column chromatography (DCM/MeOH) to give (4-(benzo[b]thiophen-4-yl)-1-(4-((2-oxo-1,2-dihydroquinolin-7-yl)oxy)butyl)piperazin-1-ium-1-yl)methyl (2-(2-methoxyethoxy)ethyl)phosphate (188 mg).

NMR1; 1.78-1.89 (2H, m), 1.89-2.01 (2H, m), 3.21 (3H, s), 3.40-3.48 (6H, m), 3.42-3.49 (4H, m), 3.53-3.68 (4H, m), 3.70-3.85 (4H, m), 4.10 (2H, t, J = 5.2 Hz), 5.09 (2H, d, J = 9.0 Hz), 6.30 (1H, d, J = 9.4 Hz), 6.82 (1H, dd, J = 2.4 Hz, 8.7 Hz), 6.89 (1H, d, J = 2.3 Hz), 7.01 (1H, d, J = 7.3 Hz), 7.32 (1H, t, J = 7.8 Hz), 7.51 (1H, d, J = 5.5 Hz), 7.56 (1H, d, J = 8.7 Hz), 7.71 (1H, d, J = 8.0 Hz), 7.77 (1H, d, J = 5.6 Hz), 7.80 (1H, d, J = 9.1 Hz), 11.67 (1H, s).

Example 8 (EX 8)

Acetone/water (9/1) (10 mL), ethyl glycolate (3 mL), and WSC•HCl (529 mg) were added at room temperature to (4-(benzo[b]thiophen-4-yl)-1-(4-((2-oxo-1,2-dihydroquinolin-7-yl)oxy)butyl)piperazin-1-ium-1-yl)methyl hydrogen phosphate (500 mg), and the mixture was stirred at 40°C for 5 hours, followed by stirring overnight at room temperature. DCM/MeOH (9/1) and water were added to the reaction mixture, and the precipitate was filtered out. The organic layer of the filtrate was concentrated and purified together with the filtered precipitate obtained above by silica gel column chromatography (DCM/MeOH) to give (4-(benzo[b]thiophen-4-yl)-1-(4-((2-oxo-1,2-dihydroquinolin-7-yl)oxy)butyl)piperazin-1-ium-1-yl)methyl (2-ethoxy-2-oxoethyl)phosphate (16 mg).

NMR1; 1.18 (3H, t, J = 7.1 Hz), 1.78-1.89 (2H, m), 1.89-2.01 (2H, m), 3.42-3.49 (4H, m), 3.51-3.68 (4H, m), 3.70-3.82 (2H, m), 4.10 (2H, J = 9.6 Hz), 4.06-4.15 (2H, m), 4.35 (2H, d, J = 9.6 Hz), 5.12 (2H, d, J = 8.8 Hz), 6.30 (1H, dd, J = 1.8 Hz, 9.4 Hz), 6.83 (1H, dd, J = 2.4 Hz, 8.7 Hz), 6.87 (1H, d, J = 2.3 Hz), 7.01 (1H, d, J = 7.5 Hz), 7.33 (1H, t, J = 7.8 Hz), 7.52 (1H, d, J = 5.5 Hz), 7.57 (1H, d, J = 9.0 Hz), 7.72 (1H, d, J = 8.0 Hz), 7.77 (1H, d, J = 5.6 Hz), 7.81 (1H, d, J = 9.1 Hz), 11.65 (1H, s).

Example 9 (EX 9)

(4-(Benzo[b]thiophen-4-yl)-1-(4-((2-oxo-1,2-dihydroquinolin-7-yl)oxy)butyl)piperazin-1-ium-1-yl)methyl hydrogen phosphate (1.0 g) was suspended in a mixture of ethylene glycol (10 mL) and water (1 mL), WSC•HCl (1.06 g) was added thereto, and the mixture was stirred at 45°C for 5 hours. Acetone and AcOEt were added to the reaction mixture, the mixture was concentrated under reduced pressure three times, and water was azeotropically removed. The concentrated residue was purified directly with medium-pressure liquid chromatography (DCM/MeOH).

Water and a mixed solvent of DCM-MeOH = 4:1 was added to the resulting product, and the mixture was stirred and extracted. The organic layer was concentrated under reduced pressure, acetone was added to the resulting oil, and the mixture was stirred at room temperature overnight. The precipitated solid was filtered out and air-dried to give (4-(benzo[b]thiophen-4-yl)-1-(4-((2-oxo-1,2-dihydroquinolin-7-yl)oxy)butyl)piperazin-1-ium-1-yl)methyl (2-hydroxyethyl) phosphate (71 mg).

NMR1; 1.78-1.88 (2H, m), 1.90-1.99 (2H, m), 3.42-3.48 (2H, m), 3.49-3.55 (2H, m), 3.56-3.68 (4H, m), 3.72-3.80 (4H, m), 4.10 (2H, t, J = 6.0 Hz), 5.11 (2H, d, J= 9.5 Hz), 6.30 (1H, dd, J = 9.5 Hz, 1.8 Hz), 6.83 (1H, dd, J = 8.6 Hz, 2.4 Hz), 6.88 (1H, d, J = 2.4 Hz), 7.00 (1H, d, J = 7.4 Hz), 7.32 (1H, t, J = 7.9 Hz), 7.51 (1H, d, J = 5.5 Hz), 7.56 (1H, d, J = 8.7 Hz), 7.71 (1H, d, J = 8.1 Hz), 7.77 (1H, d, J = 5.5 Hz), 7.80 (1H, d, J = 9.4 Hz), 11.7 (1H, s).

Example 10 (EX 10)

(4-(Benzo[b]thiophen-4-yl)-1-(4-((2-oxo-1,2-dihydroquinolin-7-yl)oxy)butyl)piperazin-1-ium-1-yl)methyl hydrogen phosphate (1.0 g) was suspended in a mixture of 1.4-butanediol (20 mL) and water (4 mL), WSC•HCl (1.06 g) was added thereto, and the mixture was stirred at 60°C for 5 hours. Acetone and AcOEt were added to the reaction mixture, the mixture was concentrated under reduced pressure three times, and water was azeotropically removed. The concentrated residue was directly purified twice by medium-pressure liquid chromatography (1st: silica gel, 2nd: amino silica gel, eluent: DCM/MeOH).

The resulting amorphous product was suspended in water (5 mL), and acetone was added for dissolution. After standing at room temperature overnight, the precipitated crystals were filtered out and air-dried at 25°C overnight to give (4-(benzo[b]thiophen-4-yl)-1-(4-((2-oxo-1,2-dihydroquinolin-7-yl)oxy)butyl)piperazin-1-ium-1-yl)methyl (4-hydroxybutyl) phosphate (438 mg).

NMR1; 1.39-1.49 (2H, m), 1.50-1.58 (2H, m), 1.78-1.88 (2H, m), 1.89-1.99 (2H, m), 3.37-3.42 (2H, m), 3.42-3.48 (2H, m), 3.53-3.67 (4H, m), 3.68-3.80 (4H, m), 4.10 (2H, t, J = 6.1 Hz), 4.43 (1H, t, J = 5.2 Hz), 5.09 (2H, d, J = 9.1 Hz), 6.30 (1H, d, J = 9.4 Hz), 6.82 (1H, dd, J = 8.6 Hz, 2.4 Hz), 6.89 (1H, d, J = 2.4 Hz), 7.01 (1H, d, J = 7.6 Hz), 7.32 (1H, t, J = 7.9 Hz), 7.51 (1H, d, J = 5.6 Hz), 7.56 (1H, d, J = 8.7 Hz), 7.71 (1H, d, J = 8.0 Hz), 7.76 (1H, d, J = 5.5 Hz), 7.80 (1H, d, J= 9.5 Hz), 11.7 (1H, s).

Example 11 (EX 11)

(4-(Benzo[b]thiophen-4-yl)-1-(4-((2-oxo-1,2-dihydroquinolin-7-yl)oxy)butyl)piperazin-1-ium-1-yl)methyl hydrogen phosphate (1.0 g) and 1.8-octanediol (3 g) were suspended in a mixed solvent of acetone (8 mL) and water (2 mL). WSC•HCl (0.71 g) was added thereto, and the mixture was stirred at 50°C for 3 hours. Acetone and AcOEt were added to the reaction mixture, the mixture was concentrated under reduced pressure, and water was azeotropically removed by carrying out this three times. The concentrated residue was directly purified twice by medium-pressure liquid chromatography (1st: silica gel, 2nd: amino silica gel, eluent: DCM/MeOH). The resulting amorphous product was suspended in water (5 mL), and acetone was added for dissolution. After standing at room temperature overnight, the precipitated crystals were filtered out and air-dried at 25°C overnight to give (4-(benzo[b]thiophen-4-yl)-1-(4-((2-oxo-1,2-dihydroquinolin-7-yl)oxy)butyl)piperazin-1-ium-1-yl)methyl (8-hydroxyoctyl) phosphate (176 mg).

NMR1; 1.18-1.29 (8H, m), 1.33-1.42 (2H, m), 1.45-1.52 (2H, m), 1.78-1.87 (2H, m), 1.88-1.98 (2H, m), 3.42-3.50 (4H, m), 3.53-3.80 (8H, m), 4.10 (2H, t, J = 6.1 Hz), 4.34 (1H, t, J = 5.2 Hz), 5.00 (2H, d, J = 9.0 Hz), 6.30 (1H, dd, J = 9.4 Hz, 1.4 Hz), 6.81 (1H, dd, J = 8.6 Hz, 2.4 Hz), 6.89 (1H, d, J = 2.4 Hz), 7.01 (1H, d, J = 7.4 Hz), 7.32 (1H, t, J = 7.8 Hz), 7.51 (1H, dd, J = 5.6 Hz, 0.4 Hz), 7.56 (1H, d, J = 8.7 Hz), 7.71 (1H, d, J = 8.1 Hz), 7.76 (1H, d, J = 5.5 Hz), 7.80 (1H, d, J = 9.5 Hz), 11.7 (1H, s).

Example 12 (EX 12)

(4-(Benzo[b]thiophen-4-yl)-1-(4-((2-oxo-1,2-dihydroquinolin-7-yl)oxy)butyl)piperazin-1-ium-1-yl)methyl hydrogen phosphate (1.0 g) was suspended in a mixture of 2-methoxyethanol (20 mL) and water (2 mL). WSC•HCl (1.06 g) was added thereto, and the mixture was stirred at 60°C for 3 hours. Acetone and AcOEt were added to the reaction mixture, the mixture was concentrated under reduced pressure, and water was azeotropically removed by carrying out this three times. The concentrated residue was directly purified twice by medium-pressure liquid chromatography (1st: silica gel, 2nd: amino silica gel, eluent: DCM/MeOH).

The resulting amorphous product was suspended in water (5 mL), and acetone was added for dissolution. After standing at room temperature overnight, the precipitated crystals were filtered out and air-dried at 25°C overnight to give (4-(benzo[b]thiophen-4-yl)-1-(4-((2-oxo-1,2-dihydroquinolin-7-yl)oxy)butyl)piperazin-1-ium-1-yl)methyl (2-methoxyethyl) phosphate (320 mg).

NMR1; 1.79-1.88 (2H, m), 1.89-1.98 (2H, m), 3.24 (3H, s), 3.40-3.48 (4H, m), 3.55-3.67 (4H, m), 4.10 (2H, t, J = 6.1 Hz), 5.09 (2H, d, J = 9.0 Hz), 6.30 (1H, d, J = 9.5 Hz), 6.82 (1H, dd, J = 8.7 Hz, 2.4 Hz), 6.89 (1H, d, J = 2.4 Hz), 7.01 (1H, d, J = 7.4 Hz), 7.32 (1H, t, J = 7.8 Hz), 7.51 (1H, d, J = 5.5 Hz), 7.56 (1H, d, J = 8.7 Hz), 7.71 (1H, d, J = 8.0 Hz), 7.77 (1H, d, J = 5.5 Hz), 7.80 (1H, d, J = 9.5 Hz), 11.7 (1H, s).

Example 13 (EX 13)

(4-(Benzo[b]thiophen-4-yl)-1-(4-((2-oxo-1,2-dihydroquinolin-7-yl)oxy)butyl)piperazin-1-ium-1-yl)methyl hydrogen phosphate (1.0 g) was suspended in a mixed solution of ethylene glycol monoisopropyl ether (20 mL) and water (2 mL). WSC•HCl (0.71 g) was added thereto, and the mixture was stirred at 50°C for 7 hours. Acetone and AcOEt were added to the reaction mixture, the mixture was concentrated under reduced pressure, and water was azeotropically

removed by carrying out this three times. The concentrated residue was directly purified twice by medium-pressure liquid chromatography (1st: silica gel, 2nd: amino silica gel, eluent: DCM/MeOH).

The resulting amorphous product was suspended in water (5 mL), and acetone (15 mL) was added for dissolution. After standing at room temperature for 5 days, the precipitated crystals were filtered out and air-dried at 25°C overnight to give (4-(benzo[b]thiophen-4-yl)-1-(4-((2-oxo-1,2-dihydroquinolin-7-yl)oxy)butyl)piperazin-1-ium-1-yl) methyl (2-isopropoxyethyl)phosphate (290 mg).

NMR1; 1.06 (6H, d, J = 6.1 Hz), 1.78-1.88 (2H, m), 1.89-2.00 (2H, m), 3.42-3.48 (4H, m), 3.49-3.68 (4H, m), 3.72-3.81 (4H, m), 4.10 (2H, t, J = 6.0 Hz), 5.10 (2H, d, J = 9.1 Hz), 6.30 (1H, dd, J = 9.4 Hz, 1.4 Hz), 6.82 (1H, dd, J = 8.6 Hz, 2.4 Hz), 6.89 (1H, d, J = 2.4 Hz), 7.01 (1H, d, J = 7.4 Hz), 7.32 (1H, t, J = 7.9 Hz), 7.51 (1H, d, J = 5.5 Hz), 7.56 (1H, d, J = 8.7 Hz), 7.71 (1H, d, J = 8.0 Hz), 7.77 (1H, d, J = 5.5 Hz), 7.80 (1H, d, J = 9.5 Hz), 11.7 (1H, s).

Example 14 (EX 14)

Morpholine (1.2 mL) was added at room temperature to a solution of (4-(benzo[b]thiophen-4-yl)-1-(4-((2-oxo-1,2-dihydroquinolin-7-yl)oxy)butyl)piperazin-1-ium-1-yl)methyl (2-bromoethyl)phosphate (300 mg) in THF (12 mL), and the mixture was stirred at 40°C for 5 hours. The reaction mixture was concentrated, and the residue was purified by silica gel column chromatography (DCM/MeOH). After the resulting product was dissolved in MeCN/$H_2O$ (1/1), MeCN was added. The precipitate was filtered out and washed with MeCN. The resulting product was dried at room temperature to give (4-(benzo[b]thiophen-4-yl)-1-(4-((2-oxo-1,2-dihydroquinolin-7-yl)oxy)butyl)piperazin-1-ium-1-yl) methyl (2-morpholinoethyl)phosphate (164 mg).

NMR1; 1.78-1.89 (2H, m), 1.89-2.01 (2H, m), 2.34-2.41 (4H, m), 2.46 (2H, t, J = 6.1 Hz), 3.42-3.49 (4H, m), 3.50-3.68 (8H, m), 3.71-3.85 (4H, m), 4.10 (2H, t, J = 6.1 Hz), 5.10 (2H, d, J = 9.1 Hz), 6.30 (1H, d, J = 9.4 Hz), 6.82 (1H, dd, J = 2.4, 8.7 Hz), 6.89 (1H, d, J = 2.3 Hz), 7.01 (1H, d, J = 7.4 Hz), 7.32 (1H, t, J = 7.8 Hz), 7.51 (1H, d, J = 5.5 Hz), 7.56 (1H, d, J = 8.7 Hz), 7.71 (1H, d, J = 8.0 Hz), 7.77 (1H, d, J = 5.6 Hz), 7.80 (1H, d, J = 9.1 Hz), 11.68 (1H, s).

Example 15 (EX 15)

Pyridine (4 mL) and acetic anhydride (2 mL) were added at room temperature to (4-(benzo[b]thiophen-4-yl)-1-(4-((2-oxo-1,2-dihydroquinolin-7-yl)oxy)butyl)piperazin-1-ium-1-yl)methyl (3-hydroxypropyl) phosphate (200 mg), and the mixture was stirred overnight. The reaction mixture was concentrated and azeotroped with toluene. The residue was purified by silica gel column chromatography (DCM/MeOH). EtOH/$H_2O$ (1/1) was added thereto, the mixture was dispersed and washed at 40°C, and the insoluble solid was filtered out. The resulting product was washed with water and dried at room temperature to give 3-acetoxypropyl ((4-(benzo[b]thiophen-4-yl)-1-(4-((2-oxo-1,2-dihydroquinolin-7-yl)oxy) butyl)piperazin-1-ium-1-yl)methyl)phosphate (126 mg). NMR3; 1.92-2.05 (7H, m), 2.04-2.16 (2H, m), 3.45-3.56 (4H, m), 3.64-3.73 (2H, m), 3.74-3.83 (2H, m), 3.86-3.93 (2H, m), 4.01 (2H, q, J = 6.2 Hz), 4.15-4.25 (4H, m), 4.52-4.65 (4H, m), 5.20( 2H, d, J = 8.1 Hz), 6.44 (1H, d, J = 9.4 Hz), 6.89 (1H, d, J = 2.4 Hz), 6.92 (1H, dd, J = 2.4, 8.7 Hz), 7.05 (1H, d, J = 7.7 Hz), 7.30 (1H, t, J = 7.9 Hz), 7.49 (1H, dd, J = 0.8, 5.6 Hz), 7.57 (1H, d, J = 5.2 Hz), 7.59 (1H, d, J = 2.0 Hz), 7.65 (1H, d, J = 8.1 Hz), 7.88 (1H, d, J = 9.4 Hz).

Example 16 (EX 16)

Chloromethyl hexanoate (494 mg) was added under stirring to a mixture of (4-(benzo[b]thiophen-4-yl)-1-(4-((2-oxo-1,2-dihydroquinolin-7-yl)oxy)butyl)piperazin-1-ium-1-yl)methyl hydrogen phosphate (632 mg), acetone (6 mL), water (2 mL), and DIPEA (0.524 mL), and the mixture was stirred at reflux. After 8 hours, the mixture was cooled to room temperature, and acetone (2 mL) was then added, followed by stirring. The precipitated solid was filtered out. The resulting product was washed with acetone/water (3:1) and then washed with acetone to obtain a crude product. Purification was performed by silica gel column chromatography (DCM/MeOH). Acetone (3 mL) and water (1 mL) were added, and the precipitated solid was filtered out. The resulting product was washed with acetone and dried to give (4-(benzo[b]thiophen-4-yl)-1-(4-((2-oxo-1,2-dihydroquinolin-7-yl)oxy)butyl)piperazin-1-ium-1-yl)methyl ((hexanoyloxy) methyl)phosphate (269 mg).

NMR1; 0.79 (3H, t, J = 6.9 Hz), 1.12-1.27 (4H, m), 1.42-1.53 (2H, m), 1.78-1.89 (2H, m), 1.89-2.01 (2H, m), 2.30 (2H, t, J = 7.5 Hz), 3.40-3.52 (4H, m), 3.52-3.70 (4H, m), 3.70-3.82 (2H, m), 4.09 (2H, t, J = 6.0 Hz), 5.09 (2H, d, J = 8.9 Hz), 5.42 (2H, d, J = 12.7 Hz), 6.30 (1H, dd, J = 1.9 Hz, 9.4 Hz), 6.82 (1H, dd, J = 2.4 Hz, 8.6 Hz), 6.86 (1H, d, J = 2.3 Hz), 7.01 (1H, d, J = 7.4 Hz), 7.32 (1H, dd, J = 7.9 Hz, 7.9 Hz), 7.52 (1H, dd, J = 0.6 Hz, 5.5 Hz), 7.56 (1H, d, J = 8.6 Hz), 7.72 (1H, d, J = 8.1 Hz), 7.77 (1H, d, J = 5.5 Hz), 7.81 (1H, d, J = 9.4 Hz), 11.64 (1H, s).

Example 17 (EX 17)

Chloromethyl decanoate (220 mg) was added under stirring to a mixture of (4-(benzo[b]thiophen-4-yl)-1-(4-((2-oxo-1,2-dihydroquinolin-7-yl)oxy)butyl)piperazin-1-ium-1-yl)methyl hydrogen phosphate (630 mg), acetone (6 mL), water (3 mL), and DIPEA (0.522 mL), and the mixture was stirred at reflux. After 1 hour, chloromethyl decanoate (220 mg) was added. After another 1 hour, chloromethyl decanoate (220 mg) was added. After stirring for another 2 hours, acetone (3 mL) was added. After another 3 hours, DIPEA (0.348 mL) and chloromethyl decanoate (220 mg) were added. After another 1 hour, chloromethyl decanoate (220 mg) was added. After another 1.5 hours, heating was stopped, and the mixture was cooled to room temperature and stirred for eight nights. After stirring at reflux for 2 hours, the mixture was cooled to room temperature and stirred for another 1 hour. After acetone (3 mL) was added, the precipitated solid was filtered out. The resulting product was washed with acetone/water (3:1) and then washed with acetone to obtain a crude product. Acetone (5 mL) and water (1 mL) were added thereto, and the mixture was stirred at reflux for 30 minutes. After cooling to room temperature, the precipitated solid was filtered out, washed with acetone/water (3:1) and then with acetone, and dried to give (4-(benzo[b]thiophen-4-yl)-1-(4-((2-oxo-1,2-dihydroquinolin-7-yl)oxy)butyl)piperazin-1-ium-1-yl)methyl ((decanoyloxy)methyl)phosphate (370 mg).

NMR1; 0.82 (3H, t, J = 6.9 Hz), 1.11-1.26 (12H, m), 1.41-1.51 (2H, m), 1.78-1.89 (2H, m), 1.89-2.01 (2H, m), 2.30 (2H, t, J = 7.5 Hz), 3.42-3.50 (4H, m), 3.53-3.69 (4H, m), 3.71-3.82 (2H, m), 4.09 (2H, t, J = 6.0 Hz), 5.09 (2H, d, J = 8.9 Hz), 5.42 (2H, d, J = 12.8 Hz), 6.30 (1H, dd, J = 1.8 Hz, 9.5 Hz), 6.82 (1H, dd, J = 2.4 Hz, 8.6 Hz), 6.85 (1H, d, J = 2.3 Hz), 7.01 (1H, d, J = 7.4 Hz), 7.32 (1H, dd, J = 7.9 Hz, 7.9 Hz), 7.52 (1H, dd, J = 0.42 Hz, 5.5 Hz), 7.56 (1H, d, J = 8.6 Hz), 7.71 (1H, d, J = 8.1 Hz), 7.77 (1H, d, J = 5.5 Hz), 7.80 (1H, d, J = 9.5 Hz), 11.63 (1H, s).

Example 18 (EX 18)

Chloromethyl cyclohexane carboxylate (530 mg) was added under stirring to a mixture of (4-(benzo[b]thiophen-4-yl)-1-(4-((2-oxo-1,2-dihydroquinolin-7-yl)oxy)butyl)piperazin-1-ium-1-yl)methyl hydrogen phosphate (632 mg), acetone (6 mL), water (2 mL), and DIPEA (0.524 mL), and the mixture was stirred at reflux. After 8 hours, the mixture was cooled to room temperature, and acetone (2 mL) was then added, followed by stirring. The precipitated solid was filtered out, and the resulting product was washed with acetone/water (3:1) and then washed with acetone to obtain a crude product. Purification was performed by silica gel column chromatography (DCM/MeOH). Acetone (3 mL) and water (1 mL) were added thereto, and the precipitated solid was filtered out. The resulting product was washed with acetone and dried to give (4-(benzo[b]thiophen-4-yl)-1-(4-((2-oxo-1,2-dihydroquinolin-7-yl)oxy)butyl)piperazin-1-ium-1-yl)methyl (((cyclohexanecarbonyl)oxy)methyl)phosphate (392 mg).

NMR1; 1.05-1.39 (5H, m), 1.46-1.55 (1H, m), 1.56-1.66 (2H, m), 1.75-1.89 (4H, m), 1.89-2.00 (2H, m), 2.23-2.34 (1H, m), 3.40-3.50 (4H, m), 3.52-3.70 (4H, m), 3.70-3.82 (2H, m), 4.09 (2H, t, J = 6.0 Hz), 5.10 (2H, d, J = 9.1 Hz), 5.42 (2H, d, J = 12.5 Hz), 6.30 (1H, dd, J = 1.8 Hz, 9.4 Hz), 6.82 (1H, dd, J = 2.4 Hz, 8.6 Hz), 6.86 (1H, d, J = 2.4 Hz), 7.01 (1H, d, J = 7.4 Hz), 7.32 (1H, dd, J = 7.9 Hz, 7.9 Hz), 7.52 (1H, dd, J = 0.4 Hz, 5.6 Hz), 7.56 (1H, d, J = 8.7 Hz), 7.72 (1H, d, J = 8.1 Hz), 7.77 (1H, d, J = 5.5 Hz), 7.81 (1H, d, J = 9.5 Hz), 11.65 (1H, s).

Example 19 (EX 19)

Chloromethyl cyclohexyl carbonate (578 mg) was added under stirring to a mixture of (4-(benzo[b]thiophen-4-yl)-1-(4-((2-oxo-1,2-dihydroquinolin-7-yl)oxy)butyl)piperazin-1-ium-1-yl)methyl hydrogen phosphate (632 mg), acetone (6 mL), water (2 mL), and DIPEA (0.524 mL), and the mixture was stirred at reflux. After 8 hours, the mixture was cooled to room temperature, and acetone (2 mL) was then added, followed by stirring. The precipitated solid was filtered out and washed with acetone/water (3:1) and then with acetone to obtain a crude product. Purification was performed by silica gel column chromatography (DCM/MeOH). Acetone (3 mL) and water (1 mL) were added thereto, and the precipitated solid was filtered out. The resulting product was washed with acetone and dried to give (4-(benzo[b]thiophen-4-yl)-1-(4-((2-oxo-1,2-dihydroquinolin-7-yl)oxy)butyl)piperazin-1-ium-1-yl)methyl ((((cyclohexyloxy)carbonyl)oxy)methyl)phosphate (296 mg).

NMR1; 1.04-1.17 (1H, m), 1.18-1.37 (4H, m), 1.37-1.47 (1H, m), 1.52-1.63 (2H, m), 1.75-1.89 (4H, m), 1.89-2.00 (2H, m), 3.42-3.50 (4H, m), 3.53-3.70 (4H, m), 3.70-3.81 (2H, m), 4.09 (2H, t, J = 6.0 Hz), 4.47-4.55 (1H, m), 5.09 (2H, d, J = 8.5 Hz), 5.43 (2H, d, J = 13.3 Hz), 6.30 (1H, dd, J = 1.9 Hz, 9.5 Hz), 6.83 (1H, dd, J = 2.4 Hz, 8.6 Hz), 6.85 (1H, d, J = 2.3 Hz), 7.00 (1H, d, J = 7.4 Hz), 7.32 (1H, dd, J = 7.8 Hz, 7.8 Hz), 7.52 (1H, dd, J = 0.4 Hz, 5.6 Hz), 7.56 (1H, d, J = 8.6 Hz), 7.72 (1H, d, J = 8.0 Hz), 7.77 (1H, d, J = 5.5 Hz), 7.81 (1H, d, J = 9.5 Hz), 11.63 (1H, s).

Example 20 (EX 20)

NaI (306 mg), $K_2CO_3$ (282 mg), and dibutyl (chloromethyl)phosphate (528 mg) were added to a solution of

7-(4-(4-(benzo[b]thiophen-4-yl)piperazin-1-yl)butoxy)-2-(tert-butoxy)quinoline (500 mg) in MeCN/DCM (30 mL) (2/1). After purging with nitrogen, the mixture was stirred at 40°C overnight under light-shielding with aluminum foil. Dibutyl (chloromethyl)phosphate (132 mg) and NaI (77 mg) were added thereto, and the mixture was stirred at 40°C for 8 hours. DCM and sat. aq. $NaHCO_3$ were added, and extracted with DCM. The organic layer was concentrated, and MeCN (10 mL), water (5 mL), and AcOH (0.5 mL) were added to the residue. The mixture was stirred at room temperature overnight. AcOEt/MeOH=9/1 and water were added to the reaction mixture and extracted. The organic layer was concentrated, and the residue was purified by silica gel column chromatography (DCM/MeOH) to give 4-(benzo[b]thiophen-4-yl)-1-(((dibutoxyphosphoryl)oxy)methyl)-1-(4-((2-oxo-1,2-dihydroquinolin-7-yl)oxy)butyl)piperazin-1-ium iodide (545 mg). NMR2; 0.92 (6H, t, J = 7.4 Hz), 1.33-1.46 (4H, m), 1.64-1.75 (4H, m), 1.94-2.04 (2H, m), 2.13-2.25 (2H m), 3.41-3.60 (4H, m), 3.85-4.18 (8H, m), 4.20 (4H, q, J = 6.6 Hz, 14.0 Hz), 5.75 (2H, d, J = 8.7 Hz), 6.42 (1H, d, J = 9.4 Hz), 6.71 (1H, dd, J = 2.3 Hz, 8.6 Hz), 6.92 (1H, d, J = 7.4 Hz), 7.12 (1H, d, J = 2.2 Hz), 7.19 (1H, t, J = 7.9 Hz), 7.30 (1H, d, J = 8.7 Hz), 7.35-7.42 (2H, m), 7.56 (1H, s), 7.58 (1H, d, J = 2.2 Hz), 11.04 (1H, s).

Reference Example 1 (REX 1)

In a nitrogen atmosphere, 7-(4-(benzo[b]thiophen-4-yl-piperazin-1-yl)butoxy)-1H-quinolin-2-one (15.0 g) and silver carbonate (20.0 g) were suspended in CPME (300 mL) in a 500 mL round-bottom flask. Then, 2-bromo-2-methylpropane (7.84 mL) was added thereto, and the mixture was stirred at 90°C for 9 hours under light-shielding conditions. The insoluble matter was filtered through Celite and washed with AcOEt (150 mL). The solvent was evaporated under reduced pressure, MeCN (180 mL) was added to the residue, and the mixture was stirred. The precipitated crystals were subjected to solid-liquid separation, and the resulting product was washed with MeCN (60 mL) and air-dried at 40°C to give 7-(4-(4-(benzo[b]thiophen-4-yl)piperazin-1-yl)butoxy)-2-(tert-butoxy)quinoline (16.0 g).

NMR2; 1.69 (9H, s), 1.75-1.83 (2H, m), 1.89-1.96 (2H, m), 2.55 (2H, t, J = 7.6 Hz), 2.73 (4H, m), 3.2 (4H, m), 4.15 (2H, t, J = 6.4 Hz), 6.64 (1H, d, J = 7.6 Hz), 6.89 (1H, d, J = 7.6 Hz), 6.99 (1H, dd, J = 8.8 Hz, J = 2.4 Hz), 7.15 (1H, d, J = 2.4 Hz), 7.27 (1H, t, J = 7.6 Hz), 7.38-7.43 (2H, m), 7.53-7.56 (2H, m), 7.82 (1H, d, J = 8.4 Hz).

Reference Example 2 (REX 2)

In a nitrogen atmosphere, 7-(4-(4-(benzo[b]thiophen-4-yl)piperazin-1-yl)butoxy)-2-(tert-butoxy)quinoline (16 g), NaI (9.8 g), and $K_2CO_3$ (9.0 g) were suspended in MeCN (240 mL) in a 1000 mL round-bottom flask, di-tert-butyl (chloromethyl)phosphate (16.9 g) was added thereto, and the mixture was stirred at 25 to 40°C for 2 days. Water (240 mL) was further added and stirred at 45°C for 1 hour. Subsequently, a large portion of the MeCN was distilled off under reduced pressure. The obtained crystals were cooled to 0°C, subjected to solid-liquid separation, washed with MeCN/water (1:4, 80 mL), and vacuum-dried at 40°C for 16 hours. The obtained crystals (19 g) were placed in a 1 L flask, and AcOEt (300 mL) and TEA (1 mL) were added thereto, followed by stirring for 30 minutes. After solid-liquid separation, the resulting product was washed with AcOEt (150 mL) and vacuum-dried at 30°C for 5 hours to give (4-(benzo[b]thiophen-4-yl)-1-(4-((2-(tert-butoxy)quinolin-7-yl)oxy)butyl)piperazin-1-ium-1-yl)methyl tert-butyl phosphate (15.73 g).

NMR3; 1.47 (9H, s), 1.66 (9H, s), 1.95-2.05 (2H, m), 2.10-2.20 (2H, m), 3.40-3.60 (4H, m), 3.65-3.70 (2H, m), 3.70-3.80 (2H, m), 3.85-3.95 (2H, m), 4.24 (2H, t, J = 5.6 Hz), 5.15 (2H, d, J = 8.0 Hz), 6.65 (2H, d, J = 8.8 Hz), 7.00 (1H, dd, J = 8.8 Hz, J = 0.8 Hz), 7.03 (1H, dd, J = 8.8 Hz, J = 2.4 Hz), 7.18 (1H, d, J = 2.4 Hz), 7.26 (1H, t, J = 8.0 Hz), 7.49 (1H, dd, J = 5.6 Hz, J = 0.8 Hz), 7.57-7.65 (3H, m), 7.92 (1H, d, J = 8.8 Hz).

Reference Example 3 (REX 3)

NaH (55% oil) (151 mg) was suspended in THF (10 mL), 7-(4-(4-benzo[b]thiophen-4-yl-piperazin-1-yl)butoxy)-1H-quinolin-2-one (500 mg) was added thereto, chloromethyl hexanoate (570 mg) was added thereto dropwise, and the mixture was stirred at 50°C for 2 hours. After cooling to 0°C, the resulting product was quenched with an aqueous ammonium chloride solution. Extraction was performed with AcOEt, followed by drying over $Na_2SO_4$. The resulting product was purified by medium-pressure silica gel column chromatography to give (7-(4-(4-(benzo[b]thiophen-4-yl)piperazin-1-yl)butoxy)-2-oxoquinolin-1(2H)-yl)methyl hexanoate (217 mg).

NMR2; 0.85 (3H, t, J = 6.8 Hz), 1.25-1.33 (4H, m), 1.58-1.69 (2H, m), 1.70-1.85 (2H, m), 1.85-1.95 (m, 2H), 2.36 (2H, t, J = 7.5 Hz), 2.54 (2H, t, J = 7.4 Hz), 2.67-2.78 (4H, m), 3.15-3.25 (4H, m), 4.08 (2H, t, J = 6.2 Hz), 6.34 (2H, brs), 6.52 (1H, d, J = 9.5 Hz), 6.84 (1H, dd, J = 2.2 Hz, 8.6 Hz), 6.84-6.92 (2H, m), 7.27 (1H, dd, J = 7.8 Hz, 7.8 Hz), 7.37-7.43 (2H, m), 7.45 (1H, d, J = 8.6 Hz), 7.55 (1H, d, J = 8.1 Hz), 7.62 (1H, d, J = 9.5 Hz).

Reference Example 4 (REX 4)

7-(4-(4-(Benzo[b]thiophen-4-yl)piperazin-1-yl)butoxy)quinolin-2(1H)-one (4.0 g) was dissolved in DCM (120

mL). Thereafter, a solution of iodomethyl piperidine-1-carboxylate (2.98 g) in DCM (10 mL) was added at room temperature with stirring, and the mixture was stirred at room temperature for 2 hours. After standing overnight, the resulting product was filtered, washed with DCM, and dried to obtain a crude product. The crude product was recrystallized in DCM/DMF (3/2) (140 mL) to give 4-(benzo[b]thiophen-4-yl)-1-(4-((2-oxo-1,2-dihydroquinolin-7-yl)oxy)butyl)-1-(((piperidine-1-carbonyl)oxy)methyl)piperazin-1-ium iodide (4.06 g).

NMR1; 1.32-1.63 (6H, m), 1.80-2.04 (4H, m), 3.25-3.40 (2H, m), 3.40-3.58 (6H, m), 3.58-3.88 (6H, m), 4.10 (2H, t, J = 5.6 Hz), 5.55 (2H, s), 6.32 (1H, dd, J = 1.4 Hz, 9.5 Hz), 6.74-6.95 (2H, m), 7.04 (1H, d, J = 7.6 Hz), 7.34 (1H, dd, J = 7.8 Hz, 7.8 Hz), 7.54 (1H, d, J = 5.5 Hz), 7.59 (1H, d, J = 9.4 Hz), 7.73 (1H, d, J = 8.1 Hz), 7.79 (1H, d, J = 5.5 Hz), 7.82 (1H, d, J = 9.5 Hz), 11.64 (1H, s).

Reference Example 5 (REX 5)

4-(Benzo[b]thiophen-4-yl)-1-(4-((2-oxo-1,2-dihydroquinolin-7-yl)oxy)butyl)-1-((propionyloxy)methyl)piperazin-1-ium iodide was obtained in the same manner as in Reference Example 4.

NMR1; 1.07 (3H, t, J = 7.4 Hz), 1.71-2.00 (4H, m), 2.56 (2H, q, J = 7.4 Hz), 3.47-3.52 (4H, m), 3.63-3.71 (2H, m), 3.71-3.86 (4H, m), 4.10 (2H, t, J = 5.6 Hz), 5.55 (2H, s), 6.32 (1H, dd, J = 1.5 Hz, 9.4 Hz), 6.77-6.87 (2H, m), 7.03 (1H, d, J = 7.6 Hz), 7.34 (1H, dd, J = 7.9 Hz, 7.9 Hz), 7.53 (1H, d, J = 5.6 Hz), 7.59 (1H, d, J = 8.5 Hz), 7.73 (1H, d, J = 8.0 Hz), 7.78 (1H, d, J = 5.5 Hz), 7.82 (1H, d, J = 9.5 Hz), 11.65 (1H, s).

Reference Example 6 (REX 6)

4-(Benzo[b]thiophen-4-yl)-1-((hexanoyloxy)methyl)-1-(4-((2-oxo-1,2-dihydroquinolin-7-yl)oxy)butyl)piperazin-1-ium iodide was obtained in the same manner as in Reference Example 4.

NMR1; 0.83 (3H, t, J = 6.9 Hz), 1.13-1.31 (4H, m), 1.46-1.58 (2H, m), 1.80-2.02 (4H, m), 2.44-2.55 (2H, m), 3.48-3.54 (4H, m), 3.64-3.88 (6H, m), 4.10 (2H, t, J = 5.5 Hz), 5.57 (2H, s), 6.29-6.37 (1H, m), 6.80-6.88 (2H, m), 7.05 (1H, d, J = 7.5 Hz), 7.35 (1H, dd, J = 7.9 Hz, 7.9 Hz), 7.54( 1H, d, J = 5.5 Hz), 7.60 (1H, d, J = 9.4 Hz), 7.74 (1H, d, J = 8.1 Hz), 7.79 (1H, d, J = 5.5 Hz), 7.84 (1H, d, J = 9.5 Hz), 11.65 (1H, s).

Reference Example 7 (REX 7)

4-(Benzo[b]thiophen-4-yl)-1-((((hexyloxy)carbonyl)oxy)methyl)-1-(4-((2-oxo-1,2-dihydroquinolin-7-yl)oxy)butyl)piperazin-1-ium iodide was obtained in the same manner as in Reference Example 4.

NMR1; 0.85 (3H, t, J = 6.9 Hz), 1.17-1.37 (6H, m), 1.50-1.67 (2H, m), 1.77-2.03 (4H, m), 3.40-3.56 (4H, m), 3.63-3.90 (6H, m), 4.09 (2H, t, J = 5.7 Hz), 4.18 (2H, t, J = 6.7 Hz), 5.61 (2H, s), 6.31 (1H, dd, J = 1.8 Hz, 9.5 Hz), 6.76-6.85 (2H, m), 7.03 (1H, d, J = 7.4 Hz), 7.34 (1H, dd, J = 7.9 Hz, 7.9 Hz), 7.54 (1H, dd, J = 0.4 Hz, 5.5 Hz), 7.58 (1H, d, J = 8.8 Hz), 7.73 (1H, d, J = 8.1 Hz), 7.79 (1H, d, J = 5.5 Hz), 7.82 (1H, d, J = 9.5 Hz), 11.63 (1H, s).

The following tables respectively show the structural formulas of Reference Example compounds (Reference Examples 1 to 7) and Example compounds (Examples 1 to 20) obtained by methods according to those described above.

Table 2A

| REX | STR |
|---|---|
| 1 | 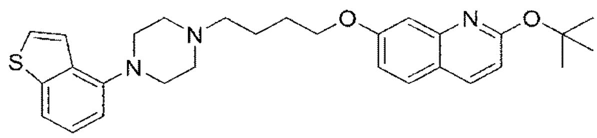 |
| 2 | 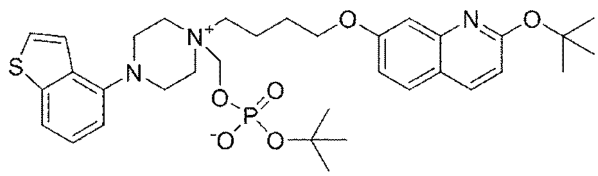 |

(continued)

| REX | STR |
|---|---|
| 3 | 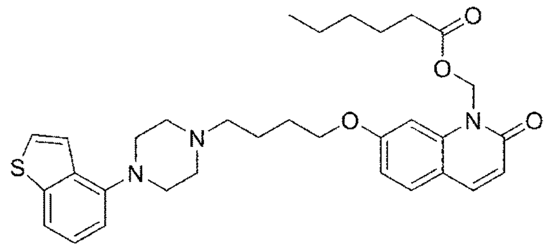 |
| 4 | 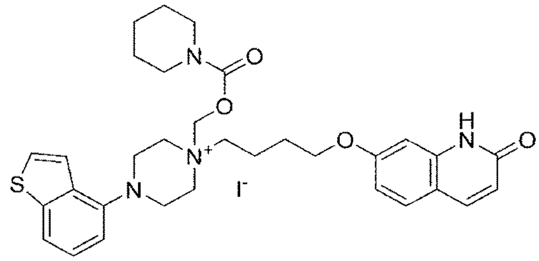 |
| 5 | 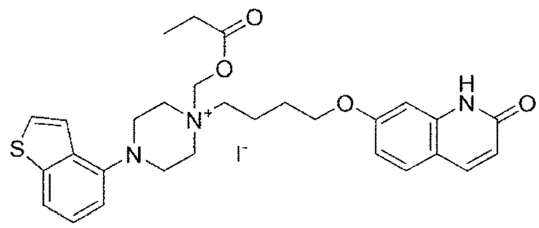 |
| 6 | 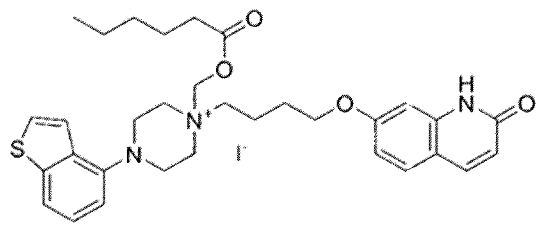 |
| 7 | 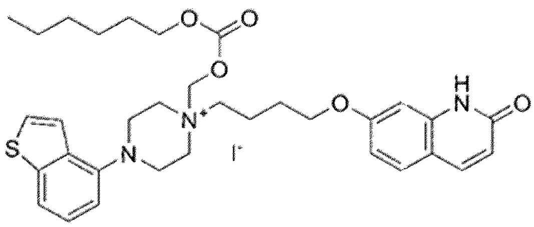 |

Table 2B

| EX | STR |
|---|---|
| 1 | 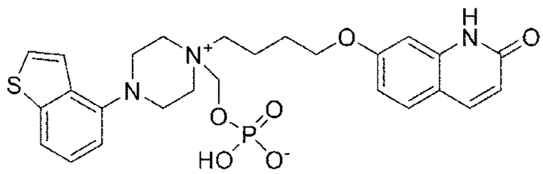 |

(continued)

| EX | STR |
|---|---|
| 2 | 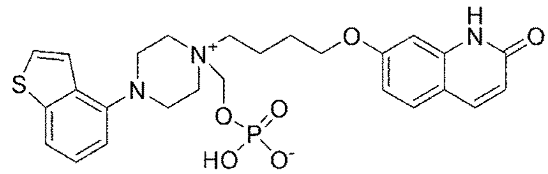 |
| 3 | 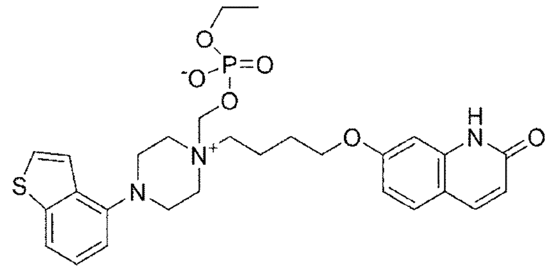 |
| 4 | 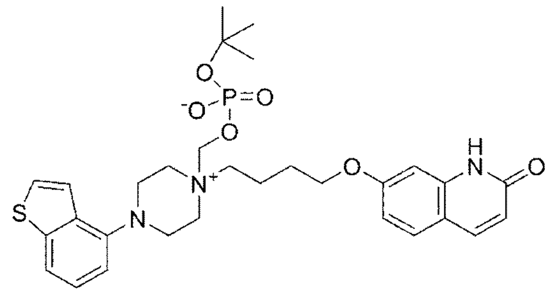 |
| 5 | 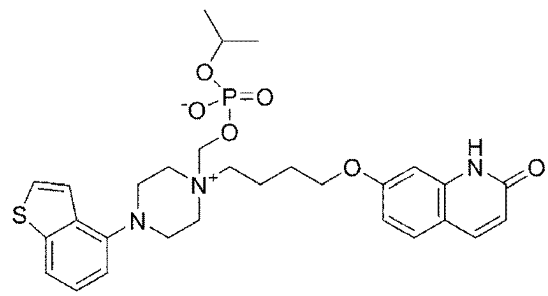 |
| 6 | 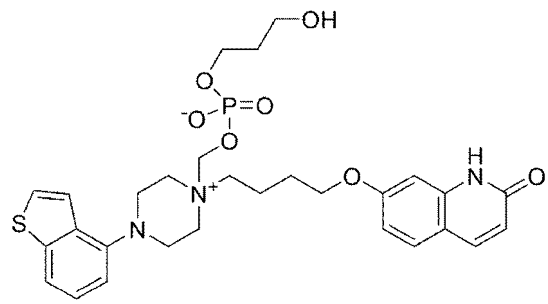 |

(continued)

| EX | STR |
|---|---|
| 7 | 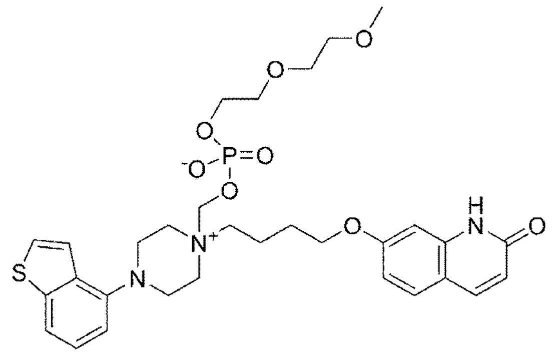 |
| 8 | 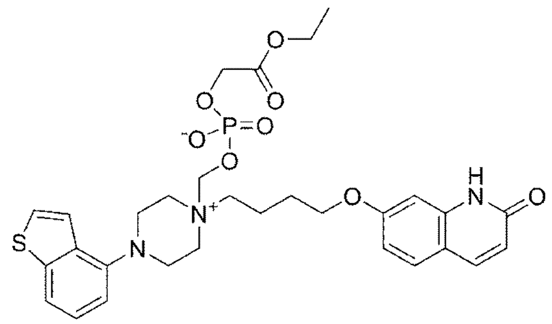 |
| 9 | 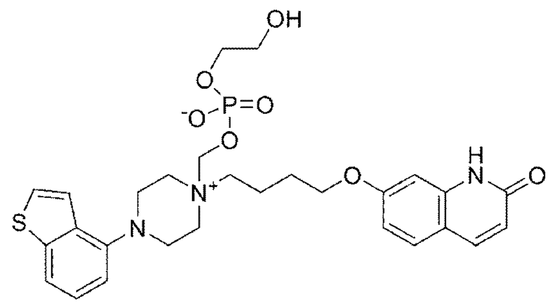 |
| 10 | 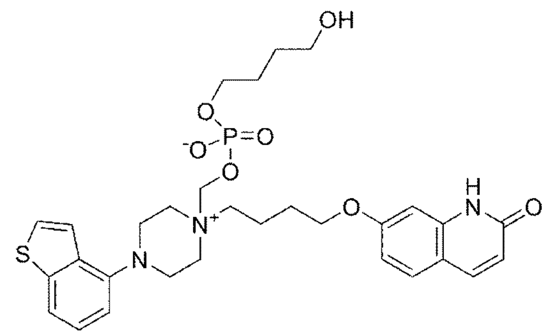 |

(continued)

| EX | STR |
|---|---|
| 11 | 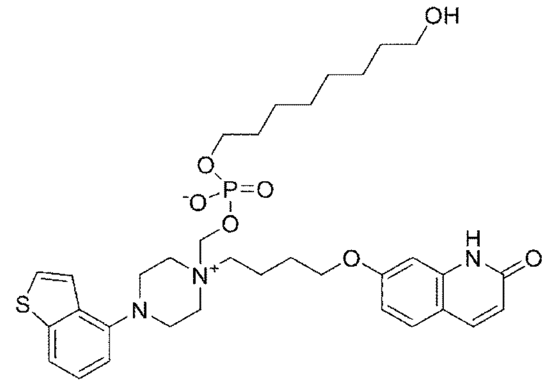 |
| 12 | 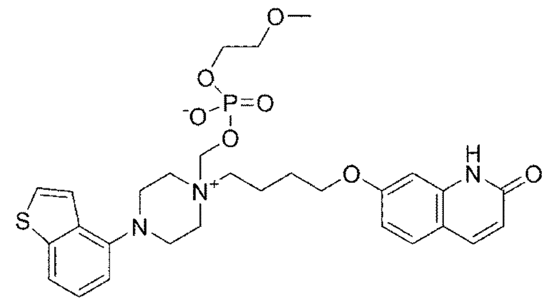 |
| 13 | 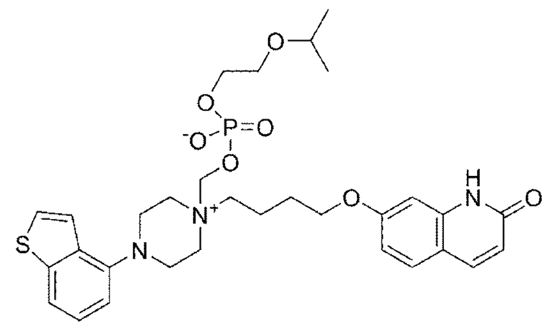 |
| 14 | 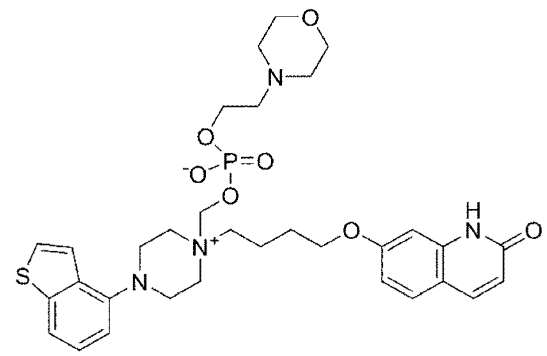 |

(continued)

| EX | STR |
|---|---|
| 15 | 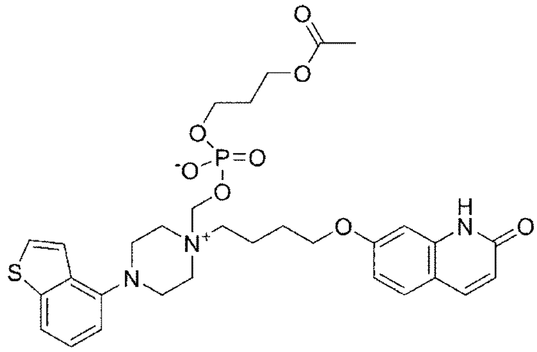 |
| 16 | 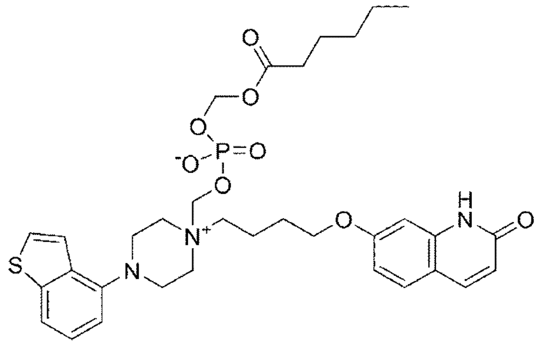 |
| 17 | 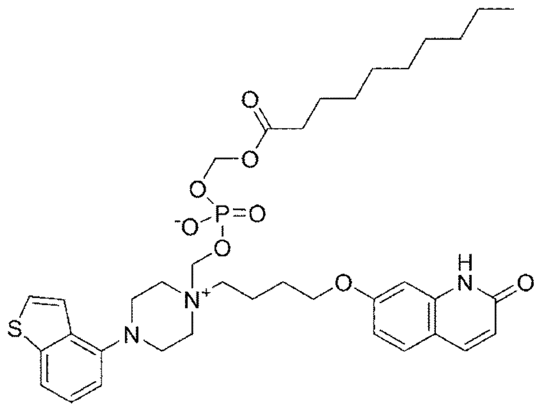 |
| 18 | 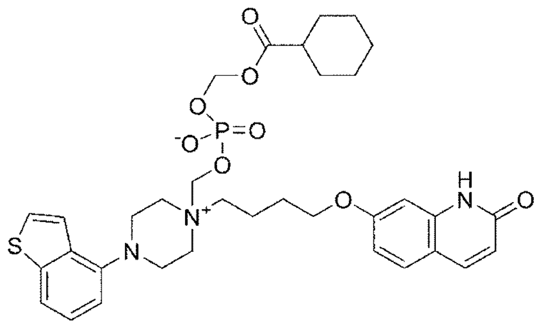 |

(continued)

| EX | STR |
|---|---|
| 19 | 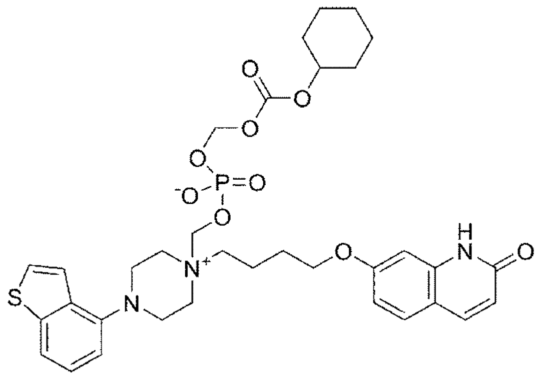 |
| 20 | 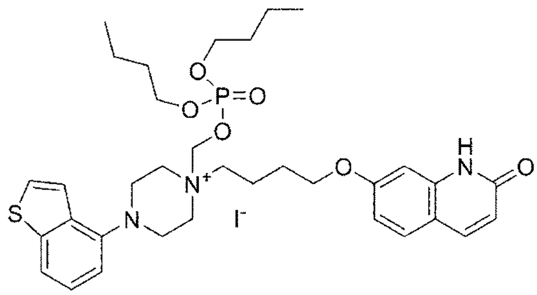 |

The compounds of the Examples other than those listed above were also synthesized and examined. The Example numbers, structural formulas, production methods, and NMR analysis data thereof are listed in the tables below. Each Example number was referred to as "EX N" (wherein N is an integer of 1 to 161), for convenience. The "EX N" may also be shown as "Example N" and the like.

Further, the compounds used in the synthesis of each Example are also listed in the tables below with their numbers, structural formulas, production methods, and NMR analysis data, as Reference Examples 8 to 67. Each Reference Example number was referred to as "REX M" (wherein M is an integer of 8 to 67), for convenience. The "REX M" may also be shown as "Reference Example M" and the like.

Table 3A

| | | | |
|---|---|---|---|
| EX21 | 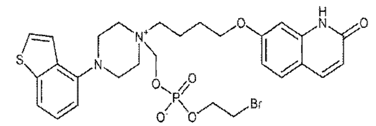 | Ethylene bromohydrin (3 mL), water (0.3 mL), and WSC·HCl (212 mg) were added at room temperature to (4-(benzo[b]thiophen-4-yl)-1-(4-((2-oxo-1,2-dihydroquinolin-7-yl)oxy)butyl)piperazin-1-ium-1-yl)methyl hydrogen phosphate (200 mg) and stirred for 4 hours. WSC·HCl (212 mg) was added and stirred overnight. Toluene was added to the reaction mixture and concentrated. The residue was purified by silica gel column chromatography (DCM/MeOH). (4-(Benzo[b]thiophen-4-yl)-1-(4-((2-oxo-1,2-dihydroquinolin-7-yl)oxy)butyl)piperazin-1-ium-1-yl)methyl (2-bromoethyl) phosphate (103 mg) was obtained. | NMR1; 1.78-1.89(2H, m), 1.89-2.01(2H, m), 3.42-3.49(4H, m), 3.53-3.68(6H, m), 3.71-3.80(2H, m), 3.96-4.05 (2H, m), 4.06-4.14(2H, m), 5.11(2H, d, J=9.0Hz), 6.30(1H, dd, J=2.0Hz, 9.4Hz), 6.83(1H, dd, J=1.6Hz, 9.4Hz), 6.87(1H, d, J=2.3Hz), 7.00(1H, d, J=7.4Hz), 7.32(1H, t, J=7.8Hz), 7.51(1H, d, J=5.5Hz), 7.56(1H, d, J=8.7Hz), 7.71(1H, d, J=8.0Hz), 7.77(1H, d, J=5.6Hz), 7.80(1H, d, J=9.1Hz), 11.65(1H, s). |
| EX22 | 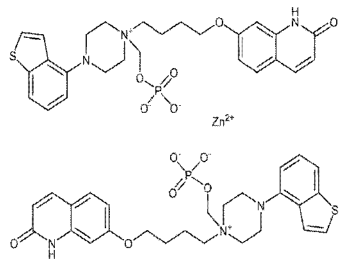 | A mixture of (4-(benzo[b]thiophen-4-yl)-1-(4-((2-oxo-1,2-dihydroquinolin-7-yl)oxy)butyl)piperazin-1-ium-1-yl)methyl hydrogen phosphate (15 g) and water (450 mL) was heated to 50°C. 1N NaOH (23.73 mL) was added, followed by cooling to room temperature. DCM (300 mL) and MeOH (30 mL) were added for liquid separation. It was separated into three phases: an organic phase, an aqueous phase, and an emulsion phase. Water (300 mL), DCM (100 mL), and MeOH (10 mL) were added to the emulsion phase for extraction. This operation was performed three times. All aqueous phases were combined and washed with Et2O (300 mL). On the other hand, zinc chloride (1.520 g) was mixed with water (150 mL) and filtered. The filtrate was slowly added dropwise into the aqueous phase obtained earlier. The mixture was stirred at room temperature for 2 hours. The solid was filtered out and washed with water and then with an acetone/water (5:1) mixed solvent. The wet crystals were dispersed in water (100 mL) and acetone (500 mL), and stirred at room temperature 5 all night. The solid was filtered out, washed with acetone/water (5:1), and then washed with acetone to obtain a white solid. The solid was air-dried at room temperature until a constant weight was reached. Then, the solid was dried under reduced pressure at 40°C to give zinc(II) (4-(benzo[b]thiophen-4-yl)-1-(4-((2-oxo-1,2-dihydroquinolin-7-yl)oxy)butyl)piperazin-1-ium-1-yl)methyl phosphate (11.8 g). | NMR4; 1.92-2.03(2H, m), 2.04-2.20(2H, m), 3.41-3.68(4H, m), 3.68-3.89(4H, m), 3.89-4.05(2H, m), 4.10-4.31(2H, m), 5.44(2H, d, J=6.6Hz), 6.71(1H, d, J=9.4Hz), 6.89(1H, s), 6.93-7.07(2H, m), 7.29(1H, dd, J=7.8Hz, 7.8Hz), 7.48(1H, d, J=5.4Hz), 7.55(1H, d, J=5.5Hz), 7.61(1H, d, J=8.8Hz), 7.66(1H, d, J=8.0Hz), 7.97(1H, d, J=9.3Hz). |
| EX23 | 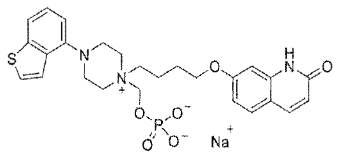 | 1N NaOH (3.32 mL) was added to a mixture of (4-(benzo[b]thiophen-4-yl)-1-(4-((2-oxo-1,2-dihydroquinolin-7-yl)oxy)butyl)piperazin-1-ium-1-yl)methyl hydrogen phosphate (2.0 g) and water (4 mL), followed by stirring at room temperature. Acetone (36 mL) was added thereto in appropriate portions and stirred for about 2 hours. The solid was filtered out, washed with water/acetone (1:5), and then washed with acetone. The solid was air-dried at room temperature to give sodium (4-(benzo[b]thiophen-4-yl)-1-(4-((2-oxo-1,2-dihydroquinolin-7-yl)oxy)butyl)piperazin-1-ium-1-yl)methyl phosphate (1.98 g). | NMR3; 1.90-2.01(2H, m), 2.03-2.14(2H, m), 3.39-3.50(2H, m), 3.50-3.59(2H, m), 3.59-3.73(4H, m), 3.85-3.95(2H, m), 4.20(2H, t, J=6.0Hz), 5.08(2H, d, J=7.2Hz), 6.43(1H, d, J=9.4Hz), 6.87-6.95(2H, m), 7.04(1H, dd, J=0.5Hz, 7.6Hz), 7.28(1H, dd, J=7.9Hz, 7.9Hz), 7.50(1H, dd, J=0.7Hz, 5.6Hz), 7.55(1H, d, J=1.8Hz), 7.58(1H, d, J=2.1Hz), 7.63(1H, d, J=8.1Hz), 7.87(1H, d, J=9.4Hz). |

| | | | |
|---|---|---|---|
| EX24 | 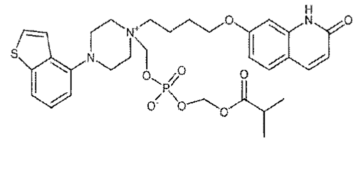 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (632 mg) and chloromethyl isobutyrate (450 mg), (4-(benzo[b]thiophen-4-yl)-1-(4-((2-oxo-1,2-dihydroquinolin-7-yl)oxy)butyl)piperazin-1-ium-1-yl)methyl ((isobutyryloxy)methyl) phosphate (477 mg) was obtained in a method similar to the synthesis method of EX 16. | NMR1; 1.08(6H, d, J=7.0Hz), 1.78-1.89(2H, m), 1.89-2.00(2H, m), 2.51-2.59(1H, m), 3.41-3.50(4H, m), 3.53-3.69(4H, m), 3.71-3.81(2H, m), 4.09(2H, t, J=6.0Hz), 5.10(2H, d, J=9.1Hz), 5.43(2H, d, J=12.5Hz), 6.30(1H, dd, J=1.9Hz, 9.5Hz), 6.82(1H, dd, J=2.4Hz, 8.6Hz), 6.85(1H, d, J=2.3Hz), 7.01(1H, J=7.3Hz), 7.33(1H, dd, J=7.9Hz, 7.9Hz), 7.50-7.59(1H, m), 7.56(1H, d, J=8.6Hz), 7.72(1H, d, J=8.1Hz), 7.77(1H, d, J=5.6Hz), 7.80(1H, d, J=9.5Hz), 11.64(1H, s). |
| EX25 | 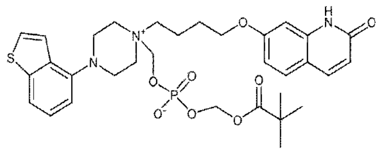 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (632 mg) and chloromethyl pivalate (519 mg), (4-(benzo[b]thiophen-4-yl)-1-(4-((2-oxo-1,2-dihydroquinolin-7-yl)oxy)butyl)piperazin-1-ium-1-yl)methyl ((pivaloyloxy)methyl) phosphate (297 mg) was obtained in a method similar to the synthesis method of EX 16. | NMR1; 1.14(9H, s), 1.78-1.88(2H, m), 1.88-2.00(2H, m), 3.41-3.50(4H, m), 3.53-3.69(4H, m), 3.71-3.81(2H, m), 4.09(2H, t, J=6.0Hz), 5.11(2H, d, J=9.4Hz), 5.43(2H, d, J=12.1Hz), 6.30(1H, dd, J=1.9Hz, 9.4Hz), 6.82(1H, dd, J=2.4Hz, 8.6Hz), 6.85(1H, d, J=2.4Hz), 7.00(1H, J=7.4Hz), 7.32(1H, dd, J=7.9Hz, 7.9Hz), 7.52(1H, dd, J=0.5Hz, 5.6Hz), 7.56(1H, d, J=8.6Hz), 7.72(1H, d, J=8.0Hz), 7.77(1H, d, J=5.5Hz), 7.81(1H, d, J=9.5Hz), 11.64(1H, s). |
| EX26 | 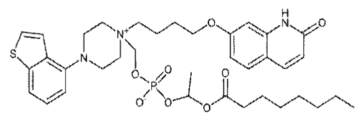 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (632 mg) and 1-chloromethyl octanoate (0.578 g), (4-(benzo[b]thiophen-4-yl)-1-(4-((2-oxo-1,2-dihydroquinolin-7-yl)oxy)butyl)piperazin-1-ium-1-yl)methyl (1-(octanoyloxy)ethyl) phosphate (297 mg) was obtained in a method similar to the synthesis method of EX 16. | NMR1; 0.79(3H, t, J=6.9Hz), 1.07-1.25(8H, m), 1.35(3H, d, J=5.2Hz), 1.40-1.52(2H, m), 1.78-1.89(2H, m), 1.89-2.00(2H, m), 2.26(2H, t, J=7.5Hz), 3.40-3.50(4H, m), 3.52-3.70(4H, m), 3.70-3.82(2H, m), 4.09(2H, t, J=6.0Hz), 5.01-5.14(2H, m), 6.25-6.34(2H, m), 6.82(1H, dd, J=2.3Hz, 8.6Hz), 6.86(1H, d, J=2.2Hz), 7.00(1H, d, J=7.6Hz), 7.32(1H, dd, J=7.8Hz, 7.8Hz), 7.52(1H, d, J=5.4Hz), 7.56(1H, d, J=8.6Hz), 7.72(1H, d, J=8.1Hz), 7.77(1H, d, J=5.6Hz), 7.80(1H, d, J=9.5Hz), 11.64(1H, s). |
| EX27 | 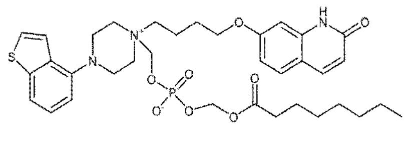 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (632 mg) and chloromethyl octanoate (578 mg), (4-(benzo[b]thiophen-4-yl)-1-(4-((2-oxo-1,2-dihydroquinolin-7-yl)oxy)butyl)piperazin-1-ium-1-yl)methyl ((octanoyloxy)methyl) phosphate (321 mg) was obtained in a method similar to the synthesis method of EX 16. | NMR1; 0.80(3H, t, J=6.9Hz), 1.10-1.25(8H, m), 1.41-1.52(2H, m), 1.78-1.89(2H, m), 1.89-2.00(2H, m), 2.30(2H, t, J=7.5Hz), 3.42-3.50(4H, m), 3.53-3.69(4H, m), 3.71-3.81(2H, m), 4.09(2H, t, J=6.0Hz), 5.09(2H, d, J=8.9Hz), 5.42(2H, d, J=12.7Hz), 6.30(1H, dd, J=1.9Hz, 9.5Hz), 6.82(1H, dd, J=2.4Hz, 8.6Hz), 6.86(1H, d, J=2.3Hz), 7.01(1H, d, J=7.4Hz), 7.32(1H, dd, J=7.9Hz, 7.9Hz), 7.52(1H, dd, J=0.4Hz, 5.6Hz), 7.56(1H, d, J=8.6Hz), 7.72(1H, d, J=8.1Hz), 7.77(1H, d, J=5.5Hz), 7.80(1H, d, J=9.5Hz), 11.63(1H, s). |
| EX28 | 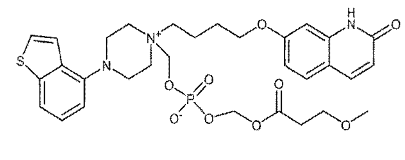 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (2.53 g) and chloromethyl 3-methoxypropanoate (2.03 g), (4-(benzo[b]thiophen-4-yl)-1-(4-((2-oxo-1,2-dihydroquinolin-7-yl)oxy)butyl)piperazin-1-ium-1-yl)methyl (((3-methoxypropanoyl)oxy)methyl) phosphate (947 mg) was obtained in a method similar to the synthesis method of EX 16. | NMR1; 1.78-1.89(2H, m), 1.89-2.00(2H, m), 2.58(2H, t, J=6.1Hz), 3.19(3H, s), 3.41-3.50(4H, m), 3.50-3.69(6H, m), 3.70-3.80(2H, m), 4.09(2H, t, J=6.0Hz), 5.09(2H, d, J=8.9Hz), 5.44(2H, d, J=12.9Hz), 6.30(1H, dd, J=1.9Hz, 9.4Hz), 6.83(1H, dd, J=2.4Hz, 8.6Hz), 6.85(1H, d, J=2.3Hz), 7.01(1H, d, J=7.4Hz), 7.33(1H, dd, J=7.9Hz, 7.9Hz), 7.52(1H, d, J=5.6Hz), 7.56(1H, d, J=8.6Hz), 7.72(1H, d, J=8.1Hz), 7.77(1H, d, J=5.5Hz), 7.81(1H, d, J=9.5Hz), 11.63(1H, s). |

| | | | |
|---|---|---|---|
| EX29 | 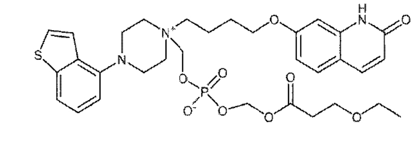 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (2.53 g) and chloromethyl 3-ethoxypropanoate (2.00 g) synthesized in REX 8, (4-(benzo[b]thiophen-4-yl)-1-(4-((2-oxo-1,2-dihydroquinolin-7-yl)oxy)butyl)piperazin-1-ium-1-yl)methyl (((3-ethoxypropanoyl)oxy)methyl) phosphate (1.22 g) was obtained in a method similar to the synthesis method of EX 16. | NMR1; 1.04(3H, t, J=7.0Hz), 1.78-1.89(2H, m), 1.89-2.00(2H, m), 2.57(2H, t, J=6.2Hz), 3.37(2H, q, J=7.0Hz), 3.41-3.50(4H, m), 3.52-3.69(6H, m), 3.70-3.80(2H, m), 4.09(2H, t, J=6.1Hz), 5.09(2H, d, J=9.0Hz), 5.44(2H, d, J=12.9Hz), 6.30(1H, dd, J=1.9Hz, 9.5Hz), 6.83(1H, dd, J=2.4Hz, 8.6Hz), 6.86(1H, d, J=2.3Hz), 7.01(1H, d, J=7.4Hz), 7.32(1H, dd, J=7.9Hz, 7.9Hz), 7.49-7.54(1H, m), 7.56(1H, d, J=8.6Hz), 7.72(1H, d, J=8.1Hz), 7.77(1H, d, J=5.5Hz), 7.81(1H, d, J=9.5Hz), 11.64(1H, s). |
| EX30 | 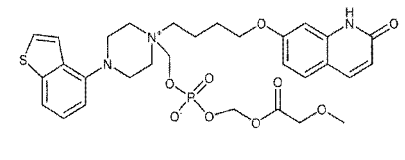 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (1.90 g) and chloromethyl 2-methoxyacetate (1.37 g), (4-(benzo[b]thiophen-4-yl)-1-(4-((2-oxo-1,2-dihydroquinolin-7-yl)oxy)butyl)piperazin-1-ium-1-yl)methyl ((2-methoxyacetoxy)methyl) phosphate (82 mg) was obtained in a method similar to the synthesis method of EX 16. | NMR1; 1.79-1.89(2H, m), 1.89-2.00(2H, m), 3.30(3H, s), 3.41-3.49(4H, m), 3.54-3.69(4H, m), 3.70-3.80(2H, m), 4.05-4.13(4H, m), 5.09(2H, d, J=9.0Hz), 5.49(2H, d, J=13.3Hz), 6.30(1H, dd, J=2.0Hz, 9.5Hz), 6.83(1H, dd, J=2.4Hz, 8.6Hz), 6.86(1H, d, J=2.3Hz), 7.01(1H, d, J=7.6Hz), 7.32(1H, dd, J=7.9Hz, 7.9Hz), 7.51(1H, d, J=5.2Hz), 7.56(1H, d, J=8.6Hz), 7.71(1H, d, J=8.0Hz), 7.77(1H, d, J=5.5Hz), 7.83(1H, d, J=9.5Hz), 11.62(1H, s). |
| EX31 | 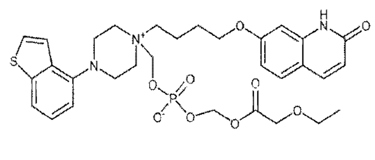 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (3.67 g) and chloromethyl 2-ethoxyacetate (2.66 g), (4-(benzo[b]thiophen-4-yl)-1-(4-((2-oxo-1,2-dihydroquinolin-7-yl)oxy)butyl)piperazin-1-ium-1-yl)methyl ((2-ethoxyacetoxy)methyl) phosphate (304 mg) was obtained in a method similar to the synthesis method of EX 16. | NMR1; 1.08(3H, t, J=7.0Hz), 1.79-1.89(2H, m), 1.89-2.00(2H, m), 3.42-3.51(6H, m), 3.54-3.69(4H, m), 3.70-3.80(2H, m), 4.06-4.13(4H, m), 5.09(2H, d, J=9.0Hz), 5.49(2H, d, J=13.2Hz), 6.30(1H, dd, J=2.0Hz, 9.5Hz), 6.83(1H, dd, J=2.4Hz, 8.6Hz), 6.86(1H, d, J=2.3Hz), 7.01(1H, d, J=7.7Hz), 7.32(1H, dd, J=7.9Hz, 7.9Hz), 7.51(1H, d, J=5.2Hz), 7.56(1H, d, J=8.6Hz), 7.72(1H, d, J=8.1Hz), 7.77(1H, d, J=5.6Hz), 7.80(1H, d, J=9.5Hz), 11.63(1H, s). |
| EX32 | 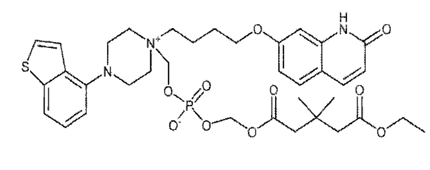 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (632 mg) and 1-(chloromethyl) 5-ethyl 3,3-dimethylpentanedioate (710 mg) synthesized in REX 9, (4-(benzo[b]thiophen-4-yl)-1-(4-((2-oxo-1,2-dihydroquinolin-7-yl)oxy)butyl)piperazin-1-ium-1-yl)methyl (((5-ethoxy-3,3-dimethyl-5-oxopentanoyl)oxy)methyl) phosphate (539 mg) was obtained in a method similar to the synthesis method of EX 16. | NMR1; 1.02(6H, s), 1.13(3H, t, J=7.1Hz), 1.79-1.89(2H, m), 1.89-2.00(2H, m), 2.34(2H, s), 2.40(2H, s), 3.42-3.50(4H, m), 3.53-3.70(4H, m), 3.71-3.81(2H, m), 4.00(2H, q, J=7.1Hz), 4.09(2H, t, J=6.0Hz), 5.10(2H, d, J=9.0Hz), 5.42(2H, d, J=12.5Hz), 6.30(1H, dd, J=1.8Hz, 9.4Hz), 6.82(1H, dd, J=2.4Hz, 8.6Hz), 6.86(1H, d, J=2.3Hz), 7.01(1H, d, J=7.4Hz), 7.32(1H, dd, J=7.9Hz, 7.9Hz), 7.52(1H, dd, J=0.6Hz, 5.6Hz), 7.56(1H, d, J=8.6Hz), 7.72(1H, d, J=8.1Hz), 7.77(1H, d, J=5.5Hz), 7.80(1H, d, J=9.5Hz), 11.64(1H, s). |
| EX33 | 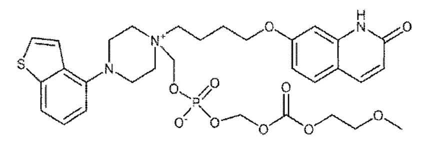 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (632 mg) and chloromethyl (2-methoxyethyl) carbonate (532 mg), (4-(benzo[b]thiophen-4-yl)-1-(4-((2-oxo-1,2-dihydroquinolin-7-yl)oxy)butyl)piperazin-1-ium-1-yl)methyl ((((2-methoxyethoxy)carbonyl)oxy)methyl) phosphate (282 mg) was obtained in a method similar to the synthesis method of EX 19. | NMR1; 1.78-1.89(2H, m), 1.89-2.00(2H, m),3.20(3H, s), 3.41-3.52(6H, m), 3.53-3.69(4H, m), 3.70-3.80(2H, m), 4.09(2H, t, J=6.0Hz), 4.18-4.23(2H, m), 5.08(2H, d, J=8.6Hz), 5.45(2H, d, J=13.5Hz), 6.30(1H, dd, J=1.9Hz, 9.4Hz), 6.80-6.87(2H, m), 7.01(1H, d, J=7.4Hz), 7.33(1H, dd, J=7.9Hz, 7.9Hz), 7.52(1H, dd, J=0.4Hz, 5.5Hz), 7.56(1H, d, J=8.5Hz), 7.72(1H, d, J=8.1Hz), 7.77(1H, d, J=5.5Hz), 7.81(1H, d, J=9.5Hz), 11.63(1H, s). |

| | | | |
|---|---|---|---|
| EX34 | 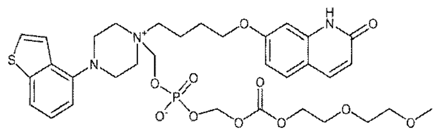 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (632 mg) and chloromethyl (2-(2-methoxyethoxy)ethyl) carbonate (701 mg), (4-(benzo[b]thiophen-4-yl)-1-(4-((2-oxo-1,2-dihydroquinolin-7-yl)oxy)butyl)piperazin-1-ium-1-yl)methyl (3-oxo-2,4,7,10-tetraoxaundecyl) phosphate (312 mg) was obtained in a method similar to the synthesis method of EX 19. | NMR1; 1.79-1.89(2H, m), 1.89-2.00(2H, m), 3.01(3H, s), 3.36-3.40(2H, m), 3.42-3.51(6H, m), 3.53-3.69(6H, m), 3.70-3.81(2H, m), 4.10(2H, t, J=5.9Hz), 4.16-4.23(2H, m), 5.09(2H, d, J=8.5Hz), 5.45(2H, d, J=13.5Hz), 6.30(1H, dd, J=1.7Hz, 9.5Hz), 6.80-6.87(2H, m), 7.01(1H, d, J=7.6Hz), 7.33(1H, dd, J=7.8Hz, 7.8Hz), 7.52(1H, d, J=5.6Hz), 7.56(1H, d, J=8.5Hz), 7.72(1H, d, J=8.0Hz), 7.77(1H, d, J=5.5Hz), 7.81(1H, d, J=9.5Hz), 11.63(1H, s). |
| EX35 | 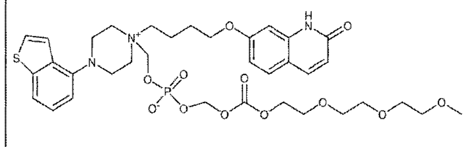 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (632 mg) and chloromethyl (2-(2-(2-methoxyethoxy)ethoxy)ethyl) carbonate (802 mg), (4-(benzo[b]thiophen-4-yl)-1-(4-((2-oxo-1,2-dihydroquinolin-7-yl)oxy)butyl)piperazin-1-ium-1-yl)methyl (3-oxo-2,4,7,10,13-pentaoxatetradecyl) phosphate (378 mg) was obtained in a method similar to the synthesis method of EX 19. | NMR1; 1.79-1.89(2H, m), 1.89-2.00(2H, m), 3.20(3H, s), 3.36-3.41(2H, m), 3.42-3.50(10H, m), 3.53-3.69(6H, m), 3.70-3.80(2H, m), 4.10(2H, t, J=6.0Hz), 4.17-4.23(2H, m), 5.09(2H, d, J=8.5Hz), 5.45(2H, d, J=13.5Hz), 6.30(1H, dd, J=1.8Hz, 9.4Hz), 6.80-6.87(2H, m), 7.01(1H, d, J=7.6Hz), 7.33(1H, dd, J=7.9Hz, 7.9Hz), 7.52(1H, d, J=5.6Hz), 7.56(1H, d, J=8.6Hz), 7.72(1H, d, J=8.1Hz), 7.77(1H, d, J=5.5Hz), 7.81(1H, d, J=9.5Hz), 11.63(1H, s). |
| EX36 | 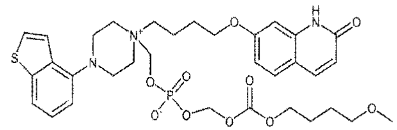 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (2.53 g) and chloromethyl (4-methoxybutyl) carbonate (2.36 g) synthesized in REX 10, (4-(benzo[b]thiophen-4-yl)-1-(4-((2-oxo-1,2-dihydroquinolin-7-yl)oxy)butyl)piperazin-1-ium-1-yl)methyl ((((4-methoxybutoxy)carbonyl)oxy)methyl) phosphate (1.38 g) was obtained in a method similar to the synthesis method of EX 19. | NMR1; 1.42-1.52(2H, m), 1.52-1.63(2H, m), 1.78-1.89(2H, m), 1.89-2.00(2H, m),3.16(3H, s), 3.23(2H, t, J=6.2Hz), 3.40-3.50(4H, m), 3.53-3.70(4H, m), 3.71-3.81(2H, m), 4.04-4.13(4H, m), 5.09(2H, d, J=8.6Hz), 5.44(2H, d, J=13.4Hz), 6.30(1H, dd, J=1.9Hz, 9.5Hz), 6.82(1H, dd, J=2.4Hz, 8.5Hz), 6.85(1H, d, J=2.3Hz), 7.01(1H, d, J=7.4Hz), 7.32(1H, dd, J=7.9Hz, 7.9Hz), 7.52(1H, dd, J=0.4Hz, 5.5Hz), 7.56(1H, d, J=8.6Hz), 7.72(1H, d, J=8.1Hz), 7.77(1H, d, J=5.6Hz), 7.81(1H, d, J=9.5Hz), 11.63(1H, s). |
| EX37 | 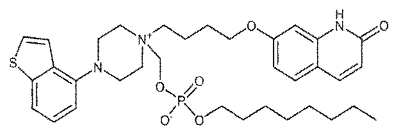 | 1-Octanol (5 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g) were mixed, and water (0.5 mL) was added and stirred. Then, WSC·HCl (0.53 g) and acetone (2 mL) were added, and the mixture was heated and stirred for 6 hours. Water and ethyl acetate were added to the reaction mixture, followed by stirring, and the precipitated solid was filtered out and purified by silica gel column chromatography (eluent: DCM-MeOH). The resulting product was dissolved in water, and acetone was added for crystallization. The resulting crystals were air-dried at 25°C to give (4-(benzo[b]thiophen-4-yl)-1-(4-((2-oxo-1,2-dihydroquinolin-7-yl)oxy)butyl)piperazin-1-ium-1-yl)methyl octyl phosphate (81 mg). | NMR1; 0.82 (3H, t, J = 6.7Hz), 1.14-1.29 (10H, m), 1.44-1.53 (2H, m) 1.78-1.88 (2H, m), 1.89-1.99 (2H, m), 3.40-3.50 (4H, m), 3.51-3.70 (8H, m), 4.10 (2H, t, J = 6.1Hz), 5.10 (2H, d, J = 9.0Hz), 6.30 (1H, dd, J = 9.5Hz, 1.8Hz), 6.82 (1H, dd, J = 8.6Hz, 2.4Hz), 6.89 (1H, d, J = 2.4Hz), 7.01 (1H, d, J = 7.4Hz), 7.32 (1H, t, J = 7.8Hz), 7.51 (1H, d, J = 5.5Hz), 7.56 (1H, d, J = 8.6Hz), 7.71 (1H, d, J = 8.0Hz), 7.77 (1H, d, J = 5.5Hz), 7.80 (1H, d, J = 9.5Hz), 11.7 (1H, s) . |

| | | | |
|---|---|---|---|
| EX38 | 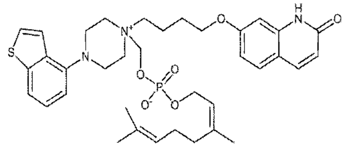 | Using nerol (5 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (Z)-(4-(benzo[b]thiophen-4-yl)-1-(4-((2-oxo-1,2-dihydroquinolin-7-yl)oxy)butyl)piperazin-1-ium-1-yl)methyl (3,7-dimethylocta-2,6-dien-1-yl) phosphate (82 mg) was obtained in a method similar to the synthesis of EX 45. | NMR1; 1.53 (3H, s) , 1.61 (3H, s), 1.67 (3H, s), 1.60-1.79 (2H, m), 1.80-2.02 (6H, m), 3.40-3.49 (4H, m), 3.53-3.68 (4H, m), 3.72-3.80 (2H, m), 4.10 (2H, t, J = 6.0Hz), 4.24 (2H, t, J = 7.0Hz), 5.04 (1H, br-s), 5.08 (2H, d, J = 9.0Hz), 5.31 (2H, t, J = 6.8Hz), 6.30 (1H, d, J = 9.5Hz), 6.82 (1H, dd, J = 8.6Hz, 2.4Hz), 6.89 (1H, d, J = 2.4Hz), 7.01 (1H, d, J = 7.4Hz), 7.32 (1H, t, J = 7.8Hz), 7.51 (1H, d, J = 5.5Hz), 7.56 (1H, d, J = 8.7Hz), 7.71 (1H, d, J = 8.0Hz), 7.77 (1H, d, J = 5.5Hz), 7.80 (1H, d, J = 9.5Hz), 11.7 (1H, s) . |
| EX39 | 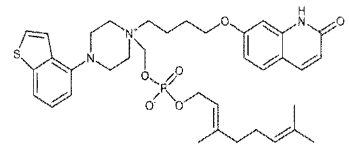 | Using geraniol (5 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (E)-(4-(benzo[b]thiophen-4-yl)-1-(4-((2-oxo-1,2-dihydroquinolin-7-yl)oxy)butyl)piperazin-1-ium-1-yl)methyl (3,7-dimethylocta-2,6-dien-1-yl) phosphate (82 mg) was obtained in a method similar to the synthesis of EX 45. | NMR1; 1.54 (3H, s), 1.60 (3H, s), 1.61 (3H, s), 1.80-1.88 (2H, m), 1.89-1.98 (4H, m), 1.99-2.07 (2H, m), 3.40-3.50 (4H, m), 3.53-3.70 (4H, m), 3.72-3.80 (2H, m), 4.10 (2H, t, J = 6.2Hz), 4.26 (2H, t, J = 6.9Hz), 5.04 (1H, t, J = 6.8Hz), 5.09 (2H, d, J = 9.0Hz), 5.30 (2H, t, J = 6.7Hz), 6.30 (1H, d, J = 9.5Hz), 6.82 (1H, dd, J = 8.6Hz, 2.4Hz), 6.89 (1H, d, J = 2.4Hz), 7.01 (1H, d, J = 7.4Hz), 7.32 (1H, t, J = 7.8Hz), 7.51 (1H, d, J = 5.5Hz), 7.56 (1H, d, J = 8.7Hz), 7.71 (1H, d, J = 8.0Hz), 7.77 (1H, d, J = 5.5Hz), 7.81 (1H, d, J = 9.5Hz), 11.7 (1H, s) . |
| EX40 | 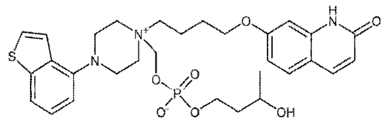 | Using 1,3-butanediol (5 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzo[b]thiophen-4-yl)-1-(4-((2-oxo-1,2-dihydroquinolin-7-yl)oxy)butyl)piperazin-1-ium-1-yl)methyl (3-hydroxybutyl) phosphate (110 mg) was obtained in a method similar to the synthesis of EX 45. | NMR1; 1.05 (2.5H, d, J = 6.2Hz), 1.17 (0.5H, d, J = 6.2Hz), 1.42-1.50 (1H, m), 1.52-1.63 (1H, m), 1.78-1.89 (2H, m), 1.90-2.00 (2H, m), 3.40-3.50 (4H, m), 3.52-3.69 (4H, m), 3.70-3.79 (2H, m), 3.80-3.88 (1H, m), 4.10 (2H, t, J = 6.1Hz), 4.80 (1H, d, J = 4.6Hz), 5.09 (2H, d, J = 9.0Hz), 6.29 (1H, dd, J = 9.5Hz, 1.5Hz), 6.82 (1H, dd, J = 8.6Hz, 2.4Hz), 6.88 (1H, d, J = 2.4Hz), 7.01 (1H, d, J = 7.4Hz), 7.32 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.80 (1H, d, J = 9.5Hz), 11.7 (1H, s). |
| EX41 | 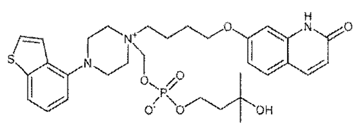 | Using 3-methylbutane-1,3-diol (5 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzo[b]thiophen-4-yl)-1-(4-((2-oxo-1,2-dihydroquinolin-7-yl)oxy)butyl)piperazin-1-ium-1-yl)methyl (3-hydroxy-3-methylbutyl) phosphate (320 mg) was obtained in a method similar to the synthesis of EX 45. | NMR1; 1.09 (6H, s), 1.66 (2H, t, J = 7.4Hz), 1.78-1.89 (2H, m), 1.90-1.99 (2H, m), 3.41-3.48 (4H, m), 3.55-3.68 (4H, m), 3.72-3.81 (2H, m), 3.83 (2H, q, J = 7.4Hz), 4.10 (2H, t, J = 5.9Hz), 4.34(1H, s), 5.10 (2H, d, J = 9.1Hz), 6.30 (1H, d, J = 9.5Hz), 6.83 (1H, dd, J = 8.6Hz, 2.4Hz), 6.89 (1H, d, J = 2.4Hz), 7.01 (1H, d, J = 7.4Hz), 7.32 (1H, t, J = 7.8Hz), 7.51 (1H, d, J = 5.5Hz), 7.56 (1H, d, J = 8.7Hz), 7.71 (1H, d, J = 8.0Hz), 7.77 (1H, d, J = 5.5Hz), 7.81 (1H, d, J = 9.5Hz), 11.7 (1H, s) . |
| EX42 | 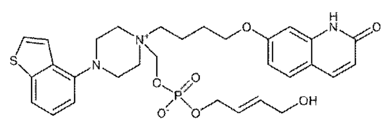 | Using trans-2-butene-1,4-diol (2.5 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (E)-(4-(benzo[b]thiophen-4-yl)-1-(4-((2-oxo-1,2-dihydroquinolin-7-yl)oxy)butyl)piperazin-1-ium-1-yl)methyl (4-hydroxybut-2-en-1-yl) phosphate (200 mg) was obtained in a method similar to the synthesis of EX 45. | NMR1; 1.79-1.88 (2H, m), 1.89-1.98 (2H, m), 3.40-3.50 (4H, m), 3.53-3.68 (4H, m), 3.70-3.80 (2H, m), 3.92 (2H, t, J = 5.3Hz), 4.10 (2H, t, J = 6.1Hz), 4.20-4.26 (2H, m), 4.72 (1H, d, J = 5.5Hz), 5.09 (2H, d, J = 8.9Hz), 6.30 (1H, dd, J = 9.5Hz, 1.6Hz), 6.82 (1H, dd, J = 8.6Hz, 2.4Hz), 6.89 (1H, d, J = 2.4Hz), 7.01 (1H, d, J = 7.5Hz), 7.32 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.81 (1H, d, J = 9.5Hz), 11.7 (1H, s). |

| | | | |
|---|---|---|---|
| EX43 | 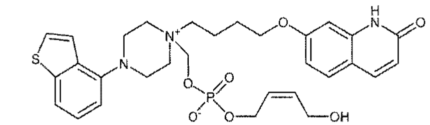 | Using cis-2-butene-1,4-diol (10 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (2.0 g), (Z)-(4-(benzo[b]thiophen-4-yl)-1-(4-((2-oxo-1,2-dihydroquinolin-7-yl)oxy)butyl)piperazin-1-ium-1-yl)methyl (4-hydroxybut-2-en-1-yl) phosphate (990 mg) was obtained in a method similar to the synthesis of EX 45. | NMR1; 1.80-1.88 (2H, m), 1.89-2.00 (2H, m), 3.40-3.50 (4H, m), 3.52-3.69 (4H, m), 3.71-3.80 (2H, m), 3.96-4.01 (2H, t, m), 4.09 (2H, t, J = 8.3Hz), 4.33 (2H, dd, J = 8.7Hz, 5.4Hz), 4.88 (1H, t, J = 5.5Hz), 5.09 (2H, d, J = 8.9Hz), 6.30 (1H, dd, J = 9.5Hz, 1.6Hz), 6.83 (1H, dd, J = 8.6Hz, 2.4Hz), 6.88 (1H, d, J = 2.4Hz), 7.01 (1H, d, J = 7.5Hz), 7.32 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.81 (1H, d, J = 9.5Hz), 11.7 (1H, s). |
| EX44 | 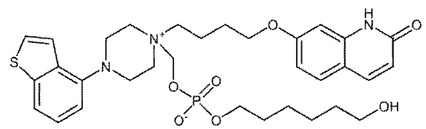 | 1,6-Hexanediol (30 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (5 g) were mixed, and acetone (40 mL) and water (10 mL) were added and stirred. Then, WSC·HCl (3.53 g) was added, and the mixture was heated and stirred at 50°C for 8 hours. Ethyl acetate was added to the reaction mixture and concentrated under reduced pressure repeatedly to azeotropically remove water. The solution was purified by silica gel column chromatography (eluent: DCM-MeOH). The resulting product was recrystallized in acetone-water to give (4-(benzo[b]thiophen-4-yl)-1-(4-((2-oxo-1,2-dihydroquinolin-7-yl)oxy)butyl)piperazin-1-ium-1-yl)methyl (6-hydroxyhexyl) phosphate (1.27 g). | NMR1; 1.21-1.32 (4H, m), 1.33-1.43 (2H, m), 1.44-1.52 (2H, m), 1.79-1.87 (2H, m), 1.90-2.00 (2H, m), 3.40-3.51 (4H, m), 3.53-3.80 (8H, m), 4.09 (2H, t, J = 6.1Hz), 4.38 (2H, t, J = 5.2Hz), 5.09 (2H, d, J = 9.1Hz), 6.30 (1H, dd, J = 9.5Hz, 1.5Hz), 6.82 (1H, dd, J = 8.6Hz, 2.4Hz), 6.90 (1H, d, J = 2.4Hz), 7.01 (1H, d, J = 7.4Hz), 7.32 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.80 (1H, d, J = 9.5Hz), 11.7 (1H, s). |
| EX45 | 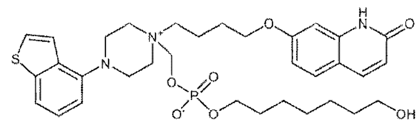 | 1,7-Heptanediol (35 g) was heated and stirred at a set temperature of 65°C, and water (4 mL) and WSC·HCl (5.29 g) were added, followed by heating and stirring. When it was in solution, (4-(benzo[b]thiophen-4-yl)-1-(4-((2-oxo-1,2-dihydroquinolin-7-yl)oxy)butyl)piperazin-1-ium-1-yl)methyl hydrogen phosphate (5 g) was added in 0.5 g portions at 5-10 minute intervals, and after addition, the mixture was further heated and stirred for 3 hours. After the reaction mixture was cooled to room temperature, ethyl acetate was added and concentrated under reduced pressure to azeotropically remove water. The reaction mixture was directly purified by silica gel column chromatography (eluent: DCM-MeOH). The resulting product was dissolved in methanol (6.6 mL), and while stirring, water-saturated ethyl acetate was gradually added. The precipitated crystals were filtered out and air-dried to give (4-(benzo[b]thiophen-4-yl)-1-(4-((2-oxo-1,2-dihydroquinolin-7-yl)oxy)butyl)piperazin-1-ium-1-yl)methyl (7-hydroxyheptyl) phosphate (3.0 g). | NMR1; 1.20-1.32 (6H, m), 1.32-1.41 (2H, m), 1.42-1.52 (2H, m), 1.78-1.88 (2H, m), 1.89-2.01 (2H, m), 3.40-3.51 (4H, m), 3.52-3.80 (8H, m), 4.10 (2H, t, J = 5.9Hz), 4.34 (2H, t, J = 5.2Hz), 5.09 (2H, d, J = 9.0Hz), 6.30 (1H, d, J = 9.5Hz), 6.82 (1H, d, J = 8.6Hz), 6.90 (1H, br-s), 7.01 (1H, d, J = 7.7Hz), 7.32 (1H, t, J = 7.8Hz), 7.51 (1H, d, J = 5.5Hz), 7.56 (1H, d, J = 8.7Hz), 7.71 (1H, d, J = 8.0Hz), 7.77 (1H, d, J = 5.5Hz), 7.80 (1H, d, J = 9.5Hz), 11.7 (1H, s). |
| EX46 | 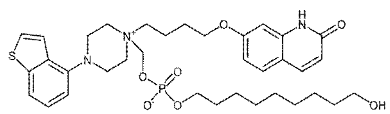 | Using 1,9-nonanediol (10.5 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (9-hydroxynonyl) phosphate (513 mg) was obtained in a method similar to the synthesis of EX 45. | NMR1; 1.18-1.30 (10H, m), 1.32-1.43 (2H, m), 1.44-1.51 (2H, m), 1.80-1.89 (2H, m), 1.90-2.01 (2H, m), 3.40-3.50 (4H, m), 3.51-3.80 (8H, m), 4.10 (2H, t, J = 6.1Hz), 4.36 (2H, t, J = 5.0Hz), 5.09 (2H, d, J = 9.0Hz), 6.30 (1H, d, J = 9.5Hz), 6.82 (1H, dd, J = 8.6Hz, 2.4Hz), 6.89 (1H, d, J = 2.4Hz), 7.01 (1H, d, J = 7.4Hz), 7.32 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.81 (1H, d, J = 9.5Hz), 11.7 (1H, s). |

| | | | |
|---|---|---|---|
| EX47 | 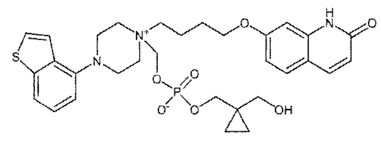 | Using cyclopropane-1,1-diyldimethanol (3.76 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl ((1-(hydroxymethyl)cyclopropyl)methyl) phosphate (380 mg) was obtained in a method similar to the synthesis of EX 44. | NMR1; 0.35 (4H, s), 1.78-1.89 (2H, m), 1.90-2.00 (2H, m), 3.40-3.50 (4H, m), 3.55-3.62 (4H, m), 3.65 (2H, d, J = 8.1Hz), 3.70-3.80 (2H, m), 4.10 (2H, t, J = 6.1Hz), 5.04 (1H, t, J = 6.2Hz), 5.09 (2H, d, J = 8.9Hz), 6.30 (1H, d, J = 9.5Hz), 6.81 (1H, dd, J = 8.6Hz, 2.4Hz), 6.87 (1H, d, J = 2.4Hz), 7.00 (1H, d, J = 7.5Hz), 7.32 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.81 (1H, d, J = 9.5Hz), 11.7 (1H, s). |
| EX48 | 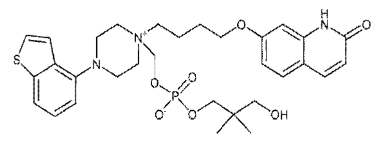 | Using 2,2-dimethyl-1,3-propanediol (3.83 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (3-hydroxy-2,2-dimethylpropyl) phosphate (230 mg) was obtained in a method similar to the synthesis of EX 44. | NMR1; 0.76 (6H, s), 1.79-1.88 (2H, m), 1.89-2.00 (2H, m), 3.13 (2H, d, J = 6.6Hz), 3.40-3.50 (6H, m), 3.55-3.68 (2H, m), 3.70-3.80 (2H, m), 4.06-4.15 (3H, m), 5.10 (2H, d, J = 8.8Hz), 5.18 (1H, t, J = 6.5Hz), 6.30 (1H, d, J = 9.5Hz), 6.82 (1H, dd, J = 8.6Hz, 2.4Hz), 6.86 (1H, d, J = 2.4Hz), 7.01 (1H, d, J = 7.5Hz), 7.32 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.81 (1H, d, J = 9.5Hz), 11.7 (1H, s). |
| EX49 | 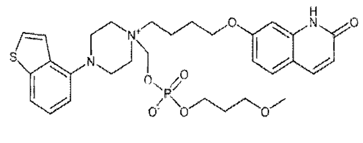 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g) and 3-methoxy-1-propanol (10 mL), (4-(benzo[b]thiophen-4-yl)-1-(4-((2-oxo-1,2-dihydroquinolin-7-yl)oxy)butyl)piperazin-1-ium-1-yl)methyl (3-methoxypropyl) phosphate (160 mg) was obtained in the same manner as in EX 44. | NMR1; 1.74 (2H, q, J = 6.5Hz), 1.78-1.88 (2H, m), 1.89-1.99 (2H, m), 3.25 (3H, s), 3.36 (2H, t, J = 6.5Hz), 3.42-3.48 (4H, m), 3.53-3.67 (4H, m), 3.72-3.79 (4H, m), 4.10 (2H, t, J = 6.1Hz), 5.09 (2H, d, J = 9.2Hz), 6.30 (1H, dd, J = 9.4Hz, 1.8Hz), 6.89 (1H, d, J = 2.4Hz), 6.89 (1H, d, J = 2.4Hz), 7.00 (1H, d, J = 7.4Hz), 7.32 (1H, t, J = 7.8Hz), 7.51 (1H, d, J = 5.5Hz), 7.56 (1H, d, J = 8.7Hz), 7.71 (1H, d, J = 8.0Hz), 7.77 (1H, d, J = 5.5Hz), 7.80 (1H, d, J = 9.4Hz), 11.7 (1H, s) . |
| EX50 | 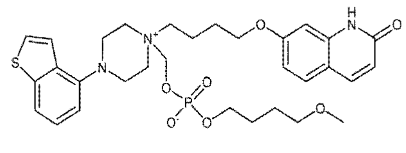 | Using (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g) and 1,4-butanediol monomethyl ether (10 mL), (4-(benzo[b]thiophen-4-yl)-1-(4-((2-oxo-1,2-dihydroquinolin-7-yl)oxy)butyl)piperazin-1-ium-1-yl)methyl (4-methoxybutyl) phosphate (106 mg) was obtained in the same manner as in EX 44. | NMR1; 1.48-1.57 (4H, m), 1.78-1.88 (2H, m), 1.89-1.98 (2H, m), 3.19 (3H, s), 3.26-3.32 (2H, t, m), 3.42-3.50 (4H, m), 3.55-3.68 (4H, m), 3.68-3.79 (4H, m), 4.09 (2H, t, J = 6.7Hz), 5.09 (2H, d, J = 9.0Hz), 6.30 (1H, d, J = 9.4Hz), 6.82 (1H, dd, J = 8.6Hz, 2.4Hz), 6.89 (1H, d, J = 2.4Hz), 7.01 (1H, d, J = 7.4Hz), 7.32 (1H, t, J = 7.9Hz), 7.51 (1H, d, J = 5.5Hz), 7.56 (1H, d, J = 8.7Hz), 7.71 (1H, d, J = 8.0Hz), 7.77 (1H, d, J = 5.5Hz), 7.81 (1H, d, J = 9.5Hz), 11.7 (1H, s). |
| EX51 | 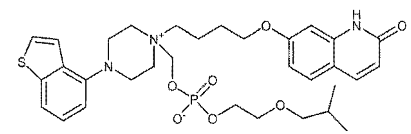 | Using ethylene glycol monoisobutyl ether (10 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (2-isobutoxyethyl) phosphate (220 mg) was obtained in a method similar to the synthesis of EX 44. | NMR1; 0.81 (6H, d, J = 6.7Hz), 1.75 (1H, sept, J = 6.7Hz), 1.80-1.89 (2H, m), 1.90-2.00 (2H, m), 3.13 (2H, d, J = 6.7Hz), 3.40-3.50 (6H, m), 3.52-3.68 (4H, m), 3.71-3.82 (4H, m), 4.10 (2H, t, J = 6.1Hz), 5.09 (2H, d, J = 9.2Hz), 6.30 (1H, dd, J = 9.5Hz, 1.6Hz), 6.82 (1H, dd, J = 8.6Hz, 2.4Hz), 6.88 (1H, d, J = 2.4Hz), 7.00 (1H, d, J = 7.2Hz), 7.32 (1H, t, J = 7.8Hz), 7.50 (1H, d, J = 5.5Hz), 7.56 (1H, d, J = 8.7Hz), 7.71 (1H, d, J = 8.0Hz), 7.77 (1H, d, J = 5.5Hz), 7.81 (1H, d, J = 9.5Hz), 11.7 (1H, s). |

| | | | |
|---|---|---|---|
| EX52 | 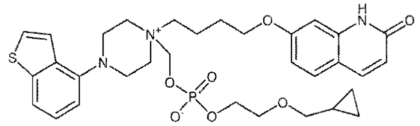 | 2-(Cyclopropylmethoxy)ethan-1-ol (1.28 g) and ethyldiisopropylamine (3.21 mL) were dissolved in DCM (40 mL), and Tf2O (2.18 mL) was slowly added dropwise under ice-cold stirring. After 1 hour, water was added to the reaction mixture and stirred, and the organic layer was separated and concentrated under reduced pressure to give triflate of 2-(cyclopropylmethoxy)ethan-1-ol. This was dissolved in acetonitrile (5 mL). (4-(benzo[b]thiophen-4-yl)-1-(4-((2-oxo-1,2-dihydroquinolin-7-yl)oxy)butyl)piperazin-1-ium-1-yl)methyl hydrogen phosphate (2.0 g) was then suspended in a mixed solvent of acetonitrile (20 mL) and water (4 mL), and DIPEA (3.21 mL) was added and stirred to bring it into solution. The solution was heated and stirred at 65°C, and the acetonitrile solution of the triflate was added, followed by heating and stirring for 1 hour. After cooling the reaction mixture to room temperature, ethyl acetate was added and concentrated under reduced pressure to azeotropically remove water. The reaction mixture was purified by silica gel column chromatography (eluent: DCM-MeOH). The resulting product recrystallized from ethyl acetate-methanol to give (4-(benzo[b]thiophen-4-yl)-1-(4-((2-oxo-1,2-dihydroquinolin-7-yl)oxy)butyl)piperazin-1-ium-1-yl)methyl (2-(cyclopropylmethoxy)ethyl) phosphate (995 mg). | NMR1; 0.08-0.15 (4H, m), 0.38-0.47 (2H, m), 0.90-1.00 (1H, m), 1.79-1.89 (2H, m), 1.90-2.00 (2H, m), 3.20 (2H, d, J = 6.8Hz), 3.40-3.51 (6H, m), 3.51-3.70 (4H, m), 3.71-3.85 (4H, m), 4.10 (2H, t, J = 6.1Hz), 5.10 (2H, d, J = 8.9Hz), 6.30 (1H, d, J = 9.5Hz), 6.82 (1H, dd, J = 8.6Hz, 2.4Hz), 6.88 (1H, d, J = 2.4Hz), 7.01 (1H, d, J = 7.4Hz), 7.33 (1H, t, J = 7.8Hz), 7.51 (1H, d, J = 5.5Hz), 7.57 (1H, d, J = 8.7Hz), 7.71 (1H, d, J = 8.1Hz), 7.77 (1H, d, J = 5.5Hz), 7.81 (1H, d, J = 9.5Hz), 11.7 (1H, s) . |
| EX53 | 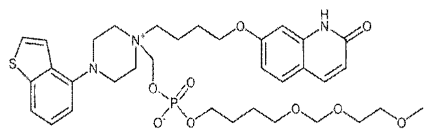 | Using 4-((2-methoxyethoxy)methoxy)butan-1-ol (3 g) synthesized in REX 12 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (4-((2-methoxyethoxy)methoxy)butyl) phosphate (81 mg) was obtained in a method similar to the synthesis of EX 44. | NMR1; 1.51-1.57 (4H, m), 1.78-1.89 (2H, m), 1.90-2.00 (2H, m), 3.23 (3H, s), 3.40-3.50 (6H, m), 3.51-3.68 (6H, m), 3.69-3.80 (4H, m), 4.10 (2H, t, J = 6.1Hz), 4.58 (2H, s), 5.09 (2H, d, J = 9.1Hz), 6.30 (1H, d, J = 9.4Hz), 6.82 (1H, dd, J = 8.6Hz, 2.4Hz), 6.89 (1H, d, J = 2.4Hz), 7.01 (1H, d, J = 7.4Hz), 7.32 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.81 (1H, d, J = 9.5Hz), 11.7 (1H, s). |
| EX54 | 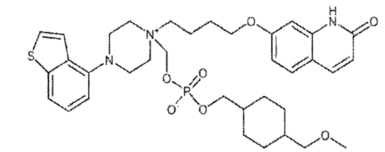 | Using (4-(methoxymethyl)cyclohexyl)methanol (0.87 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl ((4-(methoxymethyl)cyclohexyl)methyl) phosphate (81 mg) was obtained in a method similar to the synthesis of EX 52. | NMR1; 0.80-0.92 (4H, m), 1.37-1.50 (2H, m), 1.62-1.79 (4H, m), 1.80-1.89 (2H, m), 1.90-2.00 (2H, m), 3.18 (3H, s), 3.40-3.58 (4H, m), 3.51 (2H, t, J = 6.4Hz), 3.52-3.68 (4H, m), 3.70-3.82 (2H, m), 4.10 (2H, t, J = 6.1Hz), 5.08 (2H, d, J = 9.0Hz), 6.30 (1H, d, J = 9.5Hz), 6.82 (1H, dd, J = 8.7Hz, 2.4Hz), 6.89 (1H, d, J = 2.4Hz), 7.01 (1H, d, J = 7.4Hz), 7.32 (1H, t, J = 7.8Hz), 7.51 (1H, d, J = 5.5Hz), 7.56 (1H, d, J = 8.7Hz), 7.71 (1H, d, J = 8.0Hz), 7.77 (1H, d, J = 5.5Hz), 7.81 (1H, d, J = 9.5Hz), 11.7 (1H, s). |
| EX55 | 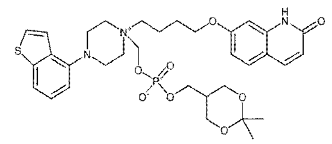 | Using (2,2-dimethyl-1,3-dioxan-5-yl)methanol (0.81 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl ((2,2-dimethyl-1,3-dioxan-5-yl)methyl) phosphate (620 mg) was obtained in a method similar to the synthesis of EX 52. | NMR1; 1.30 (6H, d, J = 10.1Hz), 1.80-1.89 (2H, m), 1.90-2.00 (2H, m), 3.40-3.50 (4H, m), 3.55-3.69 (6H, m), 3.70-3.80 (4H, m), 3.85 (2H, dd, J = 11.8Hz, 4.2Hz), 4.10 (2H, t, J = 6.1Hz), 5.10 (2H, d, J = 9.0Hz), 6.30 (1H, dd, J = 9.5Hz, 1.7Hz), 6.82 (1H, dd, J = 8.6Hz, 2.4Hz), 6.89 (1H, d, J = 2.4Hz), 7.01 (1H, d, J = 7.5Hz), 7.32 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.81 (1H, d, J = 9.5Hz), 11.7 (1H, s) . |

| | | | |
|---|---|---|---|
| EX56 | 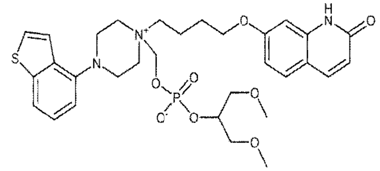 | Pyridine (0.98 mL) was dissolved in DCM (30 mL), and Tf2O (2.05 mL) was added dropwise with stirring under ice-cold conditions. After 10 minutes, a DCM (5 mL) solution of 1,3-dimethoxypropan-2-ol (1.33 mL) was added dropwise and stirred at room temperature for 10 minutes. The reaction mixture was concentrated under reduced pressure, and Et2O was added to the residue. The precipitate was filtered off and the filtrate was concentrated under reduced pressure to give a triflate brown oil. (4-(Benzo[b]thiophen-4-yl)-1-(4-((2-oxo-1,2-dihydroquinolin-7-yl)oxy)butyl)piperazin-1-ium-1-yl)methyl hydrogen phosphate (2.0 g) was then suspended in a mixed solvent of MeCN (20 mL) and water (4 mL), and DIPEA (2.57 mL) was added and stirred to bring it into solution. The solution was heated and stirred at 50°C, and an acetonitrile solution (5 mL) of the triflate was added and further stirred for 1 hour. Heating was stopped, and the reaction mixture was cooled to room temperature and concentrated under reduced pressure. The resulting crude product was purified by silica gel column chromatography (eluent: DCM-MeOH) to give (4-(benzo[b]thiophen-4-yl)-1-(4-((2-oxo-1,2-dihydroquinolin-7-yl)oxy)butyl)piperazin-1-ium-1-yl)methyl (1,3-dimethoxypropan-2-yl) phosphate (470 mg). | NMR1; 1.80-1.89 (2H, m), 1.90-2.00 (2H, m), 3.26 (6H, s), 3.40-3.50 (8H, m), 3.55-3.68 (4H, m), 3.73-3.81 (2H, m), 4.10 (2H, t, J = 6.1Hz), 4.22 (1H, sept, J = 5.2Hz), 5.08 (2H, d, J = 8.8Hz), 6.31 (1H, dd, J = 9.5Hz, 1.3Hz), 6.83 (1H, dd, J = 8.6Hz, 2.4Hz), 6.89 (1H, d, J = 2.4Hz), 7.01 (1H, d, J = 7.6Hz), 7.33 (1H, t, J = 7.8Hz), 7.52 (1H, d, J = 5.5Hz), 7.56 (1H, d, J = 8.7Hz), 7.71 (1H, d, J = 8.1Hz), 7.77 (1H, d, J = 5.5Hz), 7.81 (1H, d, J = 9.5Hz), 11.7 (1H, s) . |
| EX57 | 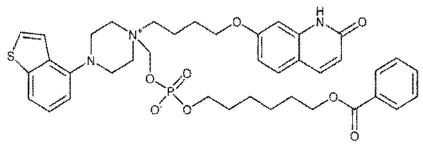 | Using 6-hydroxyhexyl benzoate (1.02 g) synthesized in REX 13 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (6-(benzoyloxy)hexyl) phosphate (610 mg) was obtained in a method similar to the synthesis of EX 56. | NMR1; 1.30-1.40 (4H, m), 1.45-1.57 (2H, m), 1.62-1.71 (2H, m), 1.78-1.88 (2H, m), 1.89-2.00 (2H, m), 3.40-3.50 (4H, m), 3.53-3.67 (4H, m), 3.68-3.70 (4H, m), 4.09 (2H, t, J = 6.1Hz), 4.24 (2H, t, J = 6.6Hz), 5.09 (2H, d, J = 9.1Hz), 6.29 (1H, d, J = 9.5Hz), 6.81 (1H, dd, J = 8.7Hz, 2.4Hz), 6.89 (1H, d, J = 2.4Hz), 7.00 (1H, d, J = 7.4Hz), 7.32 (1H, t, J = 7.8Hz), 7.48-7.57 (4H, m), 7.64 (1H, t, J = 6.2Hz), 7.71 (1H, d, J = 8.1Hz), 7.74-7.81 (2H, m), 7.95 (2H, d, J = 6.2Hz), 11.7 (1H, s) . |
| EX58 | 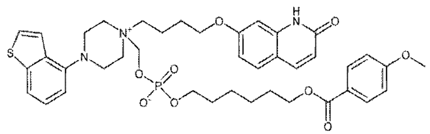 | Using 6-hydroxyhexyl 4-methoxybenzoate (1.16 g) synthesized in REX 14 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (6-((4-methoxybenzoyl)oxy)hexyl) phosphate (839 mg) was obtained in a method similar to the synthesis of EX 56. | NMR1; 1.30-1.41 (4H, m), 1.48-1.57 (2H, m), 1.61-1.72 (2H, m), 1.80-1.89 (2H, m), 1.90-2.00 (2H, m), 3.40-3.50 (4H, m), 3.55-3.69 (4H, m), 3.70-3.80 (4H, m), 3.82 (3H, s), 4.10 (2H, t, J = 6.0Hz), 4.20 (2H, t, J = 6.6Hz), 5.09 (2H, d, J = 9.1Hz), 6.30 (1H, d, J = 9.5Hz), 6.81 (1H, dd, J = 8.6Hz, 2.4Hz), 6.89 (1H, d, J = 2.4Hz), 6.98-7.06 (3H, m), 7.32 (1H, t, J = 7.8Hz), 7.51 (1H, d, J = 5.5Hz), 7.55 (1H, d, J = 8.7Hz), 7.71 (1H, d, J = 8.0Hz), 7.77 (1H, d, J = 5.5Hz), 7.80 (1H, d, J = 9.5Hz), 7.89 (2H, d, J = 6.9Hz), 11.7 (1H, s) . |

| | | | |
|---|---|---|---|
| EX59 | 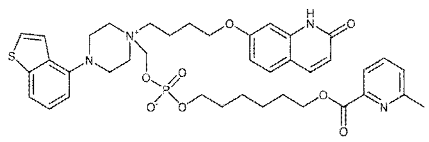 | Using 6-hydroxyhexyl 6-methylpicolinate (2.0 g) synthesized in REX 16 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (6-((6-methylpicolinoyl)oxy)hexyl) phosphate (35 mg) was obtained in a method similar to the synthesis of EX 56. | NMR1; 1.30-1.41 (4H, m), 1.47-1.56 (2H, m), 1.65-1.72 (2H, m), 1.78-1.87 (2H, m), 1.90-2.00 (2H, m), 2.52 (3H, s), 3.40-3.50 (4H, m), 3.53-3.65 (4H, m), 3.66-3.80 (4H, m), 4.09 (2H, t, J = 6.2Hz), 4.26 (2H, t, J = 6.7Hz), 5.09 (2H, d, J = 9.0Hz), 6.29 (1H, dd, J = 9.5Hz, 2.4Hz), 6.81 (1H, dd, J = 8.6Hz, 2.4Hz), 6.88 (1H, d, J = 2.4Hz), 7.00 (1H, d, J = 7.4Hz), 7.32 (1H, t, J = 7.9Hz), 7.47 (1H, dd, J = 6.4Hz, 2.3Hz), 7.51 (1H, d, J = 5.5Hz), 7.55 (1H, d, J = 8.7Hz), 7.71 (1H, d, J = 8.0Hz), 7.76 (1H, d, J = 5.5Hz), 7.79 (1H, d, J = 9.5Hz), 7.82-7.88 (2H, m), 11.7 (1H, s) . |
| EX60 | 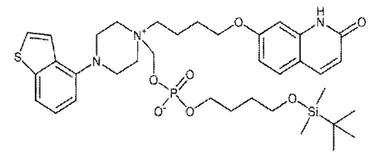 | Using 4-((tert-butyldimethylsilyl)oxy)butan-1-ol (5.0 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzo[b]thiophen-4-yl)-1-(4-((2-oxo-1,2-dihydroquinolin-7-yl)oxy)butyl)piperazin-1-ium-1-yl)methyl (4-((tert-butyldimethylsilyl)oxy)butyl) phosphate (150 mg) was obtained in a method similar to the synthesis of EX 45. | NMR1; 0.84 (9H, s), 1.41-1.58 (4H, m), 1.78-1.88 (2H, m), 1.89-2.00 (2H, m), 3.40-3.50 (4H, m), 3.52-3.67 (6H, m), 3.68-3.80 (4H, m), 4.10 (2H, t, J = 6.0Hz), 5.09 (2H, d, J = 9.1Hz), 6.31 (1H, d, J = 9.5Hz), 6.82 (1H, dd, J = 8.7Hz, 2.4Hz), 6.89 (1H, d, J = 2.4Hz), 7.01 (1H, d, J = 7.3Hz), 7.32 (1H, t, J = 7.9Hz), 7.51 (1H, d, J = 5.5Hz), 7.56 (1H, d, J = 8.7Hz), 7.71 (1H, d, J = 8.0Hz), 7.76 (1H, d, J = 5.5Hz), 7.81 (1H, d, J = 9.5Hz), 11.7 (1H, s). |
| EX61 | 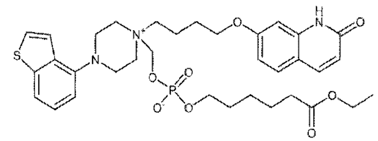 | Using ethyl 6-hydroxyhexanoate (0.99 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzo[b]thiophen-4-yl)-1-(4-((2-oxo-1,2-dihydroquinolin-7-yl)oxy)butyl)piperazin-1-ium-1-yl)methyl (6-ethoxy-6-oxohexyl) phosphate (410 mg) was obtained in a method similar to the synthesis of EX 52. | NMR1; 1.15 (3H, t, J = 7.1Hz), 1.22-1.33 (2H, m), 1.45-1.56 (4H, m), 1.78-1.89 (2H, m), 1.90-2.00 (2H, m), 3.40-3.50 (4H, m), 3.55-3.80 (4H, m), 4.01 (2H, q, J = 7.1Hz), 4.09 (2H, t, J = 6.5Hz), 5.09 (2H, d, J = 9.1Hz), 6.30 (1H, dd, J = 9.5Hz, 1.8Hz), 6.82 (1H, dd, J = 8.6Hz, 2.4Hz), 6.90 (1H, d, J = 2.4Hz), 7.01 (1H, d, J = 7.4Hz), 7.32 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.80 (1H, d, J = 9.5Hz), 11.7 (1H, s). |
| EX62 | 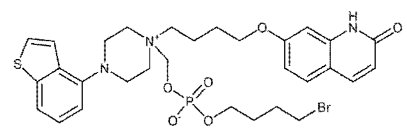 | Using 4-bromobutan-1-ol (1.06 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (4-bromobutyl) phosphate (440 mg) was obtained in a method similar to the synthesis of EX 52. | NMR1; 1.57-1.66 (2H, m), 1.79-1.89 (4H, m), 1.90-2.00 (2H, m), 3.40-3.50 (4H, m), 3.51-3.68 (6H, m), 3.70-3.80 (4H, m), 4.10 (2H, t, J = 6.0Hz), 5.09 (2H, d, J = 9.0Hz), 6.30 (1H, d, J = 9.5Hz), 6.83 (1H, dd, J = 8.6Hz, 2.4Hz), 6.88 (1H, d, J = 2.4Hz), 7.01 (1H, d, J = 7.4Hz), 7.33 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.81 (1H, d, J = 9.5Hz), 11.7 (1H, s). |

| | | | |
|---|---|---|---|
| EX63 | 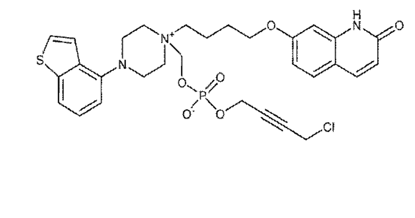 | Using 4-chlorobut-2-yn-1-ol (0.24 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzo[b]thiophen-4-yl)-1-(4-((2-oxo-1,2-dihydroquinolin-7-yl)oxy)butyl)piperazin-1-ium-1-yl)methyl (4-chlorobut-2-yn-1-yl) phosphate (230 mg) was obtained in a method similar to the synthesis of EX 56. | NMR1; 1.78-1.88 (2H, m), 1.89-2.00 (2H, m), 3.17 (2H, d, J = 5.2Hz), 3.40-3.50 (4H, m), 3.55-3.70 (4H, m), 4.08-4.13 (2H, m), 4.46-4.51 (4H, m), 5.09 (2H, d, J = 8.9Hz), 6.30 (1H, dd, J = 9.5Hz, 1.8Hz), 6.83 (1H, dd, J = 8.6Hz, 2.4Hz), 6.86 (1H, d, J = 2.4Hz), 7.01 (1H, d, J = 7.4Hz), 7.32 (1H, t, J = 7.8Hz), 7.51 (1H, d, J = 5.5Hz), 7.56 (1H, d, J = 8.7Hz), 7.71 (1H, d, J = 8.0Hz), 7.77 (1H, d, J = 5.5Hz), 7.81 (1H, d, J = 9.5Hz), 11.7 (1H, s). |
| EX64 | 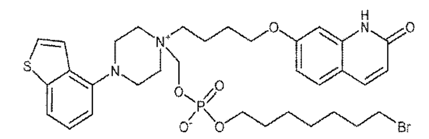 | Using 7-bromoheptan-1-ol (1.08 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (7-bromoheptyl) phosphate (640 mg) was obtained in a method similar to the synthesis of EX 52. | NMR1; 1.30-1.50 (6H, m), 1.44-1.52 (2H, m), 1.74 (2H, sext, J = 7.2Hz), 1.80-1.89 (2H, m), 1.90-2.00 (2H, m), 3.40-3.50 (6H, m), 3.52-3.80 (8H, m), 4.10 (2H, t, J = 6.0Hz), 5.09 (2H, d, J = 9.0Hz), 6.30 (1H, d, J = 9.5Hz), 6.83 (1H, dd, J = 8.6Hz, 2.4Hz), 6.89 (1H, d, J = 2.4Hz), 7.01 (1H, d, J = 7.4Hz), 7.33 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.81 (1H, d, J = 9.5Hz), 11.7 (1H, s). |
| EX65 | 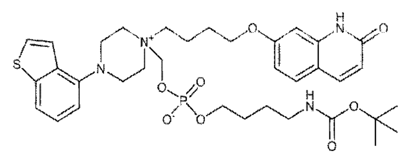 | Using tert-butyl (4-hydroxybutyl)carbamate (1.74 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (4-((tert-butoxycarbonyl)amino)butyl) phosphate (35 mg) was obtained in a method similar to the synthesis of EX 45. | NMR1; 1.35 (9H, s), 1.37-1.52 (4H, m), 1.79-1.89 (2H, m), 1.90-2.00 (2H, m), 2.91 (2H, sext, J = 6.5Hz), 3.40-3.50 (4H, m), 3.51-3.80 (8H, m), 4.10 (2H, t, J = 6.0Hz), 5.09 (2H, d, J = 9.0Hz), 6.31 (1H, dd, J = 9.5Hz, 1.3Hz), 6.80 (1H, br-t, J = 6.0Hz), 6.83 (1H, dd, J = 8.7Hz, 2.4Hz), 6.88 (1H, d, J = 2.4Hz), 7.01 (1H, d, J = 7.4Hz), 7.32 (1H, t, J = 7.8Hz), 7.51 (1H, d, J = 5.5Hz), 7.57 (1H, d, J = 8.7Hz), 7.71 (1H, d, J = 8.1Hz), 7.76 (1H, d, J = 5.5Hz), 7.81 (1H, d, J = 9.5Hz), 11.7 (1H, s). |
| EX66 | 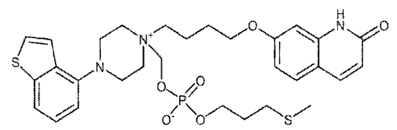 | Using 3-(methylthio)propan-1-ol (5 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzo[b]thiophen-4-yl)-1-(4-((2-oxo-1,2-dihydroquinolin-7-yl)oxy)butyl)piperazin-1-ium-1-yl)methyl (3-(methylthio)propyl) phosphate (180 mg) was obtained in a method similar to the synthesis of EX 45. | NMR1; 1.77 (2H, sext, J = 7.4Hz), 1.82-1.88 (2H, m), 1.90-2.00 (2H, m), 2.02 (3H, s), 3.40-3.50 (4H, m), 3.50-3.67 (4H, m), 3.72-3.82 (4H, m), 4.11 (2H, t, J = 6.1Hz), 5.10 (2H, d, J = 8.9Hz), 6.31 (1H, d, J = 9.5Hz), 6.83 (1H, dd, J = 8.6Hz, 2.4Hz), 6.88 (1H, d, J = 2.4Hz), 7.01 (1H, d, J = 7.4Hz), 7.32 (1H, t, J = 7.8Hz), 7.52 (1H, d, J = 5.5Hz), 7.57 (1H, d, J = 8.7Hz), 7.71 (1H, d, J = 8.1Hz), 7.76 (1H, d, J = 5.5Hz), 7.81 (1H, d, J = 9.5Hz), 11.7 (1H, s). |

| | | | |
|---|---|---|---|
| EX67 | 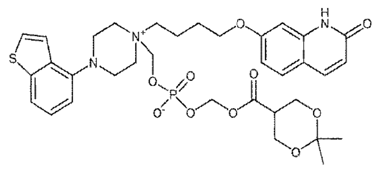 | Using chloromethyl 2,2-dimethyl-1,3-dioxane-5-carboxylate (1.15 g) synthesized in REX 20 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (((2,2-dimethyl-1,3-dioxane-5-carbonyl)oxy)methyl) phosphate (220 mg) was obtained in a method similar to the synthesis method of EX 68. | NMR1; 1.28 (6H, s), 1.79-1.89 (2H, m), 1.90-2.00 (2H, m), 2.75 (1H, sept, J = 6.5Hz), 3.40-3.50 (4H, m), 3.52-3.68 (4H, m), 3.70-3.80 (2H, m), 3.92-4.01 (4H, m), 4.09 (2H, t, J = 6.0Hz), 5.10 (2H, d, J = 9.0Hz), 5.47 (2H, d, J = 13.0Hz), 6.30 (1H, d, J = 9.5Hz), 6.82 (1H, dd, J = 8.6Hz, 2.4Hz), 6.85 (1H, d, J = 2.4Hz), 7.01 (1H, d, J = 7.6Hz), 7.32 (1H, t, J = 7.8Hz), 7.51 (1H, d, J = 5.5Hz), 7.56 (1H, d, J = 8.6Hz), 7.71 (1H, d, J = 8.0Hz), 7.77 (1H, d, J = 5.5Hz), 7.81 (1H, d, J = 9.5Hz), 11.6 (1H, s). |
| EX68 | 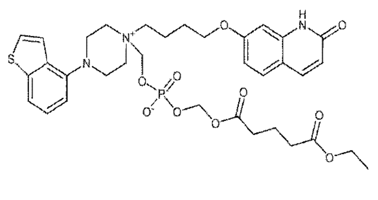 | Methyl(tert-butoxycarbonyl)-L-serinate (3 g), (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), and DIPEA (0.96 mL) were dissolved in a mixed solvent of acetone (10 mL) and water (4 mL), chloromethyl ethyl glutarate (REX 17, 1.15 g) was added, and the mixture was heated to reflux for 8 hours. The reaction mixture was cooled to room temperature, and the precipitated crystals were filtered out. The crude crystals were purified by silica gel column chromatography (eluent: DCM-MeOH). The resulting solid was recrystallized in acetone-water to give (4-(benzo[b]thiophen-4-yl)-1-(4-((2-oxo-1,2-dihydroquinolin-7-yl)oxy)butyl piperazin-1-ium-1-yl)methyl (((5-ethoxy-5-oxopentanoyl)oxy)methyl) phosphate (880 mg). | NMR1; 1.13 (3H, t, J = 7.1Hz), 1.17 (2H, sext, J = 7.3Hz), 1.80-1.89 (2H, m), 1.90-2.00 (2H, m), 2.30 (2H, t, J = 7.4Hz), 2.38 (2H, t, J = 7.4Hz), 3.40-3.50 (4H, m), 3.53-3.69 (4H, m), 3.70-3.80 (2H, m), 4.01 (2H, q, J = 7.1Hz), 4.09 (2H, t, J = 6.0Hz), 5.09 (2H, d, J = 9.0Hz), 5.43 (2H, d, J = 12.8Hz), 6.30 (1H, d, J = 9.5Hz), 6.82 (1H, dd, J = 8.6Hz, 2.4Hz), 6.86 (1H, d, J = 2.4Hz), 7.01 (1H, d, J = 7.6Hz), 7.32 (1H, t, J = 7.8Hz), 7.52 (1H, d, J = 5.5Hz), 7.56 (1H, d, J = 8.6Hz), 7.71 (1H, d, J = 8.0Hz), 7.77 (1H, d, J = 5.5Hz), 7.81 (1H, d, J = 9.5Hz), 11.6 (1H, s). |
| EX69 | 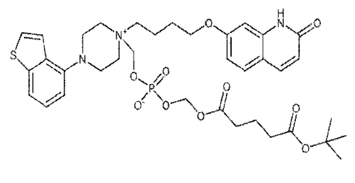 | Using 5-(tert-butoxy)-5-oxopentanoic acid (5 g), chloromethyl ester was synthesized in the same manner as in REX 17 and reacted with (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g) in the same manner as in EX 68 to give (4-(benzo[b]thiophen-4-yl)-1-(4-((2-oxo-1,2-dihydroquinolin-7-yl)oxy)butyl)piperazin-1-ium-1-yl)methyl (((5-(tert-butoxy)-5-oxopentanoyl)oxy)methyl) phosphate (3.3 g). | NMR1; 1.36 (9H, s), 1.71 (2H, sext, J = 7.5Hz), 1.79-1.88 (2H, m), 1.89-2.00 (2H, m), 2.20 (2H, t, J = 7.5Hz), 2.36 (2H, t, J = 7.5Hz), 3.40-3.50 (4H, m), 3.55-3.70 (4H, m), 3.72-3.80 (2H, m), 4.09 (2H, t, J = 6.0Hz), 5.09 (2H, d, J = 9.0Hz), 5.43 (2H, d, J = 12.8Hz), 6.30 (1H, d, J = 9.5Hz), 6.82 (1H, dd, J = 8.6Hz, 2.4Hz), 6.85 (1H, d, J = 2.4Hz), 7.01 (1H, d, J = 7.6Hz), 7.32 (1H, t, J = 7.8Hz), 7.51 (1H, d, J = 5.5Hz), 7.56 (1H, d, J = 8.6Hz), 7.71 (1H, d, J = 8.0Hz), 7.77 (1H, d, J = 5.5Hz), 7.81 (1H, d, J = 9.5Hz), 11.6 (1H, s). |
| EX70 | 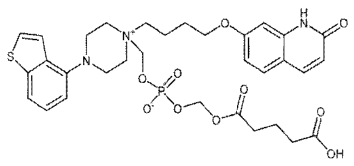 | 4-(Benzo[b]thiophen-4-yl)-1-(4-((2-oxo-1,2-dihydroquinolin-7-yl)oxy)butyl)piperazin-1-ium-1-yl)methyl (((5-(tert-butoxy)-5-oxopentanoyl)oxy)methyl) phosphate (2.5 g) synthesized in EX 69 was placed in a flask, and TFA (5 mL) was added at room temperature and stirred for 1 hour. After completion of the reaction, DCM was added to the reaction mixture and concentrated under reduced pressure twice to remove excess TFA. The residue was purified by medium-pressure liquid chromatography (eluent: DCM-MeOH) to give (4-(benzo[b]thiophen-4-yl)-1-(4-((2-oxo-1,2-dihydroquinolin-7-yl)oxy)butyl)piperazin-1-ium-1-yl)methyl (((4-carboxybutanoyl)oxy)methyl) phosphate (1.83 g). | NMR1; 1.76 (2H, sext, J = 7.4Hz), 1.79-1.88 (2H, m), 1.89-2.00 (2H, m), 2.25 (2H, t, J = 7.4Hz), 2.37 (2H, t, J = 7.4Hz), 3.40-3.50 (4H, m), 3.54-3.70 (4H, m), 3.72-3.80 (2H, m), 4.09 (2H, t, J = 6.0Hz), 5.10 (2H, d, J = 9.0Hz), 5.44 (2H, d, J = 12.8Hz), 6.30 (1H, d, J = 9.5Hz), 6.83 (1H, dd, J = 8.6Hz, 2.4Hz), 6.86 (1H, d, J = 2.4Hz), 7.01 (1H, d, J = 7.6Hz), 7.32 (1H, t, J = 7.8Hz), 7.52 (1H, d, J = 5.5Hz), 7.56 (1H, d, J = 8.6Hz), 7.71 (1H, d, J = 8.0Hz), 7.77 (1H, d, J = 5.5Hz), 7.81 (1H, d, J = 9.5Hz), 11.6 (1H, s), 12.2 (1H, br-s). |

| | | | |
|---|---|---|---|
| EX71 | 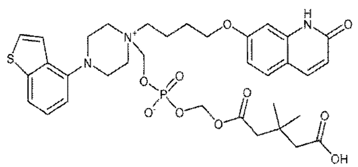 | Using (4-(benzo[b]thiophen-4-yl)-1-(4-((2-oxo-1,2-dihydroquinolin-7-yl)oxy)butyl)piperazin-1-ium-1-yl)methyl (((5-((4-methoxybenzyl)oxy)-3,3-dimethyl-5-oxopentanoyl)oxy)methyl) phosphate (0.2 g) synthesized in EX 72, (4-(benzo[b]thiophen-4-yl)-1-(4-((2-oxo-1,2-dihydroquinolin-7-yl)oxy)butyl)piperazin-1-ium-1-yl)methyl (((4-carboxy-3,3-dimethyl-5-oxopentanoyl)oxy)methyl) phosphate (120 mg) was obtained in a method similar to the synthesis method of EX 70. | NMR1; 1.04 (6H, s), 1.79-1.88 (2H, m), 1.89-2.00 (2H, m), 2.30 (2H, s), 2.41 (2H, s), 3.40-3.50 (4H, m), 3.53-3.70 (4H, m), 3.71-3.80 (2H, m), 4.09 (2H, t, J = 6.0Hz), 5.11 (2H, d, J = 9.0Hz), 5.43 (2H, d, J = 12.8Hz), 6.30 (1H, d, J = 9.5Hz), 6.82 (1H, dd, J = 8.6Hz, 2.4Hz), 6.85 (1H, d, J = 2.4Hz), 7.01 (1H, d, J = 7.6Hz), 7.32 (1H, t, J = 7.8Hz), 7.52 (1H, d, J = 5.5Hz), 7.56 (1H, d, J = 8.6Hz), 7.71 (1H, d, J = 8.0Hz), 7.77 (1H, d, J = 5.5Hz), 7.81 (1H, d, J = 9.5Hz), 11.6 (1H, s), 12.2 (1H, br-s). |
| EX72 | 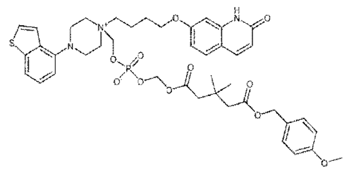 | Using 1-(chloromethyl) 5-(4-methoxybenzyl) 3,3-dimethylpentanedioate (1.82 g) synthesized in REX 19 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (((5-((4-methoxybenzyl)oxy)-3,3-dimethyl-5-oxopentanoyl)oxy)methyl) phosphate (740 mg) was obtained in a method similar to the synthesis method of EX 68. | NMR1; 1.00 (6H, s), 1.79-1.88 (2H, m), 1.89-2.00 (2H, m), 2.38 (2H, s), 2.39 (2H, s), 3.40-3.50 (4H, m), 3.54-3.67 (4H, m), 3.73 (3H, s), 3.73-3.81 (2H, m), 4.08 (2H, t, J = 6.0Hz), 4.97 (2H, s), 5.09 (2H, d, J = 9.0Hz), 5.41 (2H, d, J = 12.5Hz), 6.30 (1H, dd, J = 9.5Hz, 1.6Hz), 6.82 (1H, dd, J = 8.6Hz, 2.4Hz), 6.85 (1H, d, J = 2.4Hz), 6.90 (2H, d, J = 8.7Hz), 7.00 (1H, d, J = 7.6Hz), 7.26 (2H, d, J = 8.7Hz), 7.31 (1H, t, J = 7.8Hz), 7.51 (1H, d, J = 5.5Hz), 7.55 (1H, d, J = 8.6Hz), 7.71 (1H, d, J = 8.0Hz), 7.75 (1H, d, J = 5.5Hz), 7.80 (1H, d, J = 9.5Hz), 11.6 (1H, s). |
| EX73 | 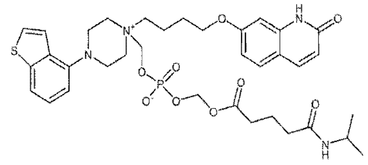 | Using (4-(benzo[b]thiophen-4-yl)-1-(4-((2-oxo-1,2-dihydroquinolin-7-yl)oxy)butyl)piperazin-1-ium-1-yl)methyl (((4-carboxybutanoyl)oxy)methyl) phosphate (300 mg) synthesized in EX 70 and isopropylamine (39 mg), (4-(benzo[b]thiophen-4-yl)-1-(4-((2-oxo-1,2-dihydroquinolin-7-yl)oxy)butyl)piperazin-1-ium-1-yl)methyl (((5-(isopropylamino)-5-oxopentanoyl)oxy)methyl) phosphate (295 mg) was obtained in the same manner as in EX 74. | NMR1; 1.00 (6H, d, J = 6.6Hz), 1.75 (2H, sext, J = 7.3Hz), 1.79-1.88 (2H, m), 1.89-2.00 (2H, m), 2.07 (2H, t, J = 7.4Hz), 2.32 (2H, t, J = 7.2Hz), 3.40-3.50 (4H, m), 3.54-3.70 (4H, m), 3.72-3.81 (3H, m), 4.03-4.12 (2H, m), 5.11 (2H, d, J = 9.0Hz), 5.44 (2H, d, J = 13.4Hz), 6.30 (1H, dd, J = 9.5Hz, 1.6Hz), 6.83 (1H, dd, J = 8.6Hz, 2.4Hz), 6.86 (1H, d, J = 2.4Hz), 7.01 (1H, d, J = 7.6Hz), 7.32 (1H, t, J = 7.8Hz), 7.52 (1H, d, J = 5.5Hz), 7.56 (1H, d, J = 8.6Hz), 7.71 (1H, d, J = 8.0Hz), 7.77 (1H, d, J = 5.5Hz), 7.80 (1H, d, J = 9.5Hz), 7.84 (1H, br-s), 11.6 (1H, s). |

| | | | |
|---|---|---|---|
| EX74 | 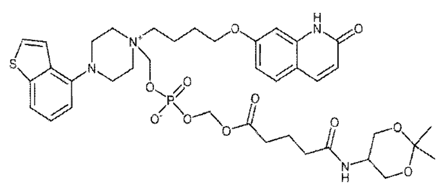 | 4-(Benzo[b]thiophen-4-yl)-1-(4-((2-oxo-1,2-dihydroquinolin-7-yl)oxy)butyl)piperazin-1-ium-1-yl)methyl (((4-carboxybutanoyl)oxy)methyl) phosphate (300 mg) synthesized in EX 70 was dissolved in a mixed solvent of DCM (5 mL) and MeOH (2 mL), and 2,2-dimethyl-1,3-dioxan-5-amine (86 mg) and DMT-MM (81 mg) were added and stirred at room temperature overnight. After completion of the reaction, the reaction mixture was concentrated under reduced pressure and purified by silica gel column chromatography (eluent: DCM-MeOH). The resulting product was crystallized in acetone-water to give (4-(benzo[b]thiophen-4-yl)-1-(4-((2-oxo-1,2-dihydroquinolin-7-yl)oxy)butyl)piperazin-1-ium-1-yl)methyl (((5-((2,2-dimethyl-1,3-dioxan-5-yl)amino)-5-oxopentanoyl)oxy)methyl) phosphate (332 mg). | NMR1; 1.28 (3H, s), 1.34 (3H, s), 1.75 (2H, sext, J = 7.4Hz), 1.80-1.88 (2H, m), 1.89-2.00 (2H, m), 2.15 (2H, t, J = 7.4Hz), 2.33 (2H, t, J = 7.4Hz), 3.40-3.50 (4H, m), 3.54-3.80 (11H, m), 4.06-4.18 (3H, m), 5.11 (2H, d, J = 8.9Hz), 5.44 (2H, d, J = 13.5Hz), 6.30 (1H, dd, J = 9.5Hz, 1.6Hz), 6.83 (1H, dd, J = 8.6Hz, 2.4Hz), 6.86 (1H, d, J = 2.4Hz), 7.01 (1H, d, J = 7.6Hz), 7.32 (1H, t, J = 7.8Hz), 7.52 (1H, d, J = 5.5Hz), 7.56 (1H, d, J = 8.6Hz), 7.71 (1H, d, J = 8.0Hz), 7.77 (1H, d, J = 5.5Hz), 7.81 (1H, d, J = 9.5Hz), 11.6 (1H, s). |
| EX75 | 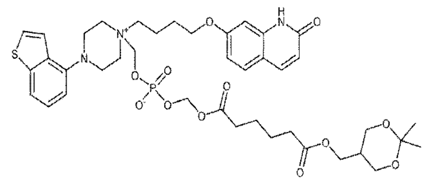 | Using chloromethyl ((2,2-dimethyl-1,3-dioxan-5-yl)methyl) adipate (1.6 g) synthesized in REX 23 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.5 g), (4-(benzo[b]thiophen-4-yl)-1-(4-((2-oxo-1,2-dihydroquinolin-7-yl)oxy)butyl)piperazin-1-ium-1-yl)methyl (((6-((2,2-dimethyl-1,3-dioxan-5-yl)methoxy)-6-oxohexanoyl)oxy)methyl) phosphate (138 mg) was obtained in a method similar to the synthesis method of EX 68. | NMR1; 1.28 (3H, s), 1.31 (3H, s), 1.46-1.53 (4H, m), 1.79-1.88 (2H, m), 1.89-2.00 (2H, m), 2.28 (2H, t, J = 6.8Hz), 2.34 (2H, t, J = 6.8Hz), 3.40-3.50 (4H, m), 3.55-3.68 (6H, m), 3.70-3.81 (2H, m), 3.86 (2H, dd, J = 11.9Hz, 4.0Hz), 4.03 (2H, d, J = 7.1Hz), 4.09 (2H, d, J = 6.1Hz), 5.09 (2H, d, J = 8.9Hz), 5.43 (2H, d, J = 12.7Hz), 6.30 (1H, dd, J = 9.5Hz, 1.7Hz), 6.85 (1H, dd, J = 8.6Hz, 2.4Hz), 6.85 (1H, d, J = 2.4Hz), 7.01 (1H, d, J = 7.6Hz), 7.32 (1H, t, J = 7.8Hz), 7.51 (1H, d, J = 5.5Hz), 7.56 (1H, d, J = 8.6Hz), 7.71 (1H, d, J = 8.0Hz), 7.77 (1H, d, J = 5.5Hz), 7.81 (1H, d, J = 9.5Hz), 11.6 (1H, s). |
| EX76 | 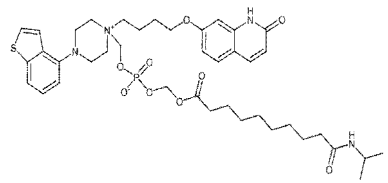 | Using chloromethyl 10-(isopropylamino)-10-oxodecanoate (1.07 g) synthesized in REX 26 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (((10-(isopropylamino)-10-oxodecanoyl)oxy)methyl) phosphate (880 mg) was obtained in a method similar to the synthesis method of EX 68. | NMR1; 1.00 (6H, d, J = 6.6Hz), 1.10-1.22 (8H, m), 1.38-1.52 (4H, m), 1.79-1.89 (2H, m), 1.90-2.00 (2H, m), 1.97 (2H, t, J = 7.4Hz), 2.30 (2H, t, J = 7.4Hz), 3.40-3.50 (4H, m), 3.53-3.69 (4H, m), 3.71-3.82 (3H, m), 4.09 (2H, t, J = 6.0Hz), 5.09 (2H, d, J = 8.9Hz), 5.43 (2H, d, J = 12.7Hz), 6.30 (1H, dd, J = 9.5Hz, 1.4Hz), 6.82 (1H, dd, J = 8.6Hz, 2.4Hz), 6.86 (1H, d, J = 2.4Hz), 7.01 (1H, d, J = 7.6Hz), 7.32 (1H, t, J = 7.8Hz), 7.52 (1H, d, J = 5.5Hz), 7.56 (1H, d, J = 8.6Hz), 7.61 (1H, br-d, J = 7.5Hz), 7.72 (1H, d, J = 8.0Hz), 7.77 (1H, d, J = 5.5Hz), 7.80 (1H, d, J = 9.5Hz), 11.6 (1H, s). |
| EX77 | 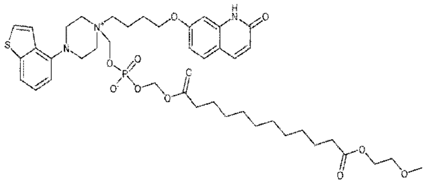 | Using 1-(chloromethyl) 12-(2-methoxyethyl) dodecanedioate (1.86 g) synthesized in REX 28 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (((12-(2-methoxyethoxy)-12-oxododecanoyl)oxy)methyl) phosphate (740 mg) was obtained in a method similar to the synthesis method of EX 68. | NMR1; 1.13-1.30 (12H, m), 1.42-1.51 (4H, m), 1.78-1.88 (2H, m), 1.89-2.00 (2H, m), 2.28 (4H, sext, J = 7.4Hz), 3.24 (3H,s), 3.40-3.50 (6H, m), 3.53-3.71 (4H, m), 3.73-3.82 (2H, m), 4.05-4.15 (4H, m), 5.10 (2H, d, J = 8.8Hz), 5.43 (2H, d, J = 12.8Hz), 6.30 (1H, dd, J = 9.5Hz, 1.6Hz), 6.82 (1H, dd, J = 8.6Hz, 2.4Hz), 6.86 (1H, d, J = 2.4Hz), 7.01 (1H, d, J = 7.6Hz), 7.32 (1H, t, J = 7.8Hz), 7.52 (1H, d, J = 5.5Hz), 7.56 (1H, d, J = 8.6Hz), 7.71 (1H, d, J = 8.0Hz), 7.77 (1H, d, J = 5.5Hz), 7.80 (1H, d, J = 9.5Hz), 11.7 (1H, s). |

| | | | |
|---|---|---|---|
| EX78 | 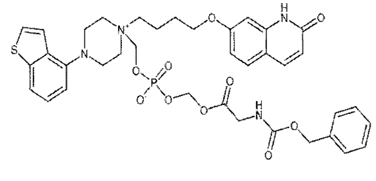 | Using chloromethyl ((benzyloxy)carbonyl)glycinate (1.42 g) synthesized in REX 29 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (((((benzyloxy)carbonyl)glycyl)oxy)methyl) phosphate (72 mg) was obtained in a method similar to the synthesis method of EX 68. | NMR1; 1.79-1.88 (2H, m), 1.89-2.00 (2H, m), 3.40-3.50 (4H, m), 3.52-3.69 (4H, m), 3.70-3.80 (2H, m), 3.84 (2H, d, J = 6.2Hz), 4.09 (2H, d, J = 6.0Hz), 5.02 (2H, s), 5.03-5.15 (2H, m), 5.48 (2H, d, J = 13.6Hz), 6.30 (1H, dd, J = 9.5Hz, 1.5Hz), 6.82 (1H, dd, J = 8.6Hz, 2.4Hz), 6.86 (1H, d, J = 2.4Hz), 7.00 (1H, d, J = 7.6Hz), 7.24-7.40 (6H, m), 7.51 (1H, d, J = 5.5Hz), 7.55 (1H, d, J = 8.6Hz), 7.71 (1H, d, J = 8.0Hz), 7.73-7.82 (3H,m), 11.6 (1H, s). |
| EX79 | 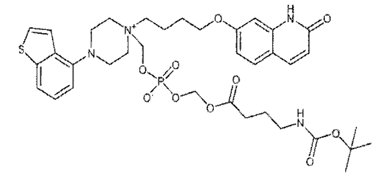 | Using chloromethyl 4-(tert-butoxycarbonyl)amino)butanoate synthesized in REX 30 (3.06 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (2.2 g), (4-(benzo[b]thiophen-4-yl)-1-(4-(2-oxo-1,2-dihydroquinolin-7-yl)oxy)butyl)piperazine-1-ium-1-yl)methyl (((4-(tert-butoxycarbonyl)amino)butanoyl)oxy)methyl) phosphate (2.12 g) was obtained in a method similar to the synthesis method of EX 68). | NMR1; 1.34 (9H, s), 1.62 (2H, sext, J = 7.3Hz), 1.80-1.89 (2H, m), 1.90-2.00 (2H, m), 2.33 (2H, t, J = 7.4Hz), 2.88-2.95 (2H, m), 3.40-3.50 (4H, m), 3.52-3.69 (4H, m), 3.70-3.80 (2H, m), 4.10 (2H, t, J = 6.0Hz), 5.09 (2H, d, J = 9.0Hz), 5.43 (2H, d, J = 12.6Hz), 6.30 (1H, dd, J = 9.5Hz, 1.5Hz), 6.83 (1H, dd, J = 8.6Hz, 2.4Hz), 6.85 (1H, d, J = 2.4Hz), 7.01 (1H, d, J = 7.6Hz), 7.32 (1H, t, J = 7.8Hz), 7.51 (1H, d, J = 5.5Hz), 7.56 (1H, d, J = 8.6Hz), 7.71 (1H, d, J = 8.0Hz), 7.77 (1H, d, J = 5.5Hz), 7.81 (1H, d, J = 9.5Hz), 11.6 (1H, s). |
| EX80 | 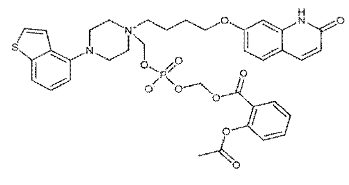 | Using chloromethyl 2-acetoxybenzoate synthesized in REX 31 (2.52 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (2.0 g), (2-acetoxybenzoyl)oxy)methyl ((4-(benzo[b]thiophen-4-yl)-1-(4-((2-oxo-1,2-dihydroquinolin-7-yl)oxy)butyl)piperazin-1-ium-1-yl)methyl) phosphate (605 mg) was obtained in a method similar to the synthesis method of EX 68. | NMR1; 1.71-1.81 (2H, m), 1.82-1.96 (2H, m), 2.31 (3H, s), 3.35-3.45 (4H, m), 3.48-3.66 (4H, m), 3.67-3.76 (2H, m), 4.03 (2H, t, J = 6.0Hz), 5.08 (2H, d, J = 9.0Hz), 5.65 (2H, d, J = 13.6Hz), 6.30 (1H, dd, J = 9.5Hz, 1.2Hz), 6.81 (1H, dd, J = 8.6Hz, 2.4Hz), 6.84 (1H, d, J = 2.4Hz), 6.93 (1H, d, J = 7.6Hz), 7.23 (1H, dd, J = 8.1Hz, 0.9Hz), 7.31 (1H, t, J = 7.8Hz), 7.38 (1H, td, J = 7.7Hz, 1.0Hz), 7.46 (1H, d, J = 5.5Hz), 7.56 (1H, d, J = 8.6Hz), 7.65 (1H, td, J = 8.0Hz, 1.7Hz), 7.71 (1H, d, J = 8.0Hz), 7.77 (1H, d, J = 5.5Hz), 7.81 (1H, d, J = 9.5Hz), 8.00 (1H, dd, J = 7.8Hz, 1.7Hz), 11.6 (1H, s). |

| | | | |
|---|---|---|---|
| EX81 | 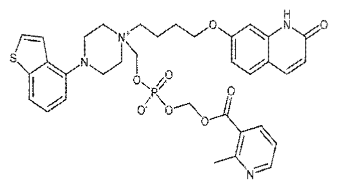 | Using chloromethyl 2-methylnicotinate synthesized in REX 32 (1.02 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-(2-oxo-1,2-dihydroquinolin-7-yl)oxy)butyl)piperazin-1-ium-1-yl)methyl (((2-methylnicotinoyl)oxy)methyl) phosphate (510 mg) was obtained in a method similar to the synthesis method of EX 68. | NMR1; 1.72-1.81 (2H, m), 1.83-1.96 (2H, m), 2.72 (3H, s), 3.38-3.48 (4H, m), 3.39-3.66 (4H, m), 3.68-3.78 (2H, m), 4.04 (2H, t, J = 6.1Hz), 5.12 (2H, d, J = 9.1Hz), 5.71 (2H, d, J = 13.4Hz), 6.30 (1H, d, J = 9.5Hz), 6.81 (1H, dd, J = 8.6Hz, 2.4Hz), 6.84 (1H, d, J = 2.4Hz), 6.96 (1H, d, J = 7.5Hz), 7.28-7.37 (2H, m), 7.49 (1H, d, J = 5.5Hz), 7.55 (1H, d, J = 8.7Hz), 7.71 (1H, d, J = 8.0Hz), 7.77 (1H, d, J = 5.5Hz), 7.80 (1H, d, J = 9.5Hz), 8.23 (1H, dd, J = 7.9Hz, 1.8Hz), 8.60 (1H, dd, J = 4.8Hz, 1.8Hz), 11.6 (1H, s). |
| EX82 | 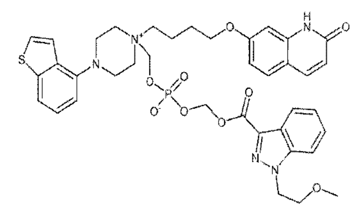 | Using chloromethyl 1-(2-methoxyethyl)-1H-indazole-3-carboxylate synthesized in REX 66 (0.74 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (((1-(2-methoxyethyl)-1H-indazole-3-carbonyl)oxy)methyl) phosphate (300 mg) was obtained in a method similar to the synthesis method of EX 68. | NMR1; 1.66-1.78 (2H, m), 1.79-1.93 (2H, m), 3.12 (3H, s), 3.35-3.45 (4H, m), 3.47-3.66 (4H, m), 3.68-3.78 (4H, m), 3.99 (2H, t, J = 6.1Hz), 4.66 (2H, t, J = 5.2Hz), 5.12 (2H, d, J = 8.8Hz), 5.89 (2H, d, J = 13.6Hz), 6.30 (1H, d, J = 9.5Hz), 6.78 (1H, dd, J = 8.6Hz, 2.4Hz), 6.82 (1H, d, J = 2.4Hz), 6.89 (1H, d, J = 7.5Hz), 7.29 (1H, t, J = 7.9Hz), 7.33 (1H, t, J = 7.9Hz), 7.42-7.49 (2H, m), 7.54 (1H, d, J = 8.7Hz), 7.71 (1H, d, J = 8.1Hz), 7.77 (1H, d, J = 5.5Hz), 7.78-7.82 (2H, m), 8.14 (1H, d, J = 8.2Hz) 11.6 (1H, s). |
| EX83 | 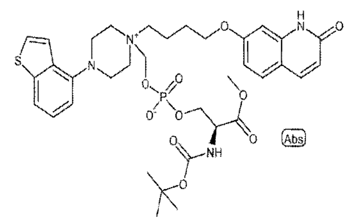 | Using methyl (tert-butoxycarbonyl)-L-serinate (3 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (S)-(4-(benzo[b]thiophen-4-yl)-1-(4-((2-oxo-1,2-dihydroquinolin-7-yl)oxy)butyl)piperazin-1-ium-1-yl)methyl (2-(tert-butoxycarbonyl)amino)-3-methoxy-3-oxopropyl) phosphate (35 mg) was obtained in a method similar to the synthesis of EX 45. | NMR1; 1.35 (9H, s), 1.80-1.89 (2H, m), 1.90-2.00 (2H, m), 3.40-3.50 (4H, m), 3.57-3.70 (7H, m), 3.70-3.80 (2H, m), 3.92-4.08 (1H, m), 4.10 (2H, t, J = 6.1Hz), 4.15-4.22 (1H, m), 5.09 (2H, d, J = 8.7Hz), 6.31 (1H, dd, J = 9.5Hz, 1.5Hz), 6.83 (1H, dd, J = 8.6Hz, 2.4Hz), 6.86 (1H, d, J = 2.4Hz), 7.01 (1H, d, J = 7.5Hz), 7.33 (1H, t, J = 7.8Hz), 7.52 (1H, d, J = 5.5Hz), 7.57 (1H, d, J = 8.6Hz), 7.72 (1H, d, J = 8.0Hz), 7.77 (1H, d, J = 5.5Hz), 7.81 (1H, d, J = 9.5Hz), 11.7 (1H, s). |

| | | | |
|---|---|---|---|
| EX84 | 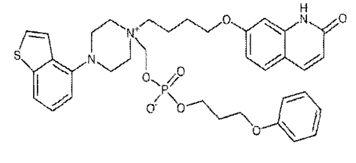 | Using 3-phenoxypropan-1-ol (5 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), 4-(benzo[b]thiophen-4-yl)-1-(4-((2-oxo-1,2-dihydroquinolin-7-yl)oxy)butyl)piperazin-1-ium-1-yl)methyl (3-phenoxypropyl) phosphate (160 mg) was obtained in a method similar to the synthesis of EX 45. | NMR1; 1.71-1.82 (2H, m), 1.85-2.01 (4H, m), 3.40-3.50 (4H, m), 3.50-3.61 (4H, m), 3.89 (2H, sext, J = 6.4Hz), 4.02 (2H, t, J = 6.3Hz), 4.08 (2H, t, J = 6.1Hz), 5.08 (2H, d, J = 9.1Hz), 6.30 (1H, d, J = 9.5Hz), 6.81 (1H, dd, J = 8.6Hz, 2.4Hz), 6.85-6.94 (4H, m), 6.99 (1H, d, J = 7.4Hz), 7.22-7.27 (2H, m), 7.32 (1H, t, J = 7.8Hz), 7.50 (1H, d, J = 5.5Hz), 7.55 (1H, d, J = 8.7Hz), 7.71 (1H, d, J = 8.1Hz), 7.77 (1H, d, J = 5.5Hz), 7.80 (1H, d, J = 9.5Hz), 11.7 (1H, s) . |
| EX85 | 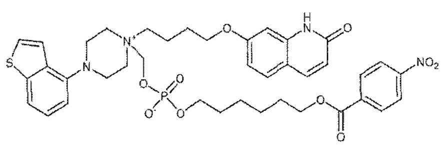 | Using 6-hydroxyhexyl 4-nitrobenzoate synthesized in REX 15 (1.2 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (6-((4-nitrobenzoyl)oxy)hexyl) phosphate (140 mg) was obtained in a method similar to the synthesis of EX 56. | NMR1; 1.30-1.45 (4H, m), 1.46-1.57 (2H, m), 1.66-1.75 (2H, m), 1.77-1.87 (2H, m), 1.90-2.00 (2H, m), 3.40-3.50 (4H, m), 3.55-3.68 (4H, m), 3.69-3.80 (4H, m), 4.09 (2H, t, J = 6.1Hz), 4.29 (2H, t, J = 6.6Hz), 5.09 (2H, d, J = 9.0Hz), 6.28 (1H, d, J = 9.5Hz), 6.80 (1H, dd, J = 8.6Hz, 2.4Hz), 6.88 (1H, d, J = 2.4Hz), 7.00 (1H, d, J = 7.4Hz), 7.31 (1H, t, J = 7.8Hz), 7.50 (1H, d, J = 5.5Hz), 7.54 (1H, d, J = 8.7Hz), 7.70 (1H, d, J = 8.0Hz), 7.76 (1H, d, J = 5.5Hz), 7.78 (1H, d, J = 9.5Hz), 8.17 (2H, d, J = 9.0Hz), 8.33 (2H, d, J = 9.0Hz), 11.7 (1H, s) . |
| EX86 | 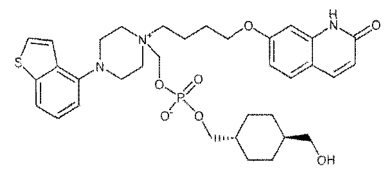 | Using trans-1,4-cyclohexanedimethanol (3.83 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g), (4-(benzo[b]thiophen-4-yl)-1-(4-((2-oxo-1,2-dihydroquinolin-7-yl)oxy)butyl)piperazin-1-ium-1-yl)methyl (((1r,4r)-4-(hydroxymethyl)cyclohexyl)methyl) phosphate (160 mg) was obtained in a method similar to the synthesis of EX 44. | NMR1; 0.75-0.95 (4H, m), 1.20-1.30 (1H, m), 1.35-1.45 (1H, m), 1.71 (4H, d, J = 8.6Hz),1.79-1.88 (2H, m), 1.89-2.00 (2H, m), 3.18 (2H, t, J = 5.4Hz), 3.40-3.49 (4H, m), 3.51 (2H, t, J = 6.4Hz), 3.55-3.67 (4H, m), 3.70-3.80 (2H, m), 4.10 (2H, t, J = 6.1Hz), 4.35 (1H, t, J = 5.3Hz), 5.08 (2H, d, J = 9.0Hz), 6.30 (1H, d, J = 9.5Hz), 6.82 (1H, dd, J = 8.6Hz, 2.4Hz), 6.89 (1H, d, J = 2.4Hz), 7.01 (1H, d, J = 7.5Hz), 7.32 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.80 (1H, d, J = 9.5Hz), 11.7 (1H, s). |
| EX87 | 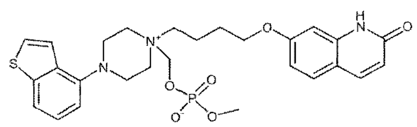 | A solution of (4-(benzo[b]thiophen-4-yl)-1-(4-((2-oxo-1,2-dihydroquinolin-7-yl)oxy)butyl)piperazin-1-ium-1-yl)methyl hydrogen phosphate (1.0 g) in a mixed solvent of MeOH (30 mL) and water (3 mL) was suspended. WSC·HCl (1.06 g) was added, and the resulting mixture was stirred at 40° C for 5 hours. Acetone and AcOEt were added to the reaction mixture, and the resulting mixture was concentrated under reduced pressure three times to azeotropically remove water. The concentrate was directly purified by silica gel column chromatography (eluent: DCM-MeOH). The resulting product was suspended in water (4.5 mL). Acetone (9 mL) was added to dissolve the suspension. The obtained solution was allowed to stand at room temperature overnight, resulting in precipitation of crystals. The crystals precipitated were collected by filtration. After air-drying at 25° C, (4-(benzo[b]thiophen-4-yl)-1-(4-((2-oxo-1,2-dihydroquinolin-7-yl)oxy)butyl)piperazin-1-ium-1-yl)methyl methyl phosphate (723 mg) was obtained. | NMR1; 1.82-1.98 (4H, m), 3.39-3.49 (7H, m), 3.53-3.67 (4H, m), 3.72-3.79 (2H, m), 3.55-3.68 (4H, m), 3.68-3.79 (4H, m), 4.10 (2H, t, J = 6.1Hz), 5.10 (2H, d, J = 8.9Hz), 6.30 (1H, dd, J = 9.4Hz, 1.7Hz), 6.82 (1H, dd, J = 8.7Hz, 2.4Hz), 6.89 (1H, d, J = 2.4Hz), 7.00 (1H, d, J = 7.4Hz), 7.32 (1H, t, J = 7.8Hz), 7.51 (1H, d, J = 5.5Hz), 7.56 (1H, d, J = 8.7Hz), 7.71 (1H, d, J = 8.1Hz), 7.77 (1H, d, J = 5.5Hz), 7.80 (1H, d, J = 9.4Hz), 11.7 (1H, s). |

| | | | |
|---|---|---|---|
| EX88 | 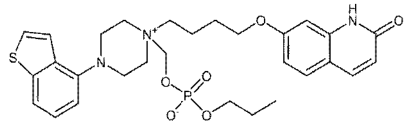 | Using 1-propyl alcohol (20 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzo[b]thiophen-4-yl)-1-(4-((2-oxo-1,2-dihydroquinolin-7-yl)oxy)butyl)piperazin-1-ium-1-yl)methyl propyl phosphate (326 mg) was obtained in a method similar to the synthesis of EX 45. | NMR1; 0.85 (3H, t, J=7.4Hz), 1.52 (2H, h, J=7.1Hz), 1.80-1.88 (2H, m), 1.92-1.97 (2H, m), 3.37-3.53 (6H, m), 3.54-3.71 (4H, m), 3.72-3.80 (2H, m), 4.11 (2H, t, J=6.1Hz), 5.09 (2H, d, J=9.1Hz), 6.30 (1H, d, J=9.3Hz), 6.83 (1H, dd, J=8.7Hz, 2.4Hz), 6.89 (1H, d, J=2.4Hz), 7.01 (1H, d, J=7.3Hz), 7.33 (1H, t, J=7.8Hz), 7.52 (1H, dd, J=5.5Hz, 0.9Hz), 7.57 (1H, d, J=8.7Hz), 7.72 (1H, d, J=8.1Hz), 7.77 (1H, d, J=5.6Hz), 7.81 (1H, d, J=9.4Hz), 11.69 (1H, s). |
| EX89 | 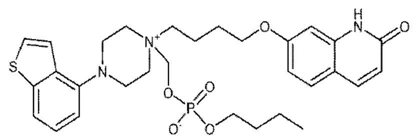 | NaI (208 mg) was added to a solution of 4-(benzo[b]thiophen-4-yl)-1-(((dibutoxyphosphoryl)oxy)methyl)-1-(4-((2-oxo-1,2-dihydroquinolin-7-yl)oxy)butyl)piperazin-1-ium iodide (545 mg) in acetone (12 mL) at room temperature, and the reaction mixture was stirred at 60° C under nitrogen atmosphere. After 3 hours, NaI (104 mg) and acetone (6 mL) were added, and the resulting mixture was stirred for 3 hours at 60° C and stirred at room temperature overnight. The resulting mixture was stirred for 3 hours at 60° C and stirred at room temperature overnight. 50 mL of DCM/MeOH = 9/1 was added. AcOH (0.398 mL) was dissolved in $H_2O$ (30 mL) and the resulting solution was added dropwise. The insoluble matter was filtered out and washed thoroughly with $H_2O$. The residue was purified by silica gel chromatography (DCM/MeOH) to give (4-(benzo[b]thiophen-4-yl)-1-(4-((2-oxo-1,2-dihydroquinolin-7-yl)oxy)butyl)piperazin-1-ium-1-yl)methyl butyl phosphate (242 mg). | NMR1; 0.86(3H, t, J=7.4Hz), 1.23-1.37(2H, m), 1.42-1.54(2H, m), 1.79-1.87(2H, m), 1.89-2.00(2H, m), 3.43-3.47(4H, m), 3.53-3.66(4H, m), 3.70(2H, q, J=6.7Hz), 3.73-3.80(2H, m), 4.10(2H, t, J=6.1Hz), 5.09(2H, d, J=9.1Hz), 6.30(1H, dd, J=9.5Hz, 2.0Hz), 6.82(1H, dd, J=8.7Hz, 2.4Hz), 6.89(1H, d, J=2.4Hz), 7.01(1H, d, J=7.2Hz), 7.33(1H, t, J=7.9Hz), 7.51(1H, dd, J=5.5Hz, 0.9Hz), 7.56(1H, d, J=8.7Hz), 7.71(1H, d, J=8.1Hz), 7.77(1H, d, J=5.5Hz), 7.81(1H, d, J=9.5Hz), 11.69(1H, s). |
| EX90 | 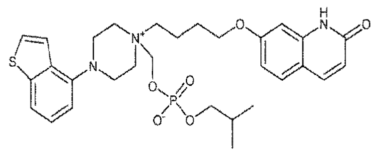 | Using isobutyl alcohol (5 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzo[b]thiophen-4-yl)-1-(4-((2-oxo-1,2-dihydroquinolin-7-yl)oxy)butyl)piperazin-1-ium-1-yl)methyl isobutyl phosphate (125 mg) was obtained in a method similar to the synthesis of EX 45. | NMR1; 0.84(6H, d, J=6.7Hz), 1.68-1.80(1H, m), 1.81-1.87(2H, m), 1.90-2.00(2H, m), 3.43-3.52(4H, m), 3.53-3.66(6H, m), 3.72-3.80(2H, m), 4.10(2H, t, J=6.1Hz), 5.09(2H, d, J=9.2Hz), 6.30(1H, dd, J=9.5Hz, 1.6Hz), 6.82(1H, dd, J=8.7Hz, 2.4Hz), 6.89(1H, d, J=2.4Hz), 7.01(1H, dd, J=7.7Hz, 0.9Hz), 7.33(1H, t, J=7.8Hz), 7.51(1H, dd, J=5.6Hz, 0.9Hz), 7.56(1H, d, J=8.7Hz), 7.71(1H, d, J=8.1Hz), 7.77(1H, d, J=5.6Hz), 7.81(1H, d, J=9.4Hz), 11.68(1H, s). |
| EX91 | 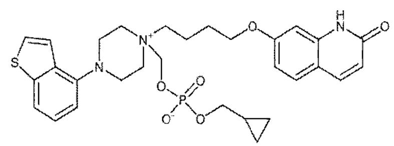 | DIPEA (0.482 mL), 1-(chloro-1-pyrrolidinylmethylene)pyrrolidinium hexafluorophosphate (918 mg), and cyclopropyl methanol (0.373 mL) were added to a solution of (4-(benzo[b]thiophen-4-yl)-1-(4-((2-oxo-1,2-dihydroquinolin-7-yl)oxy)butyl)piperazin-1-ium-1-yl)methyl hydrogen phosphate (500 mg) in DMF (5 mL) at room temperature, and stirred at 80° C for 8 hours. The reaction system was returned to room temperature. H2O was added, and the resulting mixture was stirred overnight. The precipitate was filtered out and washed thoroughly with H2O. The residue was purified by silica gel column chromatography (DCM/MeOH). Acetone/$H_2O$= 1/1 was added, and the precipitate was filtered out and allowed to stand at room temperature overnight to give (4-(benzo[b]thiophen-4-yl)-1-(4-((2-oxo-1,2-dihydroquinolin-7-yl)oxy)butyl)piperazin-1-ium-1-yl)methyl (cyclopropylmethyl) phosphate (84 mg). | NMR1; 0.14-0.22(2H, m), 0.39-0.48(2H, m), 1.80-1.87(2H, m), 1.89-2.00(2H, m), 3.30-3.41(2H, m), 3.43-3.47(4H, m), 3.55(2H, t, J=7.2Hz), 3.54-3.66(2H, m), 3.72-3.80(2H, m), 4.10(2H, t, J=6.1Hz), 5.09(2H, d, J=9.2Hz), 6.30(1H, d, J=9.4Hz), 6.82(1H, dd, J=8.7Hz, 2.4Hz), 6.89(1H, d, J=2.4Hz), 7.01(1H, d, J=7.6Hz), 7.33(1H, t, J=7.9Hz), 7.51(1H, dd, J=5.6Hz, 0.9Hz), 7.56(1H, d, J=8.7Hz), 7.71(1H, d, J=8.1Hz), 7.77(1H, d, J=5.5Hz), 7.81(1H, d, J=9.5Hz), 11.68(1H, s). |

| | | | |
|---|---|---|---|
| EX92 | 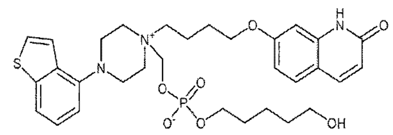 | (4-(Benzo[b]thiophen-4-yl)-1-(4-(2-oxo-1,2-dihydroquinolin-7-yl)oxy)butyl)piperazin-1-ium-1-yl)methyl hydrogen phosphate (3 g) was suspended in 1,5-pentanediol (80 mL). WSC·HCl (2.1 g) was added, and the resulting mixture was stirred at 60° C for 1 hour. Water was added to the reaction mixture, and the resulting mixture was concentrated. The concentrate was directly purified twice by medium-pressure liquid chromatography (silica gel, eluents: cOEt/DCM/MeOH). The resulting amorphous product was dissolved in a mixed solvent of acetone (8 mL) and water (8 mL). Water (4 mL) was added to the solution with stirring. The precipitated crystals were collected by filtration and air-dried at 25° C overnight to give (4-(benzo[b]thiophen-4-yl)-1-(4-((2-oxo-1,2-dihydroquinolin-7-yl)oxy)butyl)piperazin-1-ium-1-yl)methyl (4-hydroxypentyl) phosphate (2.2 g). | NMR1; 1.24-1.36(2H, m), 1.36-1.45(2H, m), 1.45-1.56(2H, m), 1.79-1.87(2H, m), 1.89-2.00(2H, m), 3.32-3.40(2H, m), 3.44(4H, d, J=5.9Hz), 3.53-3.66(4H, m), 3.70(2H, q, J=6.8Hz), 3.73-3.79(2H, m), 4.10(2H, t, J=6.1Hz), 4.39(1H, t, J=5.2Hz), 5.09(2H, d, J=9.1Hz), 6.30(1H, d, J=9.0Hz), 6.82(1H, dd, J=8.7Hz, 2.4Hz), 6.90(1H, d, J=2.4Hz), 7.00(1H, dd, J=7.8Hz, 0.8Hz), 7.32(1H, t, J=7.9Hz), 7.51(1H, dd, J=5.5Hz, 0.9Hz), 7.56(1H, d, J=8.7Hz), 7.71(1H, d, J=8.1Hz), 7.77(1H, d, J=5.6Hz), 7.80(1H, d, J=9.5Hz), 11.70(1H, s). |
| EX93 | 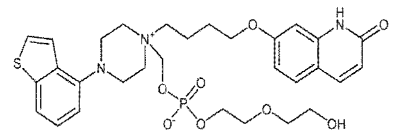 | Using diethylene glycol (20 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (2-(2-hydroxyethoxy)ethyl) phosphate (854 mg) was obtained in a method similar to the synthesis of EX 45. | NMR1; 1.80-1.88(2H, m), 1.89-2.01(2H, m), 3.36-3.55(10H, m), 3.55-3.67(4H, m), 3.72-3.79(2H, m), 3.79-3.87(2H, m), 4.10(2H, t, J=6.1Hz), 4.72(1H, t, J=5.5Hz), 5.10(2H, d, J=8.9Hz), 6.30(1H, dd, J=9.4Hz, 1.3Hz), 6.83(1H, dd, J=8.7Hz, 2.4Hz), 6.89(1H, d, J=2.5Hz), 7.01(1H, dd, J=7.8Hz, 0.9Hz), 7.32(1H, t, J=7.9Hz), 7.51(1H, dd, J=5.5Hz, 0.9Hz), 7.56(1H, d, J=8.7Hz), 7.71(1H, d, J=8.1Hz), 7.77(1H, d, J=5.5Hz), 7.81(1H, d, J=9.5Hz), 11.68(1H, s). |
| EX94 | 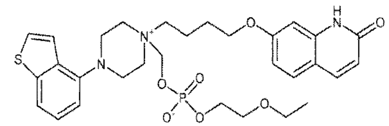 | Using 2-ethoxyethanol (3.7 mL) and (4-(benzo[b]thiophen-4-yl)-1-(4-(2-oxo-1,2-dihydroquinolin-7-yl)oxy)butyl)piperazin-1-ium-1-yl)methyl hydrogen phosphate (5 g), (4-(benzo[b]thiophen-4-yl)-1-(4-((2-oxo-1,2-dihydroquinolin-7-yl)oxy)butyl)piperazine-1-ium-1-yl)methyl (2-ethoxyethyl) phosphate (3.6 g) was obtained in a method similar to the synthesis of EX 56. | NMR1; 1.08(3H, t, J=7.0Hz), 1.80-1.87(2H, m), 1.89-2.00(2H, m), 3.41(2H, q, J=7.0Hz), 3.43-3.50(6H, m), 3.53-3.67(4H, m), 3.69-3.85(4H, m), 4.10(2H, t, J=6.1Hz), 5.09(2H, d, J=9.1Hz), 6.26-6.33(1H, m), 6.82(1H, dd, J=8.6Hz, 2.4Hz), 6.89(1H, d, J=2.4Hz), 7.01(1H, dd, J=7.6Hz, 0.8Hz), 7.33(1H, t, J=7.8Hz), 7.51(1H, dd, J=5.5Hz, 0.9Hz), 7.56(1H, d, J=8.7Hz), 7.71(1H, d, J=8.1Hz), 7.77(1H, d, J=5.5Hz), 7.80(1H, d, J=9.5Hz), 11.68(1H, s). |
| EX95 | 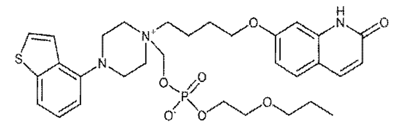 | Using ethylene glycol monopropyl ether (2 mL) and (4-(benzo[b]thiophen-4-yl)-1-(4-(2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (2-propoxyethyl) phosphate (397 mg) was obtained in a method similar to the synthesis of EX 56. | NMR1; 0.83(3H, t, J=7.4Hz), 1.48(2H, h, J=7.2Hz), 1.79-1.88(2H, m), 1.89-2.00(2H, m), 3.24-3.40(4H, m), 3.41-3.51(4H, m), 3.53-3.69(4H, m), 3.72-3.85(4H, m), 4.11(2H, t, J=6.1Hz), 5.10(2H, d, J=9.0Hz), 6.30(1H, dd, J=9.5Hz, 2.0Hz), 6.83(1H, dd, J=8.6Hz, 2.4Hz), 6.89(1H, d, J=2.4Hz), 7.01(1H, d, J=7.5Hz), 7.33(1H, t, J=7.9Hz), 7.52(1H, d, J=5.3Hz), 7.57(1H, d, J=8.6Hz), 7.72(1H, d, J=8.1Hz), 7.77(1H, d, J=5.5Hz), 7.81(1H, d, J=9.5Hz), 11.68(1H, s). |

| | | | |
|---|---|---|---|
| EX96 | 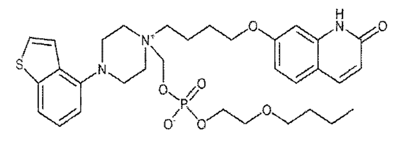 | Using ethylene glycol monobutyl ether (20 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (2-butoxyethyl) phosphate (359 mg) was obtained in a method similar to the synthesis of EX 45. | NMR3; 0.86(3H, t, J=7.4Hz), 1.25-1.38(2H, m), 1.45-1.57(2H, m), 1.94-2.04(2H, m), 2.06-2.16(2H, m), 3.45(2H, t, J=6.7Hz), 3.50-3.54(4H, m), 3.57-3.64(2H, m), 3.65-3.73(2H, m), 3.74-3.81(2H, m), 3.82-3.92(2H, m), 4.00-4.08(2H, m), 4.20(2H, t, J=5.9Hz), 5.20(2H, d, J=7.8Hz), 6.44(1H, d, J=9.4Hz), 6.89(1H, d, J=2.4Hz), 6.92(1H, dd, J=8.7Hz, 2.4Hz), 7.05(1H, dd, J=7.7Hz, 0.8Hz), 7.30(1H, t, J=7.9Hz), 7.49(1H, dd, J=5.5Hz, 0.9Hz), 7.57(1H, d, J=5.1Hz), 7.59(1H, d, J=2.0Hz), 7.65(1H, d, J=8.1Hz), 7.88(1H, d, J=9.4Hz). |
| EX97 | 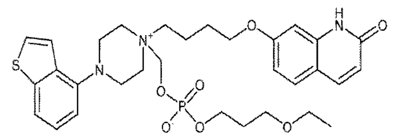 | Using 3-ethoxy-1-propanol (1 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzo[b]thiophen-4-yl)-1-(4-((2-oxo-1,2-dihydroquinolin-7-yl)oxy)butyl)piperazin-1-ium-1-yl)methyl (3-ethoxypropyl) phosphate (308 mg) was obtained in a method similar to the synthesis of EX 56. | NMR1; 1.07(3H, t, J=7.0Hz), 1.73(2H, p, J=6.4Hz), 1.79-1.87(2H, m), 1.92-1.97(2H, m), 3.36-3.42(4H, m), 3.42-3.47(4H, m), 3.51-3.67(4H, m), 3.71-3.80(4H, m), 4.10(2H, t, J=6.1Hz), 5.09(2H, d, J=9.0Hz), 6.30(1H, dd, J=9.5Hz, 2.0Hz), 6.82(1H, dd, J=8.7Hz, 2.4Hz), 6.89(1H, d, J=2.4Hz), 7.01(1H, d, J=7.5Hz), 7.32(1H, t, J=7.8Hz), 7.51(1H, dd, J=5.6Hz, 0.9Hz), 7.56(1H, d, J=8.7Hz), 7.71(1H, d, J=8.0Hz), 7.77(1H, d, J=5.5Hz), 7.80(1H, d, J=9.5Hz), 11.68(1H, s). |
| EX98 | 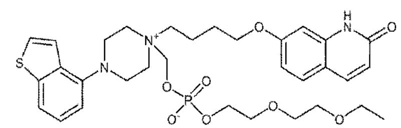 | Using diethylene glycol monoethyl ether (5 mL) and (4-(benzo[b]thiophen-4-yl)-1-(4-(2-oxo-1,2-dihydroquinolin-7-yl)oxy)butyl)piperazin-1-ium-1-yl)methyl hydrogen phosphate (0.5 g), (4-(benzo[b]thiophen-4-yl)-1-(4-((2-oxo-1,2-dihydroquinolin-7-yl)oxy)butyl)piperazin-1-ium-1-yl)methyl (2-(2-ethoxyethoxy)ethyl) phosphate (53 mg) was obtained in a method similar to the synthesis of EX 45. | NMR1; 1.06(3H, t, J=7.0Hz), 1.80-1.88(2H, m), 1.89-2.01(2H, m), 3.39(3H, q, J=7.0Hz), 3.42-3.48(7H, m), 3.48-3.54(4H, m), 3.54-3.67(2H, m), 3.71-3.85(4H, m), 4.10(2H, t, J=6.1Hz), 5.09(2H, d, J=9.0Hz), 6.30(1H, dd, J=9.5Hz, 1.7Hz), 6.82(1H, dd, J=8.7Hz, 2.4Hz), 6.88(1H, d, J=2.4Hz), 7.01(1H, d, J=7.6Hz), 7.33(1H, t, J=7.9Hz), 7.51(1H, dd, J=5.5Hz, 0.8Hz), 7.56(1H, d, J=8.7Hz), 7.71(1H, d, J=8.1Hz), 7.77(1H, d, J=5.6Hz), 7.81(1H, d, J=9.5Hz), 11.67(1H, s). |

| | | | |
|---|---|---|---|
| EX99 | 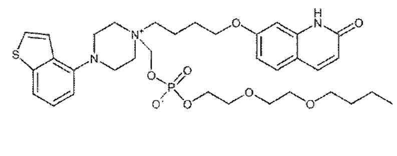 | (4-(Benzo[b]thiophen-4-yl)-1-(4-(2-oxo-1,2-dihydroquinolin-7-yl)oxy)butyl)piperazin-1-ium-1-yl)methyl hydrogen phosphate (0.5 g) was suspended in diethylene glycol monobutyl ether (20 mL) and water (1 mL). WSC·HCl (0.53 g) was added to the suspension, and the resulting mixture was stirred at 60° C for 5 hours. AcOEt was added to the reaction mixture and the resulting mixture was allowed to stand. The AcOEt layer was removed by decantation and the residue was purified twice by silica gel column chromatography (1st purification: silica gel, 2nd purification: amino silica gel, eluent: DCM/MeOH). The obtained amorphous product was suspended in water, and acetone was added to dissolve the suspension. After stirring at room temperature overnight, the crystals precipitated were collected by filtration and air-dried at 25° C overnight to give (4-(benzo[b]thiophen-4-yl)-1-(4-((2-oxo-1,2-dihydroquinolin-7-yl)oxy)butyl)piperazin-1-ium-1-yl)methyl(2-(2-butoxyethoxy)ethyl) phosphate (177 mg). | NMR1; 0.82(3H, t, J=7.3Hz), 1.18-1.32(2H, m), 1.36-1.48(2H, m), 1.80-1.88(2H, m), 1.89-2.02(2H, m), 3.23-3.37(2H, m), 3.40-3.48(6H, m), 3.48-3.54(4H, m), 3.54-3.69(4H, m), 3.71-3.85(4H, m), 4.10(2H, t, J=6.1Hz), 5.09(2H, d, J=9.1Hz), 6.30(1H, dd, J=9.4Hz, 2.0Hz), 6.82(1H, dd, J=8.6Hz, 2.4Hz), 6.89(1H, d, J=2.4Hz), 7.01(1H, dd, J=7.7Hz, 0.9Hz), 7.32(1H, t, J=7.8Hz), 7.51(1H, dd, J=5.6Hz, 0.8Hz), 7.56(1H, d, J=8.7Hz), 7.71(1H, d, J=8.1Hz), 7.77(1H, d, J=5.5Hz), 7.80(1H, d, J=9.4Hz), 11.67(1H, s). |
| EX100 | 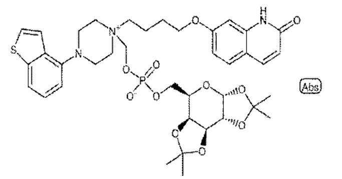 | Using 1,2:3,4-di-O-isopropylidene-α-D-galactopyranose (2.2 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (((3aR,5R,5aS,8aS,8bR)-2,2,7,7-tetramethyltetrahydro-5H-bis([1,3]dioxolo)[4,5-b:4',5'-d]pyran-5-yl)methyl)phosphate (863 mg) was obtained in a method similar to the synthesis of EX 56. | NMR1; 1.25(3H, s), 1.26(3H, s), 1.31(3H, s), 1.43(3H, s), 1.80-1.87(2H, m), 1.89-2.00(2H, m), 3.40-3.49(5H, m), 3.52-3.68(4H, m), 3.70-3.86(3H, m), 3.86-3.94(1H, m), 4.10(2H, t, J=6.1Hz), 4.23(1H, dd, J=8.0Hz, 1.9Hz), 4.32(1H, dd, J=5.0Hz, 2.4Hz), 4.57(1H, dd, J=8.0Hz, 2.3Hz), 5.04 - 5.17(2H, m), 5.44(1H, d, J=5.0Hz), 6.30(1H, dd, J=9.5Hz, 1.9Hz), 6.83(1H, dd, J=8.6Hz, 2.4Hz), 6.88(1H, d, J=2.4Hz), 7.01(1H, dd, J=7.7Hz, 0.8Hz), 7.32(1H, t, J=7.9Hz), 7.51(1H, dd, J=5.5Hz, 0.9Hz), 7.56(1H, d, J=8.7Hz), 7.71(1H, d, J=8.0Hz), 7.77(1H, d, J=5.5Hz), 7.81(1H, d, J=9.5Hz), 11.67(1H, s). |
| EX101 | 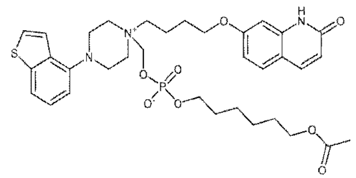 | Using (4-(benzo[b]thiophen-4-yl)-1-(4-(2-oxo-1,2-dihydroquinolin-7-yl)oxy)butyl)piperazin-1-ium-1-yl)methyl (6-hydroxyhexyl) phosphate (0.5 g), 6-acetoxyhexyl ((4-(benzo[b]thiophen-4-yl)-1-(4-((2-oxo-1,2-dihydroquinolin-7-yl)oxy)butyl)piperazin-1-ium-1-yl)methyl) phosphate (375 mg) was obtained in the same manner as in EX 15. | NMR1; 1.24-1.32(4H, m), 1.43-1.57(4H, m), 1.79-1.87(2H, m), 1.92-1.96(2H, m), 1.98(3H, s), 3.43-3.49(4H, m), 3.53-3.66(4H, m), 3.69(2H, q, J=6.7Hz), 3.72-3.79(2H, m), 3.95(2H, t, J=6.7Hz), 4.10(2H, t, J=6.1Hz), 5.09(2H, d, J=9.1Hz), 6.30(1H, d, J=9.5Hz), 6.82(1H, dd, J=8.7Hz, 2.4Hz), 6.90(1H, d, J=2.4Hz), 7.00(1H, d, J=7.6Hz), 7.32(1H, t, J=7.9Hz), 7.51(1H, dd, J=5.6Hz, 0.8Hz), 7.56(1H, d, J=8.7Hz), 7.71(1H, d, J=8.0Hz), 7.77(1H, d, J=5.5Hz), 7.80(1H, d, J=9.5Hz), 11.70(1H, s). |

| | | | |
|---|---|---|---|
| EX102 | 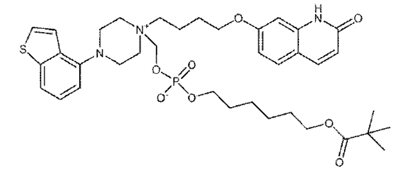 | Using 6-hydroxyhexyl pivalate (1.34 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.8 g), (4-(benzo[b]thiophen-4-yl)-1-(4-((2-oxo-1,2-dihydroquinolin-7-yl)oxy)butyl)piperazin-1-ium-1-yl)methyl (6-(pivaloyloxy)hexyl) phosphate (397 mg) was obtained in a manner similar to the synthesis in EX 56. | NMR1; 1.11(9H, s), 1.25-1.33(4H, m), 1.45-1.57(4H, m), 1.79-1.87(2H, m), 1.92-1.97(2H, m), 3.43-3.47(4H, m), 3.53-3.66(4H, m), 3.69(2H, q, J=6.7Hz), 3.72-3.79(2H, m), 3.96(2H, t, J=6.5Hz), 4.06-4.15(2H, m), 5.09(2H, d, J=9.1Hz), 6.30(1H, dd, J=9.5Hz, 1.4Hz), 6.82(1H, dd, J=8.7Hz, 2.4Hz), 6.90(1H, d, J=2.4Hz), 7.00(1H, dd, J=7.7Hz, 0.8Hz), 7.32(1H, t, J=7.9Hz), 7.51(1H, dd, J=5.6Hz, 0.8Hz), 7.56(1H, d, J=8.7Hz), 7.71(1H, d, J=8.1Hz), 7.77(1H, d, J=5.5Hz), 7.80(1H, d, J=9.5Hz), 11.70(1H, s). |
| EX103 | 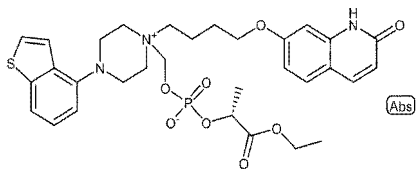 | Tf2O (3.5 mL) was added to a solution of (S)-(-)-lactic acid ethyl ester (2.1 mL) in DCM (20 mL) under ice-cold conditions and the resulting mixture was stirred for 15 minutes. Pyridine (1.8 mL) was added thereto and stirred for 15 minutes. The reaction system was concentrated, and H2O was added to the residue. The resulting mixture was extracted with Et2O. The organic layer was washed with brine, dried over Na2SO4, and concentrated to give 4.3 g of an intermediate. DIPEA (3.2 mL) was added to a suspension of (4-(benzo[b]thiophen-4-yl)-1-(4-((2-oxo-1,2-dihydroquinolin-7-yl)oxy)butyl)piperazin-1-ium-1-yl)methyl hydrogen phosphate (2 g) in MeCN/H2O (40 mL) (3/1) at room temperature and the resulting mixture was heated to 80°C. A solution of the intermediate in MeCN was added thereto. After 30 minutes, the reaction system was ice-cooled and H2O (60 mL) was added. The resulting mixture was extracted with DCM/MeOH = 9/1. The organic layer was concentrated, and the residue was purified by silica gel chromatography (DCM/MeOH). H2O (6 mL) and acetone (10 mL) were added to the amorphous product (1.3 g). While the resulting mixture was stirred, H2O (5mL) was added and stirring was performed overnight. The precipitate was filtered out, washed thoroughly with H2O, and dried at room temperature overnight to give (R)-(4-(benzo[b]thiophen-4-yl)-1-(4-((2-oxo-1,2-dihydroquinolin-7-yl)oxy)butyl)piperazin-1-ium-1-yl)methyl(1-ethoxy-1-oxopropan-2-yl) phosphate (1.27 g). | NMR1; 1.17(3H, t, J=7.1Hz), 1.32(3H, d, J=6.9Hz), 1.81-1.87(2H, m), 1.88-1.99(2H, m), 3.42-3.47(4H, m), 3.51-3.68(4H, m), 3.73-3.83(2H, m), 4.02-4.14(4H, m), 4.55-4.66(1H, m), 5.08(2H, d, J=9.0Hz), 6.30(1H, dd, J=9.5Hz, 1.7Hz), 6.83(1H, dd, J=8.6Hz, 2.4Hz), 6.87(1H, d, J=2.4Hz), 7.01(1H, d, J=7.6Hz), 7.33(1H, t, J=7.9Hz), 7.52(1H, dd, J=5.6Hz, 0.8Hz), 7.56(1H, d, J=8.7Hz), 7.72(1H, d, J=8.1Hz), 7.77(1H, d, J=5.5Hz), 7.81(1H, d, J=9.5Hz), 11.65(1H, s). |
| EX104 | 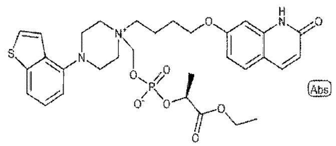 | Using (R)-(-)-lactic acid ethyl ester (2.1 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (2 g), (S)-(4-(benzo[b]thiophen-4-yl)-1-(4-((2-oxo-1,2-dihydroquinolin-7-yl)oxy)butyl)piperazin-1-ium-1-yl)methyl (1-ethoxy-1-oxopropan-2-yl) phosphate (1.14 g) was obtained in a method similar to the synthesis of EX 56. | NMR1; 1.17(3H, t, J=7.1Hz), 1.33(3H, d, J=6.8Hz), 1.79-1.87(2H, m), 1.88-1.99(2H, m), 3.41-3.50(4H, m), 3.53-3.69(4H, m), 3.70-3.83(2H, m), 4.02-4.15(4H, m), 4.55-4.66(1H, m), 5.09(2H, d, J=9.0Hz), 6.30(1H, d, J=9.5Hz), 6.83(1H, dd, J=8.6Hz, 2.4Hz), 6.87(1H, d, J=2.4Hz), 7.01(1H, d, J=7.6Hz), 7.33(1H, t, J=7.9Hz), 7.52(1H, dd, J=5.6Hz, 0.8Hz), 7.57(1H, d, J=8.7Hz), 7.72(1H, d, J=8.1Hz), 7.77(1H, d, J=5.5Hz), 7.81(1H, d, J=9.5Hz), 11.65(1H, s). |

| | | | |
|---|---|---|---|
| EX105 | 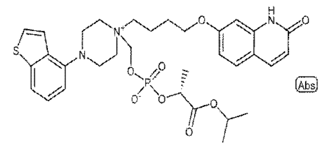 | Using (S)-(-)-lactic acid isopropyl ester synthesized in REX 33 (1.87 g) and (4-(benzo[b]thiophen-4-yl)-1-(4-((2-oxo-1,2-dihydroquinolin-7-yl)oxy)butyl)piperazine-1-ium-1-yl)methyl hydrogen phosphate (1.2 g), (R)-(4-(benzo[b]thiophen-4-yl)-1-(4-((2-oxo-1,2-dihydroquinolin-7-yl)oxy)butyl)piperazin-1-ium-1-yl)methyl (1-isopropoxy-1-oxopropan-2-yl) phosphate (425 mg) was obtained in a method similar to the synthesis of EX 56. | NMR1; 1.17(6H, dd, J=6.2Hz, 1.3Hz), 1.31(3H, d, J=6.8Hz), 1.78-1.87(2H, m), 1.89-2.00(2H, m), 3.43-3.51(4H, m), 3.53-3.70(2H, m), 3.70-3.82(2H, m), 4.09(2H, t, J=6.0Hz), 4.51-4.63(1H, m), 4.80-4.95(1H, m), 5.09(2H, d, J=9.1Hz), 6.30(1H, d, J=9.5Hz), 6.83(1H, dd, J=8.6Hz, 2.4Hz), 6.87(1H, d, J=2.3Hz), 7.01(1H, d, J=7.7Hz), 7.33(1H, t, J=7.8Hz), 7.52(1H, d, J=5.5Hz), 7.56(1H, d, J=8.7Hz), 7.72(1H, d, J=8.1Hz), 7.77(1H, d, J=5.5Hz), 7.81(1H, d, J=9.5Hz), 11.65(1H, s). |
| EX106 | 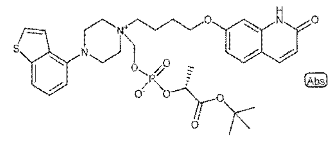 | Using (S)-tert-butyl 2-hydroxypropanoate (0.6 g) and (4-(benzo[b]thiophen-4-yl)-1-(4-(2-oxo-1,2-dihydroquinolin-7-yl)oxy)butyl)piperazin-1-ium-1-yl)methyl hydrogen phosphate (1 g), (R)-(4-(benzo[b]thiophen-4-yl)-1-(4-((2-oxo-1,2-dihydroquinolin-7-yl)oxy)butyl)piperazin-1-ium-1-yl)methyl (1-(tert-butoxy)-1-oxopropan-2-yl) phosphate (587 mg) was obtained in a method similar to the synthesis of EX 56. | NMR1; 1.29(3H, d, J=6.9Hz), 1.39(9H, s), 1.79-1.87(2H, m), 1.88-1.98(2H, m), 3.43-3.47(4H, m), 3.53-3.68(4H, m), 3.73-3.81(2H, m), 4.09(2H, t, J=6.0Hz), 4.43-4.55(1H, m), 5.09(2H, d, J=9.1Hz), 6.30(1H, dd, J=9.4Hz, 2.0Hz), 6.82(1H, dd, J=8.7Hz, 2.4Hz), 6.87(1H, d, J=2.5Hz), 7.00(1H, dd, J=7.8Hz, 0.9Hz), 7.32(1H, t, J=7.8Hz), 7.52(1H, dd, J=5.6Hz, 0.8Hz), 7.56(1H, d, J=8.7Hz), 7.71(1H, d, J=8.1Hz), 7.77(1H, d, J=5.5Hz), 7.81(1H, d, J=9.4Hz), 11.65(1H, s). |
| EX107 | 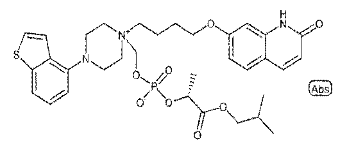 | Using (S)-(-)-lactic acid isobuyl ester synthesized in REX 34 (1.79 g) and (4-(benzo[b]thiophen-4-yl)-1-(4-(2-oxo-1,2-dihydroquinolin-7-yl)oxy)butyl)piperazin-1-ium-1-yl)methyl hydrogen phosphate (1 g), (R)-(4-(benzo[b]thiophen-4-yl)-1-(4-((2-oxo-1,2-dihydroquinolin-7-yl)oxy)butyl)piperazin-1-ium-1-yl)methyl (1-isobutoxy-1-oxopropan-2-yl) phosphate (335 mg) was obtained in a method similar to the synthesis of EX 56. | NMR1; 0.85(6H, dd, J=6.8Hz, 1.7Hz), 1.34(3H, d, J=6.9Hz), 1.79-1.89(2H, m), 1.89-1.99(2H, m), 3.42-3.47(4H, m), 3.53-3.68(4H, m), 3.76(2H, s), 3.79(1H, dd, J=10.5Hz, 6.5Hz), 3.86(1H, dd, J=10.5Hz, 6.8Hz), 4.09(2H, t, J=6.0Hz), 4.58-4.70(1H, m), 5.09(2H, d, J=9.0Hz), 6.30(1H, dd, J=9.5Hz, 1.9Hz), 6.82(1H, dd, J=8.6Hz, 2.4Hz), 6.87(2H, d, J=2.4Hz), 7.00(1H, d, J=7.6Hz), 7.32(1H, t, J=7.9Hz), 7.52(1H, dd, J=5.5Hz, 0.9Hz), 7.56(1H, d, J=8.7Hz), 7.72(1H, d, J=8.1Hz), 7.77(1H, d, J=5.5Hz), 7.80(1H, d, J=9.5Hz), 11.64(1H, s). |
| EX108 | 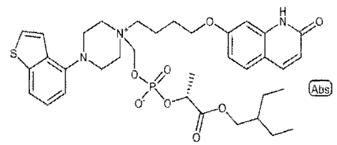 | Using 2-ethylbutyl (S)-2-hydroxypropanoate synthesized in REX 35 (947 mg) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.7 g), (R)-(4-(benzo[b]thiophen-4-yl)-1-(4-((2-oxo-1,2-dihydroquinolin-7-yl)oxy)butyl)piperazin-1-ium-1-yl)methyl (1-(2-ethylbutoxy)-1-oxopropan-2-yl) phosphate (345 mg) was obtained in a method similar to the synthesis of EX 56. | NMR1; 0.81(6H, d, J=7.4Hz), 1.18-1.30(4H, m), 1.33(3H, d, J=6.9Hz), 1.42-1.55(1H, m), 1.79-1.87(2H, m), 1.88-1.99(2H, m), 3.41-3.47(4H, m), 3.53-3.68(4H, m), 3.73-3.81(2H, m), 3.91(1H, dd, J=10.9Hz, 5.8Hz), 4.00(1H, dd, J=10.9Hz, 5.9Hz), 4.09(2H, t, J=6.1Hz), 4.57-4.69(1H, m), 5.09(2H, d, J=8.9Hz), 6.30(1H, dd, J=9.5Hz, 2.0Hz), 6.82(1H, dd, J=8.7Hz, 2.4Hz), 6.87(1H, d, J=2.4Hz), 7.00(1H, d, J=7.2Hz), 7.32(1H, t, J=7.9Hz), 7.52(1H, dd, J=5.5Hz, 0.8Hz), 7.56(1H, d, J=8.7Hz), 7.72(1H, d, J=8.0Hz), 7.77(1H, d, J=5.5Hz), 7.81(1H, d, J=9.5Hz), 11.65(1H, s). |

| | | | |
|---|---|---|---|
| EX109 | 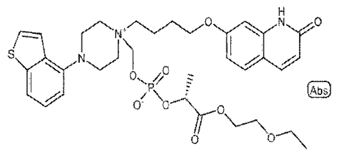 | Using 2-ethoxyethyl (S)-2-hydroxypropanoate synthesized in REX 36 (0.8 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (R)-(4-(benzo[b]thiophen-4-yl)-1-(4-((2-oxo-1,2-dihydroquinolin-7-yl)oxy)butyl)piperazin-1-ium-1-yl)methyl (1-(2-ethoxyethoxy)-1-oxopropan-2-yl) phosphate (157 mg) was obtained in a method similar to the synthesis of EX 56. | NMR1; 1.07(3H, t, J=7.0Hz), 1.33(3H, d, J=6.9Hz), 1.81–1.87(2H, m), 1.91–1.96(2H, m), 3.37–3.47(6H, m), 3.53(2H, t, J=4.8Hz), 3.54-3.61(4H, m), 3.72–3.81(2H, m), 4.06–4.24(4H, m), 4.58–4.70(1H, m), 5.08(2H, d, J=9.0Hz), 6.30(1H, dd, J=9.4Hz, 1.8Hz), 6.83(1H, dd, J=8.6Hz, 2.4Hz), 6.87(1H, d, J=2.4Hz), 7.01(1H, d, J=7.6Hz), 7.32(1H, t, J=7.8Hz), 7.52(1H, d, J=5.7Hz), 7.56(1H, d, J=8.7Hz), 7.71(1H, d, J=8.1Hz), 7.77(1H, d, J=5.5Hz), 7.80(1H, d, J=9.5Hz), 11.64(1H, s). |
| EX110 | 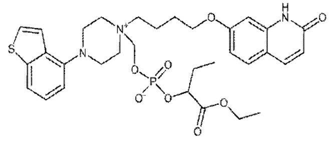 | Using ethyl 2-hydroxybutyrate (0.6 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.75 g), (4-(benzo[b]thiophen-4-yl)-1-(4-((2-oxo-1,2-dihydroquinolin-7-yl)oxy)butyl)piperazin-1-ium-1-yl)methyl (1-ethoxy-1-oxobutan-2-yl) phosphate (457 mg) was obtained in a method similar to the synthesis of EX 56. | NMR1; 0.87(3H, t, J=7.4Hz), 1.17(3H, t, J=7.1Hz), 1.58–1.78(2H, m), 1.80–1.87(2H, m), 1.88–2.00(2H, m), 3.42–3.50(4H, m), 3.54–3.68(4H, m), 3.73–3.81(2H, m), 4.01–4.18(4H, m), 4.44–4.53(1H, m), 5.09(2H, d, J=9.1Hz), 6.31(1H, dd, J=9.5Hz, 1.7Hz), 6.83(1H, dd, J=8.7Hz, 2.4Hz), 6.87(1H, d, J=2.4Hz), 7.01(1H, dd, J=7.8Hz, 0.9Hz), 7.33(1H, t, J=7.9Hz), 7.52(1H, dd, J=5.6Hz, 0.9Hz), 7.57(1H, d, J=8.7Hz), 7.72(1H, d, J=8.1Hz), 7.77(1H, d, J=5.6Hz), 7.81(1H, d, J=9.5Hz), 11.65(1H, s). |
| EX111 | 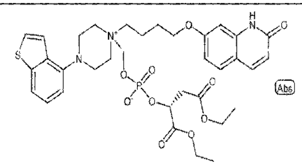 | Using L-(-)-diethyl malate (0.92 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (R)-(4-(benzo[b]thiophen-4-yl)-1-(4-((2-oxo-1,2-dihydroquinolin-7-yl)oxy)butyl)piperazin-1-ium-1-yl)methyl (1,4-diethoxy-1,4-dioxobutan-2-yl) phosphate (283 mg) was obtained in a method similar to the synthesis of EX 56. | NMR1; 1.16(6H, dt, J=8.6Hz, 7.1Hz), 1.79–1.88(2H, m), 1.89–2.00(2H, m), 2.62-2.86(2H, m), 3.43–3.47(4H, m), 3.52–3.69(4H, m), 3.73–3.80(2H, m), 3.98–4.16(6H, m), 4.82(1H, dt, J=9.6Hz, 6.0Hz), 5.09(2H, d, J=9.0Hz), 6.30(1H, dd, J=9.4Hz, 1.8Hz), 6.83(1H, dd, J=8.6Hz, 2.4Hz), 6.86(1H, d, J=2.4Hz), 7.01(1H, d, J=7.6Hz), 7.33(1H, t, J=7.9Hz), 7.52(1H, dd, J=5.5Hz, 0.8Hz), 7.56(1H, d, J=8.7Hz), 7.72(1H, d, J=8.1Hz), 7.77(1H, d, J=5.6Hz), 7.81(1H, d, J=9.5Hz), 11.63(1H, s). |
| EX112 | 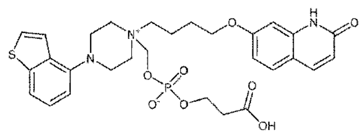 | TFA (0.1 mL) was added to a suspension of (4-(benzo[b]thiophen-4-yl)-1-(4-((2-oxo-1,2-dihydroquinolin-7-yl)oxy)butyl)piperazin-1-ium-1-yl)methyl (3-(tert-butoxy)-3-oxopropyl) phosphate (90 mg) in DCM (4 mL) under ice-cold conditions and the resulting mixture was stirred at room temperature for 2 hours. TFA (0.8 mL) was added and stirred for 2 hours. The reaction system was concentrated, and the residue was azeotroped with DCM and purified by silica gel column chromatography (DCM/MeOH) to give (4-(benzo[b]thiophen-4-yl)-1-(4-((2-oxo-1,2-dihydroquinolin-7-yl)oxy)butyl)piperazin-1-ium-1-yl)methyl (2-carboxyethyl) phosphate (45 mg). | NMR1; 1.79–1.87(2H, m), 1.89–2.01(2H, m), 2.28(2H, t, J=6.8Hz), 3.42–3.47(4H, m), 3.53–3.68(4H, m), 3.77(2H, d, J=11.5Hz), 3.93(2H, q, J=6.9Hz), 4.10(2H, t, J=6.5Hz), 5.12(2H, d, J=8.4Hz), 6.28(1H, d, J=9.5Hz), 6.78(1H, dd, J=8.6Hz, 2.4Hz), 7.01(1H, d, J=7.7Hz), 7.12(1H, s), 7.31(1H, t, J=7.9Hz), 7.49–7.56(2H, m), 7.70(1H, d, J=8.1Hz), 7.75(1H, d, J=5.5Hz), 7.78(1H, d, J=9.5Hz), 12.41(1H, s). |

| | | | |
|---|---|---|---|
| EX113 | 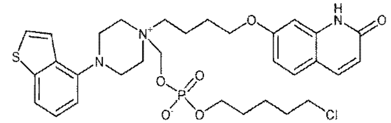 | Using 6-chlorohexanol (2 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (5-chloropentyl) phosphate (625 mg) was obtained in a method similar to the synthesis of EX 56. | NMR1; 1.24-1.41(4H, m), 1.42-1.55(2H, m), 1.60-1.72(2H, m), 1.79-1.87(2H, m), 1.89-2.01(2H, m), 3.43-3.47(4H, m), 3.52-3.65(6H, m), 3.69(2H, q, J=6.7Hz), 3.75(2H, t, J=6.9Hz), 4.06-4.15(2H, m), 5.09(2H, d, J=9.1Hz), 6.30(1H, dd, J=9.4Hz, 1.9Hz), 6.82(1H, dd, J=8.7Hz, 2.4Hz), 6.89(1H, d, J=2.4Hz), 7.01(1H, dd, J=7.7Hz, 0.8Hz), 7.32(1H, t, J=7.9Hz), 7.51(1H, dd, J=5.5Hz, 0.9Hz), 7.56(1H, d, J=8.7Hz), 7.71(1H, d, J=8.1Hz), 7.77(1H, d, J=5.5Hz), 7.80(1H, d, J=9.4Hz), 11.69(1H, s). |
| EX114 | 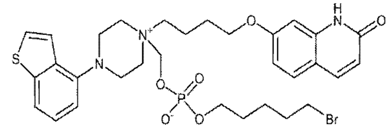 | Using 6-bromohexanol (1 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.2 g), (4-(benzo[b]thiophen-4-yl)-1-(4-(2-oxo-1,2-dihydroquinolin-7-yl)oxy)butyl)piperazin-1-ium-1-yl)methyl (5-bromopentyl) phosphate (738 mg) was obtained in a method similar to the synthesis of EX 56. | NMR1; 1.23-1.42(4H, m), 1.42-1.55(2H, m), 1.60-1.72(2H, m), 1.80-1.87(2H, m), 1.88-2.00(2H, m), 3.43-3.47(4H, m), 3.48(2H, t, J=6.7Hz), 3.53-3.66(4H, m), 3.69(2H, q, J=6.7Hz), 3.72-3.79(2H, m), 4.10(2H, t, J=6.1Hz), 5.09(2H, d, J=9.1Hz), 6.30(1H, dd, J=9.5Hz, 1.9Hz), 6.82(1H, dd, J=8.7Hz, 2.4Hz), 6.90(1H, d, J=2.4Hz), 7.00(1H, dd, J=7.7Hz, 0.9Hz), 7.33(1H, t, J=7.9Hz), 7.51(1H, dd, J=5.6Hz, 0.8Hz), 7.56(1H, d, J=8.7Hz), 7.71(1H, d, J=8.1Hz), 7.77(1H, d, J=5.5Hz), 7.80(1H, d, J=9.5Hz), 11.70(1H, s). |
| EX115 | 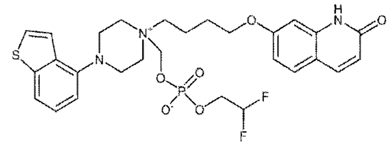 | DIPEA (0.482 mL) was added to a suspension of (4-(benzo[b]thiophen-4-yl)-1-(4-((2-oxo-1,2-dihydroquinolin-7-yl)oxy)butyl)piperazin-1-ium-1-yl)methyl hydrogen phosphate (300 mg) in MeCN/H2O (10 mL) (3/1) at room temperature, and the resulting mixture was heated to 80° C and 2,2-difluoroethyl trifluoromethanesulfonate (0.219 mL) was added. The resulting mixture was stirred for 30 minutes and then returned to room temperature. $H_2O$ (80 mL) was added and the precipitate was filtered out and washed thoroughly with $H_2O$. The residue was purified by silica gel column chromatography (DCM/MeOH). The obtained product was recrystallized in acetone/$H_2O$, and the crystals were collected by filtration and dried at room temperature to give (4-(benzo[b]thiophen-4-yl)-1-(4-(2-oxo-1,2-dihydroquinolin-7-yl)oxy)butyl)piperazin-1-ium-1-yl)methyl (2,2-difluoroethyl) phosphate (217 mg). | NMR1; 1.80-1.87 (2H, m), 1.92-1.97 (2H, m), 3.40-3.48 (4H, m), 3.53-3.68 (4H, m), 3.71-3.79 (2H, m), 3.86-4.01 (2H, m), 4.10 (2H, t, J=6.1Hz), 5.11 (2H, d, J=8.9Hz), 6.15 (1H, t, J=3.8Hz), 6.30 (1H, dd, J=9.6Hz, 1.9Hz), 6.82 (1H, dd, J=8.6Hz, 2.4Hz), 6.86 (1H, d, J=2.4Hz), 7.01 (1H, d, J=7.1Hz), 7.33 (1H, t, J=7.9Hz), 7.51 (1H, dd, J=5.6Hz, 0.8Hz), 7.56 (1H, d, J=8.6Hz), 7.71 (1H, d, J=8.1Hz), 7.77 (1H, d, J=5.5Hz), 7.80 (1H, d, J=9.4Hz), 11.64 (1H, s) |
| EX116 | 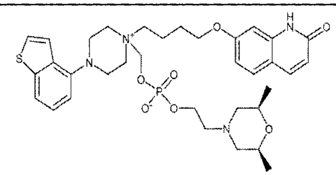 | Using cis-2,6-dimethylmorpholine (15 mL) and (4-(benzo[b]thiophen-4-yl)-1-(4-((2-oxo-1,2-dihydroquinolin-7-yl)oxy)butyl)piperazin-1-ium-1-yl)methyl (2-bromoethyl) phosphate (3 g), (4-(benzo[b]thiophen-4-yl)-1-(4-((2-oxo-1,2-dihydroquinolin-7-yl)oxy)butyl)piperazin-1-ium-1-yl)methyl (2-((2S,6R)-2,6-dimethylmorpholino)ethyl) phosphate (1.4 g) was obtained in the same manner as in Example 14. | NMR1; 0.98(3H, s), 1.00(3H, s), 1.61(2H, t, J=10.7Hz), 1.81-1.86(2H, m), 1.89-2.00(2H, m), 2.44(2H, t, J=6.2Hz), 2.70(2H, d, J=11.0Hz), 3.40-3.54(6H, m), 3.54-3.69(4H, m), 3.72-3.85(4H, m), 4.10(2H, t, J=6.1Hz), 5.10(2H, d, J=9.3Hz), 6.30(1H, dd, J=9.5Hz, 1.9Hz), 6.82(1H, dd, J=8.7Hz, 2.4Hz), 6.89(1H, d, J=2.5Hz), 7.01(1H, d, J=7.5Hz), 7.33(1H, t, J=7.8Hz), 7.51(1H, d, J=5.5Hz), 7.56(1H, d, J=8.7Hz), 7.72(1H, d, J=8.0Hz), 7.77(1H, d, J=5.6Hz), 7.80(1H, d, J=9.4Hz), 11.68(1H, s). |

| | | | |
|---|---|---|---|
| EX117 | 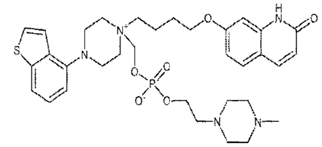 | Using 1-methylpiperazine (0.25 mL), NaI (138 mg), and (4-(benzo[b]thiophen-4-yl)-1-(4-((2-oxo-1,2-dihydroquinolin-7-yl)oxy)butyl)piperazin-1-ium-1-yl)methyl (2-bromoethyl) phosphate (0.3 g), (4-(benzo[b]thiophen-4-yl)-1-(4-((2-oxo-1,2-dihydroquinolin-7-yl)oxy)butyl)piperazin-1-ium-1-yl)methyl (2-(4-methylpiperazin-1-yl)ethyl) phosphate (33 mg) was obtained in the same manner as in Example 14. | NMR1; 1.80-1.88(2H, m), 1.92-1.97(2H, m), 2.09(3H, s), 2.23-2.27(4H, m), 2.35-2.40(4H, m), 2.45(2H, t, J=6.2Hz), 3.43-3.48(4H, m), 3.53-3.67(4H, m), 3.72-3.84(4H, m), 4.10(2H, t, J=6.1Hz), 5.10(2H, d, J=9.1Hz), 6.30(1H, d, J=9.4Hz), 6.82(1H, dd, J=8.7Hz, 2.4Hz), 6.89(1H, d, J=2.4Hz), 7.01(1H, d, J=7.7Hz), 7.33(1H, t, J=7.9Hz), 7.51(1H, dd, J=5.5Hz, 0.8Hz), 7.56(1H, d, J=8.7Hz), 7.71(1H, d, J=8.1Hz), 7.77(1H, d, J=5.5Hz), 7.80(1H, d, J=9.5Hz), 11.68(1H, s). |
| EX118 | 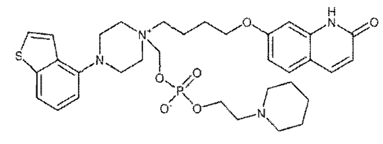 | Using piperidine (1 mL), DIPEA (0.12 mL), and (4-(benzo[b]thiophen-4-yl)-1-(4-((2-oxo-1,2-dihydroquinolin-7-yl)oxy)butyl)piperazin-1-ium-1-yl)methyl (2-bromoethyl) phosphate (0.22 g), (4-(benzo[b]thiophen-4-yl)-1-(4-((2-oxo-1,2-dihydroquinolin-7-yl)oxy)butyl)piperazin-1-ium-1-yl)methyl (2-(piperidin-1-yl)ethyl) phosphate (101 mg) was obtained in the same manner as in Example 14. | NMR1; 1.30-1.36(2H, m), 1.39-1.48(4H, m), 1.81-1.87(2H, m), 1.92-1.97(2H, m), 2.30-2.34(4H, m), 2.41(2H, t, J=6.3Hz), 3.40-3.47(4H, m), 3.53-3.67(4H, m), 3.69-3.82(4H, m), 4.10(2H, t, J=6.1Hz), 5.10(2H, d, J=9.1Hz), 6.30(1H, dd, J=9.5Hz, 1.9Hz), 6.82(1H, dd, J=8.7Hz, 2.4Hz), 6.89(1H, d, J=2.4Hz), 7.01(1H, d, J=7.6Hz), 7.33(1H, t, J=7.8Hz), 7.51(1H, dd, J=5.6Hz, 0.8Hz), 7.56(1H, d, J=8.6Hz), 7.71(1H, d, J=8.0Hz), 7.77(1H, d, J=5.5Hz), 7.80(1H, d, J=9.4Hz), 11.68(1H, s). |
| EX119 | 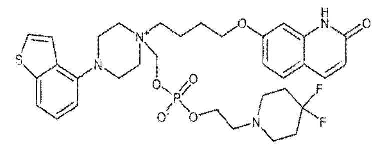 | $K_2CO_3$ (63.7 mg) was added to a suspension of 4,4-difluoropiperidine hydrochloride (48.4 mg) in MeCN (4 mL) at room temperature, and the resulting mixture was stirred for 15 minutes. (4-(Benzo[b]thiophen-4-yl)-1-(4-((2-oxo-1,2-dihydroquinolin-7-yl)oxy)butyl)piperazin-1-ium-1-yl)methyl (2-bromoethyl) phosphate (100 mg) was added, and the resulting mixture was stirred at 50° C. DMF (1 mL) was added, and the resulting mixture was heated to 50° C. 4,4-Difluoropiperidine ester hydrochloride (48.4 mg) and $K_2CO_3$ (105 mg) were added, and the resulting mixture was heated to 60° C. NaI (46.1 mg) was added and the resulting mixture was stirred at 70° C overnight. $H_2O$ was added to the reaction system, and the resulting mixture was extracted with DCM/MeOH = 9/1. The organic layer was collected and concentrated. The residue was purified by silica gel column chromatography (DCM/MeOH) to give (4-(benzo[b]thiophen-4-yl)-1-(4-((2-oxo-1,2-dihydroquinolin-7-yl)oxy)butyl)piperazin-1-ium-1-yl)methyl (2-(4,4-difluoropiperidin-1-yl)ethyl) phosphate (36 mg). | NMR1; 1.81-2.01(8H, m), 2.55(2H, t, J=6.1Hz), 3.29-3.39(4H, m), 3.44-3.48(4H, m), 3.53-3.68(4H, m), 3.70-3.85(4H, m), 4.10(2H, t, J=6.1Hz), 5.10(2H, d, J=9.1Hz), 6.30(1H, d, J=9.5Hz), 6.82(1H, dd, J=8.7Hz, 2.4Hz), 6.89(1H, d, J=2.4Hz), 7.01(1H, d, J=7.6Hz), 7.33(1H, t, J=7.9Hz), 7.51(1H, dd, J=5.5Hz, 0.8Hz), 7.56(1H, d, J=8.7Hz), 7.71(1H, d, J=8.1Hz), 7.77(1H, d, J=5.5Hz), 7.80(1H, d, J=9.5Hz), 11.68(1H, s). |
| EX120 | 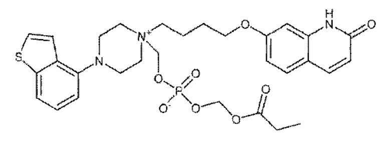 | Using chloromethylpropanoate (3.2 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl ((propionyloxy)methyl) phosphate (747 mg) was obtained in a method similar to the synthesis of EX 121. | NMR1; 1.00(3H, t, J=7.5Hz), 1.80-1.88(2H, m), 1.92-1.96(2H, m), 2.35(2H, q, J=7.5Hz), 3.42-3.49(4H, m), 3.54-3.68(4H, m), 3.72-3.80(2H, m), 4.10(2H, t, J=6.0Hz), 5.09(2H, d, J=8.9Hz), 5.43(2H, d, J=12.8Hz), 6.30(1H, d, J=9.4Hz), 6.83(1H, dd, J=8.6Hz, 2.4Hz), 6.86(1H, d, J=2.4Hz), 7.01(1H, dd, J=7.7Hz, 0.8Hz), 7.33(1H, t, J=7.9Hz), 7.52(1H, dd, J=5.6Hz, 0.8Hz), 7.57(1H, d, J=8.6Hz), 7.72(1H, d, J=8.1Hz), 7.77(1H, d, J=5.5Hz), 7.81(1H, d, J=9.5Hz), 11.64(1H, s). |

| | | | |
|---|---|---|---|
| EX121 | 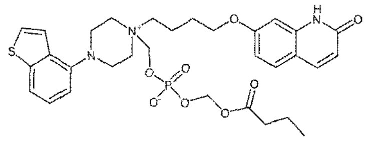 | Chloromethylbutyrate (6 mL) was added to a mixture of (4-(benzo[b]thiophen-4-yl)-1-(4-((2-oxo-1,2-dihydroquinolin-7-yl)oxy)butyl)piperazin-1-ium-1-yl)methyl hydrogen phosphate (8 g), acetone (120 mL), water (40 mL), and DIPEA (7 mL) with stirring, and then the resulting mixture was stirred under reflux. After 18 hours, the mixture was cooled to room temperature. Water (100 mL) was then added, and the resulting mixture was stirred. The precipitated solid was collected by filtration and washed with water to obtain a crude product. The crude product was purified by silica gel column chromatography (DCM/MeOH). Acetone (800 mL) and water (240 mL) were added and the resulting mixture was heated to 60° C to dissolve the purified product. The temperature was then reduced to 0° C with stirring. The precipitated solid was filtered out, washed with water, and dried to give (4-(benzo[b]thiophen-4-yl)-1-(4-((2-oxo-1,2-dihydroquinolin-7-yl)oxy)butyl)piperazin-1-ium-1-yl)methyl ((butyloxy)methyl) phosphate (5.8 g). | NMR1; 0.85(3H, t, J=7.4Hz), 1.52(2H, h, J=7.4Hz), 1.80-1.88(2H, m), 1.92-1.97(2H, m), 2.30(2H, t, J=7.3Hz), 3.44-3.48(4H, m), 3.54-3.68(4H, m), 3.72-3.80(2H, m), 4.10(2H, t, J=6.0Hz), 5.09(2H, d, J=9.0Hz), 5.43(2H, d, J=12.7Hz), 6.30(1H, d, J=9.3Hz), 6.82(1H, dd, J=8.6Hz, 2.4Hz), 6.86(1H, d, J=2.4Hz), 7.01(1H, dd, J=7.8Hz, 0.9Hz), 7.32(1H, t, J=7.9Hz), 7.52(1H, dd, J=5.5Hz, 0.9Hz), 7.56(1H, d, J=8.6Hz), 7.72(1H, d, J=8.1Hz), 7.77(1H, d, J=5.5Hz), 7.81(1H, d, J=9.5Hz), 11.63(1H, s). |
| EX122 | 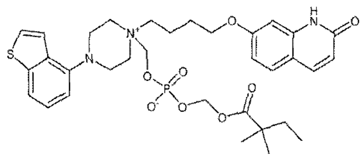 | Using iodomethyl 2,2-dimethylbutanoate synthesized in REX 38 (1.48 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (((2,2-dimethylbutanoyl)oxy)methyl) phosphate (731 mg) was obtained in a method similar to the synthesis of EX 121. | NMR1; 0.78 (3H, t, J=7.5Hz), 1.09 (6H, s), 1.50 (2H, q, J=7.5Hz), 1.81-1.87 (2H, m), 1.92-1.96 (2H, m), 3.43-3.48 (4H, m), 3.52-3.69 (4H, m), 3.72-3.80 (2H, m), 4.09 (2H, t, J=6.1Hz), 5.10 (2H, d, J=9.4Hz), 5.43 (2H, d, J=11.9Hz), 6.30 (1H, d, J=9.5Hz), 6.82 (1H, dd, J=8.5Hz, 2.5Hz), 6.85 (1H, d, J=2.4Hz), 7.01 (1H, d, J=7.5Hz), 7.33 (1H, t, J=7.9Hz), 7.52 (1H, dd, J=5.5Hz, 0.8Hz), 7.56 (1H, d, J=8.6Hz), 7.72 (1H, d, J=8.1Hz), 7.77 (1H, d, J=5.5Hz), 7.81 (1H, d, J=9.5Hz), 11.64 (1H, s). |
| EX123 | 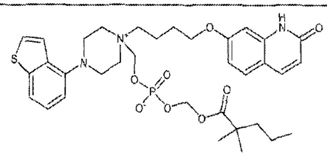 | Using chloromethyl 2,2-dimethylpentanoate synthesized in REX 39 (4.6 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (3.5 g), (4-(benzo[b]thiophen-4-yl)-1-(4-((2-oxo-1,2-dihydroquinolin-7-yl)oxy)butyl)piperazin-1-ium-1-yl)methyl (((2,2-dimethylpentanoyl)oxy)methyl) phosphate (2.62 g) was obtained in a method similar to the synthesis of EX 121. | NMR1; 0.82(3H, t, J=7.3Hz), 1.10(6H, s), 1.12-1.25(2H, m), 1.39-1.48(2H, m), 1.80-1.88(2H, m), 1.92-1.97(2H, m), 3.43-3.49(4H, m), 3.53-3.69(4H, m), 3.72-3.80(2H, m), 4.09(2H, t, J=6.1Hz), 5.11(2H, d, J=9.3Hz), 5.42(2H, d, J=11.7Hz), 6.30(1H, dd, J=9.5Hz, 1.8Hz), 6.82(1H, dd, J=8.6Hz, 2.4Hz), 6.85(1H, d, J=2.4Hz), 7.01(1H, dd, J=7.7Hz, 0.8Hz), 7.33(1H, t, J=7.9Hz), 7.52(1H, dd, J=5.6Hz, 0.8Hz), 7.56(1H, d, J=8.6Hz), 7.72(1H, d, J=8.1Hz), 7.77(1H, d, J=5.6Hz), 7.81(1H, d, J=9.5Hz), 11.64(1H, s). |
| EX124 | 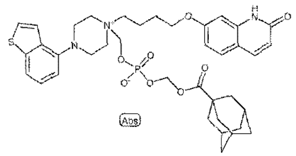 | Using chloromethyl (3r,5r,7r)-adamantane-1-carboxylate synthesized in REX 40 (1.4 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (((1s,3s)-adamantane-1-carbonyl)oxy)methyl ((4-(benzo[b]thiophen-4-yl)-1-(4-((2-oxo-1,2-dihydroquinolin-7-yl)oxy)butyl)piperazin-1-ium-1-yl)methyl) phosphate (760 mg) was obtained in a method similar to the synthesis of EX 121. | NMR1; 1.56-1.68 (6H, m), 1.77-1.88 (7H, m), 1.88-2.02 (6H, m), 3.41-3.49 (4H, m), 3.52-3.70 (4H, m), 3.73-3.81 (2H, m), 4.10 (2H, t, J=6.1Hz), 5.11 (2H, d, J=9.2Hz), 5.42 (2H, d, J=12.1Hz), 6.30 (1H, dd, J=9.4Hz, 1.7Hz), 6.83 (1H, dd, J=8.6Hz, 2.4Hz), 6.86 (1H, d, J=2.4Hz), 7.01 (1H, dd, J=7.8Hz, 0.9Hz), 7.33 (1H, t, J=7.9Hz), 7.52 (1H, dd, J=5.5Hz, 0.9Hz), 7.57 (1H, d, J=8.6Hz), 7.72 (1H, d, J=8.1Hz), 7.77 (1H, d, J=5.5Hz), 7.81 (1H, d, J=9.5Hz), 11.65 (1H, s). |

| | | | |
|---|---|---|---|
| EX125 | 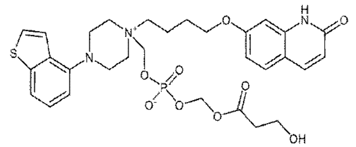 | PPTS (163 mg) was added to a solution of (4-(benzo[b]thiophen-4-yl)-1-(4-((2-oxo-1,2-dihydroquinolin-7-yl)oxy)butyl)piperazin-1-ium-1-yl)methyl (((3-(tetrahydro-2H-pyran-2-yl)oxy)propanoyl)oxy)methyl)phosphate (790 mg) synthesized in a method similar to the synthesis of EX 16 (Example 16) in EtOH (10 mL) at room temperature, and the resulting mixture was stirred at 50° C for 5 hours. The reaction system was concentrated and the residue was purified by silica gel column chromatography (DCM/MeOH). After $H_2O$ (5 mL) and acetone (5 mL) were added, $H_2O$ (5 mL) was added and the resulting mixture was heated to 40° C. The precipitate was filtered out and dried at room temperature to give (4-(benzo[b]thiophen-4-yl)-1-(4-((2-oxo-1,2-dihydroquinolin-7-yl)oxy)butyl)piperazin-1-ium-1-yl)methyl (((3-hydroxypropanoyl)oxy)methyl) phosphate (448 mg). | NMR1; 1.80-1.88(2H, m), 1.92-1.96(2H, m), 2.47(2H, d, J=6.0Hz), 3.38-3.50(4H, m), 3.53-3.62(2H, m), 3.62-3.70(4H, m), 3.71-3.79(2H, m), 4.10(2H, t, J=6.1Hz), 4.96(1H, t, J=5.4Hz), 5.10(2H, d, J=8.9Hz), 5.45(2H, d, J=13.5Hz), 6.30(1H, dd, J=9.5Hz, 1.2Hz), 6.83(1H, dd, J=8.5Hz, 2.4Hz), 6.86(1H, d, J=2.4Hz), 7.01(1H, dd, J=7.7Hz, 0.9Hz), 7.32(1H, t, J=7.8Hz), 7.52(1H, dd, J=5.6Hz, 0.8Hz), 7.57(1H, d, J=8.6Hz), 7.72(1H, d, J=8.0Hz), 7.77(1H, d, J=5.5Hz), 7.81(1H, d, J=9.5Hz), 11.63(1H, s). |
| EX126 | 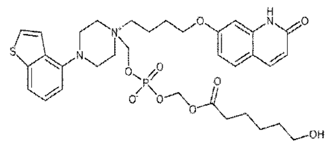 | Using (4-(benzo[b]thiophen-4-yl)-1-(4-((2-oxo-1,2-dihydroquinolin-7-yl)oxy)butyl)piperazin-1-ium-1-yl)methyl (((6-(tetrahydro-2H-pyran-2-yl)oxy)hexanoyl)oxy)methyl) phosphate (1.1 g) synthesized in a method similar to the synthesis of EX 16 (Example 16) (1.1 g), (4-(benzo[b]thiophen-4-yl)-1-(4-((2-oxo-1,2-dihydroquinolin-7-yl)oxy)butyl)piperazin-1-ium-1-yl)methyl (6-hydroxyhexanoyl)oxy)methyl) phosphate (620 mg) was obtained in a method similar to the synthesis of EX 125. | NMR1; 1.19-1.31(2H, m), 1.31-1.42(2H, m), 1.41-1.56(2H, m), 1.80-1.88(2H, m), 1.92-1.97(2H, m), 2.31(2H, t, J=7.4Hz), 3.30-3.37(2H, m), 3.43-3.48(4H, m), 3.49-3.68(4H, m), 3.72-3.80(2H, m), 4.10(2H, t, J=6.0Hz), 4.40(1H, t, J=5.2Hz), 5.09(2H, d, J=8.9Hz), 5.43(2H, d, J=12.7Hz), 6.30(1H, d, J=9.5Hz), 6.83(1H, dd, J=8.6Hz, 2.4Hz), 6.86(1H, d, J=2.4Hz), 7.01(1H, dd, J=7.8Hz, 0.8Hz), 7.32(1H, t, J=7.9Hz), 7.52(1H, dd, J=5.5Hz, 0.9Hz), 7.56(1H, d, J=8.6Hz), 7.72(1H, d, J=8.2Hz), 7.77(1H, d, J=5.5Hz), 7.81(1H, d, J=9.5Hz), 11.64(1H, s). |
| EX127 | 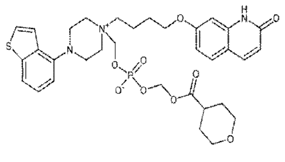 | Using iodomethyltetrahydro-2H-pyran-4-carboxylate synthesized in REX 46 (647 mg) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzo[b]thiophen-4-yl)-1-(4-((2-oxo-1,2-dihydroquinolin-7-yl)oxy)butyl)piperazin-1-ium-1-yl)methyl (((tetrahydro-2H-pyran-4-carbonyl)oxy)methyl) phosphate (448 mg) was obtained in a method similar to the synthesis of EX 121. | NMR1; 1.48-1.62(2H, m), 1.69-1.77(2H, m), 1.79-1.87(2H, m), 1.92-1.96(2H, m), 2.58(1H, tt, J=11.1Hz, 4.1Hz), 3.18-3.33(2H, m), 3.43-3.48(4H, m), 3.54-3.68(4H, m), 3.72-3.82(4H, m), 4.09(2H, t, J=6.0Hz), 5.10(2H, d, J=9.1Hz), 5.45(2H, d, J=12.6Hz), 6.30(1H, dd, J=9.4Hz, 1.9Hz), 6.83(1H, dd, J=8.6Hz, 2.4Hz), 6.86(1H, d, J=2.4Hz), 7.01(1H, dd, J=7.7Hz, 0.8Hz), 7.33(1H, t, J=7.8Hz), 7.52(1H, dd, J=5.6Hz, 0.8Hz), 7.56(1H, d, J=8.7Hz), 7.72(1H, d, J=8.1Hz), 7.77(1H, d, J=5.5Hz), 7.81(1H, d, J=9.5Hz), 11.64(1H, s). |
| EX128 | 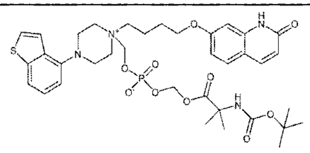 | Using chloromethyl 2-(tert-butoxycarbonyl)amino)-2-methylpropanoate synthesized in REX 47 (1.6 g) and (4-(benzo[b]thiophen-4-yl)-1-(4-(2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (((2-(tert-butoxycarbonyl)amino)-2-methylpropanoyl)oxy)methyl) phosphate (280 mg) was obtained in a method similar to the synthesis of EX 121. | NMR1; 1.33(6H, s), 1.34(9H, s), 1.81-1.86(2H, m), 1.88-1.97(2H, m), 3.43-3.48(4H, m), 3.54-3.69(4H, m), 3.71-3.76(2H, m), 4.09(2H, t, J=6.2Hz), 5.10(2H, d, J=9.2Hz), 5.42(2H, d, J=12.3Hz), 6.30(1H, dd, J=9.4Hz, 1.8Hz), 6.82(1H, dd, J=8.6Hz, 2.4Hz), 6.85(1H, d, J=2.4Hz), 7.00(1H, d, J=7.6Hz), 7.27(1H, s), 7.32(1H, t, J=7.9Hz), 7.51(1H, d, J=5.5Hz), 7.56(1H, d, J=8.6Hz), 7.72(1H, d, J=8.1Hz), 7.77(1H, d, J=5.5Hz), 7.80(1H, d, J=9.5Hz), 11.63(1H, s). |

| | | | |
|---|---|---|---|
| EX129 | 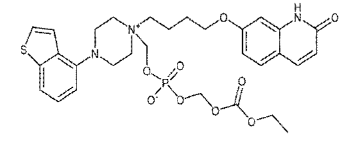 | Using chloromethyl ethyl carbonate (3 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (3 g), (4-(benzo[b]thiophen-4-yl)-1-(4-(((2-oxo-1,2-dihydroquinolin-7-yl)oxy)butyl)piperazin-1-ium-1-yl)methyl (((ethoxycarbonyl)oxy)methyl) phosphate (1.9 g) was obtained in a method similar to the synthesis of EX 121. | NMR1; 1.18(3H, t, J=7.1Hz), 1.81–1.88(2H, m), 1.92–1.96(2H, m), 3.42–3.47(4H, m), 3.53–3.68(4H, m), 3.72–3.80(2H, m), 4.04–4.17(2H, m), 4.12(2H, q, J=7.1Hz), 5.09(2H, d, J=8.6Hz), 5.44(2H, d, J=13.3Hz), 6.30(1H, d, J=9.4Hz), 6.83(1H, dd, J=8.5Hz, 2.4Hz), 6.85(1H, d, J=2.4Hz), 7.01(1H, d, J=7.5Hz), 7.33(1H, t, J=7.8Hz), 7.52(1H, dd, J=5.6Hz, 0.8Hz), 7.56(1H, d, J=8.6Hz), 7.72(1H, d, J=8.1Hz), 7.77(1H, d, J=5.6Hz), 7.81(1H, d, J=9.5Hz), 11.62(1H, s). |
| EX130 | 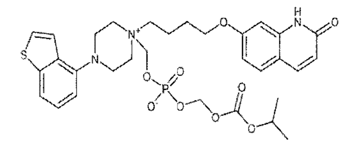 | Using chloromethyl isopropyl carbonate (1 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.7 g), (4-(benzo[b]thiophen-4-yl)-1-(4-((2-oxo-1,2-dihydroquinolin-7-yl)oxy)butyl)piperazin-1-ium-1-yl)methyl ((isopropoxycarbonyl)oxy)methyl) phosphate (540 mg) was obtained in a method similar to the synthesis of EX 121. | NMR1; 1.19(6H, d, J=6.2Hz), 1.82–1.86(2H, m), 1.92–1.96(2H, m), 3.40–3.47(4H, m), 3.55–3.59(2H, m), 3.64–3.68(2H, m), 3.72–3.80(2H, m), 4.09(2H, t, J=6.0Hz), 4.70–4.83(1H, m), 5.09(2H, d, J=8.6Hz), 5.42(2H, d, J=13.2Hz), 6.30(1H, dd, J=9.5Hz, 1.9Hz), 6.83(1H, dd, J=8.5Hz, 2.4Hz), 6.85(1H, d, J=2.0Hz), 7.01(1H, d, J=7.8Hz), 7.32(1H, t, J=7.8Hz), 7.51(1H, dd, J=5.6Hz, 0.7Hz), 7.56(1H, d, J=8.5Hz), 7.72(1H, d, J=8.0Hz), 7.77(1H, d, J=5.5Hz), 7.81(1H, d, J=9.5Hz), 11.63(1H, s). |
| EX131 | 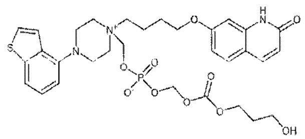 | Using (4-(benzo[b]thiophen-4-yl)-1-(4-((2-oxo-1,2-dihydroquinolin-7-yl)oxy)butyl)piperazin-1-ium-1-yl)methyl ((((3-((tetrahydro-2H-pyran-2-yl)oxy)propoxy)carbonyl)oxy)methyl)phosphate (747 mg), (4-(benzo[b]thiophen-4-yl)-1-(4-((2-oxo-1,2-dihydroquinolin-7-yl)oxy)butyl)piperazin-1-ium-1-yl)methyl ((((3-hydroxypropoxy)carbonyl)oxy)methyl) phosphate (516 mg) was obtained in a method similar to the synthesis of EX 125. | NMR1; 1.63-1.78(2H, m), 1.80–1.89(2H, m), 1.92–1.96(2H, m), 3.37–3.49(6H, m), 3.53–3.69(4H, m), 3.72–3.80(2H, m), 4.10(2H, t, J=6.0Hz), 4.16(2H, t, J=6.5Hz), 4.60(1H, t, J=5.2Hz), 5.09(2H, d, J=8.6Hz), 5.44(2H, d, J=13.4Hz), 6.30(1H, dd, J=9.5Hz, 1.4Hz), 6.83(1H, dd, J=8.5Hz, 2.4Hz), 6.85(1H, d, J=2.4Hz), 7.01(1H, dd, J=7.7Hz, 0.9Hz), 7.32(1H, t, J=7.9Hz), 7.52(1H, dd, J=5.6Hz, 0.8Hz), 7.56(1H, d, J=8.6Hz), 7.72(1H, d, J=8.0Hz), 7.77(1H, d, J=5.5Hz), 7.81(1H, d, J=9.5Hz), 11.63(1H, s). |
| EX132 | 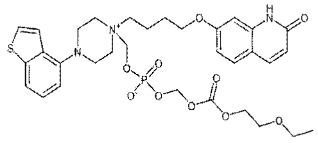 | Using chloromethyl (2-ethoxyethyl) carbonate (2.5 g) synthesized in REX 49 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl ((((2-ethoxyethoxy)carbonyl)oxy)methyl) phosphate (613 mg) was obtained in a method similar to the synthesis of EX 121. | NMR1; 1.04(3H, t, J=7.0Hz), 1.85(2H, d, J=6.9Hz), 1.92–1.96(2H, m), 3.36–3.41(4H, m), 3.44(4H, d, J=6.9Hz), 3.49–3.54(2H, m), 3.54–3.60(2H, m), 3.60–3.68(2H, m), 3.72–3.79(2H, m), 4.09(2H, t, J=6.1Hz), 4.16–4.22(2H, m), 5.09(2H, d, J=8.6Hz), 5.45(2H, d, J=13.5Hz), 6.30(1H, dd, J=9.4Hz, 2.0Hz), 6.79–6.87(2H, m), 7.01(1H, d, J=7.5Hz), 7.32(1H, t, J=7.9Hz), 7.52(1H, dd, J=5.6Hz, 0.8Hz), 7.56(1H, d, J=8.5Hz), 7.72(1H, d, J=8.1Hz), 7.77(1H, d, J=5.5Hz), 7.81(1H, d, J=9.5Hz), 11.63(1H, s). |

| | | | |
|---|---|---|---|
| EX133 | 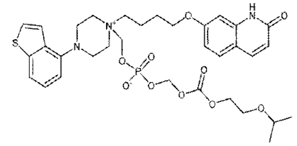 | Iodomethyl (2-isopropoxyethyl) carbonate (9.3 g) was added to a mixture of (4-(benzo[b]thiophen-4-yl)-1-(4-((2-oxo-1,2-dihydroquinolin-7-yl)oxy)butyl)piperazin-1-ium-1-yl)methyl hydrogen phosphate (3 g), acetone (45 mL), water (15 mL), and DIPEA (6 mL) with stirring, and then stirred under reflux. After 1 hour, the reaction system was concentrated and the residue was purified by silica gel column chromatography (DCM/MeOH). Acetone (5 mL) and water (5 mL) were added, and the resulting mixture was heated to 50° C to dissolve the purified residue. Water (10 mL) was further added and the resulting mixture was ice-cooled. The precipitated solid was filered out, washed with water, and air-dried at room temperature to give (4-(benzo[b]thiophen-4-yl)-1-(4-((2-oxo-1,2-dihydroquinolin-7-yl)oxy)butyl)piperazin-1-ium-1-yl)methyl ((((2-isopropoxyethyloxy)carbonyl)oxy)methyl) phosphate (2.9 g). | NMR1; 1.02(6H, d, J=6.1Hz), 1.80–1.88(2H, m), 1.92–1.96(2H, m), 3.42–3.54(7H, m), 3.54–3.69(4H, m), 3.72–3.80(2H, m), 4.10(2H, t, J=6.1Hz), 4.13–4.19(2H, m), 5.09(2H, d, J=8.5Hz), 5.45(2H, d, J=13.4Hz), 6.30(1H, d, J=9.1Hz), 6.83(1H, dd, J=8.5Hz, 2.4Hz), 6.85(1H, d, J=2.4Hz), 7.01(1H, dd, J=7.8Hz, 0.9Hz), 7.33(1H, t, J=7.9Hz), 7.52(1H, dd, J=5.6Hz, 0.9Hz), 7.57(1H, d, J=8.5Hz), 7.72(1H, d, J=8.0Hz), 7.77(1H, d, J=5.5Hz), 7.81(1H, d, J=9.5Hz), 11.63(1H, s). |
| EX134 | 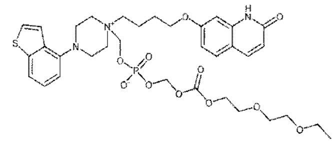 | Using chloromethyl (2-(2-ethoxyethoxy)ethyl) carbonate synthesized in REX 52 (2.5 g) and (4-(benzo[b]thiophen-4-yl)-1-(4-(2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (3-oxo-2,4,7,10-tetraoxadodecyl) phosphate (730 mg) was obtained in a method similar to the synthesis of EX 121. | NMR1; 1.05(3H, t, J=7.0Hz), 1.82–1.86(2H, m), 1.92–1.96(2H, m), 3.20–3.41(6H, m), 3.42–3.50(6H, m), 3.53–3.68(4H, m), 3.72–3.76(2H, m), 4.09(2H, t, J=6.1Hz), 4.16–4.23(2H, m), 5.09(2H, d, J=8.6Hz), 5.45(2H, d, J=13.4Hz), 6.30(1H, d, J=9.7Hz), 6.79–6.87(2H, m), 7.01(1H, d, J=7.5Hz), 7.33(1H, t, J=7.8Hz), 7.52(1H, d, J=5.8Hz), 7.56(1H, d, J=8.5Hz), 7.72(1H, d, J=8.1Hz), 7.77(1H, d, J=5.5Hz), 7.81(1H, d, J=9.5Hz), 11.62(1H, s). |
| EX135 | 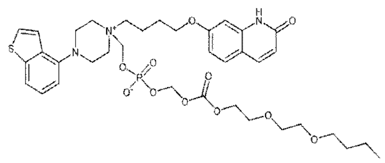 | Using 2-(2-butoxyethoxy)ethyl (iodomethyl) carbonate (10 g) synthesized in REX 54 and (4-(benzo[b]thiophen-4-yl)-1-(4-((2-oxo-1,2-dihydroquinolin-7-yl)oxy)butyl)piperazin-1-ium-1-yl)methyl hydrogen phosphate (3 g), (4-(benzo[b]thiophen-4-yl)-1-(4-((2-oxo-1,2-dihydroquinolin-7-yl)oxy)butyl)piperazin-1-ium-1-yl)methyl (3-oxo-2,4,7,10-tetraoxatetradecyl) phosphate (3.1 g) was obtained in a method similar to the synthesis of EX 121. | NMR1; 0.83(3H, t, J=7.3Hz), 1.18–1.32(2H, m), 1.35–1.47(2H, m), 1.80–1.88(2H, m), 1.92–1.96(2H, m), 3.30(2H, t, J=6.6Hz), 3.34–3.44(4H, m), 3.44–3.50(4H, m), 3.53–3.68(6H, m), 3.72–3.79(2H, m), 4.09(2H, t, J=6.0Hz), 4.16–4.23(2H, m), 5.09(2H, d, J=8.6Hz), 5.45(2H, d, J=13.5Hz), 6.30(1H, d, J=9.5Hz), 6.83(1H, dd, J=8.5Hz, 2.4Hz), 6.85(1H, d, J=2.4Hz), 7.01(1H, dd, J=7.7Hz, 0.9Hz), 7.32(1H, t, J=7.9Hz), 7.52(1H, dd, J=5.6Hz, 0.8Hz), 7.56(1H, d, J=8.6Hz), 7.72(1H, d, J=8.1Hz), 7.77(1H, d, J=5.5Hz), 7.80(1H, d, J=9.5Hz), 11.63(1H, s). |
| EX136 | 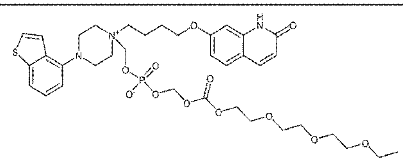 | Using chloromethyl (2-(2-(2-ethoxyethoxy)ethoxy)ethyl) carbonate (2.5 g) synthesized in REX 55 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (3-oxo-2,4,7,10,13-pentaoxapentadecyl) phosphate (415 mg) was obtained in a method similar to the synthesis of EX 121. | NMR1; 1.06(3H, t, J=7.0Hz), 1.81–1.88(2H, m), 1.92–1.96(2H, m), 3.38(2H, q, J=7.0Hz), 3.42–3.51(12H, m), 3.53–3.68(6H, m), 3.72–3.79(2H, m), 4.09(2H, t, J=6.1Hz), 4.17–4.23(2H, m), 5.09(2H, d, J=8.6Hz), 5.45(2H, d, J=13.4Hz), 6.30(1H, dd, J=9.5Hz, 1.8Hz), 6.83(1H, dd, J=8.5Hz, 2.4Hz), 6.85(1H, d, J=2.3Hz), 7.01(1H, d, J=7.6Hz), 7.32(1H, t, J=7.9Hz), 7.52(1H, dd, J=5.5Hz, 0.9Hz), 7.56(1H, d, J=8.6Hz), 7.72(1H, d, J=8.1Hz), 7.77(1H, d, J=5.6Hz), 7.80(1H, d, J=9.5Hz), 11.62(1H, s). |

| | | | |
|---|---|---|---|
| EX137 | 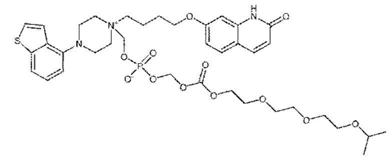 | Using chloromethyl (2-(2-(2-isopropoxyethoxy)ethoxy)ethyl) carbonate (1.6 g) synthesized in REX 56 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (14-methyl-3-oxo-2,4,7,10,13-pentaoxapentadecyl) phosphate (604 mg) was obtained in a method similar to the synthesis of EX 121. | NMR1; 1.04(6H, d, J=6.1Hz), 1.80-1.88(2H, m), 1.92-1.96(2H, m), 3.38-3.52(14H, m), 3.55-3.68(6H, m), 3.72-3.79(2H, m), 4.10(2H, t, J=6.0Hz), 4.17-4.23(2H, m), 5.09(2H, d, J=8.6Hz), 5.45(2H, d, J=13.5Hz), 6.83(1H, dd, J=8.5Hz, 2.4Hz), 6.85(1H, d, J=2.5Hz), 7.01(1H, dd, J=7.7Hz, 0.9Hz), 7.33(1H, t, J=7.9Hz), 7.52(1H, dd, J=5.6Hz, 0.8Hz), 7.56(1H, d, J=8.5Hz), 7.72(1H, d, J=8.1Hz), 7.77(1H, d, J=5.5Hz), 7.81(1H, d, J=9.5Hz), 11.63(1H, s). |
| EX138 | 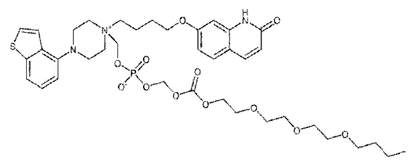 | Using 2-(2-(2-butoxyethoxy)ethoxy)ethyl (chloromethyl) carbonate (1.8 g) synthesized in REX 57 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (3-oxo-2,4,7,10,13-pentaoxaheptadecyl) phosphate (705 mg) was obtained in a method similar to the synthesis of EX 121. | NMR1; 0.84(3H, t, J=7.3Hz), 1.20-1.34(2H, m), 1.35-1.48(2H, m), 1.80-1.88(2H, m), 1.92-1.96(2H, m), 3.28-3.43(6H, m), 3.42-3.53(8H, m), 3.53-3.68(6H, m), 3.73-3.77(1H, m), 4.10(2H, t, J=6.1Hz), 4.17-4.23(2H, m), 5.09(2H, d, J=8.6Hz), 5.45(2H, d, J=13.5Hz), 6.30(2H, dd, J=9.5Hz, 2.0Hz), 6.83(1H, dd, J=8.5Hz, 2.4Hz), 6.85(1H, d, J=2.4Hz), 7.01(1H, dd, J=7.8Hz, 0.9Hz), 7.32(1H, t, J=7.9Hz), 7.52(1H, dd, J=5.6Hz, 0.8Hz), 7.56(1H, d, J=8.4Hz), 7.72(1H, d, J=8.1Hz), 7.77(1H, d, J=5.5Hz), 7.81(1H, d, J=9.5Hz), 11.63(1H, s). |
| EX139 | 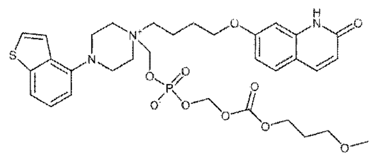 | Using chloromethyl (3-methoxypropyl) carbonate (7 g) synthesized in REX 58 and (4-(benzo[b]thiophen-4-yl)-1-(4-((2-oxo-1,2-dihydroquinolin-7-yl)oxy)butyl)piperazin-1-ium-1-yl)methyl hydrogen phosphate (3.5 g), (4-(benzo[b]thiophen-4-yl)-1-(4-((2-oxo-1,2-dihydroquinolin-7-yl)oxy)butyl)piperazin-1-ium-1-yl)methyl ((((3-methoxypropoxy)carbonyl)oxy)methyl) phosphate (2.3 g) was obtained in a method similar to the synthesis of EX 121. | NMR1; 1.70-1.81(2H, m), 1.82-1.88(2H, m), 1.92-1.96(2H, m), 3.17(3H, s), 3.31(2H, t, J=6.2Hz), 3.43-3.47(4H, m), 3.53-3.69(4H, m), 3.72-3.80(2H, m), 4.10(2H, t, J=6.1Hz), 4.12(2H, t, J=6.6Hz), 5.09(2H, d, J=8.6Hz), 5.44(2H, d, J=13.4Hz), 6.31(1H, d, J=9.4Hz), 6.83(1H, dd, J=8.5Hz, 2.4Hz), 6.86(1H, d, J=2.5Hz), 7.01(1H, dd, J=7.7Hz, 0.9Hz), 7.33(1H, t, J=7.9Hz), 7.52(1H, dd, J=5.5Hz, 0.9Hz), 7.57(1H, d, J=8.5Hz), 7.72(1H, d, J=8.0Hz), 7.77(1H, d, J=5.5Hz), 7.81(1H, d, J=9.5Hz), 11.64(1H, s). |
| EX140 | 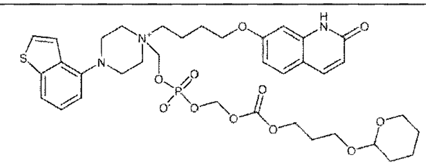 | Using chloromethyl (3-((tetrahydro-2H-pyran-2-yl)oxy)propyl) carbonate (3.6 g) synthesized in REX 48 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.2 g), (4-(benzo[b]thiophen-4-yl)-1-(4-((2-oxo-1,2-dihydroquinolin-7-yl)oxy)butyl)piperazin-1-ium-1-yl)methyl ((((3-((tetrahydro-2H-pyran-2-yl)oxy)propoxy)carbonyl)oxy)methyl) phosphate (1.12 g) was obtained in a method similar to the synthesis of EX 121. | NMR1; 1.34-1.46(2H, m), 1.49-1.60(1H, m), 1.60-1.70(1H, m), 1.76-1.89(4H, m), 1.92-1.96(2H, m), 3.18-3.34(4H, m), 3.41-3.48(4H, m), 3.53-3.71(6H, m), 3.72-3.80(2H, m), 4.09(2H, t, J=6.1Hz), 4.16(2H, t, J=6.5Hz), 4.48(1H, t, J=3.6Hz), 5.09(2H, d, J=8.6Hz), 5.44(2H, d, J=13.4Hz), 6.30(1H, d, J=9.5Hz), 6.83(1H, dd, J=8.5Hz, 2.4Hz), 6.85(1H, d, J=2.4Hz), 7.01(1H, d, J=7.6Hz), 7.32(1H, t, J=7.9Hz), 7.52(1H, dd, J=5.5Hz, 0.8Hz), 7.56(1H, d, J=8.5Hz), 7.72(1H, d, J=8.0Hz), 7.77(1H, d, J=5.5Hz), 7.81(1H, d, J=9.5Hz), 11.63(1H, s). |

| | | | |
|---|---|---|---|
| EX141 | 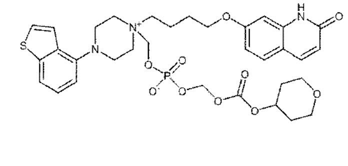 | Using iodomethyl (tetrahydro-2H-pyran-4-yl) carbonate (1.36 g) synthesized in REX 60 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl ((((tetrahydro-2H-pyran-4-yl)oxy)carbonyl)oxy)methyl) phosphate (982 mg) was obtained in a method similar to the synthesis of EX 121. | NMR1; 1.52(2H, m), 1.82-1.90(4H, m), 1.92-1.96(2H, m), 3.32-3.41(2H, m), 3.43-3.47(4H, m), 3.53-3.69(4H, m), 3.69-3.80(4H, m), 4.09(2H, t, J=6.0Hz), 4.73(1H, tt, J=8.8Hz, 4.2Hz), 5.09(2H, d, J=8.5Hz), 5.45(2H, d, J=13.3Hz), 6.30(1H, dd, J=9.5Hz, 1.5Hz), 6.83(1H, dd, J=8.5Hz, 2.4Hz), 6.85(1H, d, J=2.4Hz), 7.01(1H, dd, J=7.7Hz, 0.9Hz), 7.33(1H, t, J=7.9Hz), 7.51(1H, dd, J=5.5Hz, 0.9Hz), 7.56(1H, d, J=8.5Hz), 7.72(1H, d, J=8.1Hz), 7.77(1H, d, J=5.6Hz), 7.81(1H, d, J=9.5Hz), 11.63(1H, s). |
| EX142 | 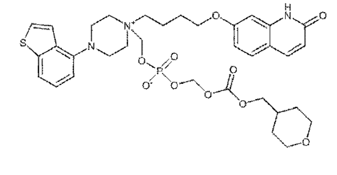 | Using iodomethyl ((tetrahydro-2H-pyran-4-yl)methyl) carbonate (1.34 g) synthesized in REX 62 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (((((tetrahydro-2H-pyran-4-yl)methoxy)carbonyl)oxy)methyl) phosphate (705 mg) was obtained in a method similar to the synthesis of EX 121. | NMR1; 1.09-1.24(2H, m), 1.43-1.50(2H, m), 1.73-1.87(3H, m), 1.92-1.96(2H, m), 3.17(2H, td, J=11.8Hz, 2.1Hz), 3.43-3.47(4H, m), 3.53-3.68(4H, m), 3.72-3.80(4H, m), 3.93(2H, d, J=6.6Hz), 4.09(2H, t, J=6.0Hz), 5.09(2H, d, J=8.5Hz), 5.44(2H, d, J=13.4Hz), 6.30(1H, dd, J=9.5Hz, 1.7Hz), 6.83(1H, dd, J=8.5Hz, 2.4Hz), 6.85(1H, d, J=2.4Hz), 7.01(1H, d, J=7.6Hz), 7.33(1H, t, J=7.8Hz), 7.51(1H, dd, J=5.6Hz, 0.9Hz), 7.57(1H, d, J=8.5Hz), 7.72(1H, d, J=8.1Hz), 7.77(1H, d, J=5.5Hz), 7.81(1H, d, J=9.5Hz), 11.63(1H, s). |
| EX143 | 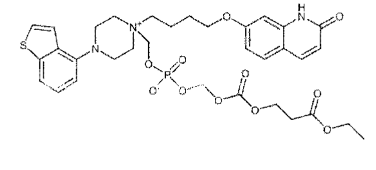 | Using ethyl 3-(((chloromethoxy)carbonyl)oxy)propanoate (2 g) synthesized in REX 63 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl ((((3-ethoxy-3-oxopropoxy)carbonyl)oxy)methyl) phosphate (159 mg) was obtained in a method similar to the synthesis of EX 121. | NMR1; 1.13(3H, t, J=7.1Hz), 1.81-1.87(2H, m), 1.91-1.96(2H, m), 2.67(2H, t, J=6.1Hz), 3.43-3.47(4H, m), 3.53-3.68(4H, m), 3.71-3.79(2H, m), 4.04(2H, q, J=7.1Hz), 4.09(2H, t, J=6.1Hz), 4.29(2H, t, J=6.1Hz), 5.08(2H, d, J=8.7Hz), 5.44(2H, d, J=13.5Hz), 6.30(1H, dd, J=9.5Hz, 2.0Hz), 6.79-6.87(2H, m), 7.01(1H, d, J=7.4Hz), 7.32(1H, t, J=7.9Hz), 7.52(1H, dd, J=5.5Hz, 0.9Hz), 7.56(1H, d, J=8.5Hz), 7.72(1H, d, J=8.1Hz), 7.77(1H, d, J=5.5Hz), 7.80(1H, d, J=9.5Hz), 11.62(1H, s). |
| EX144 | 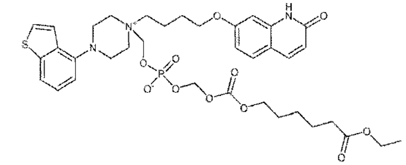 | Using ethyl 6-(((chloromethoxy)carbonyl)oxy)hexanoate (2 g) synthesized in REX 64 and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl ((((3-ethoxy-3-oxopropoxy)carbonyl)oxy)methyl) phosphate (682 mg) was obtained in a method similar to the synthesis of EX 121. | NMR1; 1.15(3H, t, J=7.1Hz), 1.20-1.30(2H, m), 1.40-1.59(4H, m), 1.81-1.87(2H, m), 1.92-1.96(2H, m), 2.21(2H, t, J=7.4Hz), 3.43-3.47(4H, m), 3.51-3.61(2H, m), 3.61-3.68(2H, m), 3.72-3.80(2H, m), 3.97-4.13(6H, m), 5.09(2H, d, J=8.5Hz), 5.44(2H, d, J=13.4Hz), 6.30(1H, dd, J=9.4Hz, 2.0Hz), 6.82(1H, dd, J=8.5Hz, 2.5Hz), 6.85(1H, d, J=2.4Hz), 7.00(1H, d, J=7.4Hz), 7.32(1H, t, J=7.9Hz), 7.51(1H, dd, J=5.5Hz, 0.9Hz), 7.56(1H, d, J=8.5Hz), 7.71(1H, d, J=8.1Hz), 7.77(1H, d, J=5.5Hz), 7.80(1H, d, J=9.5Hz), 11.63(1H, s). |

| | | | |
|---|---|---|---|
| EX145 | 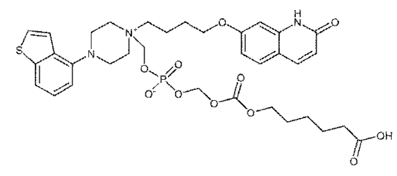 | TFA (5 mL) was added to (4-(benzo[b]thiophen-4-yl)-1-(4-((2-oxo-1,2-dihydroquinolin-7-yl)oxy)butyl)piperazin-1-ium-1-yl)methyl (((((6-((4-methoxybenzyl)oxy)-6-oxohexyl)oxy)carbonyl)oxy)methyl) phosphate (1.43 g) synthesized in REX 65 under ice-cooling. The mixture was stirred at room temperature. After 3 hours, TFA (3 mL) was added. After 2 hours, the reaction system was concentrated and azeotroped three times with DCM. The residue was purified by silica gel column chromatography (DCM/MeOH). Acetone (1 mL) and $H_2O$ (1 mL) were added, and $H_2O$ (5 mL) was further added. The mixture was heated to 40° C, and the insoluble matter was filtered out. Washing with $H_2O$ was performed to give (4-(benzo[b]thiophen-4-yl)-1-(4-((2-oxo-1,2-dihydroquinolin-7-yl)oxy)butyl)piperazin-1-ium-1-yl)methyl (((((5-carboxypentyl)oxy)carbonyl)oxy)methyl) phosphate (580 mg). | NMR1; 1.19-1.31(2H, m), 1.38-1.62(4H, m), 1.80-1.87(2H, m), 1.92-1.96(2H, m), 2.16(2H, t, J=7.3Hz), 3.43-3.47(4H, m), 3.53-3.68(4H, m), 3.76(2H, d, J=13.5Hz), 4.05(2H, t, J=6.6Hz), 4.09(2H, t, J=6.2Hz), 5.09(2H, d, J=8.6Hz), 5.44(2H, d, J=13.4Hz), 6.30(1H, d, J=10.2Hz), 6.82(1H, dd, J=8.5Hz, 2.4Hz), 6.85(1H, d, J=2.4Hz), 7.00(1H, d, J=8.0 Hz), 7.32(1H, t, J=7.9Hz), 7.51(1H, dd, J=5.5Hz, 0.8Hz), 7.56(1H, d, J=8.6Hz), 7.71(1H, d, J=8.0Hz), 7.77(1H, d, J=5.6Hz), 7.80(1H, d, J=9.5Hz), 11.62(1H, s), 12.03(1H, s). |
| EX146 | 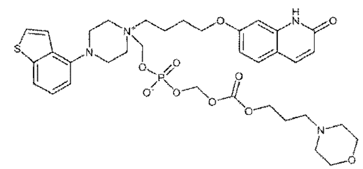 | NaI (142 mg) and morpholine (0.9 mL) were added to a suspension of (4-(benzo[b]thiophen-4-yl)-1-(4-((2-oxo-1,2-dihydroquinolin-7-yl)oxy)butyl)piperazin-1-ium-1-yl)methyl ((((3-chloropropoxy)carbonyl)oxy)methyl) phosphate (657 mg) synthesized in EX 161 in THF (20 mL) at room temperature. The mixture was heated to 60° C and stirred for 5 hours. The mixture was stirred at room temperature overnight. The reaction system was concentrated, and the residue was purified by silica gel column chromatography (DCM/MeOH). The residue was dissolved in acetone (1 mL) and $H_2O$ (1 mL), followed by stirring. $H_2O$ was added. The precipitated crystals were filtered out. Washing with $H_2O$ was performed, and drying was performed at room temperature overnight. (4-(Benzo[b]thiophen-4-yl)-1-(4-((2-oxo-1,2-dihydroquinolin-7-yl)oxy)butyl)piperazin-1-ium-1-yl)methyl ((((3-morpholinopropoxy)carbonyl)oxy)methyl) phosphate (56 mg) was obtained. | NMR1; 1.60-1.76(2H, m), 1.81-1.88(2H, m), 1.92-1.96(2H, m), 2.21-2.26(4H, m), 3.28-3.37(2H, m), 3.43-3.47(4H, m), 3.48(4H, t, J=4.7Hz), 3.53-3.60(2H, m), 3.61-3.68(2H, m), 3.72-3.80(2H, m), 4.06-4.14(4H, m), 5.09(2H, d, J=8.5Hz), 5.44(2H, d, J=13.3Hz), 6.30(1H, dd, J=9.5Hz, 1.9Hz), 6.83(1H, dd, J=8.5Hz, 2.4Hz), 6.85(1H, d, J=2.4Hz), 7.01(1H, d, J=7.5Hz), 7.33(1H, t, J=7.8Hz), 7.52(1H, dd, J=5.5Hz, 0.8Hz), 7.56(1H, d, J=8.6Hz), 7.72(1H, d, J=8.0Hz), 7.77(1H, d, J=5.5Hz), 7.81(1H, d, J=9.5Hz), 11.63(1H, s). |
| EX147 | 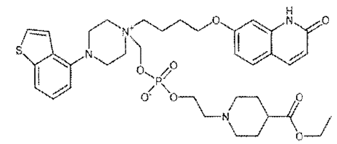 | Using ethyl 4-piperidinecarboxylate (2 mL) and (4-(benzo[b]thiophen-4-yl)-1-(4-((2-oxo-1,2-dihydroquinolin-7-yl)oxy)butyl)piperazin-1-ium-1-yl)methyl (2-bromoethyl) phosphate (0.4 g) synthesized in EX 21, (4-(benzo[b]thiophen-4-yl)-1-(4-((2-oxo-1,2-dihydroquinolin-7-yl)oxy)butyl)piperazin-1-ium-1-yl)methyl (2-(4-(ethoxycarbonyl)piperidin-1-yl)ethyl) phosphate (294 mg) was obtained in the same manner as in EX 14. | NMR1; 1.15(3H, t, J=7.1Hz), 1.44-1.58(1H, m), 1.68-1.77(1H, m), 1.78-1.87(1H, m), 1.90-2.04(3H, m), 2.45(2H, t, J=6.2Hz), 2.74-2.81(2H, m), 3.24-3.42(5H, m), 3.43-3.47(4H, m), 3.54-3.68(4H, m), 3.72-3.83(4H, m), 4.03(2H, q, J=7.1Hz), 4.10(2H, t, J=6.1Hz), 5.10(2H, d, J=9.1Hz), 6.30(1H, dd, J=9.4Hz, 1.6Hz), 6.82(1H, dd, J=8.7Hz, 2.4Hz), 6.89(1H, d, J=2.4Hz), 7.01(1H, d, J=7.5Hz), 7.32(1H, t, J=7.9Hz), 7.51(1H, d, J=5.5Hz), 7.56(1H, d, J=8.7Hz), 7.71(1H, d, J=8.1Hz), 7.77(1H, d, J=5.5Hz), 7.80(1H, d, J=9.5Hz), 11.67(1H, s). |

| | | | |
|---|---|---|---|
| EX148 | 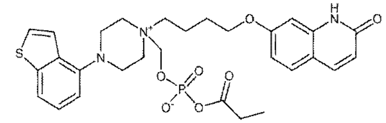 | Pyridine (5 mL), propionic anhydride (3 mL), and DMAP (112 mg) were added to (4-(benzo[b]thiophen-4-yl)-1-(4-((2-oxo-1,2-dihydroquinolin-7-yl)oxy)butyl)piperazin-1-ium-1-yl)methyl hydrogen phosphate (500 mg) at room temperature, and the mixture was stirred overnight. The reaction system was concentrated and azeotroped with toluene. $H_2O$ was added to the residue, and ultrasonic treatment was performed. The white precipitate was filtered out. Washing with $H_2O$ was performed, and drying under reduced pressure was performed. Acetone/$H_2O$=1/1 (10 mL) was added to the residue. Acetone (10 mL) was further added, and the precipitate was filtered out. (4-(benzothiophen-4-yl)-1-(4-((2-oxo-1H-quinolin-7-yl)oxy)butyl)piperazin-1-ium-1-yl)methyl propanoyl phosphate (480 mg) was obtained. | NMR1; 0.99(3H, t, J=7.4Hz), 1.79-1.87(2H, m), 1.92-1.96(2H, m), 2.36(2H, q, J=7.4Hz), 3.37-3.49(4H, m), 3.54-3.69(4H, m), 3.72-3.80(2H, m), 4.09(2H, t, J=6.1Hz), 5.19(2H, d, J=9.2Hz), 6.30(1H, dd, J=9.4Hz, 1.8Hz), 6.79-6.88(2H, m), 7.01(1H, dd, J=7.7Hz, 0.9Hz), 7.32(1H, t, J=7.9Hz), 7.52(1H, dd, J=5.5Hz, 0.9Hz), 7.56(1H, d, J=8.5Hz), 7.72(1H, d, J=8.1Hz), 7.77(1H, d, J=5.5Hz), 7.81(1H, d, J=9.5Hz), 11.62(1H, s). |
| EX149 | 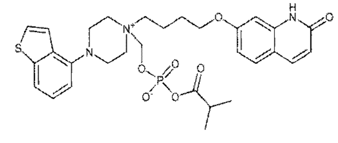 | Using isobutyric anhydride (3 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzothiophen-4-yl)-1-(4-((2-oxo-1H-quinolin-7-yl)oxy)butyl)piperazin-1-ium-1-yl)methyl 2-methylpropanoyl phosphate (493 mg) was obtained in a method similar to the synthesis of EX 148. | NMR1; 1.08(6H, d, J=7.0Hz), 1.81-1.87(2H, m), 1.92-1.96(2H, m), 3.39-3.49(4H, m), 3.52-3.68(5H, m), 3.72-3.80(2H, m), 4.09(2H, t, J=6.1Hz), 5.19(2H, d, J=9.3Hz), 6.30(1H, dd, J=9.5Hz, 1.9Hz), 6.83(2H, dd, J=10.5Hz, 2.0Hz), 7.01(1H, d, J=7.5Hz), 7.32(1H, t, J=7.9Hz), 7.52(1H, dd, J=5.5Hz, 0.9Hz), 7.56(1H, d, J=8.5Hz), 7.72(1H, d, J=8.1Hz), 7.77(1H, d, J=5.6Hz), 7.81(1H, d, J=9.5Hz), 11.62(1H, s). |
| EX150 | 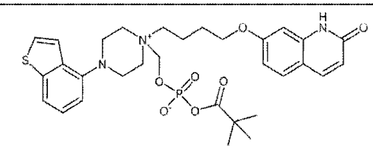 | Using pivalic anhydride (3 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzothiophen-4-yl)-1-(4-((2-oxo-1H-quinolin-7-yl)oxy)butyl)piperazin-1-ium-1-yl)methyl 2,2-dimethylpropanoyl phosphate (287 mg) was obtained in a method similar to the synthesis of EX 148. | NMR1; 1.13(9H, s), 1.81-1.88(2H, m), 1.92-1.96(2H, m), 3.39-3.49(4H, m), 3.54-3.69(4H, m), 3.73-3.81(2H, m), 4.10(2H, t, J=6.0Hz), 5.20(2H, d, J=9.4Hz), 6.30(1H, dd, J=9.4Hz, 1.9Hz), 6.79 - 6.88(2H, m), 7.01(1H, d, J=7.6Hz), 7.33(1H, t, J=7.9Hz), 7.52(1H, d, J=5.5Hz), 7.57(1H, d, J=8.6Hz), 7.72(1H, d, J=8.1Hz), 7.77(1H, d, J=5.5Hz), 7.81(1H, d, J=9.5Hz), 11.62(1H, s). |
| EX151 | 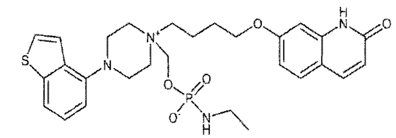 | Using ethylamine (2.8 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.3 g), (4-(benzo[b]thiophen-4-yl)-1-(4-((2-oxo-1,2-dihydroquinolin-7-yl)oxy)butyl)piperazin-1-ium-1-yl)methyl ethylphosphoramidate (156 mg) was obtained in a method similar to the synthesis of EX 152. | NMR1; 0.94-1.02(3H, m), 1.79-1.87(2H, m), 1.92-1.96(2H, m), 2.66-2.81(2H, m), 3.08(1H, q, J=6.6Hz), 3.38-3.52(4H, m), 3.52-3.65(4H, m), 3.73-3.81(2H, m), 4.11(2H, t, J=6.2Hz), 5.03(2H, d, J=9.0Hz), 6.30(1H, dd, J=9.4Hz, 1.5Hz), 6.82(1H, dd, J=8.7Hz, 2.4Hz), 6.96(1H, d, J=2.4Hz), 7.01(1H, dd, J=7.7Hz, 0.8Hz), 7.33(1H, t, J=7.9Hz), 7.52(1H, dd, J=5.5Hz, 0.9Hz), 7.56(1H, d, J=8.7Hz), 7.71(1H, d, J=8.1Hz), 7.76(1H, d, J=5.5Hz), 7.80(1H, d, J=9.5Hz), 11.82(1H, s). |

| | | | |
|---|---|---|---|
| EX152 | 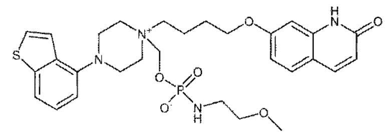 | DIPEA (0.289 mL) and HATU (420 mg) were added to a solution of (4-(benzo[b]thiophen-4-yl)-1-(4-((2-oxo-1,2-dihydroquinolin-7-yl)oxy)butyl)piperazin-1-ium-1-yl)methyl hydrogen phosphate (300 mg) in DMF (2 mL) at room temperature. After 10 minutes, 1-amino-2-methoxyethane (0.240 mL) was added, and the mixture was stirred for 1 hour. The reaction mixture was concentrated, and the residue was purified by silica gel column chromatography (DCM/MeOH) and suspended in acetone/H2O=1/1 (6 mL), and acetone (15 mL) was added while heating to 50° C, followed by stirring at room temperature overnight. The insoluble matter was filtered out to give (4-(benzo[b]thiophen-4-yl)-1-(4-((2-oxo-1,2-dihydroquinolin-7-yl)oxy)butyl)piperazin-1-ium-1-yl)methyl (2-methoxyethyl)phosphoramidate (230 mg). | NMR1; 1.79-1.87(2H, m), 1.92-1.96(2H, m), 2.81-2.93(2H, m), 3.07(1H, q, J=6.8Hz), 3.21(3H, s), 3.30(2H, t, J=6.3Hz), 3.42-3.46(4H, m), 3.51-3.66(4H, m), 3.73-3.81(2H, m), 4.11(2H, t, J=6.1Hz), 5.04(2H, d, J=9.0Hz), 6.29(1H, dd, J=9.4Hz, 1.9Hz), 6.82(1H, dd, J=8.7Hz, 2.4Hz), 6.98(1H, d, J=2.4Hz), 7.01(1H, dd, J=7.7Hz, 0.9Hz), 7.32(1H, t, J=7.9Hz), 7.51(1H, dd, J=5.6Hz, 0.9Hz), 7.55(1H, d, J=8.7Hz), 7.71(1H, d, J=8.0Hz), 7.77(1H, d, J=5.5Hz), 7.80(1H, d, J=9.5Hz), 11.83(1H, s). |
| EX153 | 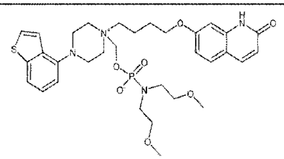 | Using bis(2-methoxyethyl)amine (0.4 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.3 g), (4-(benzo[b]thiophen-4-yl)-1-(4-((2-oxo-1,2-dihydroquinolin-7-yl)oxy)butyl)piperazin-1-ium-1-yl)methyl bis(2-methoxyethyl)phosphoramidate (224 mg) was obtained in a method similar to the synthesis of EX 152. | NMR1; 1.80-1.87(2H, m), 1.92-1.97(2H, m), 3.07(4H, m), 3.21(6H, s), 3.39(4H, d, J=6.6Hz), 3.42-3.46(4H, m), 3.53-3.65(4H, m), 3.73-3.81(2H, m), 4.11(2H, t, J=6.1Hz), 5.04(2H, d, J=9.1Hz), 6.30(1H, dd, J=9.5Hz, 1.4Hz), 6.81(1H, dd, J=8.7Hz, 2.4Hz), 6.95(1H, d, J=2.4Hz), 7.01(1H, d, J=7.6Hz), 7.32(1H, t, J=7.9Hz), 7.52(1H, dd, J=5.6Hz, 0.8Hz), 7.56(1H, d, J=8.8Hz), 7.71(1H, d, J=8.0Hz), 7.76(1H, d, J=5.6Hz), 7.80(1H, d, J=9.5Hz), 11.79(1H, s). |
| EX154 | 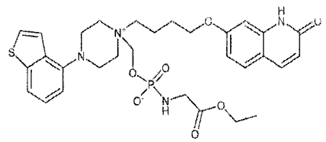 | Using glycine ethyl ester hydrochloride (154 mg) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.3 g), (4-(benzo[b]thiophen-4-yl)-1-(4-((2-oxo-1,2-dihydroquinolin-7-yl)oxy)butyl)piperazin-1-ium-1-yl)methyl (2-ethoxy-2-oxoethyl)phosphoramidate (175 mg) was obtained in a method similar to the synthesis of EX 152. | NMR1; 1.14(3H, t, J=7.1Hz), 1.78-1.86(2H, m), 1.91-1.96(2H, m), 3.42-3.47(5H, m), 3.50-3.65(6H, m), 3.73-3.82(2H, m), 4.03(2H, q, J=7.1Hz), 4.10(2H, t, J=6.1Hz), 5.04(2H, d, J=8.7Hz), 6.30(1H, dd, J=9.5Hz, 1.9Hz), 6.82(1H, dd, J=8.6Hz, 2.4Hz), 6.96(1H, d, J=2.5Hz), 7.01(1H, dd, J=7.8Hz, 0.9Hz), 7.32(1H, t, J=7.9Hz), 7.52(1H, dd, J=5.6Hz, 0.8Hz), 7.56(1H, d, J=8.7Hz), 7.71(1H, d, J=8.0Hz), 7.77(1H, d, J=5.5Hz), 7.80(1H, d, J=9.5Hz), 11.79(1H, s). |
| EX155 | 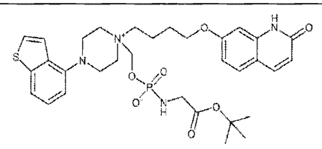 | Using glycine tert-butyl ester (0.25 mL) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.5 g), (4-(benzo[b]thiophen-4-yl)-1-(4-((2-oxo-1,2-dihydroquinolin-7-yl)oxy)butyl)piperazin-1-ium-1-yl)methyl (2-(tert-butoxy)-2-oxoethyl)phosphoramidate (540 mg) was obtained in a method similar to the synthesis of EX 152. | NMR1; 1.37(9H, s), 1.78-1.86(2H, m), 1.91-1.95(2H, m), 3.25-3.35(1H, m), 3.40-3.49(6H, m), 3.51-3.65(4H, m), 3.74-3.83(2H, m), 4.10(2H, t, J=6.1Hz), 5.04(2H, d, J=8.6Hz), 6.30(1H, d, J=9.5Hz), 6.82(1H, dd, J=8.7Hz, 2.4Hz), 6.96(1H, d, J=2.4Hz), 7.00(1H, d, J=7.5Hz), 7.32(1H, t, J=7.8Hz), 7.52(1H, dd, J=5.6Hz, 0.8Hz), 7.56(1H, d, J=8.7Hz), 7.71(1H, d, J=8.2Hz), 7.77(1H, d, J=5.6Hz), 7.80(1H, d, J=9.5Hz), 11.80(1H, s). |

| | | | |
|---|---|---|---|
| EX156 | 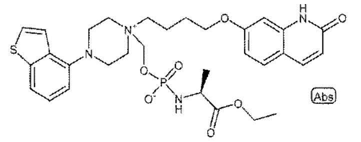 | Using L-alanine ethyl ester hydrochloride (0.6 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1 g), (4-(benzo[b]thiophen-4-yl)-1-(4-((2-oxo-1,2-dihydroquinolin-7-yl)oxy)butyl)piperazin-1-ium-1-yl)methyl (S)-(1-ethoxy-1-oxopropan-2-yl)phosphoramidate (608 mg) was obtained in a method similar to the synthesis of EX 152. | NMR1; 1.15(3H, t, J=7.1Hz), 1.20(3H, d, J=7.1Hz), 1.80-1.86(2H, m), 1.90-1.95(2H, m), 3.41-3.46(5H, m), 3.50-3.64(4H, m), 3.74-3.82(3H, m), 3.95-4.07(2H, m), 4.10(2H, t, J=6.2Hz), 5.01(2H, d, J=9.0Hz), 6.30(1H, dd, J=9.4Hz, 1.9Hz), 6.82(1H, dd, J=8.7Hz, 2.4Hz), 6.95(1H, d, J=2.4Hz), 7.00(1H, d, J=7.7Hz), 7.32(1H, t, J=7.9Hz), 7.52(1H, d, J=5.5Hz), 7.56(1H, d, J=8.7Hz), 7.71(1H, d, J=8.1Hz), 7.77(1H, d, J=5.5Hz), 7.80(1H, d, J=9.5Hz), 11.80(1H, s). |
| EX157 | 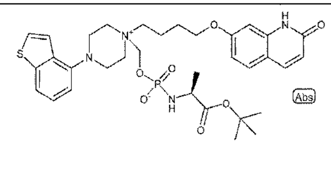 | Using L-alanine tert-butyl ester hydrochloride (1.2 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (1.6 g), (4-(benzo[b]thiophen-4-yl)-1-(4-((2-oxo-1,2-dihydroquinolin-7-yl)oxy)butyl)piperazin-1-ium-1-yl)methyl (S)-(1-(tert-butoxy)-1-oxopropan-2-yl)phosphoramidate (1.3 g) was obtained in a method similar to the synthesis of EX 152. | NMR1; 1.18(3H, d, J=7.0Hz), 1.37(9H, s), 1.80-1.86(2H, m), 1.88-1.95(2H, m), 3.42-3.46(4H, m), 3.49-3.72(6H, m), 3.74-3.83(2H, m), 4.10(2H, t, J=6.1Hz), 5.01(2H, d, J=9.1Hz), 6.30(1H, dd, J=9.5Hz, 1.9Hz), 6.82(1H, dd, J=8.7Hz, 2.4Hz), 6.93(1H, d, J=2.5Hz), 7.00(1H, d, J=7.6Hz), 7.32(1H, t, J=7.9Hz), 7.52(1H, dd, J=5.6Hz, 0.8Hz), 7.56(1H, d, J=8.7Hz), 7.71(1H, d, J=8.0Hz), 7.77(1H, d, J=5.5Hz), 7.80(1H, d, J=9.5Hz), 11.77(1H, s). |
| EX158 | 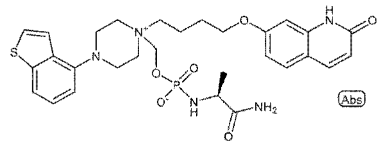 | Using L-alaninamide hydrochloride (0.4 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.7 g), (4-(benzo[b]thiophen-4-yl)-1-(4-((2-oxo-1,2-dihydroquinolin-7-yl)oxy)butyl)piperazin-1-ium-1-yl)methyl (S)-(1-amino-1-oxopropan-2-yl)phosphoramidate (554 mg) was obtained in a method similar to the synthesis of EX 152. | NMR1; 1.16(3H, d, J=6.8Hz), 1.79-1.86(2H, m), 1.91-1.95(2H, m), 3.39-3.66(10H, m), 3.73-3.81(2H, m), 4.10(2H, t, J=6.1Hz), 5.03(2H, d, J=8.9Hz), 6.30(1H, dd, J=9.4Hz, 1.8Hz), 6.82(1H, dd, J=8.7Hz, 2.5Hz), 6.84(1H, s), 6.93(1H, d, J=2.4Hz), 7.00(1H, dd, J=7.8Hz, 0.9Hz), 7.32(1H, t, J=7.9Hz), 7.52(1H, dd, J=5.6Hz, 0.8Hz), 7.56(1H, d, J=8.6Hz), 7.56(1H, s), 7.71(1H, d, J=8.0Hz), 7.76(1H, d, J=5.5Hz), 7.80(1H, d, J=9.5Hz), 11.80(1H, s). |
| EX159 | 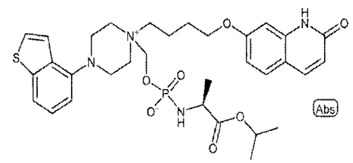 | Using L-alanine isopropyl ester hydrochloride (0.4 g) and (4-(benzo[b]thiophen-4-yl)-1-(4-((2-oxo-1,2-dihydroquinolin-7-yl)oxy)butyl)piperazin-1-ium-1-yl)methyl hydrogen phosphate (0.6 g), (4-(benzo[b]thiophen-4-yl)-1-(4-((2-oxo-1,2-dihydroquinolin-7-yl)oxy)butyl)piperazin-1-ium-1-yl)methyl (S)-(1-isopropoxy-1-oxopropan-2-yl)phosphoramidate (392 mg) was obtained in a method similar to the synthesis of EX 152. | NMR1; 1.15(6H, dd, J=6.3Hz, 1.5Hz), 1.19(3H, d, J=7.1Hz), 1.78-1.86(2H, m), 1.88-1.98(2H, m), 3.30-3.40(1H, m), 3.41-3.48(4H, m), 3.50-3.65(4H, m), 3.68-3.82(2H, m), 4.10(2H, t, J=6.0Hz), 4.77-4.93(1H, m), 5.01(2H, d, J=9.0Hz), 6.30(1H, dd, J=9.4Hz, 1.6Hz), 6.82(1H, dd, J=8.7Hz, 2.4Hz), 6.94(1H, d, J=2.4Hz), 7.00(1H, dd, J=7.8Hz, 0.9Hz), 7.33(1H, t, J=7.8Hz), 7.52(1H, dd, J=5.6Hz, 0.9Hz), 7.56(1H, d, J=8.7Hz), 7.71(1H, d, J=8.0Hz), 7.77(1H, d, J=5.5Hz), 7.80(1H, d, J=9.5Hz), 11.77(1H, s). |

| | | | |
|---|---|---|---|
| EX160 | 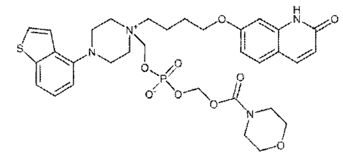 | DIPEA (0.689 mL) and chloromethyl morpholine-4-carboxylate (0.710 g) were added dropwise to a mixture of (4-(benzo[b]thiophen-4-yl)-1-(4-((2-oxo-1,2-dihydroquinolin-7-yl)oxy)butyl)piperazin-1-ium-1-yl)methyl hydrogen phosphate (0.500 g) and acetone/water (6/1) (7 mL) at room temperature. The mixture was heated to 60° C and stirred for 3 hours. The mixture was then returned to room temperature and stirred over the weekend. The solid was filtered out, and washing with acetone/water (3/1) was performed. Thereafter, air-drying was performed overnight to give (4-(benzo[b]thiophen-4-yl)-1-(4-((2-oxo-1,2-dihydroquinolin-7-yl)oxy)butyl)piperazin-1-ium-1-yl)methyl (((morpholine-4-carbonyl)oxy)methyl) phosphate (0.52 g). | NMR1; 1.79-2.00(4H, m), 3.38-3.70(16H, m), 3.72-3.79(2H, m), 4.10(2H, t, J=6.1Hz), 5.10(2H, d, J=9.0Hz), 5.43(2H, d, J=11.9Hz), 6.30(1H, dd, J=1.9Hz, 9.4Hz), 6.83(1H, dd, J=2.4Hz, 8.6Hz), 6.86(1H, d, J=2.3Hz), 7.01(1H, d, J=7.4Hz), 7.33(1H, t, J=7.9Hz), 7.52(1H, d, J=5.5Hz), 7.56(1H, d, J=8.6Hz), 7.72(1H, d, J=8.1Hz), 7.77(1H, d, J=5.6Hz), 7.80(1H, d, J=9.5Hz), 11.64(1H, s). |
| EX161 | 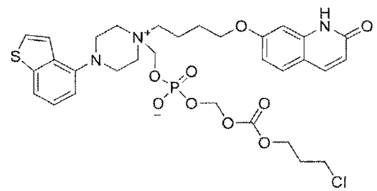 | Using chloromethyl (3-chloropropyl) carbonate (4.2 g) synthesized in REX 67 and (4-(benzo[b]thiophen-4-yl)-1-(4-((2-oxo-1,2-dihydroquinolin-7-yl)oxy)butyl)piperazin-1-ium-1-yl)methyl hydrogen phosphate (3.5 g), (4-(benzo[b]thiophen-4-yl)-1-(4-((2-oxo-1,2-dihydroquinolin-7-yl)oxy)butyl)piperazin-1-ium-1-yl)methyl ((((3-chloropropoxy)carbonyl)oxy)methyl) phosphate (1.93 g) was obtained in the same manner as in EX 121. | NMR1; 1.80-1.88 (2H, m), 1.92-1.97 (2H, m), 1.95-2.09 (2H, m), 3.42-3.49 (4H, m), 3.54-3.69 (6H, m), 3.72-3.80 (2H, m), 4.10 (2H, t, J=6.1Hz), 4.21 (2H, t, J=6.3Hz), 5.10 (2H, d, J=8.5Hz), 5.46 (2H, d, J=13.4Hz), 6.31 (1H, d, J=9.4Hz), 6.83 (1H, dd, J=8.5Hz, 2.4Hz), 6.86 (1H, d, J=2.4Hz), 7.01 (1H, d, J=7.5Hz), 7.33 (1H, t, J=7.8Hz), 7.52 (1H, dd, J=5.5Hz, 0.8Hz), 7.57 (1H, d, J=8.6Hz), 7.72 (1H, d, J=8.0Hz), 7.77 (1H, d, J=5.5Hz), 7.81 (1H, d, J=9.5Hz), 11.64 (1H, s). |

Table 3B

| | | | |
|---|---|---|---|
| REX 8 | 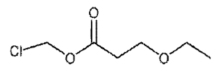 | Chloromethyl 3-ethoxypropanoate (10 g) was dissolved in DCM (180 mL) and water (280 mL), and tetra-n-butylammonium hydrogen sulfate (2.87 g) was added. After $NaHCO_3$ (28.4 g) was added thereto, a solution of chloromethyl sulfurochloridate (11.35 mL) in DCM (100 mL) was added, and the mixture was stirred at room temperature for 12 hours. The reaction solution was separated to obtain an organic layer. DCM was added to the aqueous layer, and extraction was performed to separate an organic layer. The organic layers were combined, washed with water, and dried over $MgSO_4$. After filtration, the solvent was concentrated under reduced pressure. After the residue was dissolved in hexane (80 mL) and DCM (40 mL), silica gel (neutral) (20 g) was added, followed by stirring and then filtration. The filtrate was concentrated under reduced pressure to give chloromethyl 3-ethoxypropanoate (5.34 g). | NMR2; 1.19(3H, t, J=7.0Hz), 2.66(2H, t, J=6.3Hz), 3.51(2H, q, J=7.0Hz), 3.72(2H, t,, J=6.3Hz), 5.72(2H, s). |
| REX 9 | 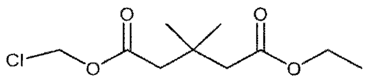 | 5-Ethoxy-3,3-dimethyl-5-oxopentanoic acid (7.0 g) was dissolved in DCM (70 mL), and $NaHCO_3$ (12.5 g), tetra-n-butylammonium hydrogen sulfate (1.26 g), and water (70 mL) were added, followed by stirring. Chloromethyl sulfurochlorochloridate was slowly added thereto, and the mixture was stirred at room temperature overnight. The organic layer was separated and concentrated under reduced pressure. The crude product obtained was purified by silica gel column chromatography (hexane/AcOEt) to give 1-(chloromethyl) 5-ethyl 3,3-dimethylpentanedioate (7.0 g). | NMR2; 1.14(6H, s), 1.26(3H, t, J=7.1Hz), 2.42(2H, s), 2.52(2H, s), 4.13(2H, q, J=7.1Hz), 5.70(2H, s). |
| REX 10 | 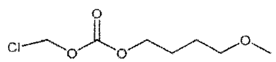 | 4-Methoxybutan-1-ol (6.16 g) was dissolved in DCM (50 mL), pyridine (5.01 mL) was added under ice-cooling, and a solution of chloromethyl chloroformate (5 mL) in DCM (50 mL) was slowly added, followed by stirring at room temperature for 3 hours. 1N HCl (50 mL) was added for liquid separation, and the organic layer was separated. The organic layer was washed with water, dried over $MgSO_4$, and filtered, and the solvent was distilled off under reduced pressure to give chloromethyl (4-methoxybutyl) carbonate (10.52 g). | NMR2; 1.62-1.71(2H, m), 1.74-1.84(2H, m), 3.33(3H, s), 3.40(2H, t, J=6.2Hz), 4.26(2H, t, J=6.5Hz), 5.73(2H, s). |
| REX 11 | 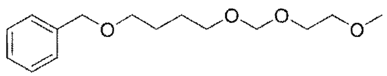 | 4-(Benzyloxy)butan-1-ol (5 g) and diisopropylethylamine (14.5 mL) were dissolved in DCM (50 mL), 2-methoxyethoxymethyl chloride was added, and the mixture was stirred at room temperature for 2 days. Water was added to the reaction solution, followed by stirring. The organic layer was separated and concentrated under reduced pressure. The crude product obtained was purified by silica gel column chromatography (hexane/AcOEt) to give 13-phenyl-2,5,7,12-tetraoxatridecane (5.76 g). | NMR2; 1.65-1.74 (4H, m), 3.39 (3H, s), 3.47-3.54 (2H, m), 3.53-3.59 (4H, m), 3.66-3.71 (2H, m), 4.50 (2H, s), 4.71 (2H, s), 7.25-7.35 (5H, m). |
| REX 12 | 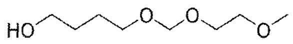 | 13-Phenyl-2,5,7,12-tetraoxatridecane (5.76 g) synthesized in REX 11 was dissolved in EtOH (100 mL), 10% Pd/C (1.0 g) was added, and the mixture was stirred at room temperature under a hydrogen atmosphere for 5 hours. The catalyst was filtered off and the filtrate was concentrated under reduced pressure to give 4-((2-methoxyethoxy)methoxy)butan-1-ol (3.61 g). | NMR2; 1.56-1.72 (4H, m), 1.93 (1H, br-s), 3.39 (3H, s), 3.50-3.64 (4H, m), 3.65-3.74 (4H, m), 4.73 (2H, s). |

| | | | |
|---|---|---|---|
| REX 13 | 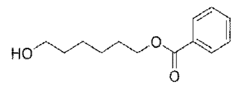 | Benzoic acid (3.03 g) and $K_2CO_3$ (6.87 g) were added to DMF (30 mL) and stirred, then 1-bromo-6-hydroxyhexane (3.0 g) was added, and the mixture was heated with stirring at 80° C for 3 hours. The reaction solution was cooled to room temperature. AcOEt and water were added to the reaction solution and stirred. After washing the organic layer twice, the organic layer was separated and concentrated under reduced pressure. The crude product obtained was purified by silica gel column chromatography (hexane/AcOEt) to give 6-hydroxyhexyl benzoate (2.73 g). | NMR2; 1.40-1.51 (4H, m), 1.52-1.64 (2H, m), 1.70-1.85 (2H, m), 3.66 (2H, q, J = 6.1Hz), 4.33 (2H, t, J = 6.6Hz), 7.44 (2H, t, J = 6.6Hz), 7.56 (1H, t, J = 6.2Hz), 8.04 (2H, d, J = 6.2Hz). |
| REX 14 | 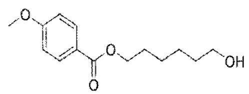 | Using 4-methoxybenzoic acid (3.0 g) and 1-bromo-6-hydroxyhexane (5.36 g), 6-hydroxyhexyl 4-methoxybenzoate (4.9 g) was obtained in the same manner as in REX 13. | NMR2; 1.26 (1H, br-s), 1.40-1.53 (4H, m), 1.54-1.65 (2H, m), 1.72-1.80 (2H, m), 3.66 (2H, q, J = 6.0Hz), 3.86 (3H, s), 4.29 (2H, t, J = 6.6Hz), 6.92 (2H, d, J = 6.9Hz), 7.99 (2H, d, J = 6.9Hz). |
| REX 15 | 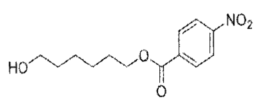 | 1,6-Hexanediol (3.0 g) and DIPEA (13.3 mL) were dissolved in DCM (90 mL), and 4-nitrobenzoyl chloride (4.2 g) was gradually added at room temperature with stirring. After 1 hour, water was added to the reaction solution, the mixture was stirred, and then the organic layer was separated and concentrated under reduced pressure. The crude product obtained was purified by silica gel column chromatography (hexane/AcOEt) to give 6-hydroxyhexyl 4-nitrobenzoate (1.21 g). | NMR2; 1.29 (1H, br-s), 1.42-1.55 (4H, m), 1.56-1.65 (2H, m), 1.82 (2H, sext, J = 7.0Hz), 3.67 (2H, q, J = 6.3Hz), 4.38 (2H, t, J = 6.7Hz), 8.21 (2H, d, J = 9.0Hz), 8.29 (2H, d, J = 9.0Hz). |
| REX 16 | 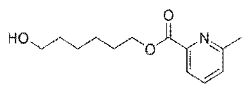 | Using 6-methylpyridine-2-carboxylic acid (5.0 g) and 6-chlorohexan-1-ol (5.98 g), 6-hydroxyhexyl 6-methylpicolinate (1.07 g) was obtained in the same manner as in REX 13. | NMR2; 1.35-1.52 (5H, m), 1.55-1.63 (2H, m), 1.78-1.87 (2H, m), 2.66 (3H, s), 3.62-3.70 (2H, m), 4.41 (2H, t, J = 6.8Hz), 7.33 (2H, d, J = 7.8Hz), 7.71 (2H, t, J = 7.8Hz), 7.91 (2H, d, J = 7.8Hz). |
| REX 17 | 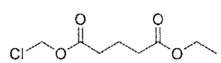 | 5-Ethoxy-5-oxopentanoic acid (5 mL) was dissolved in DCM (60 mL), $NaHCO_3$ (11.7 g), tetra-n-butylammonium hydrogensulfate (1.18 g), and water (60 mL) were added, and the mixture was stirred. Chloromethyl sulfurochloridate (4.3 mL) was then slowly added, and the mixture was stirred at room temperature overnight. After completion of the reaction, the organic layer was separated and concentrated under reduced pressure, and the crude product obtained was purified by silica gel column chromatography (hexane/AcOEt) to give chloromethyl ethyl glutarate (5.2 g). | NMR2; 1.26 (3H, t, J = 7.1Hz), 2.00 (2H, sext, J = 7.3Hz), 2.39 (2H, t, J = 7.3Hz), 2.48 (2H, t, J = 7.3Hz), 4.14 (2H, q, J = 7.1Hz), 5.71 (2H, s). |

| | | | |
|---|---|---|---|
| REX 18 | 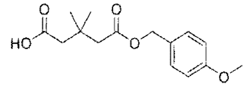 | (4-Methoxyphenyl)methanol (10.7 g) and 4,4-dimethyldihydro-2H-pyran-2,6(3H)-dione (10 g) were dissolved in DCM (200 mL), pyridine (15.4 mL) and DMAP (0.86 g) were added, and the mixture was stirred at room temperature overnight. After completion of the reaction, 2N-HCl was added to the reaction solution, and the mixture was stirred. The organic layer was separated and washed with brine. The organic layer was separated, and the solvent was distilled off under reduced pressure to give a pale yellow oil. The oil obtained was purified by silica gel column chromatography (hexane/AcOEt) to give 5-((4-methoxybenzyl)oxy)-3,3-dimethyl-5-oxopentanoic acid (13.4 g). | NMR2; 1.12 (6H, s), 2.45 (2H, s), 2.46 (2H, s), 2.60 (1H, s), 3.81 (3H, s), 5.06 (2H, s), 6.89 (2H, d, J = 6.8Hz), 7.29 (2H, d, J = 6.8Hz). |
| REX 19 | 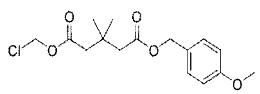 | Using 5-((4-methoxybenzyl)oxy)-3,3-dimethyl-5-oxopentanoic acid (7.0 g) synthesized in REX 18, 1-(chloromethyl) 5-(4-methoxybenzyl) 3,3-dimethylpentanedioate (5.38 g) was obtained in a method similar to the synthesis method of REX 17. | NMR2; 1.12 (6H, s), 2.45 (2H, s), 2.49 (2H, s), 3.81 (3H, s), 5.04 (2H, s), 5.66 (2H, s), 6.89 (2H, d, J = 6.8Hz), 7.29 (2H, d, J = 6.8Hz). |
| REX 20 | 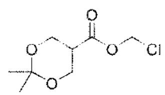 | Using 2,2-dimethyl-1,3-dioxane-5-carboxylic acid (1.8 g), chloromethyl 2,2-dimethyl-1,3-dioxane-5-carboxylate (1.7 g) was obtained in a method similar to the synthesis method of REX 17. | NMR2; 1.43 (3H, s), 1.44 (3H, s), 2.80-2.89 (1H, m), 4.04-4.16 (4H, m), 5.73 (2H, s). |
| REX 21 | 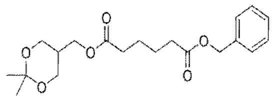 | 6-(Benzyloxy)-6-oxohexanoic acid (3 g) was dissolved in MeCN (60 mL), CDI (2.47 g) was added, and the mixture was stirred at room temperature for 1 hour. To this solution, (2,2-dimethyl-1,3-dioxan-5-yl)methanol (2 g) was added, and the mixture was heated with stirring at 90° C for 7 hours. The reaction solution was cooled to room temperature and concentrated under reduced pressure. AcOEt and water were added to the residue, and the organic layer was washed twice with water. The organic layer was separated and concentrated under reduced pressure. The crude product obtained was purified by silica gel column chromatography (hexane/AcOEt) to give benzyl ((2,2-dimethyl-1,3-dioxan-5-yl)methyl) adipate (3.8 g). | NMR2; 1.40 (3H, s), 1.44 (3H, s), 1.62-1.73 (4H, m), 1.92-2.01 (1H, m), 2.30-2.40 (4H, m), 3.71 (1H, d, J = 5.8Hz), 3.74 (1H, d, J = 5.8Hz), 3.97 (1H, d, J = 4.1Hz), 4.00 (1H, d, J = 4.1Hz), 4.17 (2H, d, J = 7.1Hz), 5.11 (2H, s), 7.30-7.45 (5H, m). |
| REX 22 | 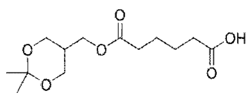 | Benzyl ((2,2-dimethyl-1,3-dioxan-5-yl)methyl) adipate (3.8 g) synthesized in REX 21 was dissolved in EtOH (40 mL), 10% Pd/C (0.56 g) was added, and the mixture was stirred at room temperature under a hydrogen atmosphere for 3 hours. The catalyst was filtered off and the filtrate was concentrated under reduced pressure to give 6-((2,2-dimethyl-1,3-dioxan-5-yl)methoxy)-6-oxohexanoic acid (2.6 g). | NMR2; 1.40 (3H, s), 1.44 (3H, s), 1.65-1.74 (4H, m), 1.94-2.03 (1H, m), 2.32-2.37 (2H, m), 2.38-2.44 (2H, m), 3.72 (1H, d, J = 5.8Hz), 3.75 (1H, d, J = 5.8Hz), 3.98 (1H, d, J = 4.0Hz), 4.01 (1H, d, J = 4.0Hz), 5.70 (2H, s). |

| | | | |
|---|---|---|---|
| REX 23 | 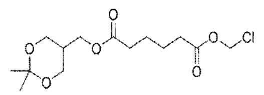 | Using 6-((2,2-dimethyl-1,3-dioxan-5-yl)methoxy)-6-oxohexanoic acid (2.6 g) synthesized in REX 22, chloromethyl ((2,2-dimethyl-1,3-dioxan-5-yl)methyl) adipate (1.64 g) was obtained in a method similar to the synthesis method of REX 17. | NMR2; 1.40 (3H, s), 1.44 (3H, s), 1.65-1.74 (4H, m), 1.94-2.03 (1H, m), 2.32-2.37 (2H, m), 2.38-2.44 (2H, m), 3.72 (1H, d, J = 5.8Hz), 3.75 (1H, d, J = 5.8Hz), 3.98 (1H, d, J = 4.0Hz), 4.01 (1H, d, J = 4.0Hz), 5.70 (2H, s). |
| REX 24 | 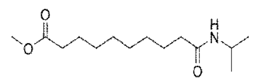 | 10-Methoxy-10-oxodecanoic acid (5.0 g) was suspended in MeCN (100 mL), isopropylamine (3.96 mL), HOBt (5.31 g), and WSC·HCl (6.65 g) were added, and the mixture was stirred at room temperature overnight. The reaction solution was concentrated under reduced pressure, and AcOEt and water were added to perform extraction. After washing the organic layer twice with water, the organic layer was separated and concentrated under reduced pressure to give methyl 10-(isopropylamino)-10-oxodecanoate (6.16 g). | NMR2; 1.14 (6H, d, J = 6.6Hz), 1.26-1.34 (8H, m), 1.56-1.64 (4H, m), 2.11 (2H, t, J = 7.4Hz), 2.30 (2H, t, J = 7.4Hz), 3.67 (3H, s), 4.10 (1H, sept, J = 6.6Hz), 5.23 (1H, br-s). |
| REX 25 | 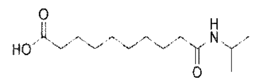 | 10-(Isopropylamino)-10-oxodecanoate (5.95 g) synthesized in REX 24 was dissolved in MeOH (60 mL), a 5N-NaOH solution (13.9 mL) was added, and the mixture was stirred at room temperature overnight. The reaction solution was concentrated under reduced pressure to remove the MeOH, and the resulting aqueous solution was neutralized with 5N-HCl. Crystals were precipitated out, filtered, and dried to give 10-(isopropylamino)-10-oxodecanoic acid (5.35 g). | NMR2; 1.14 (6H, d, J = 6.6Hz), 1.27-1.38 (8H, m), 1.55-1.67 (4H, m), 2.13 (2H, t, J = 7.4Hz), 2.34 (2H, t, J = 7.4Hz), 4.09 (1H, sept, J = 6.6Hz), 5.31 (1H, br-s). |
| REX 26 | 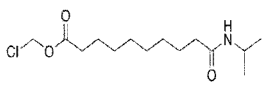 | Using 10-(isopropylamino)-10-oxodecanoic acid (3.0 g) synthesized in REX 25, chloromethyl 10-(isopropylamino)-10-oxodecanoate (1.64 g) was obtained in a method similar to the synthesis method of REX 17. | NMR2; 1.14 (6H, d, J = 6.6Hz), 1.26-1.35 (8H, m), 1.57-1.69 (4H, m), 2.11 (2H, t, J = 7.4Hz), 2.38 (2H, t, J = 7.4Hz), 4.09 (1H, sept, J = 6.6Hz), 5.23 (1H, br-s), 5.70 (2H, s). |
| REX 27 | 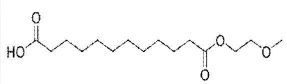 | Dodecanedioyl dichloride (25 g) was dissolved in DCM (450 mL), and a solution (50 mL) of 2-methoxyethanol (7.4 mL) in DCM was slowly added dropwise under ice-cold stirring. After completion of the dropwise addition, the mixture was stirred at room temperature overnight. To the reaction solution, water (200 mL) was added, and the mixture was stirred for 3 hours. The organic layer was separated and concentrated under reduced pressure for solidification. The crude product obtained was purified by silica gel column chromatography (hexane/AcOEt) to give 12-(2-methoxyethoxy)-12-oxodecanoic acid (11.8 g). | NMR2; 1.20-1.38 (12H, m), 1.55-1.67 (4H, m), 2.30-2.38 (4H, m), 3.39 (3H,s), 3.59 (2H, t, J = 4.7Hz), 4.23 (2H, t, J = 4.7Hz). |

| | | | |
|---|---|---|---|
| REX 28 | 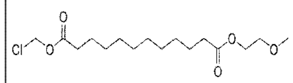 | Using 12-(2-methoxyethoxy)-12-oxodecanoic acid (10 g) synthesized in REX 27, 1-(chloromethyl) 12-(2-methoxyethyl) dodecanedioate (7.22 g) was obtained in a method similar to the synthesis method of REX 17. | NMR2; 1.22-1.36 (12H, m), 1.57-1.67 (4H, m), 2.36 (4H, sext, J = 7.9Hz), 3.39 (3H,s), 3.59 (2H, t, J = 4.7Hz), 4.23 (2H, t, J = 4.7Hz), 5.70 (2H, s). |
| REX 29 | 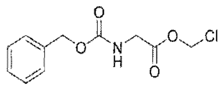 | Using ((benzyloxy)carbonyl)glycine (6.5 g), chloromethyl ((benzyloxy)carbonyl)glycinate (5.1 g) was obtained in a method similar to the synthesis method of REX 17. | NMR2; 4.09 (2H, s), 5.14 (2H, s), 5.26 (1H, br-s), 5.75 (2H, s), 7.26-7.40 (5H, m). |
| REX 30 | 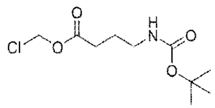 | Using 4-((tert-butoxycarbonyl)amino)butanoic acid (3.0 g), chloromethyl 4-((tert-butoxycarbonyl)amino)butanoate (3.3 g) was obtained in a method similar to the synthesis method of REX 17. | NMR2; 1.44 (9H, s), 1.85 (2H, sext, J = 7.1Hz), 2.44 (2H, t, J = 7.3Hz), 3.13-3.22 (2H, m), 4.61 (1H, br-s), 5.71 (2H,s). |
| REX 31 | 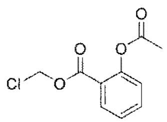 | Using 2-acetoxybenzoic acid (3.0 g), chloromethyl 2-acetoxybenzoate (3.3 g) was obtained in a method similar to the synthesis method of REX 17. | NMR2; 2.38 (3H, s), 5.90 (2H, s), 7.14 (1H, dd, J = 8.2Hz, 1.1Hz), 7.35 (1H, td, J = 7.7Hz, 1.2Hz), 7.62 (1H, td, J = 7.6Hz, 1.7Hz), 8.06 (1H, dd, J = 8.0Hz, 1.7Hz). |
| REX 32 | 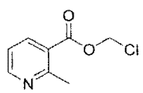 | Using 2-methylnicotinic acid (9.7 g), chloromethyl 2-methylnicotinate (4.67 g) was obtained in a method similar to the synthesis method of REX 17. | NMR2; 2.88 (3H, s), 5.95 (2H, s), 7.23-7.28 (1H, m), 8.26 (1H, dd, J = 8.0Hz, 1.8Hz), 8.67 (1H, dd, J = 4.8Hz, 1.8Hz). |

| | | | |
|---|---|---|---|
| REX 33 | 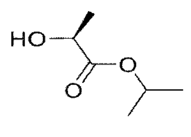 | Thionyl chloride (5 mL) was added to a solution of L-lactic acid (2.459 mL) in isopropyl alcohol (50 mL) at room temperature, and the mixture was stirred at 80° C for 10 hours. The reaction system was concentrated and the residue was ice-cooled. After dissolving in AcOEt, a saturated sodium bicarbonate solution was added. Extraction was performed with AcOEt, the organic layer was washed with brine, dried over $Na_2SO_4$, and concentrated to give isopropyl (S)-2-hydroxypropanoate (1.87 g). | NMR2; 1.28 (6H, dd, J=6.3Hz, 2.5Hz), 1.40 (3H, d, J=6.9Hz), 2.83 (1H, d, J=5.3Hz), 4.16-4.30 (1H, m), 5.09 (1H, hept, J=6.3Hz). |
| REX 34 | 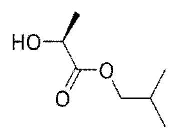 | L-Lactic acid (2.6 mL), isobutyl alcohol (4.1 mL), and p-toluenesulfonic acid monohydrate (0.67 g) were added to toluene (60 mL), and the mixture was stirred at 120° C overnight. A saturated sodium bicarbonate solution was added to the reaction system, followed by washing with AcOEt and extraction. The organic layer was washed with brine, dried over $Na_2SO_4$, and concentrated. The residue was purified by silica gel chromatography (hexane/AcOEt) to give isobutyl (S)-2-hydroxypropanoate (1.79 g). | NMR2; 0.95 (6H, dd, J=6.7Hz, 0.6 Hz), 1.43 (3H, d, J=6.9Hz), 1.89-2.06 (1H, m), 2.82 (1H, d, J=5.3Hz), 3.94 (1H, dd, J=10.5Hz, 6.6Hz), 4.01 (1H, dd, J=10.6Hz, 6.7Hz), 4.16-4.36 (1H, m). |
| REX 35 | 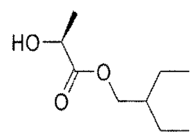 | Using 2-ethyl-1-butanol (4.1 mL) and L-lactic acid (1.639 mL), 2-ethylbutyl (S)-2-hydroxypropanoate (947 mg) was obtained in the same manner as in REX 34. | NMR2; 0.90 (6H, t, J=7.5Hz), 1.28-1.41 (4H, m), 1.42 (3H, d, J=6.9Hz), 1.49-1.62 (1H, m), 2.81 (1H, d, J=5.2Hz), 4.09 (1H, dd, J=10.9Hz, 5.7Hz), 4.15 (1H, dd, J=10.8Hz, 5.9Hz), 4.18-4.38 (1H, m). |
| REX 36 | 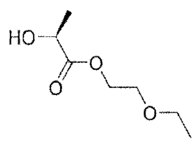 | Using 2-ethoxyethanol (10 mL) and L-lactic acid (3 mL), 2-ethoxyethyl (S)-2-hydroxypropanoate (3.1 g) was obtained in the same manner as in REX 34. | NMR2; 1.21 (3H, t, *J*=7.0Hz), 1.43 (3H, d, *J*=6.9Hz), 2.81 (1H, d, *J*=5.3Hz), 3.53 (2H, q, J=7.0Hz), 3.63-3.76 (2H, m), 4.28-4.35 (3H, m). |
| REX 37 | 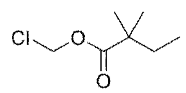 | 2,2-Dimethylbutyric acid (1 mL), $NaHCO_3$ (2.68 g), and tetrabutylammonium hydrogen sulfate (0.271 g) were added to a mixed solvent of DCM (20 mL) and $H_2O$ (20 mL), and chloromethyl chlorosulfonate (1 mL) was added dropwise, followed by vigorous stirring at room temperature overnight. The organic layer of the reaction solution was separated and concentrated at water temperature. The residue was dissolved in hexane/AcOEt = 2/5 (70 mL), and silica gel was added. After 30 minutes, filtration with Celite was performed. The filtrate was concentrated to give chloromethyl 2,2-dimethylbutanoate (1.16 g). | NMR2; 0.86 (3H, t, *J*=7.5Hz), 1.19 (6H, s), 1.60 (2H, q, *J*=7.5Hz), 5.72 (2H, s). |
| REX 38 | 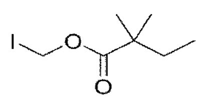 | MeCN (10 mL) and NaI (2.1 g) were added to chloromethyl 2,2-dimethylbutanoate (1.16 g) synthesized in REX 37, and the mixture was stirred at room temperature overnight. The reaction system was concentrated at water temperature, brine was added, and extraction with AcOEt was performed. The organic layer was washed with a 10% sodium thiosulfate aqueous solution, dried over $Na_2SO_4$, and concentrated at water temperature to give iodomethyl 2,2-dimethylbutanoate (1.48 g). | NMR2; 0.86 (3H, t, *J*=7.5Hz), 1.16 (6H, s), 1.59 (2H, q, *J*=7.5Hz), 5.93 (2H, s). |

| | | | |
|---|---|---|---|
| REX 39 | 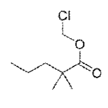 | Using 2,2-dimethylpentanoic acid (10 mL) and chloromethyl chlorosulfonate (9.3 mL), chloromethyl 2,2-dimethylpentanoate (11.5 g) was obtained in the same manner as in REX 37. | NMR2; 0.90 (3H, t, $J$=7.3Hz), 1.20 (6H, s), 1.26 (2H, t, $J$=7.3Hz), 1.48–1.57 (2H, m), 5.72 (2H, s). |
| REX 40 | 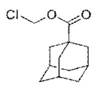 | Using 1-adamantanecarboxylic acid (2 g) and chloromethyl chlorosulfonate (1.34 mL), chloromethyl (3r,5r,7r)-adamantane-1-carboxylate (2.3 g) was obtained in the same manner as in REX 37. | NMR2; 1.65–1.79 (6H, m), 1.89–1.94 (6H, m), 2.00–2.08 (3H, m), 5.71 (2H, s). |
| REX 41 | 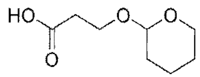 | Using methyl 3-hydroxypropanoate (1.7 g), 3-((tetrahydro-2H-pyran-2-yl)oxy)propanoic acid (2.2 g) was obtained in the same manner as in REX 43. | NMR2; 1.47–1.66 (4H, m), 1.63–1.88 (2H, m), 2.67 (2H, t, $J$=6.3Hz), 3.48–3.57 (1H, m), 3.72 (1H, dt, $J$=10.1Hz, 6.3Hz), 3.82–3.92 (1H, m), 4.01 (1H, dt, $J$=10.1Hz, 6.2Hz), 4.65 (1H, dd, $J$=4.2Hz, 2.8Hz). |
| REX 42 | 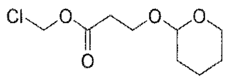 | Using 3-((tetrahydro-2H-pyran-2-yl)oxy)propanoic acid (2.2 g) synthesized in REX 41 and chloromethyl chlorosulfonate (1.66 mL), chloromethyl 3-((tetrahydro-2H-pyran-2-yl)oxy)propanoate (2 g) was obtained in the same manner as in REX 37. | NMR2; 1.45–1.88 (6H. m), 2.70 (2H, t, $J$=6.3Hz), 3.47–3.59 (1H. m), 3.72 (1H, dt, $J$=10.1Hz, 6.3Hz), 3.80–3.89 (1H, m), 4.03 (1H, dt, $J$=10.1Hz, 6.2Hz), 4.64 (1H, dd, $J$=4.0Hz, 2.9Hz), 5.67–5.79 (2H, m). |
| REX 43 | 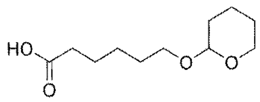 | PPTS (0.309 g) and DHP (1.7 mL) were added to a solution of ethyl 6-hydroxyhexanoate (2 mL) in DCM (120 mL) at room temperature, and the mixture was stirred overnight. $H_2O$ and brine were added to the reaction system, and extraction with DCM was performed. The organic layer was washed with brine, dried over $Na_2SO_4$, and concentrated. The intermediate (3.9 g) was dissolved in MeOH (100 mL), a 1N NaOH aqueous solution (100 mL) was added, and the mixture was stirred at 60° C for 3 hours. The reaction system was concentrated, and the aqueous layer was washed with DCM. Citric acid was added to the aqueous layer to make it slightly acidic, followed by extraction with AcOEt. The organic layer was dried over $Na_2SO_4$ and concentrated to give 6-((tetrahydro-2H-pyran-2-yl)oxy)hexanoic acid (2.7 g). | NMR2; 1.37–1.48 (2H, m), 1.48–1.76 (9H, m), 1.76–1.89 (1H, m), 2.37 (2H, t, $J$=7.5Hz), 3.39 (1H, dt, $J$=9.7Hz, 6.5Hz), 3.46–3.56 (1H, m), 3.74 (1H, dt, $J$=9.6Hz, 6.7Hz), 3.81–3.92 (1H, m), 4.58 (1H, dd, $J$=4.5Hz, 2.8Hz). |
| REX 44 | 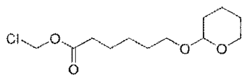 | Using 6-((tetrahydro-2H-pyran-2-yl)oxy)hexanoic acid (2.7 g) synthesized in REX 43 and chloromethyl chlorosulfonate (1.69 mL), chloromethyl 6-((tetrahydro-2H-pyran-2-yl)oxy)hexanoate (3.1 g) was obtained in the same manner as in REX 37. | NMR2; 1.35–1.48 (2H, m), 1.48–1.76 (9H, m), 1.76–1.88 (1H, m), 2.40 (2H, t, $J$=7.5Hz), 3.39 (1H, dt, $J$=9.7Hz, 6.5Hz), 3.45–3.55 (1H, m), 3.74 (1H, dt, $J$=9.7Hz, 6.7Hz), 3.81–3.91 (1H, m), 4.57 (1H, dd, $J$=4.6Hz, 2.7Hz), 5.70 (2H, s). |
| REX 45 | 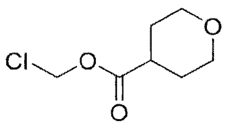 | Using tetrahydropyran-4-carboxylic acid (5 g) and chloromethyl chlorosulfonate (5 mL), chloromethyl tetrahydro-2H-pyran-4-carboxylate (2.76 g) was obtained in the same manner as in REX 37. | NMR2; 1.72–1.93 (4H, m), 2.63 (1H, tt, $J$=10.6Hz, 4.5Hz), 3.38–3.50 (2H, m), 3.91–4.02 (2H, m), 5.73 (2H, s). |

| | | | |
|---|---|---|---|
| REX 46 | 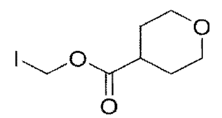 | Using chloromethyl tetrahydro-2H-pyran-4-carboxylate (1 g) synthesized in REX 45 and NaI (1 g), iodomethyl tetrahydro-2H-pyran-4-carboxylate (647 mg) was obtained in the same manner as in REX 38. | NMR2; 1.71–1.92 (4H, m), 2.52–2.69 (1H, m), 3.38–3.50 (2H, m), 3.97 (2H, dt, $J$=11.6Hz, 3.7 Hz), 5.94 (2H, s). |
| REX 47 | 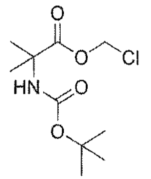 | Using 2-(tert-butoxycarbonylamino)isobutyric acid (5 g) and chloromethyl chlorosulfonate (3 mL), chloromethyl 2-((tert-butoxycarbonyl)amino)-2-methylpropanoate (5.8 g) was obtained in the same manner as in REX 37. | NMR2; 1.43 (9H, s), 1.51 (6H, s), 4.91 (1H, s), 5.75 (2H, s). |
| REX 48 | 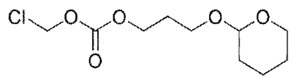 | Pyridine (2 mL) was added to a solution of 1,3-propanediol (5 mL) in DCM (50 mL) under ice-cooling, a DCM solution (20 mL) of chloromethyl chloroformate (2 mL) was added thereto with a dropping funnel, and the mixture was stirred overnight. A 1N HCl aqueous solution was added to the reaction system, and extraction was performed with DCM. The organic layer was washed with $H_2O$, dried over $Na_2SO_4$, and concentrated. PPTS (0.452 g) and DHP (2.5 mL) were added to a solution of the intermediate (2.94 g) in DCM (100 mL) at room temperature, and the mixture was stirred overnight. $H_2O$ and brine were added to the reaction system, and extraction was performed with DCM. The organic layer was washed with brine and concentrated. The residue was purified by silica gel chromatography (hexane/AcOEt) to give chloromethyl (3-((tetrahydro-2H-pyran-2-yl)oxy)propyl) carbonate (3.6 g). | NMR2; 1.46–1.65 (4H, m), 1.65–1.87 (2H, m), 1.92-2.07 (2H, m), 3.43–3.56 (2H, m), 3.78–3.90 (2H, m), 4.36 (2H, t, J=6.4Hz), 4.59 (1H, dd, J=4.4Hz, 2.8Hz), 5.73 (2H, s). |
| REX 49 | 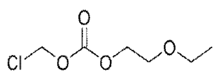 | Chloromethyl chloroformate (2 mL) and pyridine (2 mL) were added to a solution of 2-ethoxyethanol (2.5 mL) in DCM (50 mL) under ice-cooling, and the mixture was stirred overnight. A 1N HCl aqueous solution was added to the reaction system, and extraction with DCM was performed. The organic layer was washed with $H_2O$ and concentrated to give chloromethyl (2-ethoxyethyl) carbonate (4 g). | NMR2; 1.22 (3H, t, $J$=7.0Hz), 3.54 (2H, q, $J$=7.0Hz), 3.66–3.71 (2H, m), 4.33–4.40 (2H, m), 5.74 (2H, s). |
| REX 50 | 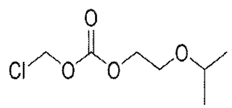 | Using ethylene glycol monoisopropyl ether (6 mL) and chloromethyl chloroformate (4 mL), chloromethyl (2-isopropoxyethyl) carbonate (8.9 g) was obtained in the same manner as in REX 49. | NMR2; 1.17 (6H, d, $J$=6.1Hz), 3.55–3.67 (1H, m), 3.63–3.70 (2H, m), 4.31–4.38 (2H, m), 5.74 (2H, s). |
| REX 51 | 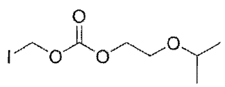 | Using chloromethyl (2-isopropoxyethyl) carbonate (8.9 g) synthesized in REX 50 and NaI (9 g), iodomethyl (2-isopropoxyethyl) carbonate (12.6 g) was obtained in the same manner as in REX 38. | NMR2; 1.17 (6H, d, $J$=6.1Hz), 3.56–3.70 (3H, m), 4.30–4.37 (2H, m), 5.96 (2H, s). |

| | | | |
|---|---|---|---|
| REX 52 | 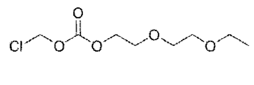 | Using diethylene glycol monoethyl ether (3.6 mL) and chloromethyl chloroformate (2 mL), chloromethyl (2-(2-ethoxyethoxy)ethyl) carbonate (5.08 g) was obtained in the same manner as in REX 49. | NMR2; 1.21 (3H, t, $J$=7.0Hz), 3.53 (2H, q, $J$=7.0Hz), 3.57–3.63 (2H, m), 3.63–3.69 (2H, m), 3.74–3.80 (2H, m), 4.35–4.42 (2H, m), 5.73 (2H, s). |
| REX 53 | 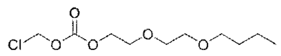 | Using diethylene glycol monobutyl ether (6.9 mL) and chloromethyl chloroformate (3 mL), 2-(2-butoxyethoxy)ethyl (chloromethyl) carbonate (8 g) was obtained in the same manner as in REX 49. | NMR2; 0.92 (3H, t, $J$=7.3Hz), 1.30–1.43 (2H, m), 1.51–1.63 (2H, m), 3.46 (2H, t, $J$=6.7Hz), 3.55–3.62 (2H, m), 3.62–3.69 (2H, m), 3.72–3.79 (2H, m), 4.34–4.41 (2H, m), 5.73 (2H, s). |
| REX 54 | 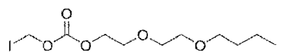 | Using 2-(2-butoxyethoxy)ethyl (chloromethyl) carbonate (9.9 g) synthesized in REX 53 and NaI (10 g), 2-(2-butoxyethoxy)ethyl (iodomethyl) carbonate (12.29 g) was obtained in the same manner as in REX 38. | NMR2; 0.92 (3H, t, $J$=7.4Hz), 1.30–1.43 (2H, m), 1.51–1.63 (2H, m), 3.46 (2H, t, $J$=6.7Hz), 3.56–3.61 (2H, m), 3.62 – 3.67 (2H, m), 3.73 – 3.77 (2H, m), 4.33 – 4.41 (2H, m), 5.95 (2H, s). |
| REX 55 | 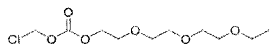 | Using triethylene glycol monoethyl ether (5 mL) and chloromethyl chloroformate (2 mL), chloromethyl (2-(2-(2-ethoxyethoxy)ethoxy)ethyl) carbonate (4.9 g) was obtained in the same manner as in REX 49. | NMR2; 1.21 (3H, t, $J$=7.0Hz), 3.53 (2H, q, $J$=7.0Hz), 3.57–3.62 (2H, m), 3.62–3.69 (2H, m), 3.67 (4H, s), 3.72–3.79 (2H, m), 4.34–4.41 (2H, m), 5.73 (2H, s). |
| REX 56 | 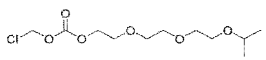 | Using triethylene glycol monoisopropyl ether (5 mL) and chloromethyl chloroformate (2 mL), chloromethyl (2-(2-(2-isopropoxyethoxy)ethoxy)ethyl) carbonate (5.79 g) was obtained in the same manner as in REX 49. | NMR2; 1.16 (6H, d, $J$=6.1Hz), 3.54–3.70 (5H, m), 3.67 (4H, s), 3.72-3.79 (2H, m), 4.34–4.41 (2H, m), 5.73 (2H, s). |
| REX 57 | 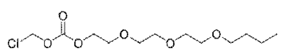 | Using triethylene glycol monobutyl ether (6 mL) and chloromethyl chloroformate (2 mL), 2-(2-(2-butoxyethoxy)ethyl (chloromethyl) carbonate (6.27 g) was obtained in the same manner as in REX 49. | NMR2; 0.91 (3H, t, $J$=7.4Hz), 1.29–1.43 (2H, m), 1.51–1.62 (2H, m), 3.46 (2H, t, $J$=6.7Hz), 3.54–3.61 (2H, m), 3.61–3.70 (2H, m), 3.66 (4H, s), 3.72–3.79 (2H, m), 4.34–4.41 (2H, m), 5.73 (2H, s). |
| REX 58 | 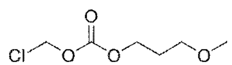 | Using 3-methoxy-1-propanol (5 mL) and chloromethyl chloroformate (4 mL), chloromethyl (3-methoxypropyl) carbonate (8.04 g) was obtained in the same manner as in REX 49. | NMR2; 1.90-2.05 (2H, m), 3.34 (3H, s), 3.47 (2H, t, J=6.1Hz), 4.33 (2H, t, J=6.4Hz), 5.73 (2H, s). |

| | | | |
|---|---|---|---|
| REX 59 | 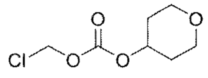 | Using tetrahydro-4-pyranol (3.9 g) and chloromethyl chloroformate (3 mL), chloromethyl (tetrahydro-2H-pyran-4-yl) carbonate (6.49 g) was obtained in the same manner as in REX 49. | NMR2; 1.70-1.84 (2H, m), 1.96-2.06 (2H, m), 3.55 (2H, ddd, *J*=11.8Hz, 8.8Hz, 3.1Hz), 3.89-4.00 (2H, m), 4.91 (1H, tt, *J*=8.5Hz, 4.1Hz), 5.74 (2H, s). |
| REX 60 | 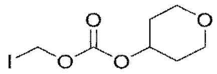 | Using chloromethyl (tetrahydro-2H-pyran-4-yl) carbonate (1 g) synthesized in REX 59 and NaI (1 g), iodomethyl (tetrahydro-2H-pyran-4-yl) carbonate (1.36 g) was obtained in the same manner as in REX 38. | NMR2; 1.69-1.84 (2H, m), 1.95-2.05 (2H, m), 3.48-3.60 (2H, m), 3.88-4.00 (2H, m), 4.90 (1H, tt, *J*=8.5Hz, 4.1Hz), 5.96 (2H, s). |
| REX 61 | 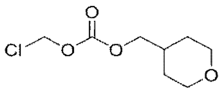 | Using tetrahydropyran-4-methanol (4.1 g) and chloromethyl chloroformate (2.8 mL), chloromethyl ((tetrahydro-2H-pyran-4-yl)methyl) carbonate (7.1 g) was obtained in the same manner as in REX 49. | NMR2; 1.28-1.47 (2H, m), 1.60-1.70 (2H, m), 1.91-2.07 (1H, m), 3.40 (2H, td, *J*=11.8Hz, 2.1Hz), 3.95-4.04 (2H, m), 4.09 (2H, d, *J*=6.7Hz), 5.73 (2H, s). |
| REX 62 | 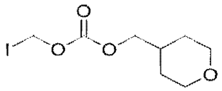 | Using chloromethyl ((tetrahydro-2H-pyran-4-yl)methyl) carbonate (1 g) synthesized in REX 61 and NaI (1 g), iodomethyl ((tetrahydro-2H-pyran-4-yl)methyl) carbonate (1.34 g) was obtained in the same manner as in REX 38. | NMR2; 1.31-1.46 (2H, m), 1.59-1.69 (2H, m), 1.90-2.07 (1H, m), 3.40 (2H, td, *J*=11.9Hz, 2.2Hz), 3.95-4.04 (2H, m), 4.08 (2H, d, *J*=6.7Hz), 5.96 (2H, s). |
| REX 63 | 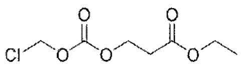 | Using ethyl 3-hydroxypropanoate (5 g) and chloromethyl chloroformate (3.6 mL), ethyl 3-(((chloromethoxy)carbonyl)oxy)propanoate (8.4 g) was obtained in the same manner as in REX 49. | NMR2; 1.27 (3H, t, *J*=7.2Hz), 2.72 (2H, t, *J*=6.3Hz), 4.18 (2H, q, *J*=7.2Hz), 4.50 (2H, t, *J*=6.3Hz), 5.73 (2H, s). |
| REX 64 | 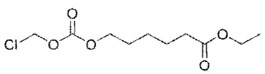 | Using ethyl 6-hydroxyhexanoate (4.2 mL) and chloromethyl chloroformate (2 mL), ethyl 6-(((chloromethoxy)carbonyl)oxy)hexanoate (5.4 g) was obtained in the same manner as in REX 49. | NMR2; 1.26 (3H, t, *J*=7.1Hz), 1.36-1.49 (2H, m), 1.61-1.78 (4H, m), 2.31 (2H, t, *J*=7.4Hz), 4.13 (2H, q, *J*=7.1Hz), 4.23 (2H, t, *J*=6.6Hz), 5.73 (2H, s). |
| REX 65 | 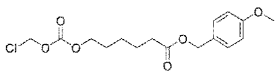 | Using 4- methoxybenzyl 6-hydroxyhexanoate (3 g) and chloromethyl chloroformate (1.3 mL), 4-methoxybenzyl 6-(((chloromethoxy)carbonyl)oxy)hexanoate (3.8 g) was obtained in the same manner as in REX 49. | NMR2; 1.34-1.46 (2H, m), 1.61-1.76 (4H, m), 2.34 (2H, t, *J*=7.4Hz), 3.81 (3H, s), 4.20 (2H, t, *J*=6.6Hz), 5.05 (2H, s), 5.72 (2H, s), 6.85-6.93 (2H, m), 7.25-7.32 (2H, m). |

| | | | |
|---|---|---|---|
| REX 66 | 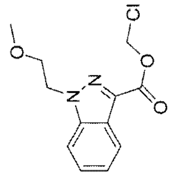 | 1H-Indazole-3-carboxylic acid (4.0 g) was suspended in acetone (100 mL), and 5N-NaOH (18.5 mL) and water (20 mL) were added, followed by stirring. 1-Bromo-2-methoxyethane (12.9 g) was slowly added thereto, and the mixture was heated under reflux for 3 hours. The reaction solution was cooled to room temperature and concentrated under reduced pressure. The concentrated solution was stirred under ice-cooling, and 5N-HCl was added to make it slightly acidic. The color of the solution turned from orange to yellow, and crystals precipitated. They were filtered out, and washing with water was performed until the filtrate was neutral. The obtained crude crystals were purified by medium-pressure liquid chromatography (DCM-MeOH) to give 1-(2-methoxyethoxy)-1H-indazole-3-carboxylic acid (4.0 g). Using 1-(2-methoxyethoxy)-1H-indazole-3-carboxylic acid (4.0 g), chloromethyl 1-(2-methoxyethoxy)-1H-indazole-3-carboxylate (3.96 g) was obtained in a method similar to the synthesis method of REX 17. | NMR2; 3.28 (3H, s), 3.88 (2H, t, J = 5.4Hz), 4.67 (2H, t, J = 5.4Hz), 6.07 (2H, s), 7.36 (1H, td, J = 6.1Hz, 0.8Hz), 7.47 (1H, td, J = 6.8Hz, 1.0Hz), 7.60 (1H, dd, J = 7.8Hz, 0.8Hz), 8.23 (1H, dt, J = 8.2Hz, 1.0Hz). |
| REX 67 | 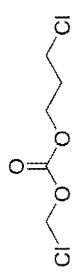 | Using 3-chloro-1-propanol (2 mL) and chloromethyl chloroformate (2.5 mL), chloromethyl (3-chloropropyl) carbonate (4.33 g) was obtained in the same manner as in REX 49. | NMR2; 2.1-2.23 (2H, m), 3.64 (2H, t, J=6.3Hz), 4.40 (2H, t, J=6.0Hz), 5.74 (2H, s). |

Each obtained compound was used in each of the following Test Examples. The compounds of Reference

Examples 3 and 4 were the compounds respectively described in Examples 58 and 706 of PTL 2 (WO2013/035892) above.

Cytotoxicity Test using HL-60 Cells

(1) Cell Culture

An HL-60 cell line (human acute promyelocytic leukemia cells) was successively cultured in RPMI 1640 containing 20% fetal bovine serum (FBS) at 37°C in 95% atmosphere/5% $CO_2$. Before evaluation, the cells were pre-cultured for about 72 hours in the presence of 10 nM phorbol 12-myristate 13-acetate (PMA) to induce macrophage-like differentiation.

(2) Evaluation Compound Treatment

Evaluation compounds were dissolved in DMSO to obtain 30 mmol/L solutions (or suspensions). Each DMSO solution was diluted 100-fold with RPMI 1640 containing 2% FBS (final concentration of the compound: 300 μmol/L) to obtain a treatment solution. The culture supernatant of the differentiated HL-60 cells was removed, and each treatment solution was added and cultured for about 24 hours, followed by cytotoxicity evaluation. The Reference Example 3 compound, which showed irritation when subcutaneously administered to dogs, was used as the positive control compound, while Risperidone, whose subcutaneous injection is on the market, was used as the negative control compound.

(3) Cytotoxicity Evaluation

The activity of lactate dehydrogenase (LDH) was measured in the treatment solutions exposed to the cells for about 24 hours by using a commercial kit (Cytotoxicity LDH Assay Kit-WST, Dojindo Laboratories) and a plate reader (Tecan Infinite M1000). The obtained measurement values were used to calculate cytotoxicity (%) as comparison values for each evaluation compound-treated group, with the no-treatment group taken as 0% and the cell lysis buffer-treated group taken as 100%. The test was conducted in triplicate in three wells each, and the average values were used as adopted values. The test was considered feasible when the positive control compound-treated group showed obvious cytotoxicity while the negative control compound-treated group showed almost no cytotoxicity.

(4) Test Results (Discussion)

The table below shows the test results for cytotoxicity (%).

Table 4

| Evaluation compound | Evaluation compound results | Negative control (Risperidone) | Positive control (Reference Example 3 compound) |
|---|---|---|---|
| EX 1, EX 2 | -7 | 9 | 44 |
| EX 3 | -7 | 8 | 38 |
| EX 4 | -5 | 8 | 38 |
| EX 5 | -4 | 21 | 45 |
| EX 6 | 16 | 21 | 45 |
| EX 7 | -4 | 21 | 45 |
| EX 8 | 15 | 21 | 45 |
| EX 9 | -2 | 21 | 45 |
| EX 10 | -6 | 15 | 44 |
| EX 11 | 16 | 15 | 44 |
| EX 12 | -7 | 21 | 45 |
| EX 13 | -5 | 21 | 45 |
| EX 14 | -10 | 15 | 44 |

(continued)

| Evaluation compound | Evaluation compound results | Negative control (Risperidone) | Positive control (Reference Example 3 compound) |
|---|---|---|---|
| EX 15 | 3 | 15 | 44 |
| EX 16 | 17 | 15 | 44 |
| EX 17 | 8 | 15 | 44 |
| EX 18 | 8 | 15 | 44 |
| EX 19 | 16 | 15 | 44 |
| EX 24 | 10 | 16 | 58 |
| EX 26 | -5 | 16 | 58 |
| EX 28 | -8 | 26 | 53 |
| EX 29 | -4 | 26 | 53 |
| EX 30 | 14 | 7 | 44 |
| EX 31 | -8 | 26 | 53 |
| EX 32 | 5 | 16 | 52 |
| EX 33 | -7 | 16 | 52 |
| EX 34 | -10 | 16 | 52 |
| EX 35 | -9 | 16 | 52 |
| EX 36 | -10 | 26 | 53 |
| EX 38 | 0 | 7 | 35 |
| EX 39 | 2 | 7 | 35 |
| EX 40 | -5 | 16 | 43 |
| EX 41 | -3 | 9 | 39 |
| EX 42 | 8 | 16 | 43 |
| EX 43 | -6 | 16 | 43 |
| EX 44 | -8 | 16 | 58 |
| EX 45 | 3 | 7 | 35 |
| EX 46 | 14 | 7 | 35 |
| EX 47 | -5 | 8 | 39 |
| EX 48 | -9 | 7 | 44 |
| EX 49 | -7 | 16 | 58 |
| EX 50 | -8 | 16 | 58 |
| EX 51 | -9 | 16 | 43 |
| EX 52 | -1 | 8 | 44 |
| EX 53 | -3 | 7 | 35 |
| EX 54 | -10 | 8 | 44 |
| EX 55 | -7 | 21 | 45 |
| EX 56 | -6 | 16 | 43 |
| EX 57 | 4 | 9 | 39 |
| EX 59 | 1 | 16 | 43 |
| EX 60 | 16 | 7 | 35 |
| EX 61 | 5 | 16 | 52 |

(continued)

| Evaluation compound | Evaluation compound results | Negative control (Risperidone) | Positive control (Reference Example 3 compound) |
|---|---|---|---|
| EX 62 | 3 | 7 | 35 |
| EX 64 | 9 | 7 | 35 |
| EX 66 | -2 | 9 | 39 |
| EX 67 | 1 | 7 | 44 |
| EX 68 | -6 | 26 | 53 |
| EX 70 | -7 | 9 | 49 |
| EX 71 | -9 | 26 | 53 |
| EX 74 | -1 | 8 | 39 |
| EX 77 | 6 | 9 | 49 |
| EX 80 | 7 | 9 | 49 |
| EX 81 | 8 | 26 | 53 |
| EX 82 | -2 | 9 | 49 |
| EX 83 | -5 | 16 | 43 |
| EX 84 | 1 | 12 | 39 |
| EX 90 | -6 | 9 | 49 |
| EX 91 | 17 | 9 | 49 |
| EX 92 | -7 | 21 | 45 |
| EX 93 | -6 | 16 | 43 |
| EX 94 | -8 | 16 | 58 |
| EX 95 | -4 | 16 | 52 |
| EX 96 | 12 | 16 | 43 |
| EX 97 | -2 | 16 | 43 |
| EX 98 | -8 | 16 | 52 |
| EX 99 | -6 | 16 | 52 |
| EX 100 | -3 | 7 | 35 |
| EX 101 | 16 | 9 | 39 |
| EX 102 | 16 | 9 | 39 |
| EX 103 | 3 | 8 | 39 |
| EX 104 | -5 | 7 | 44 |
| EX 105 | -6 | 8 | 44 |
| EX 106 | 10 | 8 | 44 |
| EX 107 | -6 | 8 | 44 |
| EX 108 | -7 | 8 | 44 |
| EX 110 | -3 | 7 | 35 |
| EX 111 | 0 | 9 | 39 |
| EX 112 | -7 | 16 | 52 |
| EX 113 | 10 | 9 | 39 |
| EX 114 | 14 | 7 | 35 |
| EX 116 | -9 | 16 | 58 |

(continued)

| Evaluation compound | Evaluation compound results | Negative control (Risperidone) | Positive control (Reference Example 3 compound) |
|---|---|---|---|
| EX 117 | -7 | 16 | 52 |
| EX 118 | -8 | 16 | 58 |
| EX 119 | 2 | 7 | 44 |
| EX 120 | 13 | 8 | 39 |
| EX 123 | -8 | 16 | 58 |
| EX 125 | -7 | 7 | 44 |
| EX 126 | -7 | 7 | 44 |
| EX 127 | 0 | 8 | 39 |
| EX 128 | 2 | 9 | 49 |
| EX 129 | -4 | 21 | 45 |
| EX 130 | 1 | 21 | 45 |
| EX 131 | 10 | 7 | 44 |
| EX 132 | -7 | 21 | 45 |
| EX 133 | -5 | 21 | 45 |
| EX 134 | -7 | 21 | 45 |
| EX 135 | -4 | 21 | 45 |
| EX 136 | -9 | 26 | 53 |
| EX 137 | -8 | 26 | 53 |
| EX 138 | -7 | 26 | 53 |
| EX 139 | -4 | 21 | 45 |
| EX 140 | 9 | 7 | 44 |
| EX 141 | 19 | 8 | 39 |
| EX 142 | 8 | 8 | 39 |
| EX 143 | -5 | 26 | 53 |
| EX 144 | 18 | 21 | 45 |
| EX 145 | -9 | 7 | 44 |
| EX 146 | 1 | 9 | 49 |
| EX 147 | -6 | 9 | 49 |
| EX 148 | -5 | 15 | 44 |
| EX 149 | -3 | 15 | 44 |
| EX 150 | 10 | 16 | 58 |
| EX 151 | -6 | 9 | 49 |
| EX 152 | -10 | 16 | 58 |
| EX 153 | -7 | 16 | 52 |
| EX 154 | 4 | 16 | 52 |
| EX 155 | -3 | 21 | 45 |
| EX 156 | -9 | 7 | 44 |
| EX 157 | -4 | 8 | 44 |
| EX 158 | -3 | 9 | 39 |

(continued)

| Evaluation compound | Evaluation compound results | Negative control (Risperidone) | Positive control (Reference Example 3 compound) |
|---|---|---|---|
| EX 159 | -1 | 9 | 39 |
| EX 160 | 9 | 9 | 49 |
| REX 4 (Control compound) | 83 | 9 | 44 |

The Example compounds in the above table all showed cytotoxicity comparable to or less than that of the negative control, i.e., Risperidone. Further, the Reference Example 4 compound (REX 4), which was a quaternary ammonium compound, showed stronger cytotoxicity than that of the Reference Example 3 compound (REX 3), which was set as the positive control. These results indicated that the cytotoxicity of the Example compounds was lower than that of the Reference Example 4 compound.

Study for Confirmation of Conversion to Brexpiprazole

The conversion from the evaluation compounds to brexpiprazole in a buffer (i.e., the stability of the evaluation compounds) was evaluated.
The Example 1 compound was dissolved in acetonitrile/water (1:1, v/v) to 10 mmol/L and further diluted with acetonitrile/water (1:1, v/v) to 100 μmol/L for use.
The other Example compounds and the Reference Example 5 compound to Reference Example 7 compound were each dissolved in DMSO to 10 mmol/L and further diluted with acetonitrile to 100 μmol/L for use, except for the following compounds.
The compounds of Examples 52, 95, 96, 117, and 130 (EX 52, EX 95, EX 96, EX 117, and EX 130) were dissolved in acetonitrile/water (8:2, v/v) to 10 mmol/L and further diluted with acetonitrile to 100 μmol/L for use.
The compound of Example 97 (EX 97) was dissolved in acetonitrile/water (8:2, v/v) to 10 mmol/L and further diluted with acetonitrile/water (8:2, v/v) to 100 μmol/L for use.
The compounds of Examples 15 and 116 (EX 15 and EX 116) were dissolved in DMSO to 1 mmol/L and further diluted with acetonitrile to 100 μmol/L for use.
These evaluation compound solutions were prepared at the time of use.
The reaction was conducted by preparing each reaction solution such that the final concentration of each component was as follows. The test was conducted in duplicate.

| Composition | Final concentration |
|---|---|
| Tris-hydrochloric acid buffer (pH 7.5) | 50 mmol/L |
| Magnesium chloride | 5 mmol/L |
| Evaluation compound solution | 1 μmol/L |

For the compounds of Examples 1 to 8, 10 to 19, 26 to 27, 37, 50, 87 to 89, 91, 116, 121, 123, 150, and 151 (EX 1 to EX 8, EX 10 to EX 19, EX 26 to EX 27, EX 37, EX 50, EX 87 to EX 89, EX 91, EX 116, EX 121, EX 123, EX 150, and EX 151), and the compounds of Reference Examples 5 to 7 (REX 5 to REX 7), the reaction solution without the evaluation compound (198 μl) was pre-incubated at 37°C for 3 minutes, and then each of the evaluation compound solutions (2 μl) was added and stirred; thereafter, the reaction was started at 37°C. At 0 minutes or 60 minutes after the start of the reaction, the reaction was stopped by adding and stirring a reaction stop solution (1000 μl) composed of acetonitrile/isopropanol (4:1, v/v) containing internal standard substances. However, the 0-minute reaction sample (198 μl) was prepared by adding the evaluation compound (2 μl) after adding the reaction stop solution (1000 μl). The samples after stopping the reaction were used as the measurement samples. Calibration curve samples with the same composition were prepared (final concentrations of the evaluation compounds and brexpiprazole: 0.01, 0.1, and 1 μmol/L, with the proviso that for the evaluation compounds only, the 0-minute reaction samples were also used as calibration curve samples for 1 μmol/L).
For the compounds of Examples 24 to 25, 28 to 29, 32 to 36, 40 to 49, 51 to 60, 62 to 64, 66 to 77, 80 to 86, 90, 92 to 111, 113, 115, 117 to 120, 122, 124 to 130, 132 to 147, and 152 to 160 (EX 24 to EX 25, EX 28 to EX 29, EX 32 to EX 36, EX 40 to EX 49, EX 51 to EX 60, EX 62 to EX 64, EX 66 to EX 77, EX 80 to EX 86, EX 90, EX 92 to EX 111, EX 113, EX 115, EX 117 to EX 120, EX 122, EX 124 to EX 130, EX 132 to EX 147, and EX 152 to EX 160), the reaction solution without the evaluation compound (148.5 μl) was pre-incubated at 37°C for 3 minutes, and then each of the evaluation compound solutions (1.5 μl) was added and stirred; thereafter, the reaction was started at 37°C. At 0 minutes or 60 minutes after the

start of the reaction, the reaction was stopped by adding and stirring a reaction stop solution (600 μl) composed of acetonitrile/isopropanol (4:1, v/v) containing internal standard substances. However, the 0-minute reaction sample (148.5 μl) was prepared by adding the evaluation compound (1.5 μl) after adding the reaction stop solution (600 μl). The samples after stopping the reaction were used as the measurement samples. Calibration curve samples with the same composition were prepared (final concentrations of the evaluation compounds and brexpiprazole: 0.01, 0.1, and 1 μmol/L, with the proviso that for the evaluation compounds only, the 0-minute reaction samples were also used as calibration curve samples for 1 μmol/L).

For the compounds of Examples 1 to 8, 10 to 19, 26 to 27, 37, 50, 87 to 89, 91, 116, 121, 123, 150, and 151 (EX 1 to EX 8, EX 10 to EX 19, EX 26 to EX 27, EX 37, EX 50, EX 87 to EX 89, EX 91, EX 116, EX 121, EX 123, EX 150, and EX 151), and the compounds of Reference Examples 5 to 7 (REX 5 to REX 7), the obtained measurement samples and calibration curve samples were centrifuged, the supernatants were appropriately diluted with a mixture of acetonitrile/isopropanol (4:1, v/v), and the resulting samples were each injected into a liquid chromatography-tandem mass spectrometer (HPLC: Shimadzu Prominence UFLC system, MS: SCIEX API 4000) to measure the evaluation compound, brexpiprazole, and internal standard substances.

For the compounds of Examples 24 to 25, 28 to 29, 32 to 36, 40 to 49, 51 to 60, 62 to 64, 66 to 77, 80 to 86, 90, 92 to 111, 113, 115, 117 to 120, 122, 124 to 130, 132 to 147, and 152 to 160 (EX 24 to EX 25, EX 28 to EX 29, EX 32 to EX 36, EX 40 to EX 49, EX 51 to EX 60, EX 62 to EX 64, EX 66 to EX 77, EX 80 to EX 86, EX 90, EX 92 to EX 111, EX 113, EX 115, EX 117 to EX 120, EX 122, EX 124 to EX 130, EX 132 to EX 147, and EX 152 to EX 160), the obtained measurement samples and calibration curve samples were centrifuged, and the supernatants were appropriately diluted with ultrapure water and a mixture of acetonitrile/isopropanol (4:1, v/v) so that the proportion of ultrapure water was 20%. The thus-obtained diluted measurement samples were each injected into a liquid chromatography-tandem mass spectrometer (HPLC: Shimadzu Prominence UFLC system, MS: SCIEX Triple Quad 4500) to measure the evaluation compound, brexpiprazole, and internal standard substances.

For ionization, an electrospray ionization method was performed by a positive ion detection method, and a selected reaction monitoring method was performed by setting the precursor ion and product ion. For the internal standard substances, a deuterium of brexpiprazole (Brexpiprazole-$d_8$) etc. were used. The residual amount of the evaluation compound and the amount of brexpiprazole formed in the 0-minute reaction samples and 60-minute reaction samples were calculated.

The difference between the 0-minute reaction sample and the 60-minute reaction sample in terms of the residual amount of the evaluation compound was used to calculate the amount of decrease in the 60-minute reaction sample. Based on the change in the amount of the evaluation compound, the stability of the evaluation compound in the buffer was evaluated.

The conversion to brexpiprazole in the 60-minute reaction sample was evaluated from the amount of brexpiprazole formed in the 60-minute reaction sample. If brexpiprazole was detected in the 0-minute reaction sample, the difference between the 0-minute reaction sample and the 60-minute reaction sample in terms of the amount of brexpiprazole formed was used to calculate the amount of increase in the 60-minute reaction sample. Based on the change in the amount of brexpiprazole, the stability of the evaluation compound in the buffer was evaluated.

The table below shows the study results. The concentration of the evaluation compound decreased (μmol/L) and the concentration of brexpiprazole formed (μmol/L) were determined in duplicate, and the average values were used.

Table 5

| Evaluation compound | Concentration of evaluation compound decreased (μM) | Concentration of brexpiprazole formed (μM) |
|---|---|---|
| EX 1, EX 2 | 0.00 | 0.00 |
| EX 3 | 0.01 | 0.00 |
| EX 4 | 0.02 | 0.00 |
| EX 5 | 0.07 | 0.00 |
| EX 6 | -0.11 | 0.00 |
| EX 7 | -0.06 | 0.00 |
| EX 8 | 0.07 | 0.01 |
| EX 10 | -0.02 | 0.00 |
| EX 11 | 0.03 | 0.00 |
| EX 12 | 0.02 | 0.00 |

(continued)

| Evaluation compound | Concentration of evaluation compound decreased (μM) | Concentration of brexpiprazole formed (μM) |
|---|---|---|
| EX 13 | 0.03 | 0.00 |
| EX 14 | 0.08 | 0.00 |
| EX 15 | 0.00 | 0.00 |
| EX 16 | 0.01 | 0.00 |
| EX 17 | 0.07 | 0.00 |
| EX 18 | -0.01 | 0.00 |
| EX 19 | -0.07 | 0.00 |
| EX 24 | -0.11 | 0.00 |
| EX 25 | -0.01 | 0.00 |
| EX 26 | 0.05 | 0.00 |
| EX 27 | 0.00 | 0.00 |
| EX 28 | -0.07 | -0.01 |
| EX 29 | -0.01 | 0.00 |
| EX 32 | 0.01 | 0.00 |
| EX33 | 0.00 | 0.00 |
| EX 34 | 0.01 | 0.00 |
| EX 35 | -0.02 | 0.00 |
| EX 36 | -0.04 | 0.00 |
| EX 37 | 0.06 | 0.00 |
| EX 40 | 0.06 | 0.00 |
| EX 41 | 0.07 | 0.00 |
| EX 42 | 0.03 | 0.00 |
| EX 43 | 0.12 | 0.00 |
| EX 44 | -0.05 | 0.00 |
| EX 45 | 0.07 | 0.00 |
| EX 46 | 0.04 | 0.00 |
| EX 47 | 0.00 | 0.00 |
| EX 48 | -0.03 | 0.00 |
| EX 49 | 0.05 | 0.00 |
| EX 50 | 0.01 | 0.00 |
| EX 51 | 0.05 | 0.00 |
| EX 52 | -0.04 | 0.00 |
| EX 53 | 0.06 | 0.00 |
| EX 54 | 0.14 | 0.00 |
| EX 55 | 0.07 | 0.00 |
| EX 56 | 0.04 | 0.00 |
| EX 57 | 0.09 | 0.00 |
| EX 58 | 0.04 | 0.00 |
| EX 59 | 0.05 | 0.00 |

(continued)

| Evaluation compound | Concentration of evaluation compound decreased (μM) | Concentration of brexpiprazole formed (μM) |
|---|---|---|
| EX 60 | 0.09 | 0.00 |
| EX 62 | 0.18 | 0.00 |
| EX 63 | 0.02 | 0.00 |
| EX 64 | 0.03 | 0.00 |
| EX 66 | 0.17 | 0.00 |
| EX 67 | 0.09 | 0.00 |
| EX 68 | 0.09 | 0.00 |
| EX 69 | -0.01 | 0.00 |
| EX 70 | 0.00 | 0.00 |
| EX 71 | 0.10 | 0.00 |
| EX 72 | 0.15 | 0.00 |
| EX 73 | 0.13 | 0.00 |
| EX 74 | 0.05 | 0.00 |
| EX 75 | 0.07 | 0.00 |
| EX 76 | 0.00 | 0.00 |
| EX 77 | 0.11 | 0.00 |
| EX 80 | 0.17 | 0.00 |
| EX 81 | -0.03 | 0.00 |
| EX 82 | 0.02 | 0.00 |
| EX 83 | 0.07 | 0.00 |
| EX 84 | 0.01 | 0.00 |
| EX 85 | 0.04 | 0.00 |
| EX 86 | 0.05 | 0.00 |
| EX 87 | 0.00 | 0.00 |
| EX 88 | -0.01 | 0.00 |
| EX 89 | -0.07 | 0.00 |
| EX 90 | -0.03 | 0.00 |
| EX 91 | 0.12 | 0.00 |
| EX 92 | 0.11 | 0.00 |
| EX 93 | -0.01 | 0.00 |
| EX 94 | -0.07 | 0.00 |
| EX 95 | -0.01 | 0.00 |
| EX 96 | 0.03 | 0.00 |
| EX 97 | 0.09 | 0.00 |
| EX 98 | -0.02 | 0.00 |
| EX 99 | 0.00 | 0.00 |
| EX 100 | 0.05 | 0.00 |
| EX 101 | 0.08 | 0.00 |
| EX 102 | 0.07 | 0.00 |

(continued)

| Evaluation compound | Concentration of evaluation compound decreased (μM) | Concentration of brexpiprazole formed (μM) |
|---|---|---|
| EX 103 | -0.01 | 0.00 |
| EX 104 | 0.20 | 0.00 |
| EX 105 | 0.05 | 0.00 |
| EX 106 | 0.07 | 0.00 |
| EX 107 | 0.07 | 0.00 |
| EX 108 | 0.08 | 0.00 |
| EX 109 | -0.02 | 0.00 |
| EX 110 | 0.07 | 0.00 |
| EX 111 | 0.02 | 0.00 |
| EX 113 | 0.16 | 0.00 |
| EX 115 | 0.05 | 0.00 |
| EX 116 | 0.09 | 0.00 |
| EX 117 | 0.05 | 0.00 |
| EX 118 | 0.06 | 0.00 |
| EX 119 | 0.14 | 0.00 |
| EX 120 | 0.05 | 0.00 |
| EX 121 | 0.09 | 0.00 |
| EX 122 | 0.05 | 0.00 |
| EX 123 | -0.11 | 0.00 |
| EX 124 | 0.10 | 0.00 |
| EX 125 | 0.13 | 0.00 |
| EX 126 | 0.16 | 0.00 |
| EX 127 | 0.04 | 0.00 |
| EX 128 | -0.03 | 0.00 |
| EX 129 | 0.09 | 0.00 |
| EX 130 | -0.02 | 0.00 |
| EX 132 | 0.13 | 0.00 |
| EX 133 | 0.14 | 0.00 |
| EX 134 | 0.07 | 0.00 |
| EX 135 | 0.07 | 0.00 |
| EX 136 | 0.00 | 0.00 |
| EX 137 | 0.09 | 0.00 |
| EX 138 | 0.09 | 0.00 |
| EX 139 | 0.12 | 0.00 |
| EX 140 | -0.02 | 0.00 |
| EX 141 | 0.02 | 0.00 |
| EX 142 | -0.07 | 0.00 |
| EX 143 | 0.05 | 0.00 |
| EX 144 | 0.12 | 0.00 |

(continued)

| Evaluation compound | Concentration of evaluation compound decreased (μM) | Concentration of brexpiprazole formed (μM) |
|---|---|---|
| EX 145 | 0.03 | 0.00 |
| EX 146 | 0.11 | 0.00 |
| EX 147 | 0.07 | 0.00 |
| EX 150 | 0.11 | 0.00 |
| EX 151 | -0.01 | 0.00 |
| EX 152 | 0.01 | 0.00 |
| EX 153 | -0.01 | 0.00 |
| EX 154 | 0.02 | 0.00 |
| EX 155 | 0.16 | 0.00 |
| EX 156 | 0.17 | 0.00 |
| EX 157 | 0.08 | 0.00 |
| EX 158 | 0.07 | 0.00 |
| EX 159 | 0.06 | 0.00 |
| EX 160 | -0.01 | 0.00 |
| REX 5 (Control compound) | 0.20 | 0.26 |
| REX 6 (Control compound) | 0.22 | 0.21 |
| REX 7 (Control compound) | 0.27 | 0.24 |

Discussion

Although the Example compounds were the same quaternary ammonium compounds as the control compounds (Reference Example 5, Reference Example 6, and Reference Example 7), the Example compounds were stable in the buffer and did not convert to brexpiprazole. In contrast, the control compounds clearly degraded to brexpiprazole. These results indicated that the Example compounds of this application are stable for drug formulation, showing the possibility that they can be used stably as a prodrug of brexpiprazole.

Preparation of Pharmaceutical Composition of Example 1 Compound - 1

Example A: 1.25 mg/mL Solution Formulation of the Example 1 Compound

The Example 1 compound was dissolved at a concentration of 1.25 mg/mL in phosphate-buffered saline (pH: 7.4) containing 10% dimethyl sulfoxide and 0.09% polysorbate 80.

Example B: 0.5 mg/mL Solution Formulation of the Example 1 Compound

About 80% of the final preparation amount of water for injection was weighed, and D-mannitol and monosodium phosphate monohydrate were added and stirred for dissolution. The Example 1 compound was added thereto and stirred for dispersion. A 1 M sodium hydroxide solution was added gradually to dissolve the Example 1 compound, and the pH was adjusted to 8.0. After adding an insufficient amount of water for injection so as to reach the final preparation amount, the mixture was aseptically filtered through a filter (0.2 μm, PVDF membrane). The table below shows the composition per milliliter.

Table 6

| Composition of Example B (0.5 mg/mL solution formulation of the Example 1 compound) | | |
|---|---|---|
| Component name | Purpose of blending | Amount per milliliter |
| Example 1 Compound* | Main drug | 0.5 mg |

(continued)

| Composition of Example B (0.5 mg/mL solution formulation of the Example 1 compound) | | |
|---|---|---|
| Component name | Purpose of blending | Amount per milliliter |
| D-Mannitol | Isotonic agent | 45.0 mq |
| Monosodium phosphate monohydrate | pH buffer | 1.38 mg |
| Sodium hydroxide | pH adjuster | q.s. to pH 8.0 |
| Water for injection | Solvent | q.s. to 1.0 mL |
| *in terms of anhydride | | |

Example C: 5.0 mg/mL Solution Formulation of the Example 1 Compound

About 80% of the final preparation amount of water for injection was weighed, and D-mannitol and monosodium phosphate monohydrate were added and stirred for dissolution. The Example 1 compound was added thereto and stirred for dispersion. A 1M sodium hydroxide solution was added gradually to dissolve the Example 1 compound, and the pH was adjusted to 8.9. After adding an insufficient amount of water for injection so as to reach the final preparation amount, the mixture was aseptically filtered through a filter (0.2 μm, PVDF membrane). The table below shows the composition per milliliter.

Table 7

| Composition of Example C (5.0 mg/mL solution formulation of the Example 1 compound) | | |
|---|---|---|
| Component name | Purpose of blending | Amount per milliliter |
| Example 1 Compound* | Main drug | 5.0 mq |
| D-Mannitol | Isotonic agent | 45.0 mq |
| Monosodium phosphate monohydrate | pH buffer | 1.38 mq |
| Sodium hydroxide | pH adjuster | q.s. to pH 8.9 |
| Water for injection | Solvent | q.s. to 1.0 mL |
| *in terms of anhydride | | |

Evaluation of Pharmaceutical Composition of Example A

The pharmaceutical composition of Example A (1.25 mg/mL solution formulation of the Example 1 compound) was subcutaneously administered to male rats, and the plasma concentrations of the Example 1 compound and brexpiprazole were measured. The male rats were purchased from Japan SLC, Inc. at 7 weeks of age and used in the experiments after preliminary breeding. The breeding environment was as follows. Food and water intake: unrestricted, number of animals per cage: 3, temperature: 23±2°C, humidity: 60±10%, and lighting time: 7:00-19:00.

Subcutaneous administration was performed to the back of the rats under isoflurane anesthesia using a plastic syringe. The dose was 1.25 mg/kg. About 0.3 mL each of blood was collected from the jugular vein at 5 minutes, 10 minutes, 20 minutes, 30 minutes, 45 minutes, 1 hour, 2 hours, 4 hours, 6 hours, and 8 minutes after administration. The blood was immediately heparinized and ice-cooled. The plasma was separated by centrifugation, and the compound concentration was quantified by LC-MS.

Fig. 1 shows the changes in the plasma drug concentration after subcutaneous administration of the pharmaceutical composition of Example A (1.25 mg/mL solution formulation of the Example 1 compound). Since the Example 1 compound and brexpiprazole were detected in the plasma 5 minutes after administration, it is presumed that the Example 1 compound immediately reached the blood and the whole body from the subcutaneous site, and that brexpiprazole was immediately formed in the blood. The plasma concentration of brexpiprazole reached maximum at one hour after subcutaneous administration. Thus, an immediate pharmacological effect is expected to be obtained. Therefore, the pharmaceutical composition in the form of a solution containing the Example 1 compound is expected to serve as a pharmaceutical composition useful, for example, as a therapeutic agent for alleviating symptoms in patients with agitation in schizophrenia in the acute stage or Alzheimer's dementia, since brexpiprazole is immediately formed in vivo after subcutaneous administration, and the formed brexpiprazole exerts a pharmacological effect.

Preparation of Pharmaceutical Composition of Example 1 Compound - 2

Example D: 150 mg/g aqueous suspension formulation of the Example 1 Compound

Sodium carboxymethyl cellulose (CMC-Na) (1.0% (w/w)), sodium chloride (0.9% (w/w)), and sodium dihydrogen phosphate monohydrate (0.074% (w/w)) were dissolved in water for injection so that the concentrations of these components were as stated. The pH was adjusted to 6.8 with sodium hydroxide, and the resulting aqueous solution was aseptically filtered with a filter (0.2 μm, PES membrane) in a laminar flow cabinet. The Example 1 compound was added to the aqueous solution to the indicated concentration (150 mg/g) to prepare a primary aqueous suspension. An autoclave-sterilized magnetic stirrer and 1 g of autoclave-sterilized zirconia beads with a diameter of 1.0 mm were added to the primary aqueous suspension, and after sealing the container, stirring was performed for 21 hours at 5°C, followed by wet-milling to prepare a secondary aqueous suspension. From the zirconia beads mixture, only the secondary aqueous suspension was collected with a sterile pipette. The particle size distribution of the secondary aqueous suspension was measured with a SALD-3100 laser diffraction particle size analyzer (Shimadzu Corporation). After ultrasonic irradiation for one minute with the built-in device, the average particle diameter was measured by setting the refractive index to 2.00 to 0.20i; the average particle diameter was 1.9 μm.

Evaluation of Pharmaceutical Composition of Example D

The pharmaceutical composition of Example D (150 mg/g aqueous suspension formulation of the Example 1 compound) was subcutaneously administered to male rats, and the plasma concentrations of the Example 1 compound and brexpiprazole were measured.

The male rats were purchased from Japan SLC, Inc. at 7 weeks of age and used in the experiments after preliminary breeding. The breeding environment was as follows. Food and water intake: unrestricted, number of animals per cage: 3, temperature: 23±2°C, humidity: 60±10%, and lighting time: 7:00-19:00.

Subcutaneous administration was performed to the back of the rats under isoflurane anesthesia using a plastic syringe. The dose was adjusted to be 25 mg/kg in terms of brexpiprazole. About 0.5 mL each of blood was collected at 2 hours, 1 day, 3 days, 6 days, 9 days, and 14 days after administration. The blood was immediately heparinized and ice-cooled. The plasma was separated by centrifugation, and the compound concentration was quantified by LC-MS.

Fig. 2 shows the changes in the plasma drug concentration after subcutaneous administration of the pharmaceutical composition of Example D (150 mg/g aqueous suspension formulation of the Example 1 compound) to the male rats.

When the pharmaceutical composition of Example D was subcutaneously administered, the Example 1 compound and brexpiprazole were detected at 2 hours after administration. The plasma concentration of brexpiprazole remained almost constant from 2 hours to 24 hours after administration, and decreased rapidly on day 3 onwards. Therefore, the aqueous suspension injection of the Example 1 compound is expected to exert a pharmacological effect by maintaining brexpiprazole in the plasma for about 1 day after subcutaneous administration. Accordingly, the pharmaceutical composition obtained by suspending the Example 1 compound in water for injection is expected to immediately form brexpiprazole in vivo after subcutaneous administration, maintain the plasma level of brexpiprazole almost constant for one day after administration, and thus exert the pharmacological effect of brexpiprazole. Therefore, this pharmaceutical composition is expected to serve as a pharmaceutical composition that is useful and highly convenient as an injection for subcutaneous administration for daily use, for example, for patients with agitation in schizophrenia, depression, and Alzheimer's dementia who show a tendency to refuse medication.

Preparation of Pharmaceutical Composition of Example 1 Compound - 3

Example E: 159 mg/g aqueous suspension formulation of the zinc salt of the Example 1 compound

Sodium carboxymethyl cellulose (CMC-Na) (1.0% (w/w)), D-mannitol (4.5% (w/w)), and sodium dihydrogen phosphate monohydrate (0.074% (w/w)) were dissolved in water for injection so that the concentrations of these components were as stated. The pH was adjusted to 6.0 with sodium hydroxide, and the resulting aqueous solution was aseptically filtered with a filter (0.2 μm, PES membrane) in a laminar flow cabinet to prepare a suspension medium. The zinc salt of the Example 1 compound (757 mg) and the suspension medium (3873 mg) were weighed and mixed. The average particle diameter of the suspended particles thus obtained was 3.9 μm. The average particle diameter was measured with a SALD-3100 laser diffraction particle size analyzer (Shimadzu Corporation) by setting the refractive index to 2.00 to 0.20i after ultrasonic irradiation for one minute with the built-in device.

Evaluation of Pharmaceutical Composition of Example E

The pharmaceutical composition of Example E (159 mg/g aqueous suspension formulation of the zinc salt of the Example 1 compound) was subcutaneously administered to male rats, and the plasma concentrations of the Example 1 compound and brexpiprazole were measured.

The male rats were purchased from Japan SLC, Inc. at 7 weeks of age and used in the experiments after preliminary breeding. The breeding environment was as follows. Food and water intake: unrestricted, number of animals per cage: 3, temperature: 23±2°C, humidity: 60±10%, and lighting time: 7:00-19:00.

Subcutaneous administration was performed to the back of the rats under isoflurane anesthesia using a glass syringe. The dose was adjusted to be 25 mg/kg in terms of brexpiprazole. About 0.5 mL each of blood was collected at 2 hours, 1 day, 3 days, 6 days, 9 days, 14 days, 21 days, and 28 days after administration. The blood was immediately heparinized and ice-cooled. The plasma was separated by centrifugation, and the compound concentration was quantified by LC-MS. Fig. 3 shows the changes in the plasma drug concentration after subcutaneous administration of the pharmaceutical composition of Example E (159 mg/g aqueous suspension formulation of the zinc salt of the Example 1 compound) to the male rats.

When the pharmaceutical composition of Example E was subcutaneously administered, the Example 1 compound and brexpiprazole were detected 2 hours after administration, and the plasma drug concentration reached maximum at 24 hours. Compared with Example D, the plasma drug concentration was suppressed at 2 hours and 24 hours, confirming the sustained-release effect of the zinc salt. The plasma drug concentration was maintained at a constant level from day 3 onward until before day 14. Therefore, the injection of the aqueous suspension of the zinc salt of the Example 1 compound is expected to exert a pharmacological effect by maintaining brexpiprazole in the plasma for 1 to 14 days after subcutaneous administration. Accordingly, the pharmaceutical composition obtained by suspending the zinc salt of the Example 1 compound in water for injection is expected to immediately form brexpiprazole in vivo after subcutaneous administration, maintain brexpiprazole in the plasma for several days or more after administration, and exert the pharmacological effect of brexpiprazole. This pharmaceutical composition is thus expected to serve as a pharmaceutical composition that is useful and highly convenient as an injection for subcutaneous administration for daily use and for weekly use for patients with agitation in schizophrenia, depression, and Alzheimer's dementia who show a tendency to refuse medication.

Preparation of Pharmaceutical Composition of Example 1 Compound - 4

Example F: 150 mg/g liquid crystal/lipid gel formulation of the Example 1 compound

Soybean lecithin (soybean phosphatidylcholine (SPC) (LIPOID S 100, Lipoid) and glycerol dioleate (GDO) (Isomeric Mix 1,2/1,3-GDO, Merck) were added to ethanol, and the mixture was heated at 40 to 50°C for dissolution. The resulting solution was filtered through a 0.2 μm disc filter, the Example 1 compound was added to the filtrate, and the mixture was mixed by vortexing for homogenization. The table below shows the composition of the resulting formulation.

Table 8

| Composition of Example F (150 mg/g liquid crystal/lipid gel formulation of the Example 1 compound) | | |
|---|---|---|
| Component name | Purpose of blending | Amount (w/w%) |
| Example 1 Compound | Main drug | 17.5* |
| SPC | Sustained-release base | 29.0 |
| GDO | Sustained-release base | 43.5 |
| Ethanol | Solvent | 10.0 |
| *The amount of the Example 1 compound (anhydrous) was 15.0 w/w% with a water content etc. of 2.5 w/w%. The amount in terms of brexpiprazole was 12.0 w/w%. | | |

Example G: 159 mg/g liquid crystal/lipid gel formulation of the zinc salt of the Example 1 compound

Soybean lecithin (soybean phosphatidylcholine (SPC) (LIPOID S 100, Lipoid) and glycerol dioleate (GDO) (Isomeric Mix 1,2/1,3-GDO, Merck) were added to a mixture of DMSO and ethanol and heated at 40 to 50°C for dissolution. The resulting solution was filtered through a 0.2 μm disc filter, the zinc salt of the Example 1 compound was added to the filtrate, and the mixture was mixed by vortexing for homogenization. The table below shows the composition of the

resulting formulation.

Table 9

| Composition of Example G (159 mg/g liquid crystal/lipid gel formulation of the zinc salt of the Example 1 compound) | | |
|---|---|---|
| Component name | Purpose of blending | Amount (w/w%) |
| Zinc salt of Example 1 compound | Main drug | 16.4* |
| SPC | Sustained-release base | 16.0 |
| GDO | Sustained-release base | 24.0 |
| Ethanol | Solvent | 10.0 |
| DMSO | Solvent | 33.6 |
| *The amount of the zinc salt of the Example 1 compound (anhydrous) was 15.9 w/w% with a water content etc. of 0.5 w/w%. The amount in terms of brexpiprazole was 12.0 w/w%. | | |

Evaluation of Pharmaceutical Compositions of Examples F and G Viscosity Measurement

The viscosity of the formulation F at a shear rate of 9000 to 10000 (1/s) was 402 mPa·s, as measured with a rheometer (discovery hybrid rheometer (DHR)-2, manufacturer: TA Instruments). The conditions for the viscosity measurement were as follows.

- Shear rate in the measurement range: 0.1 → 10000 (1/s)
- Measurement temperature: 20°C
- A 20 mm flat plate was used
- Gap: 50 μm (40 mm flat plate)

Changes in Plasma Drug Concentration

The pharmaceutical compositions (formulations) of Examples F and G were subcutaneously administered to male rats, and the plasma concentrations of the Example 1 compound and brexpiprazole were measured.

The male rats were purchased from Japan SLC, Inc. at 7 weeks of age and used in the experiments after preliminary breeding. The breeding environment was as follows. Food and water intake: unrestricted, number of animals per cage: 3, temperature: 23±2°C, humidity: 60±10%, and lighting time: 7:00-19:00.

Subcutaneous administration was performed to the back of the rats under isoflurane anesthesia using a glass syringe. The dose was adjusted to be 25 mg/kg in terms of brexpiprazole. About 0.5 mL each of blood was collected at 2 hours, 1 day, 3 days, 6 days, 9 days, 14 days, 21 days, and 28 days after administration. The blood was immediately heparinized and ice-cooled. The plasma was separated by centrifugation, and the compound concentration was quantified by LC-MS. Figs. 4 and 5 respectively show the changes in the plasma drug concentration after subcutaneous administration of the pharmaceutical compositions of Examples F and G to the male rats.

Example F maintained a relatively high plasma concentration of brexpiprazole in the initial stage after administration and also showed a relatively high plasma concentration of brexpiprazole even at 14 days after administration onwards; the plasma concentration of brexpiprazole was maintained for 14 days or more. Compared with the aqueous suspension of Example D, the plasma drug concentration was even more suppressed, confirming the sustained-release effect of the liquid crystal/lipid gel formulation.

Example G maintained a relatively high plasma concentration of brexpiprazole in the initial stage after administration and also showed a relatively high plasma concentration of brexpiprazole even at 21 days after administration onwards; the plasma concentration of brexpiprazole was maintained for 21 days or more. Compared with the aqueous suspension of the zinc salt of Example E, the plasma drug concentration was even more suppressed, confirming the sustained-release effect of the liquid crystal/lipid gel formulation. These results suggest that the pharmaceutical composition that forms the liquid crystal/lipid gel of the Example 1 compound or a salt thereof can control the release of the Example 1 compound from the formulation by combining an appropriate sustained-release base with a metal salt, and can maintain the brexpiprazole exposure to the blood at a level sufficient to exert a therapeutic effect for 14 to 21 days or more by converting the Example 1 compound to brexpiprazole in vivo. Since such a pharmaceutical composition maintains a relatively high blood concentration of brexpiprazole in the early stage after administration, the blood drug concentration can be maintained without the need for a combined use of oral agents or without the need for shorter

injection intervals to compensate for the blood drug concentration at the start of treatment with a sustained-release injection. Such a pharmaceutical composition is thus expected to serve as a pharmaceutical composition useful, for example, for preventing recurrence of schizophrenia etc.

Preparation of Pharmaceutical Composition of Example 1 Compound - 5

Examples H, I, J, K, L, M, N, and O: 300-400 mg/g microspheres of the Example 1 compound

According to the compositions shown in the following tables, the Example 1 compound and a poly(lactide-co-glycolide) copolymer (lactic acid-glycolic acid copolymer (PLGA)) as a biodegradable polymer, such as Resomer RG 505, Resomer RG 504, Resomer RG 503, Resomer RG 502, Resomer RG 752 H, or Lactel 85:15 poly(DL-lactide-co-glycolide), ester terminated (PLGE), inherent viscosity range (IV): 0.76-0.85 dL/g (all of the Resomer products and Lactel products being from Evonik), were dissolved in dichloromethane (FUJIFILM Wako Pure Chemical Corporation) and acetic acid (FUJIFILM Wako Pure Chemical Corporation) to prepare an oil phase. Polyvinyl alcohol 40-88 (Merck) was dissolved in purified water to 0.5% to prepare an aqueous phase (continuous phase). The oil phase was added dropwise while homogenizing the aqueous phase at a rotational speed of 2000 to 3000 rpm with a T.K. Robomix (PRIMIX Corporation) equipped with a homomixer. The aqueous phase was then dried in liquid for 5 hours while stirring at 200 to 500 rpm with a stirrer to prepare microspheres, and the microspheres after drying in liquid were observed under a polarized light microscope. After drying in liquid, a washing method was additionally performed as necessary by adding 99.5% ethanol or an alkali, such as a 1N sodium hydroxide solution. After drying in liquid, the microspheres were filtered out by aspiration with a filtering apparatus in which a 20 μm nylon mesh filter is sandwiched to remove the aqueous phase. Purified water in an amount of about 1/5 of the amount of the aqueous phase used was poured on the microspheres filtered out with the filtering apparatus, and the PVA was thoroughly washed away to collect the microspheres. The microspheres were collected in Falcon tubes, placed in a freezer at -20°C or lower to freeze, and then freeze-dried using a LyoStar 3 lyophilizer (SP Scientific) to collect a microsphere powder.

The molar ratio of lactic acid to glycolic acid, characteristics, weight average molecular weight, and inherent viscosity range (dL/g) of each PLGA used were as follows.

Resomer RG 505: lactide:glycolide 50:50, ester terminated, Mw 54,000-69,000
Resomer RG 504: lactide:glycolide 50:50, ester terminated, Mw 38,000-54,000, 0.45-0.60
Resomer RG 503: lactide:glycolide 50:50, ester terminated, Mw 24,000-38,000, 0.32-0.44
Resomer RG 502: lactide:glycolide 50:50, ester terminated, Mw 7,000-17,000, 0.16-0.24
Resomer RG 752 H: lactide:glycolide 75:25, acid terminated, Mw 4,000-15,000, 0.14-0.22
Lactel 85:15 PLGE IV 0.76-0.85: lactide:glycolide 85:15, ester terminate, unknown, 0.76-0.85

Table 10

Composition of Examples H to O (microspheres of the Example 1 compound) and rotational speed during homogenization

| Composition | Example H | Example I | Example J | Example K | Example L |
|---|---|---|---|---|---|
| Example 1 compound* | 2.0 g | 1.0 g | 1.0 g | 1.0 g | 1.0 g |
| Resomer RG 505 | 3.0 g | 2.0 g | - | - | - |
| Resomer RG 504 | - | - | 2.0 g | - | - |
| Resomer RG 503 | - | - | - | 1.5 g | 2.0 g |
| Resomer RG 502 | - | - | - | - | - |
| Dichloromethane | 20 mL | 14 mL | 14 mL | 10 mL | 14 mL |
| Acetic acid | 3 mL | 2 mL | 2 mL | 1.5 mL | 2 mL |
| 0.5% PVA solution | 3 L | 1.6 L | 1.6 L | 1.6 L | 1.6 L |
| Rotational speed (rpm) | 2700 | 2000 | 2000 | 2000 | 2000 |

| Composition | Example M | Example N | Example O |
|---|---|---|---|
| Example 1 compound free form* | 1.0 g | 1.0 g | 1.0 g |

| | | | |
|---|---|---|---|
| Resomer RG 502 | 1.5 g | - | - |
| Lactel 85:15 PLGE IV 0.76-0.85 | - | 2.0 g | - |
| Resomer RG 752 H | - | - | 1.5 g |
| Dichloromethane | 6 mL | 14 mL | 10 mL |
| Acetic acid | 1 mL | 2 mL | 1.5 mL |
| 0.5% PVA solution | 1.5 L | 1.6 L | 1.6 L |
| Rotational speed (rpm) | 2000 | 2000 | 2000 |

*Amounts that were a 5% increase from the indicated amounts were used (For example, 2.0 g in Example H was actually 2.0 × 1.05 = 2.1 g.)

Evaluation of Pharmaceutical Compositions of Examples H to O Polarized Light Microscopy Observation

The microspheres of Examples I, J, and K after drying in liquid were observed under a polarized light microscope. Figs. 6, 7, and 8 show the results. The results all confirmed the formation of microspheres.

Measurement of Average Particle Diameter

The obtained microspheres were suspended in a 0.5% carmellose sodium solution, and the particle diameter was measured by a laser diffraction scattering method using a SALD-3100 laser diffraction particle size analyzer (Shimadzu Corporation). The measurement was performed using purified water as the solvent, and using a batch cell or circulation cell. For the batch cell, the stirring speed was set to maximum, and for the circulation cell, the circulation speed was set to 5. The formulation was added dropwise to purified water so that the concentration was optimal, and the refractive index was set to 2.00 to 0.20i to measure the average particle diameter. The table below shows the results. The average particle diameters were 50 to 85 μm, and the average particle diameters in the Production Examples were suitable for maintaining the blood concentration for one month or more.

Table 11

| Average particle diameter of Examples H to O (microspheres of the Example 1 compound) | | | | | |
|---|---|---|---|---|---|
| | Example H | Example I | Example J | Example K | Example L |
| Average particle diameter (μm) | 63.0 | 52.3 | 52.7 | 77.9 | 60.9 |
| | Example M | Example N | Example O | | |
| Average particle diameter (μm) | 59.1 | 82.2 | 64.9 | | |

Percentage of Drug Inclusion

The obtained microspheres (20 mg) were dissolved in 10 mL of a dimethyl sulfoxide/acetic acid (4/1) solution, and the percentage of drug inclusion in the microspheres was determined by HPLC using a test solution in which 1 mL of the resulting solution was diluted 10-fold with acetonitrile. The percentage of drug inclusion was determined by HPLC for each Production Example. The table below shows the results. As shown in the table below, the percentages of inclusion of Examples H to O were 80% or more.

Table 12

| Percentage of drug inclusion (%) of Examples H to O (microspheres of the Example 1 compound) | | | | | |
|---|---|---|---|---|---|
| | Example H | Example I | Example J | Example K | Example L |
| Percentage of inclusion (%) | 91.7[*1] | 89.2[*2] | 90.7[*2] | 90.0[*1] | 89.7[*2] |
| | Example M | Example N | Example O | | |
| Percentage of inclusion (%) | 84.9[*1] | 87.6[*2] | 91.7[*1] | | |

*1:

$$\text{Percentage of drug inclusion (\%)} = \frac{\text{Concentration of the Example 1 compound in microspheres (mg/g)}}{\text{Theoretical concentration of the Example 1 compound in microspheres (400 mg/g)}} \times 100$$

*2:

$$\text{Percentage of drug inclusion (\%)} = \frac{\text{Concentration of the Example 1 compound in microspheres (mg/g)}}{\text{Theoretical concentration of the Example 1 compound in microspheres (333 mg/g)}} \times 100$$

Since the microspheres were prepared through the drying step, the denominator (theoretical concentration of the Example 1 compound in microspheres) in the above formulas for the percentage of drug inclusion (%) substantially represents the concentration of the Example 1 compound with respect to the total amount of the Example 1 compound and PLGA used.

Changes in the Plasma Drug Concentration

For administration to animals, MCT suspensions of Examples H, J, K, and L were prepared by adding medium-chain fatty acid triglyceride (MCT, obtained from Croda and used as a solvent) to the microsphere powders of Examples H, J, K, and L to a concentration of 300 mg/mL. These suspensions were subcutaneously administered to male rats, and the plasma concentrations of the Example 1 compound and brexpiprazole were measured.

The male rats were purchased from Japan SLC, Inc. at 7 weeks of age and used in the experiments after preliminary breeding. The breeding environment was as follows. Food and water intake: unrestricted, number of animals per cage: 2 to 3, temperature: 23±2°C, humidity: 60±10%, and lighting time: 7:00-19:00.

Subcutaneous administration was performed to the back of the rats under isoflurane anesthesia using a glass syringe. For all of the formulations, the dose was adjusted to be 25 mg/kg in terms of brexpiprazole. After administration, 0.5 mL each of blood was collected from the jugular vein over time. For Example H, the blood was collected at 2 hours, 1 day, 3 days, 6 days, 9 days, 14 days, 21 days, and 28 days after administration. For Examples J, K, and L, the blood was collected at 2 hours, 1 day, 3 days, 7 days, 10 days, 14 days, 21 days, 28 days, 35 days, 42 days, and 56 days after administration. The collected blood was immediately heparinized and ice-cooled. The plasma was separated by centrifugation, and the concentrations of the Example compounds and brexpiprazole were determined by LC-MS.

Figs. 9, 10, 11, and 12 show the changes in the plasma drug concentration after subcutaneous administration of the pharmaceutical compositions of Examples H, J, K, and L to the male rats. All of the cases confirmed an increase in the blood concentration immediately after administration and also confirmed that the blood concentration of brexpiprazole was maintained for one month or more. These results suggest that the pharmaceutical compositions of the microspheres of the Example 1 compound can control the release of the Example 1 compound from the formulation by preparing the microspheres containing the Example 1 compound using an appropriate biodegradable polymer, and can maintain the brexpiprazole exposure to the blood at a level sufficient to exert a therapeutic effect for one month or more by converting the Example 1 compound to brexpiprazole in vivo. Since such pharmaceutical compositions maintain a relatively high blood concentration of brexpiprazole in the early stage after administration, the blood drug concentration can be maintained without the need for a combined use of oral agents or without the need for shorter injection intervals to compensate for the blood drug concentration at the start of treatment with a sustained-release injection. Such pharmaceutical compositions are thus expected to serve as a pharmaceutical composition useful, for example, for preventing recurrence of schizophrenia etc.

Preparation of Pharmaceutical Composition of Example 3 Compound Example P

Sodium carboxymethyl cellulose (CMC-Na) (1.23 % (w/v)), sucrose (5.75 % (w/v)), sodium dihydrogen phosphate monohydrate (0.12 % (w/v)), anhydrous citric acid (0.16 % (w/v)) were dissolved in water for injection so that the concentrations of these components were as stated. The pH was adjusted to 5.0 by adding an appropriate amount

of sodium hydroxide to prepare a suspension medium. The Example 3 compound (712 mg) was added to the suspension medium (3288 mg), and the mixture was mixed to prepare a primary suspension. Zirconia beads with a diameter of 2 mm (4 g) were added to the primary suspension, and the particles of the Example 3 compound were milled by stirring with a stirrer (milling time: 8 hours). The zirconia beads were removed with a sterile pipette to prepare an aqueous suspension formulation of the Example 3 compound.

Preparation of Pharmaceutical Composition of Example 10 Compound Example Q

Sodium carboxymethyl cellulose (CMC-Na) (0.796 % (w/v)), sodium chloride (0.716 % (w/v)), and sodium dihydrogen phosphate monohydrate (0.055 % (w/v)) were dissolved in water for injection so that the concentrations of these components were as stated. The pH was adjusted to 6.0 by adding an appropriate amount of sodium hydroxide to prepare a suspension medium. The Example 10 compound (816 mg) was added to the suspension medium (3184 mg), and the mixture was mixed and stirred to prepare a primary suspension. Zirconia beads with a diameter of 2 mm (4 g) were added to the primary suspension, and the particles of the Example 10 compound were milled by stirring with a stirrer (milling time: 80 minutes). The zirconia beads were removed with a sterile pipette to prepare an aqueous suspension formulation of the Example 10 compound.

Preparation of Pharmaceutical Composition of Example 12 Compound Example R

Sodium carboxymethyl cellulose (CMC-Na) (0.813 % (w/v)), sodium chloride (0.732 % (w/v)), and sodium dihydrogen phosphate monohydrate (0.056 % (w/v)) were dissolved in water for injection so that the concentrations of these components were as stated. The pH was adjusted to 6.0 by adding an appropriate amount of sodium hydroxide to prepare a suspension medium. The Example 12 compound (748 mg) was added to the suspension medium (3252 mg), and the mixture was mixed and stirred to prepare a primary suspension. Zirconia beads with a diameter of 2 mm (4 g) were added to the primary suspension, and the particles of the Example 12 compound were milled by stirring with a stirrer (milling time: 2 hours). The zirconia beads were removed with a sterile pipette to prepare an aqueous suspension formulation of the Example 12 compound.

Preparation of Pharmaceutical Composition of Example 13 Compound Example S

Sodium carboxymethyl cellulose (CMC-Na) (0.805 % (w/v)), sodium chloride (0.725 % (w/v)), and sodium dihydrogen phosphate monohydrate (0.056 % (w/v)) were dissolved in water for injection so that the concentrations of these components were as stated. The pH was adjusted to 6.0 by adding an appropriate amount of sodium hydroxide to prepare a suspension medium. The Example 13 compound (780 mg) was added to the suspension medium (3220 mg), and the mixture was mixed and stirred to prepare a primary suspension. Zirconia beads with a diameter of 2 mm (4 g) were added to the primary suspension, and the particles of the Example 13 compound were milled by stirring with a stirrer (milling time: 3 hours). The zirconia beads were removed with a sterile pipette to prepare an aqueous suspension formulation of the Example 13 compound.

Preparation of Pharmaceutical Composition of Example 44 Compound Example T

Sodium carboxymethyl cellulose (CMC-Na) (0.791 % (w/v)), sodium chloride (0.712 % (w/v)), and sodium dihydrogen phosphate monohydrate (0.055 % (w/v)) were dissolved in water for injection so that the concentrations of these components were as stated. The pH was adjusted to 6.0 by adding an appropriate amount of sodium hydroxide to prepare a suspension medium. The Example 44 compound (836 mg) was added to the suspension medium (3164 mg), and the mixture was mixed and stirred to prepare a primary suspension. Zirconia beads with a diameter of 2 mm (4 g) were added to the primary suspension, and the particles of the Example 44 compound were milled by stirring with a stirrer (milling time: 4 hours). The zirconia beads were removed with a sterile pipette to prepare an aqueous suspension formulation of the Example 44 compound.

Preparation of Pharmaceutical Composition of Example 92 Compound Example U

Sodium carboxymethyl cellulose (CMC-Na) (0.803 % (w/v)), sodium chloride (0.723 % (w/v)), and sodium dihydrogen phosphate monohydrate (0.055 % (w/v)) were dissolved in water for injection so that the concentrations of these components were as stated. The pH was adjusted to 6.0 by adding an appropriate amount of sodium hydroxide to prepare a suspension medium. The Example 92 compound (788 mg) was added to the suspension medium (3152 mg), and the mixture was mixed and stirred to prepare a primary suspension. Zirconia beads with a diameter of 2 mm (4 g) were added to the primary suspension, and the particles of the Example 92 compound were milled by stirring with a stirrer

(milling time: 24 hours). The zirconia beads were removed with a sterile pipette to prepare an aqueous suspension formulation of the Example 92 compound.

Preparation of Pharmaceutical Composition of Example 121 Compound Example V

Hydroxypropyl cellulose (HPC) (4.02 % w/v), glycerin (2.01 % w/v), and histidine (0.06 % w/v) were dissolved in water for injection so that the concentrations of these components were as stated. The pH was adjusted to 7.0 by adding an appropriate amount of hydrochloric acid to prepare a suspension medium. The Example 121 compound (784 mg) was added to the suspension medium (3216 mg), and the mixture was mixed and stirred to prepare a primary suspension. Zirconia beads with a diameter of 0.5 mm (4 g) were added to the primary suspension, and the particles of the Example 121 compound were milled by stirring with a stirrer (milling time: 24 hours). The zirconia beads were removed with a sterile pipette to prepare an aqueous suspension formulation of the Example 121 compound.

Evaluation of Pharmaceutical Composition of Examples P to V Measurement of Average Particle Diameter

The particle diameter of the obtained aqueous suspension formulations was measured by a laser diffraction scattering method by using a SALD-3100 laser diffraction particle size analyzer (Shimadzu Corporation) and by setting the refractive index to 2.00 to 0.20i. The table below shows the results.

Table 13

| Average particle diameter of Examples P to V | | | | | | | |
|---|---|---|---|---|---|---|---|
| | Example P | Example Q | Example R | Example S | Example T | Example U | Example V |
| Average particle diameter (μm) | 3.8 | 4.5 | 4.6 | 4.2 | 4.4 | 3.4 | 1.1 |

Changes in the Plasma Drug Concentration

The aqueous suspensions of Examples P to V were subcutaneously administered to male rats, and the plasma concentrations of each Example compound and brexpiprazole were measured.

The male rats were purchased from Japan SLC, Inc. at 7 weeks of age and used in the experiments after preliminary breeding. The breeding environment was as follows. Food and water intake: unrestricted, number of animals per cage: 3, temperature: 23±2°C, humidity: 60±10%, and lighting time: 7:00-19:00.

Subcutaneous administration was performed to the back of the rats under isoflurane anesthesia using a plastic syringe or glass syringe. For all of the formulations, the dose was adjusted to be 25 mg/kg in terms of brexpiprazole. After administration, 0.5 mL each of blood was collected from the jugular vein over time. The blood was collected at 2 hours, 1 day, 3 days, 6 days, 9 days, 14 days, 21 days, and 28 days after administration. The collected blood was immediately heparinized and ice-cooled. To suppress hydrolysis in the samples, 1% phosphatase inhibitor cocktail (Nacalai Tesque, Inc.) and 10 mM 2-thenoyltrifluoroacetone (TTFA) was added to the blood samples of the animals dosed with Examples P - U and Example V, respectively. The plasma was separated by centrifugation, and the concentrations of each Example compound and brexpiprazole were determined by LC-MS. Figs. 13, 14, 15, 16, 17, 18, and 19 show the changes in the plasma drug concentration after subcutaneous administration of the pharmaceutical compositions of Examples P, Q, R, S, T, U, and V to the male rats, wherein the results are shown as the mean ± standard deviation of 3 to 5 examples. Compared with the pharmaceutical composition of Example D (aqueous suspension formulation of the Example 1 compound), all cases confirmed that the concentration of brexpiprazole was maintained in an even more significant manner, and that the blood concentration of brexpiprazole maintained for 2 weeks or more to 4 weeks or more. These results suggest that an appropriate structural conversion of the Example 1 compound enables control of the release of the Example compound from the formulation, and that conversion of the Example compound to brexpiprazole in vivo makes it possible to maintain the brexpiprazole exposure to the blood at a level sufficient to exert a therapeutic effect for two weeks or more to four weeks or more. Thus, these products are expected to serve as a pharmaceutical composition useful, for example, for preventing recurrence of schizophrenia etc.

Dog irritation study

Method

Male beagle dogs were purchased at the age of 5-7 months from Kitayama Labes Co., Ltd. and used in the

experiment after acclimatization. The breeding environment was as follows: feeding: 250 g of solid feed once a day, water intake: unlimited, number of animals per cage: 1, temperature: 21-25°C, humidity: 50-70%, lighting time: 7:00-19:00. The test formulation was subcutaneously administered to dogs anesthetized with 25 mg/kg thiopental sodium using a plastic syringe at a dose of 10 mg/kg per formulation, changing the position on the back. After administration, dogs were observed for 14 days, then sacrificed by bleeding under anesthesia with 25 mg/kg thiamylal sodium, and tissues of injection sites were collected. The collected tissues were fixed in 10% neutral buffered formalin, and hematoxylin and eosin-stained specimens were prepared for histopathological examination.

Results and Discussion

For some compounds, external observation of the injection site and histopathological examination revealed that the changes in the injection site were physiological responses to foreign bodies and that the irritation was within an acceptable range.

## Claims

1. A compound represented by formula (I) or a salt thereof:

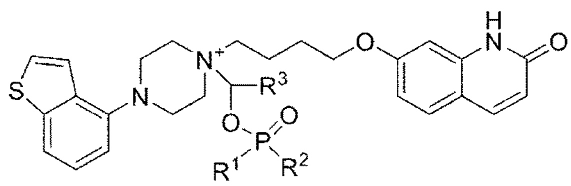

(I)

wherein

$R^1$ and $R^2$ each independently represent $-O^-$, $-OR^4$, or - $NR^{4Na}R^{4Nb}$,
$R^3$ represents hydrogen or alkyl,
$R^4$, $R^{4Na}$, and $R^{4Nb}$ each independently represent hydrogen, or alkyl, alkenyl, alkadienyl, or alkynyl each optionally having 1 to 3 substituents, wherein alkyl represented by $R^4$, $R^{4Na}$, or $R^{4Nb}$ may have a structure in which one or more methylene groups ($-CH_2-$) are replaced with -O-, -S-, -CO-, -NH-, $-SiR^{sia}R^{sib}-$, or -(CO)O-,
$R^{sia}$ and $R^{sib}$ may be identical or different and represent hydrogen or $C_{1-6}$ alkyl,
if $R^1$ and $R^2$ both represent $-OR^4$, $R^1$ and $R^2$ may be identical or different, and
if $R^1$ and $R^2$ both represent $-NR^{4Na}R^{4Nb}$, $R^1$ and $R^2$ may be identical or different.

2. The compound or salt thereof according to claim 1, wherein

$R^1$ represents $-O^-$,
$R^2$ represents $-OR^4$ or $-NR^{4Na}R^{4Nb}$ wherein $R^4$, $R^{4Na}$, and $R^{4Nb}$ are as defined above.

3. A compound represented by formula (I) or a salt thereof:

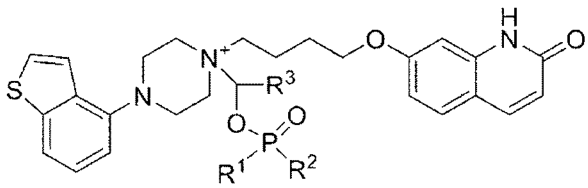

(I)

wherein

$R^1$ represents $-O^-$,
$R^2$ represents $-OR^4$ or $-NR^{4Na}R^{4Nb}$,
$R^3$ represents hydrogen or $C_{1-6}$ alkyl,
$R^4$ represents hydrogen or any of the following (0-1) to (5),

$-NR^{4Na}R^{4Nb}$ represents the following (i) or (ii) :

(0-1): $C_{1-18}$ alkenyl optionally having 1 to 3 substituents selected from halogen, hydroxy, $C_{1-6}$ alkyl, and $C_{1-6}$ alkoxy,
(0-2): $C_{1-18}$ alkadienyl optionally having 1 to 3 substituents selected from halogen, hydroxy, $C_{1-6}$ alkyl, and $C_{1-6}$ alkoxy,
(0-3): $C_{1-18}$ alkynyl optionally having 1 to 3 substituents selected from halogen, hydroxy, $C_{1-6}$ alkyl, and $C_{1-6}$ alkoxy,
(1-1): $R^{4a1}$,

wherein $R^{4a1}$ represents

$-C_nH_{2n+1}$,

$-C_nH_{2n-1}$,

$-C_nH_{2n-3}$,

$-C_nH_{2n}-OH$,

$-C_nH_{2n-2}-OH$,

or

$-C_nH_{2n-4}-OH$,

wherein n represents 1 to 24, with the proviso that in $-C_nH_{2n-1}$, n is 2 or more; in $-C_nH_{2n-3}$, n is 2 or more; in $-C_nH_{2n-2}-$, n is 2 or more; and in $-C_nH_{2n-4}-$, n is 2 or more,

(1-2): $R^{4a2}$,

wherein $R^{4a2}$ represents

$-CHX^1X^2$,

$-C_{n-1}H_{2n-2}-CHX^1X^2$,

$-C_{n-1}H_{2n-4}-CHX^1X^2$,

or

$-C_{n-1}H_{2n-6}-CHX^1X^2$,

wherein
n represents 2 to 24, with the proviso that in $-C_{n-1}H_{2n-4}-$, n represents 3 or more; in $-C_{n-1}H_{2n-6}-$, n represents 3 or more,
$X^1$ and $X^2$ are identical or different and represent hydrogen or halogen (F, Cl, Br, or I), with the proviso that either $X^1$ or $X^2$, or both, represent halogen,

**(1-3) :** $R^{4a3}$,

wherein $R^{4a3}$ represents

$-C_nH_{2n}-R^{4-1}$,

$-C_{n-1}H_{2n-2}-CHR^{4-1a}R^{4-1b}$**,**

$-C_nH_{2n-2}-R^{4-1}$,

or

$-C_nH_{2n-4}-R^{4-1}$,

wherein
n represents 1 to 24, with the proviso that in $-C_nH_{2n-2}-$, n represents 2 or more; in $-C_nH_{2n-4}-$, n represents 2 or more,
$R^{4-1}$ represents $C_{1-6}$ alkoxy, -O-phenyl, or a heterocyclic group optionally substituted with $C_{1-6}$ alkyl or halogen,
$R^{4-1a}$ and $R^{4-1b}$ are identical or different and represent $-C_{1-3}$ alkylene-$C_{1-3}$ alkoxy, or identical or different and represent $-COO-C_{1-3}$ alkyl or $-CH_2-CO-O-C_{1-3}$ alkyl,

(2): $R^{4b}$,

wherein $R^{4b}$ represents

$-(C_pH_{2p}-O)_q-C_rH_{2r}-R^{4-2}$,

$-(C_pH_{2p}-O)_q-C_rH_{2r-2}-R^{4-2}$,

$-(C_pH_{2p-2}-O)q-C_rH_{2r}-R^{4-2}$,

or

$-(C_pH_{2p-2}-O)_q-C_rH_{2r-2}-R^{4-2}$,

wherein p represents 1 to 4, q represents 1 to 4, r represents 1 to 4, with the proviso that in $C_pH_{2p-2}$, p represents 2 or more, in $C_rH_{2r-2}$, r represents 2 or more,
when q is 2 to 4, 2 to 4 repeating structures, the number of repetitions of which is represented by q, may be identical or different,
$R^{4-2}$ represents hydrogen, hydroxy, or cycloalkyl, phenyl, benzyl, or a heterocyclic group each optionally having 1 to 3 substituents selected from $C_{1-6}$ alkyl, $C_{1-6}$ alkoxy, $-C_{1-3}$ alkylene-$C_{1-3}$ alkoxy, and nitro,

(3) : $-C_\alpha H_{2\alpha}-CO-O-R^{COO}$ or $-C_\alpha H_{2\alpha}-CO-O-CH_2-R^{COO}$ wherein $\alpha$ represents 1 to 10,
wherein $-R^{COO}$ represents hydrogen, $C_{1-6}$ alkyl or $C_{1-6}$ alkoxy each optionally substituted with 1 to 3 $C_{1-6}$ alkoxy groups, or cycloalkyl, phenyl, benzyl, or a heterocyclic group each optionally having 1 to 3 substituents selected from $C_{1-6}$ alkyl, $C_{1-6}$ alkoxy, $-C_{1-3}$ alkylene-$C_{1-3}$ alkoxy, and nitro,
(4): $-C_\beta H_{2\beta}-O-CO-R^{OCO}$ wherein $\beta$ represents 1 to 10,

wherein $-R^{OCO}$ represents $R^{4a1}$, $R^{4b}$, or $-C_\alpha H_{2\alpha}-CO-O-R^{COO}$, or $-C_\alpha H_{2\alpha}-CO-NH-R^{COO}$, $-C_\alpha H_{2\alpha}-NH-CO-R^{COO}$, or $-C_\alpha H_{2\alpha}-NH-CO-O-R^{COO}$ wherein - $R^{COO}$ is as defined above, or
cycloalkyl, phenyl, benzyl, or a heterocyclic group each optionally having 1 to 3 substituents selected from $C_{1-6}$ alkyl, $C_{1-6}$ alkoxy, $-C_{1-3}$ alkylene-$C_{1-3}$ alkoxy, and nitro,

(5): $-C_\gamma H_{2\gamma}-O-CO-O-R^{OCOO}$ wherein $\gamma$ represents 1 to 10,

wherein $-R^{OCOO}$ represents hydrogen, $R^{4a1}$, $R^{4b}$, or $-C_\alpha H_{2\alpha}-CO-O-R^{COO}$ wherein $-R^{COO}$ is as defined above, or
cycloalkyl, phenyl, benzyl, or a heterocyclic group each optionally having 1 to 3 substituents selected from $C_{1-6}$ alkyl, $C_{1-6}$ alkoxy, $-C_{1-3}$ alkylene-$C_{1-3}$ alkoxy, and nitro,

(i) $R^{4Na}$ and $R^{4Nb}$ are identical or different and represent $C_{1-6}$ alkyl optionally substituted with 1 to 3 $C_{1-6}$ alkoxy groups,
(ii) $R^{4Na}$ represents hydrogen, and $R^{4Nb}$ represents $C_{1-6}$ alkyl optionally substituted with 1 to 3 $C_{1-6}$ alkoxy groups, or represents $-C_\alpha H_{2\alpha}-CO-O-R^{COO}$ or $-C_\alpha H_{2\alpha}-CO-NH-R^{COO}$

wherein $-R^{COO}$ is as defined above.

4. The compound or salt thereof according to any one of claims 1 to 3, wherein the heterocyclic group has a structure in which a hydrogen atom bound to a constituent atom of a heterocycle is removed from the heterocycle, the heterocycle being selected from the group consisting of furan, tetrahydropyran, tetrahydrofuran, 1,3-dioxane, 1,4-dioxane, pyrrole, imidazole, pyrazole, pyridine, pyrimidine, pyrazine, pyridazine, pyrrolidine, imidazolidine, thiophene, piperidine, piperazine, oxazole, isoxazole, oxadiazole, morpholine, indole, indazole, benzimidazole, and quinoline.

5. The compound or salt thereof according to claim 1 or 2, wherein -$OR^4$ represents any of the groups listed in the following table when asterisk "*" represents the side opposite to $R^4$ relative to the oxygen atom (*-$OR^4$):

| |
|---|
| *-OH |
| 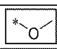 |
| 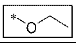 |
| 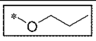 |
| 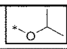 |
| 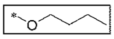 |
| 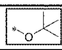 |
| 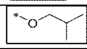 |
| 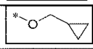 |
| 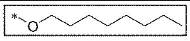 |
| 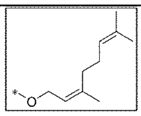 |
| 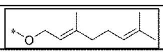 |
| 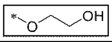 |
| 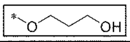 |
| 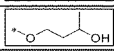 |
| 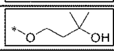 |
| 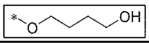 |
| 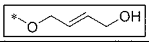 |
| 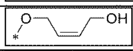 |
| 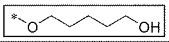 |
| 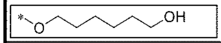 |
| 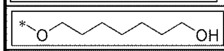 |
| 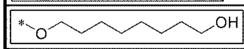 |
| 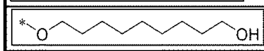 |
| 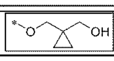 |

| |
|---|
| 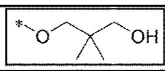 |
| 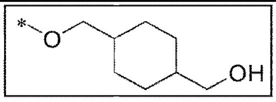 |
| 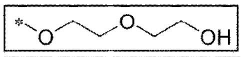 |
| 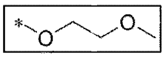 |
| 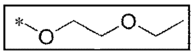 |
| 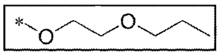 |
| 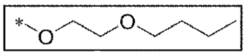 |
| 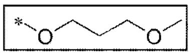 |
| 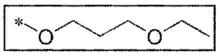 |
| 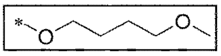 |
| 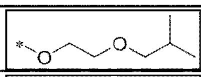 |
| 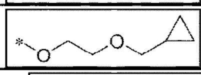 |
| 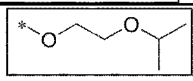 |
| 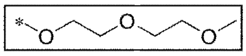 |
| 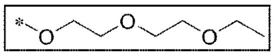 |
| 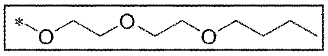 |
| 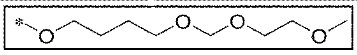 |
| 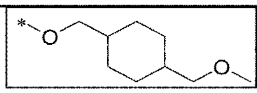 |
| 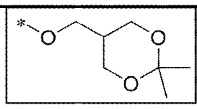 |
| 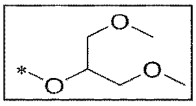 |
| 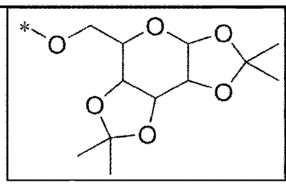 |

| |
|---|
| 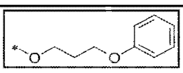 |
| 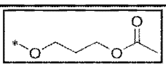 |
| 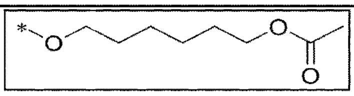 |
| 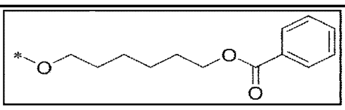 |
| 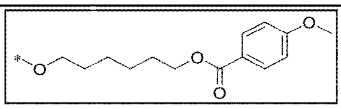 |
| 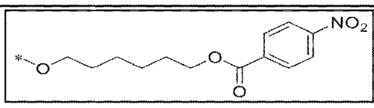 |
| 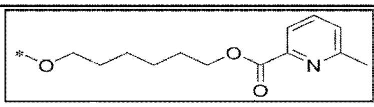 |
| 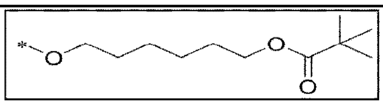 |
| 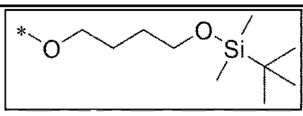 |
| 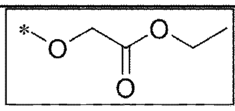 |
| 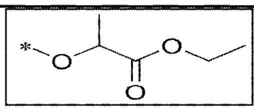 |
| 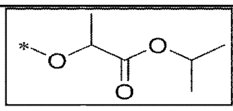 |
| 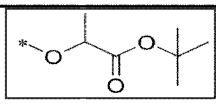 |
| 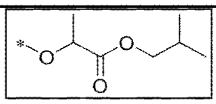 |

| |
|---|
| 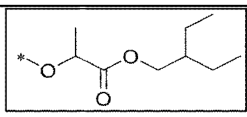 |
| 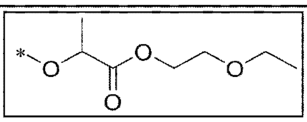 |
| 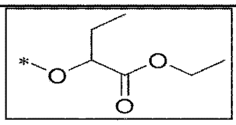 |
| 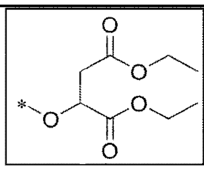 |
| 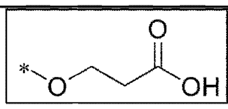 |
| 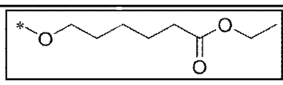 |
| 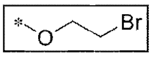 |
| 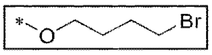 |
| 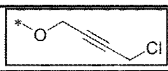 |
| 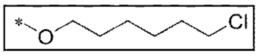 |
| 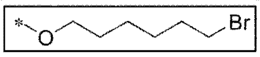 |
| 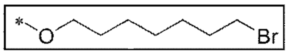 |
| 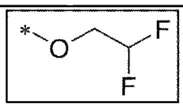 |
| 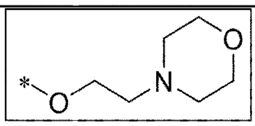 |
| 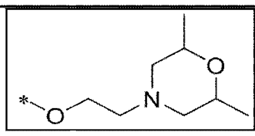 |

| |
|---|
| 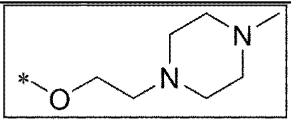 |
| 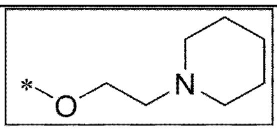 |
| 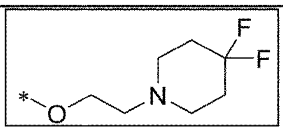 |
| 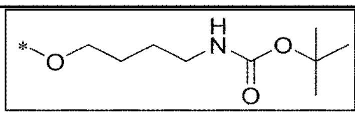 |
| 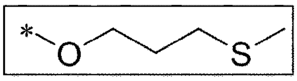 |
| 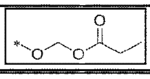 |
| 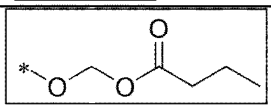 |
| 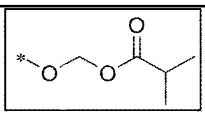 |
| 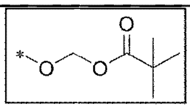 |
| 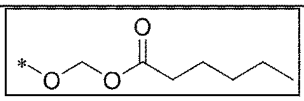 |
| 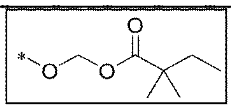 |
| 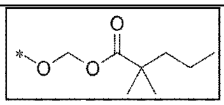 |
| 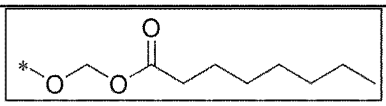 |

| |
|---|
| 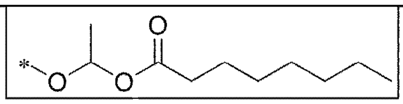 |
| 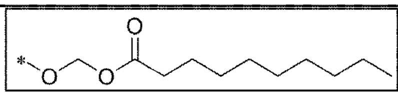 |
| 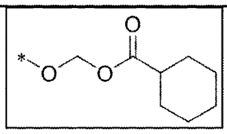 |
| 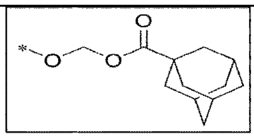 |
| 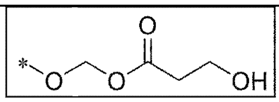 |
| 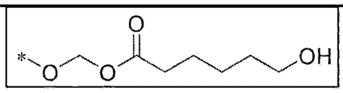 |
| 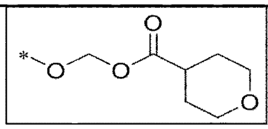 |
| 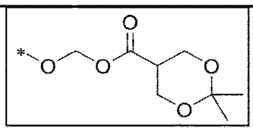 |
| 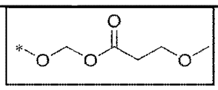 |
| 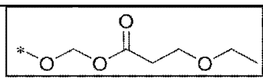 |
| 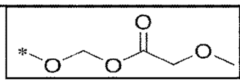 |
| 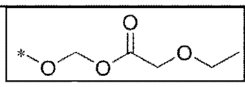 |
| 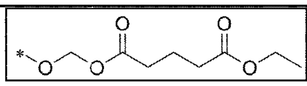 |
| 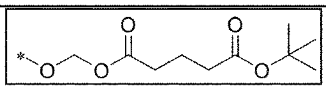 |

| |
|---|
| 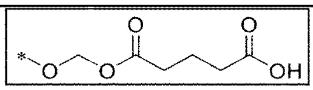 |
| 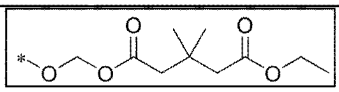 |
| 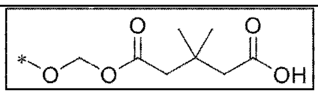 |
| 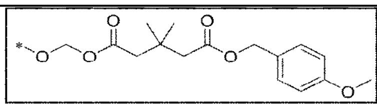 |
| 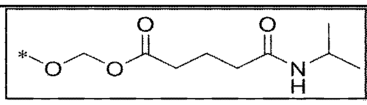 |
| 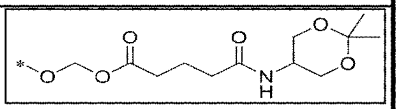 |
| 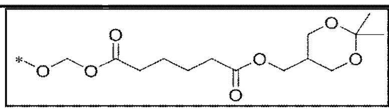 |
| 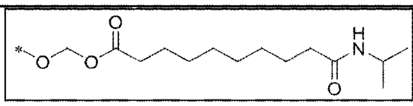 |
| 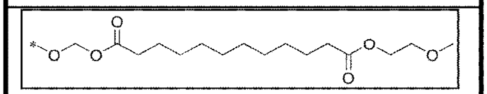 |
| 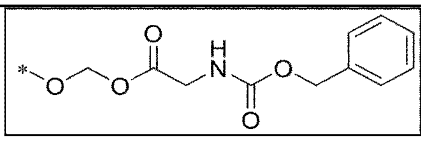 |
| 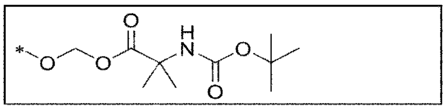 |
| 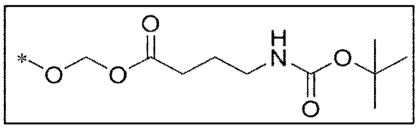 |

| |
|---|
| 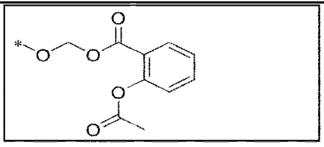 |
| 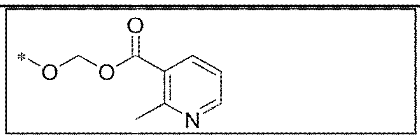 |
| 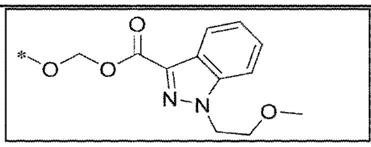 |
| 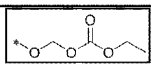 |
| 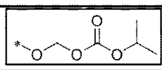 |
| 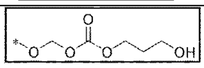 |
| 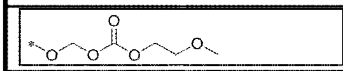 |
| 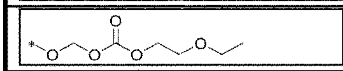 |
| 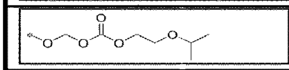 |
| 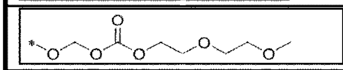 |
| 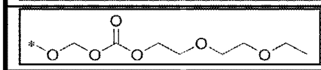 |
| 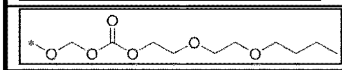 |
| 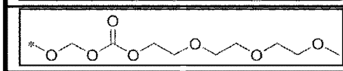 |
| 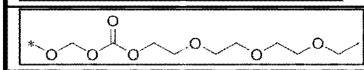 |
| 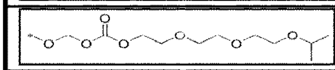 |
| 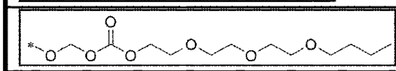 |
| 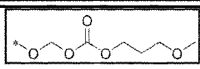 |
| 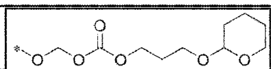 |
| 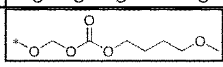 |
| 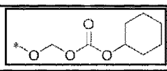 |
| 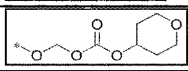 |
| 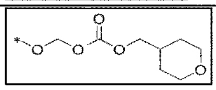 |

| |
|---|
| 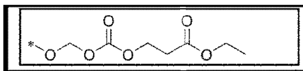 |
| 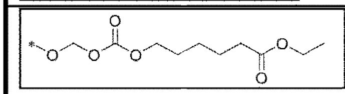 |
| 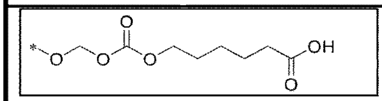 |
| 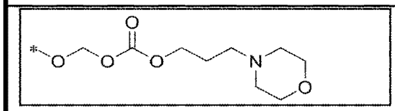 |
| 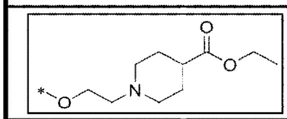 |
| 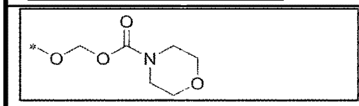 |
| 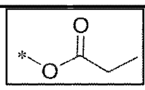 |
| 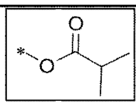 |
| 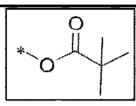 |
| 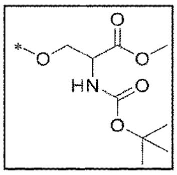 |
| 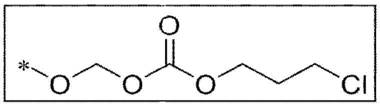 |

and $-NR^{4Na}R^{4Nb}$ represents any of the groups listed in the following table when asterisk "*" represents the side opposite to $R^{4Na}R^{4Nb}$ relative to the nitrogen atom ($*-NR^{4Na}R^{4Nb}$):

| |
|---|
| 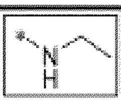 |
| 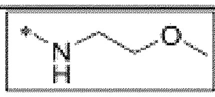 |
| 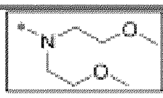 |
| 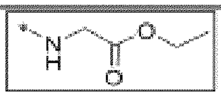 |
| 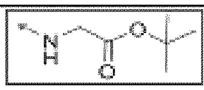 |
| 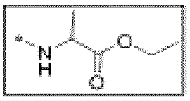 |
| 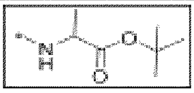 |
| 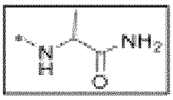 |
| 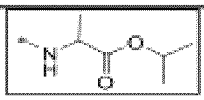 |

.

**6.** A compound represented by formula (II) or a salt thereof:

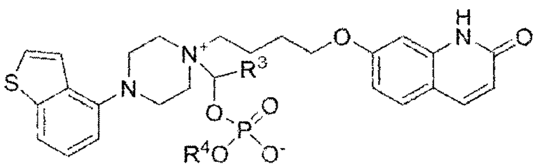

(II)

wherein,

$R^3$ represents hydrogen or $C_{1-6}$ alkyl,
$R^4$ represents hydrogen or $C_{1-18}$ alkyl optionally having 1 to 3 substituents,
the substituent independently represents halogen, hydroxy, $C_{1-6}$ alkoxy, -O- $(CH_2)_n$-$OR^5$, -O(CO)- $(CH_2)_n$-$OR^{5a}$, -(CO)$OR^6$, - (CO)$NR^7R^8$, -O(CO)$R^9$, -O(CO)$OR^{10}$, -O(CO)$(CR^{11}R^{12})_n$(CO)$OR^{13}$, $C_{3-7}$ cycloalkyl, or an optionally substituted heterocyclic group,
$R^5$, $R^{5a}$, $R^6$, $R^9$, $R^{10}$, and $R^{13}$ independently represent hydrogen, $C_{1-16}$ alkyl, or $C_{3-7}$ cycloalkyl,
$R^7$ and $R^8$ independently represent hydrogen or $C_{1-6}$ alkyl optionally having one or two methylene groups replaced with an oxygen atom or nitrogen atom,
$R^7$ and $R^8$ may be bound to form a ring,
$R^{11}$ and $R^{12}$ independently represent hydrogen or $C_{1-16}$ alkyl, wherein if two or more $R^{11}$s are present, the two or more $R^{11}$s independently represent hydrogen or $C_{1-16}$ alkyl, and if two or more $R^{12}$s are present, the two or more $R^{12}$s independently represent hydrogen or $C_{1-16}$ alkyl, and
n represents an integer of 1 to 10.

**7.** The compound or salt thereof according to claim 6, wherein

$R^4$ represents hydrogen or $-(A-Y)_m-R^{14}$,
A represents $C_{1-18}$ alkylene,
Y represents -O-, -(CO)O-, -O(CO)O-, or -(CO)NH-,
$R^{14}$ represents hydrogen, $C_{1-18}$ alkyl, hydroxy, cycloalkyl, or a heterocyclic group, and
m represents an integer of 0 to 5.

**8.** The compound or salt thereof according to claim 6, wherein

$R^4$ represents hydrogen or $C_{1-8}$ alkyl optionally having one substituent,
the substituent independently represents hydroxy, $C_{1-6}$ alkoxy, $-O-(CH_2)_n-OR^5$, $-O(CO)-(CH_2)_n-OR^{5a}$, -(CO)OR$^6$, -O(CO)R$^9$, - O(CO)OR$^{10}$, -O(CO) -A-(CO) OR$^{13}$, or morpholinyl optionally having two $C_{1-6}$ alkyl groups,
n represents an integer of 1 to 10,
$R^5$, $R^{5a}$, $R^6$, $R^9$, $R^{10}$, and $R^{13}$ independently represent hydrogen, $C_{1-16}$ alkyl, an optionally substituted heterocyclic group, or $C_{3-7}$ cycloalkyl, and
A represents $C_{1-6}$ alkylene.

**9.** The compound or salt thereof according to claim 6, wherein

$R^4$ represents hydrogen or $C_{1-8}$ alkyl optionally having one substituent,
the substituent independently represents hydroxy, $C_{1-6}$ alkoxy, $-O-(CH_2)_n-OR^5$, $-O(CO)-(CH_2)_n-OR^{5a}$, -(CO)OR$^6$, -O(CO)R$^9$, - O(CO)-A-(CO)OR$^{13}$, or a heterocyclic group optionally having one or two $C_{1-6}$ alkyl groups,
n represents an integer of 1 to 10,
$R^5$ represents $C_{1-6}$ alkyl,
$R^{5a}$ represents $C_{1-6}$ alkyl,
$R^6$ represents $C_{1-6}$ alkyl,
$R^9$ represents $C_{1-16}$ alkyl,
$R^{13}$ represents $C_{1-6}$ alkyl or pyridyl optionally having $C_{1-6}$ alkyl, and
A represents $C_{1-6}$ alkylene.

**10.** A pharmaceutical composition comprising the compound or salt thereof of any one of claims 1 to 9 and a pharmaceutically acceptable carrier.

**11.** The pharmaceutical composition according to claim 10, comprising the compound or salt thereof of any one of claims 1 to 9, a suspending agent, and a dispersion medium, wherein the pharmaceutical composition is in the form of a suspension.

**12.** The pharmaceutical composition according to claim 11, wherein the average particle diameter of particles in the suspension is 0.5 to 30 $\mu$m.

**13.** The pharmaceutical composition according to claim 11, wherein the average particle diameter of particles in the suspension is 50 to 500 nm.

**14.** The pharmaceutical composition according to any one of claims 11 to 13, wherein the suspending agent is carboxymethyl cellulose or a salt thereof, and the dispersion medium is a liquid containing water for injection.

**15.** A pharmaceutical composition comprising a viscous mixture of

a) the compound or salt thereof of any one of claims 1 to 9,
b) two or more liquid crystal-forming lipids or gel-forming lipids, and
c) a biocompatible organic solvent,

wherein the pharmaceutical composition is a precursor preparation used to form a liquid crystal phase structure or lipid gel by bringing the pharmaceutical composition into contact with an aqueous fluid in vivo.

**16.** The pharmaceutical composition according to claim 15, wherein b) the two or more liquid crystal-forming lipids or gel-forming lipids contain

b-1) at least one diacylglycerol, and

b-2) at least one phosphatidylcholine.

17. A pharmaceutical composition comprising a viscous mixture of

a) the compound or salt thereof of any one of claims 1 to 9,
b-1) at least one diacylglycerol,
b-2) at least one phosphatidylcholine, and
c) a biocompatible organic solvent.

18. A microsphere comprising the compound or salt thereof of any one of claims 1 to 9 as an active ingredient.

19. The microsphere according to claim 18, comprising the compound of any one of claims 1 to 9 and a biodegradable polymer.

20. The microsphere according to claim 19, wherein the biodegradable polymer is at least one member selected from the group consisting of polylactic acid and a lactic acid-glycol copolymer.

21. The microsphere according to any one of claims 18 to 20, having an average particle diameter of 5 to 150 $\mu$m.

22. A pharmaceutical composition comprising the microsphere of any one of claims 18 to 21 in the form of a suspension.

23. A pharmaceutical composition comprising the microsphere of any one of claims 18 to 21 in the form of a suspension in oil.

24. The pharmaceutical composition according to claim 23, wherein the oil is a medium-chain fatty acid triglyceride.

25. The pharmaceutical composition according to any one of claims 10 to 17 and 22 to 24, which is for use in intramuscular administration or subcutaneous administration.

26. The pharmaceutical composition according to any one of claims 10 to 17 and 22 to 25, which is for use in the prevention and/or treatment of a central nervous system disease.

27. The pharmaceutical composition according to claim 26, wherein the central nervous system disease is selected from the group consisting of schizophrenia, treatment-resistant, refractory, or chronic schizophrenia, schizoaffective disorder, psychotic disorder, mood disorder, bipolar disorder, mania, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, Alzheimer's disease, Parkinson's disease, cognitive impairment, cognitive impairment associated with neurodegenerative disease, cognitive impairment due to neurodegenerative disease, cognitive impairment in schizophrenia, cognitive impairment caused by treatment-resistant, refractory, or chronic schizophrenia, vomiting, motion sickness, obesity, migraine, pain, mental retardation, autism disorder, Tourette's disorder, tic disorder, attention deficit hyperactivity disorder, conduct disorder, Down syndrome, impulsive symptoms associated with dementia, and borderline personality disorder.

Fig. 1

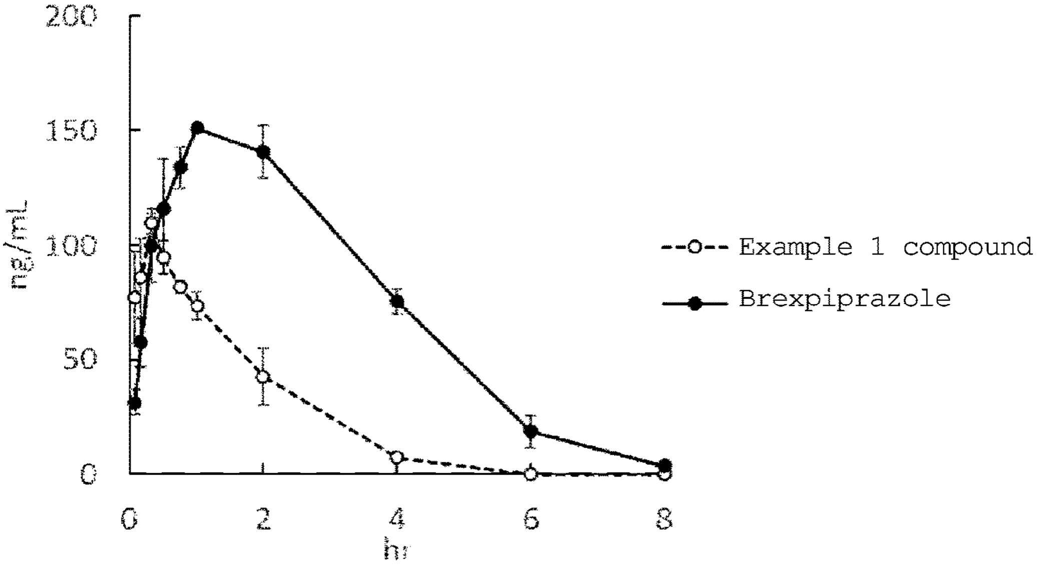
200
150
100
50
0
ng/mL
0
2
4
6
8
hr
Example 1 compound
Brexpiprazole

Fig. 2

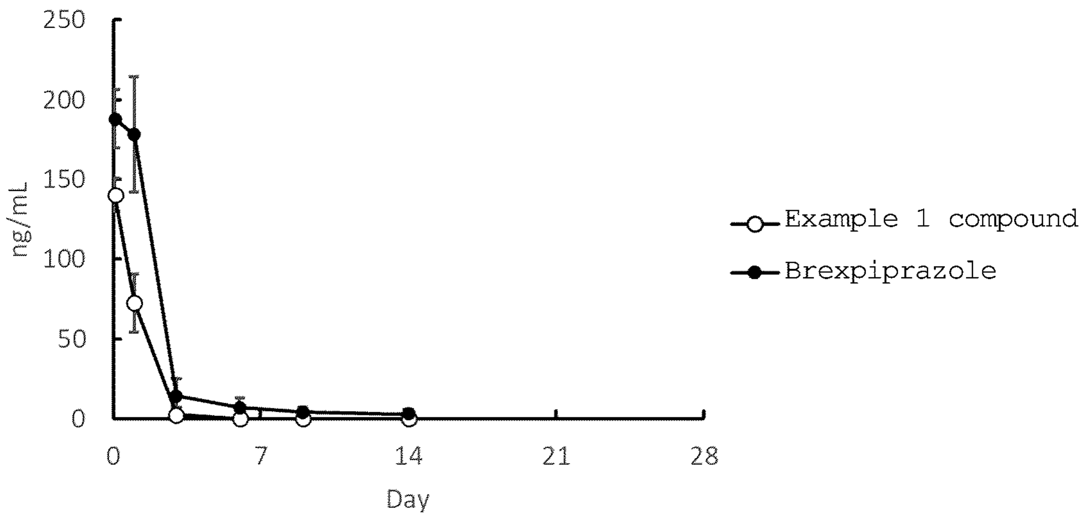
250
200
150
100
50
0
ng/mL
0
7
14
21
28
Day
Example 1 compound
Brexpiprazole

Fig. 3

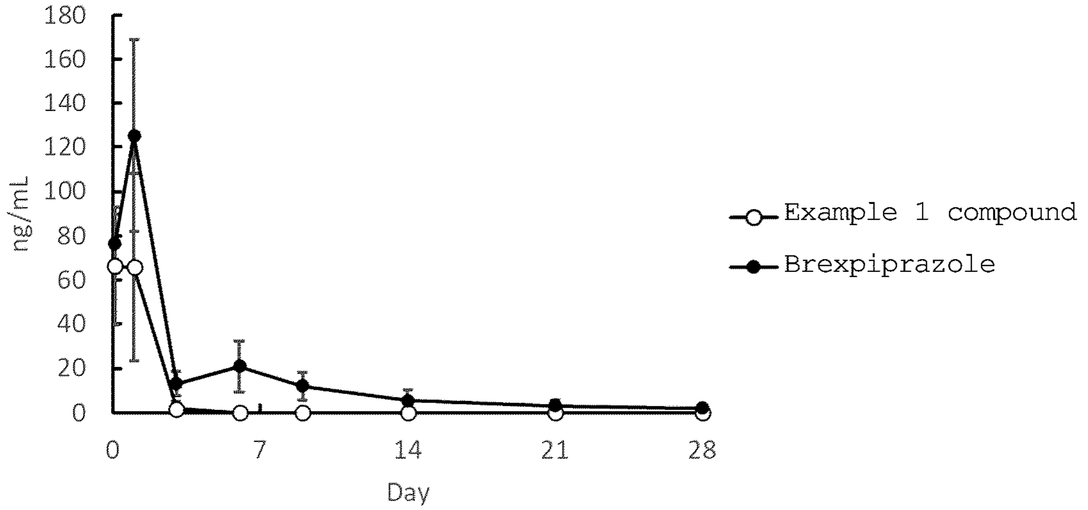


Fig. 4

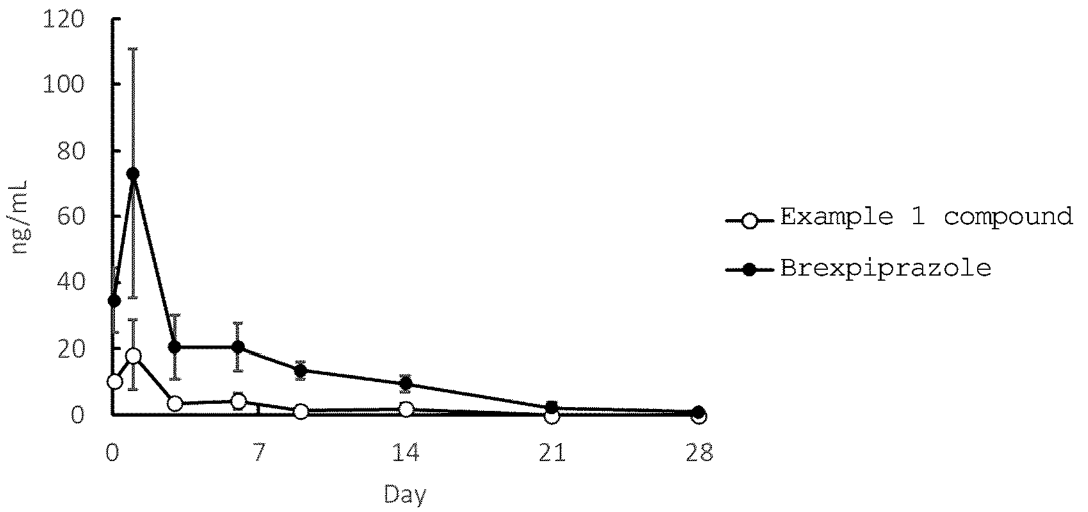


Fig. 5

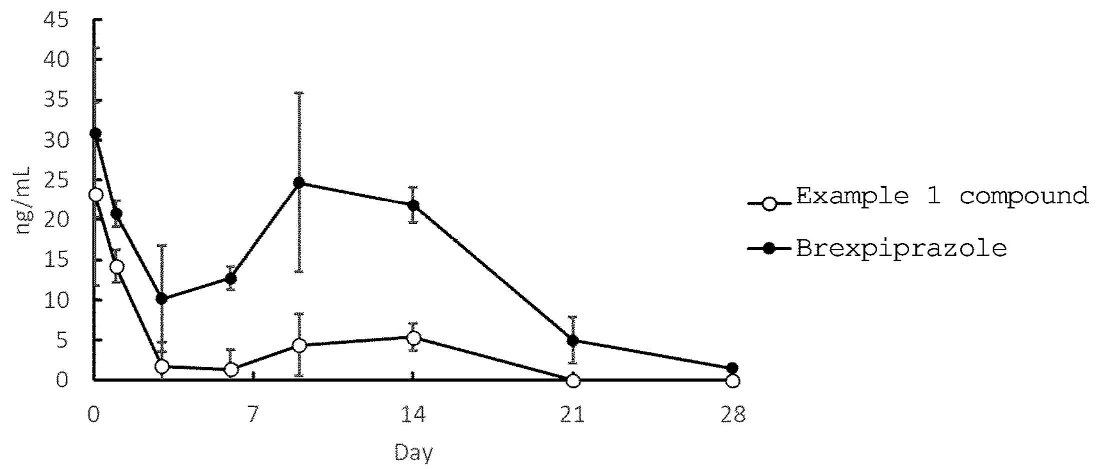

Fig. 6

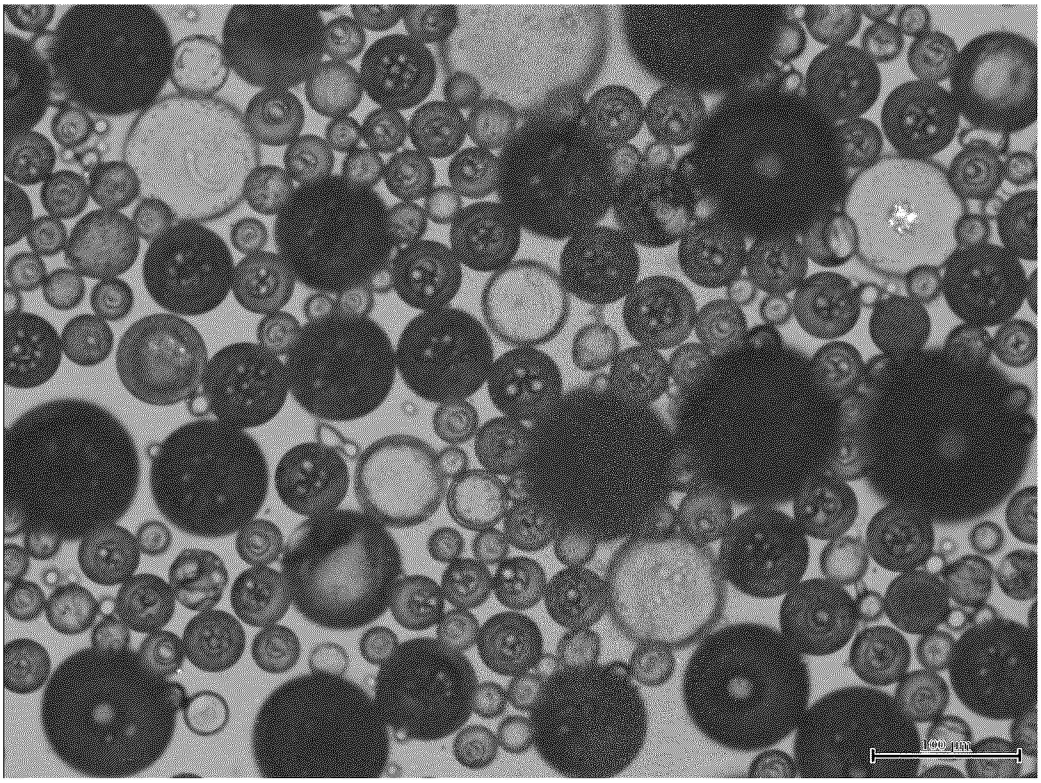


Fig. 7

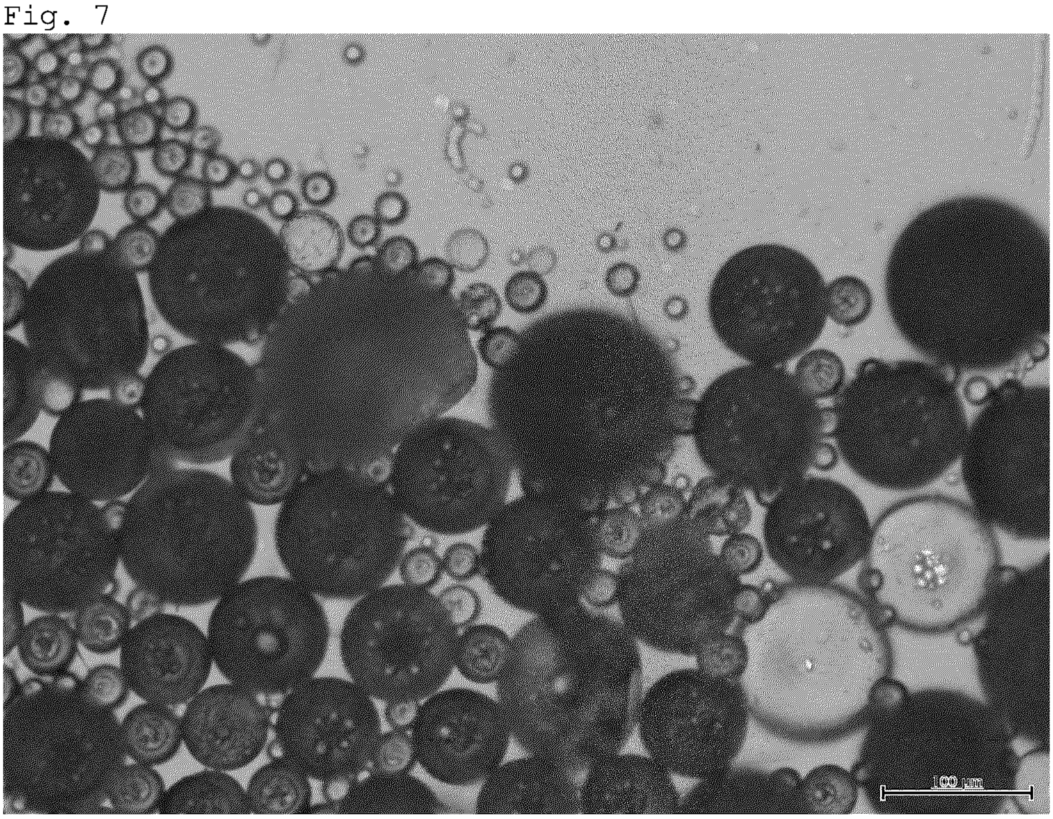

Fig. 8

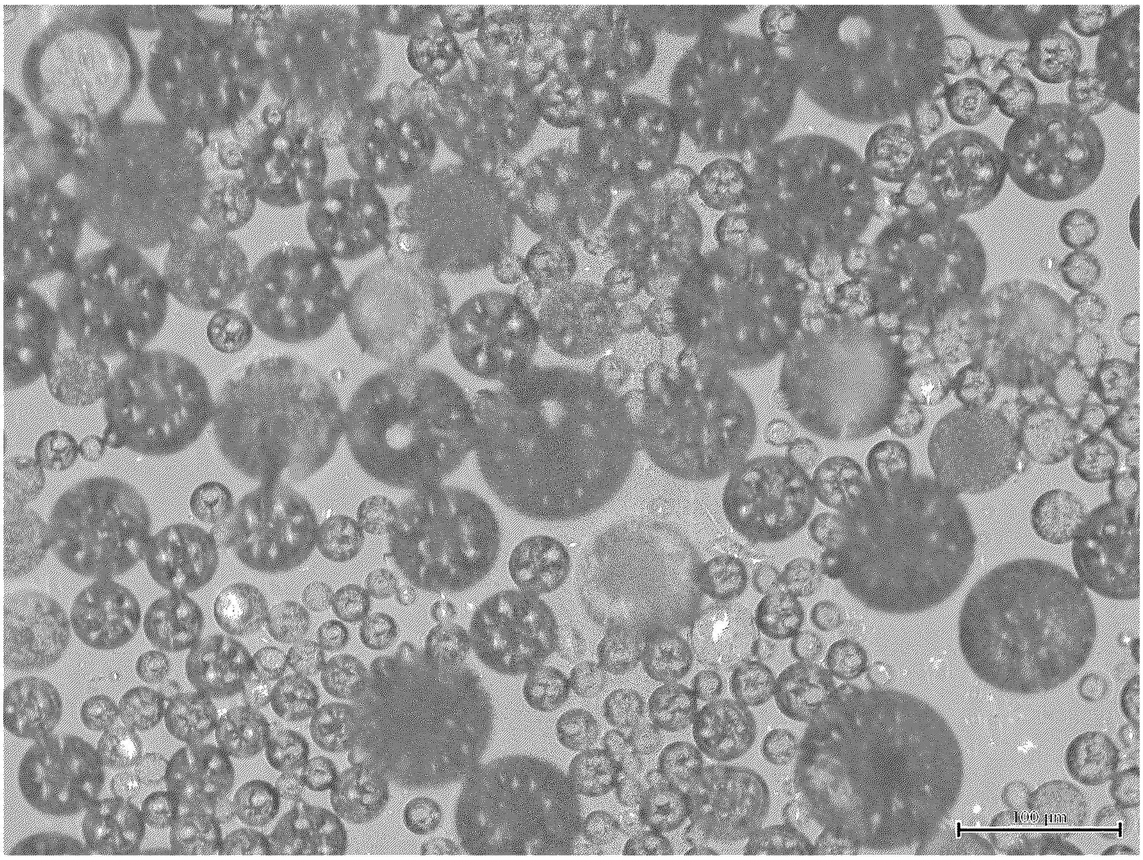
100 μm

Fig. 9

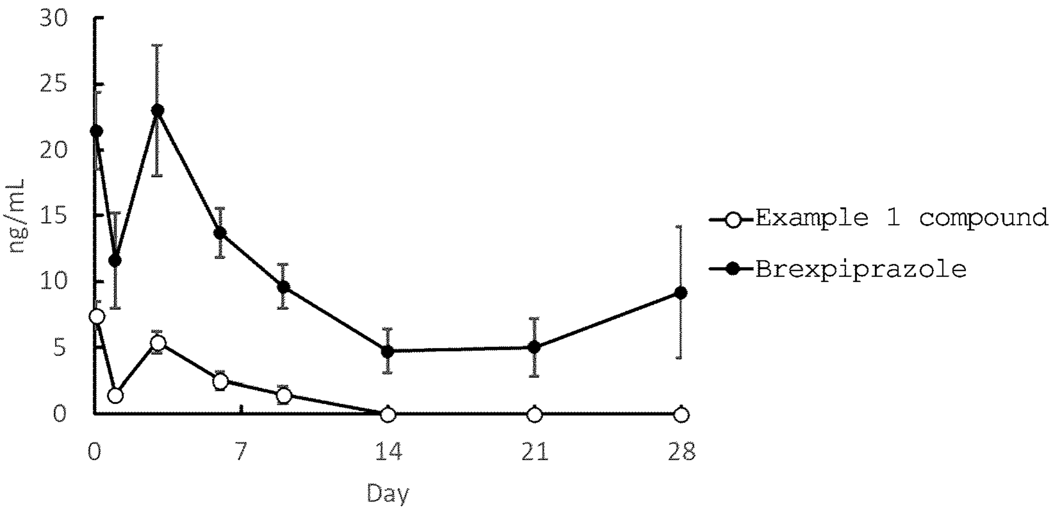
30
25
20
15
10
5
0
ng/mL
0
7
14
21
28
Day
Example 1 compound
Brexpiprazole

Fig. 10

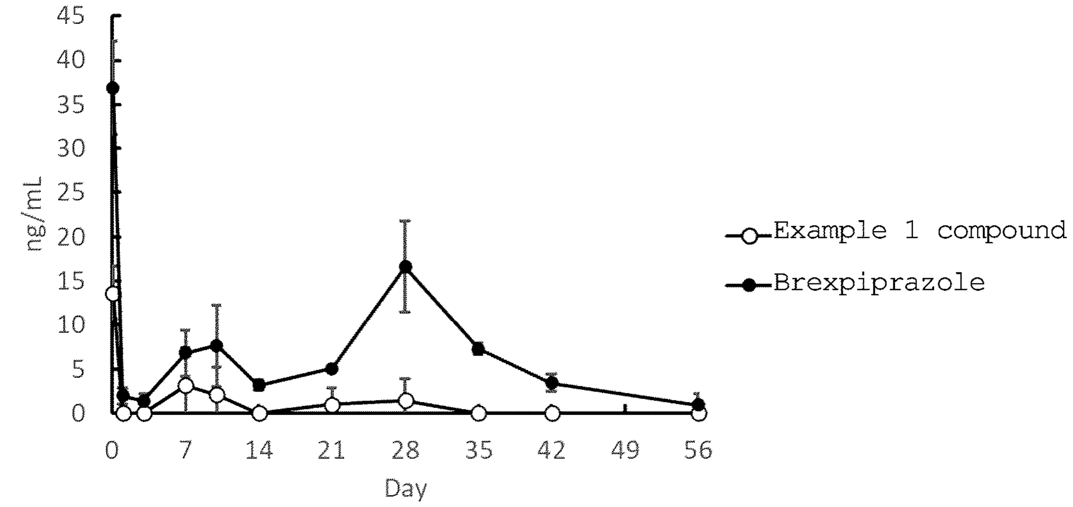


Fig. 11

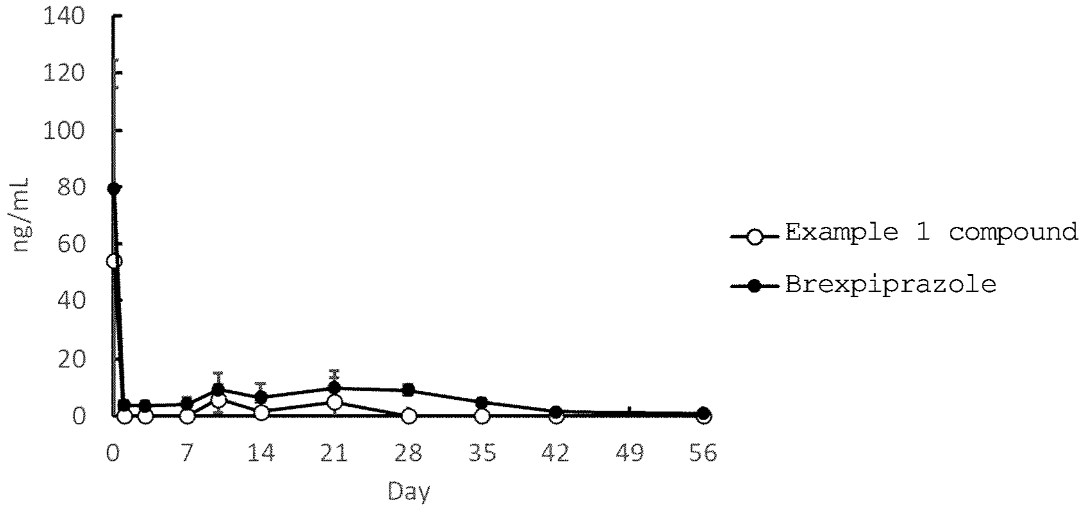


Fig. 12

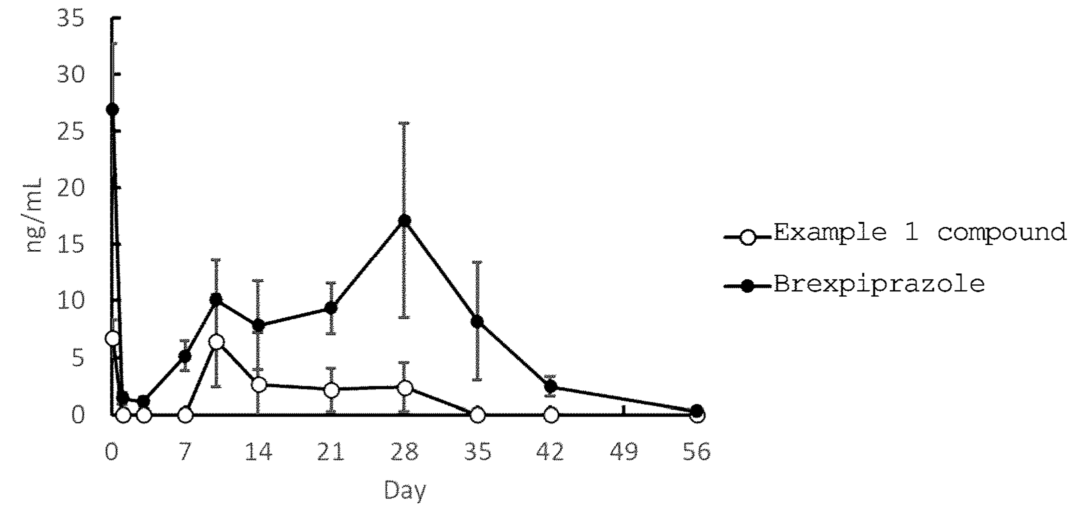

Fig. 13

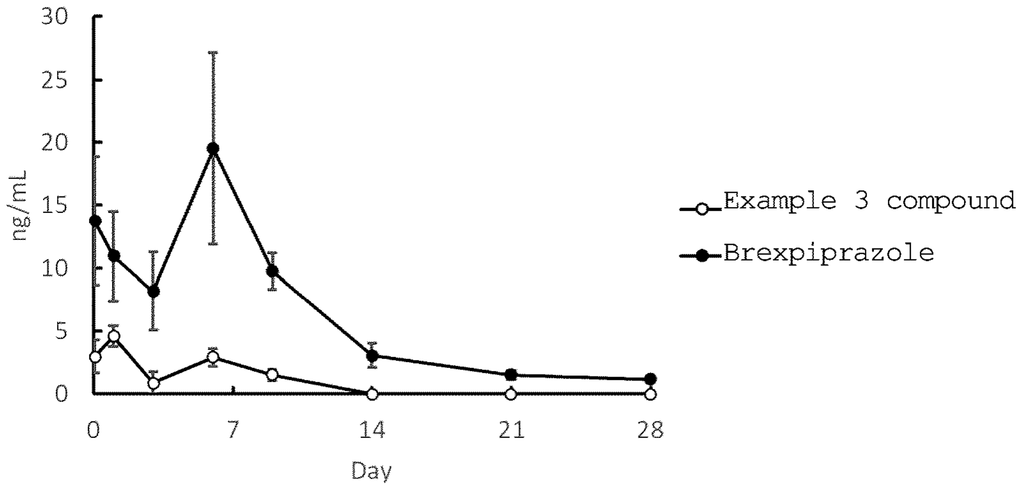


Fig. 14

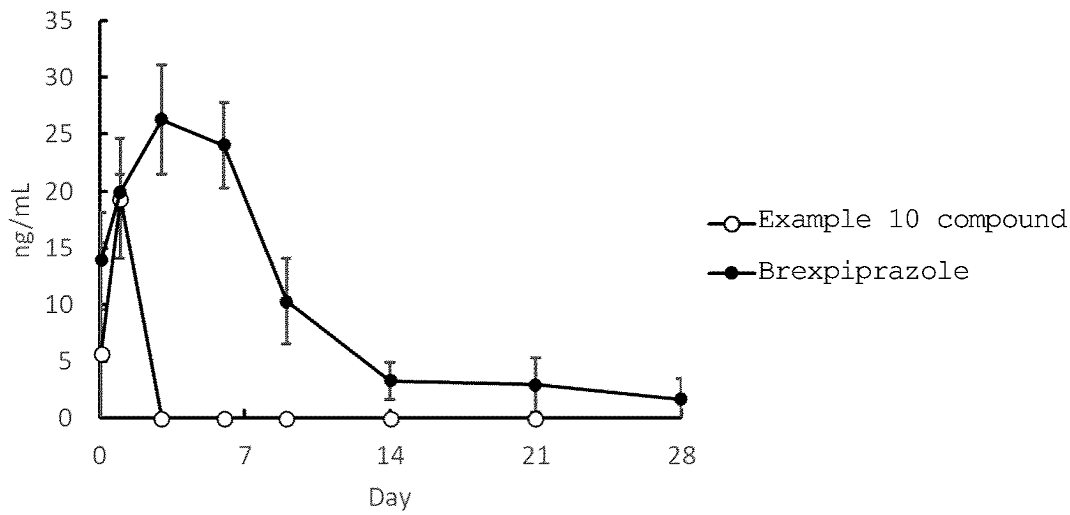


Fig. 15

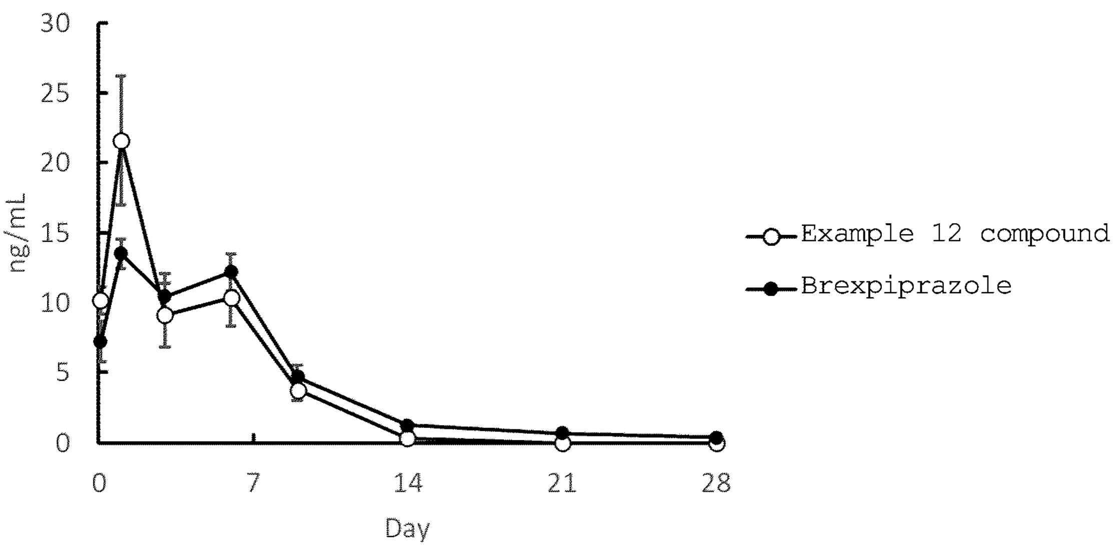

Fig. 16

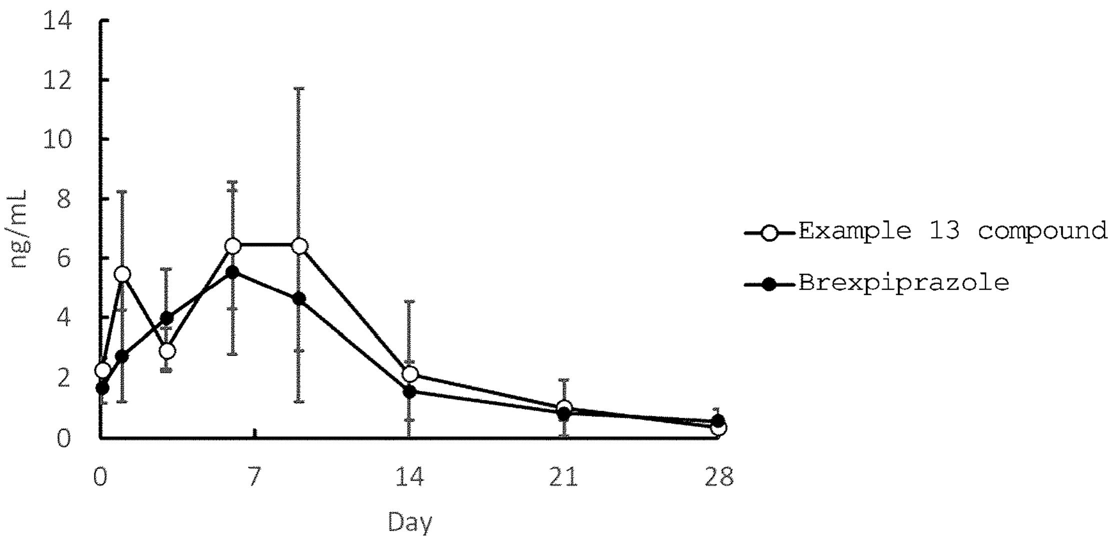
14
12
10
8
6
4
2
0
ng/mL
0
7
14
21
28
Day
Example 13 compound
Brexpiprazole

Fig. 17

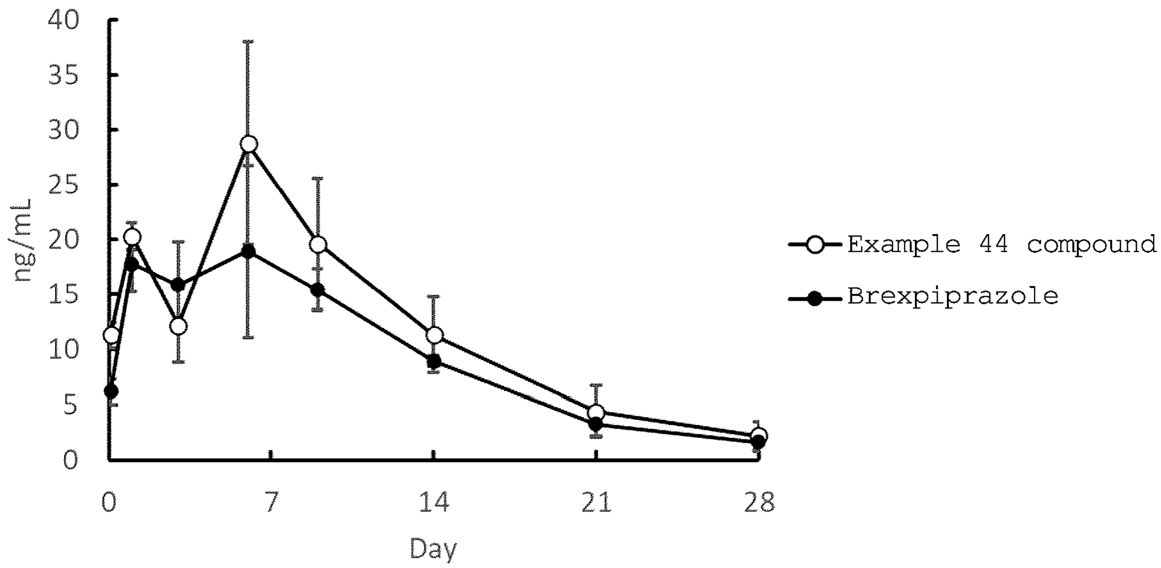
40
35
30
25
20
15
10
5
0
ng/mL
0
7
14
21
28
Day
Example 44 compound
Brexpiprazole

Fig. 18

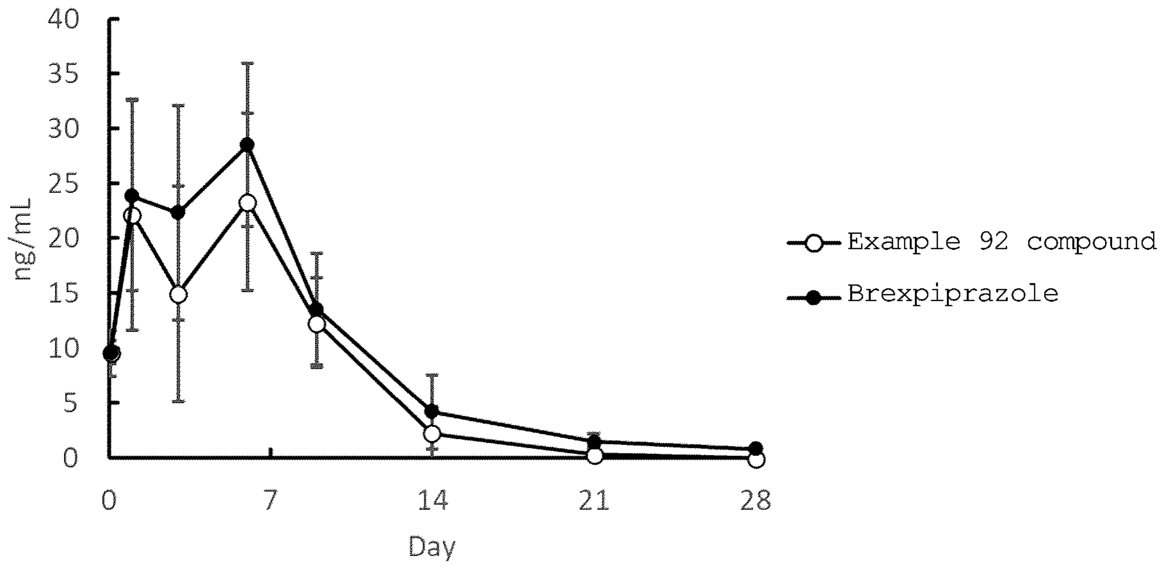
40
35
30
25
20
15
10
5
0
ng/mL
0
7
14
21
28
Day
Example 92 compound
Brexpiprazole

Fig. 19

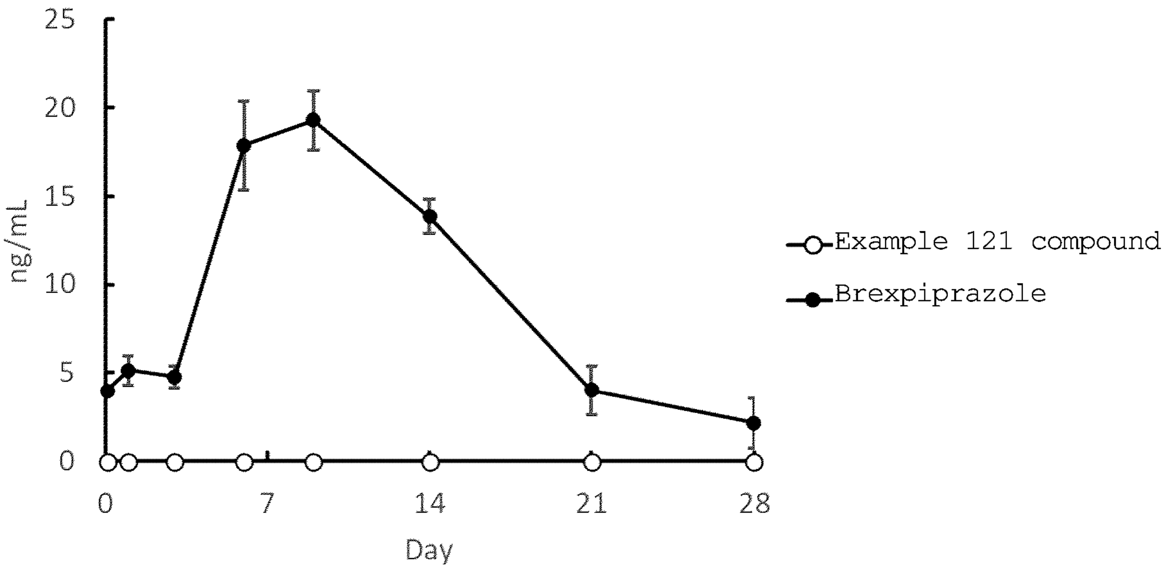
25
20
15
10
5
0
ng/mL
0
7
14
21
28
Day
Example 121 compound
Brexpiprazole

**INTERNATIONAL SEARCH REPORT**

International application No. **PCT/JP2024/016879**

**A. CLASSIFICATION OF SUBJECT MATTER**

***C07F 9/6561***(2006.01)i; ***A61K 31/675***(2006.01)i; ***A61P 25/00***(2006.01)i; ***A61P 25/16***(2006.01)i; ***A61P 25/18***(2006.01)i; ***A61P 25/20***(2006.01)i; ***A61P 25/22***(2006.01)i; ***A61P 25/24***(2006.01)i; ***A61P 25/28***(2006.01)i; ***A61P 43/00***(2006.01)i
FI: C07F9/6561 Z CSP; A61K31/675; A61P25/00; A61P25/16; A61P25/18; A61P25/20; A61P25/22; A61P25/24; A61P25/28; A61P43/00 111

According to International Patent Classification (IPC) or to both national classification and IPC

**B. FIELDS SEARCHED**

Minimum documentation searched (classification system followed by classification symbols)

C07F9/6561; A61K31/675; A61P25/00; A61P25/16; A61P25/18; A61P25/20; A61P25/22; A61P25/24; A61P25/28; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN); PubMed

**C. DOCUMENTS CONSIDERED TO BE RELEVANT**

| Category* | Citation of document, with indication, where appropriate, of the relevant passages | Relevant to claim No. |
|---|---|---|
| A | JP 2014-526435 A (OTSUKA PHARMACEUTICAL CO., LTD.) 06 October 2014 (2014-10-06) claims, examples 200-202, 383, 404, 451, 581-582, 652, 678-712 | 1-27 |
| A | JP 2012-531429 A (ALKERMES, INC.) 10 December 2012 (2012-12-10) claims, paragraph [0002], table 1, examples 98-100, 247-249 | 1-27 |
| A | FULMALI, A. et al., Phosphate moiety in FDA - approved pharmaceutical salts and prodrugs, Drug Development Research, 2022, vol. 83, no. 5, pages 1059-1074, DOI: 10.1002/ddr.21953 fig. 3-8 | 1-27 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 June 2024** | **02 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/016879**

| Patent document cited in search report | Publication date (day/month/year) | Patent family member(s) | Publication date (day/month/year) |
|---|---|---|---|
| JP 2014-526435 A | 06 October 2014 | US 2015/0045356 A1<br>claims, examples 200-202, 383, 404, 451, 581-582, 652, 678-712<br>WO 2013/035892 A1<br>EP 2753617 A1<br>CN 103781783 A<br>KR 10-2014-0069109 A | |
| JP 2012-531429 A | 10 December 2012 | WO 2010/151689 A1<br>claims, pages 1, 31-32, examples 98-100, 247-249<br>US 2011/0003828 A1<br>EP 2445343 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006316052 A
- WO 2013035892 A


### Non-patent literature cited in the description


- Organic Functional Group Preparations. Academic Press, Inc., 1989
- Comprehensive Organic Transformations. VCH Publishers, Inc., 1989
- **T. W. GREENE**. Greene's Protective Groups in Organic Synthesis. John Wiley & Sons, 1991